# EUROPEAN PATENT APPLICATION

(11) **EP 2 567 967 A2**
(43) Date of publication of application: **13.03.2013**
(21) Application number: 12180322.5
(22) Date of filing: 12.04.2005
(51) Int. Cl.: C07K 14/16, C12N 15/861, A61K 39/21

(54) **Method of using adenoviral vectors to induce an immune response**

(30) Priority: 12.04.2004 US 561341 P
(62) Divisional of application: 05778761.6
(71) Applicant: GOVERNMENT OF THE UNITED STATES OF AMERICA, as represented by THE SECRETARY, DEPARTMENT OF HEALTH AND HUMAN SERVICES, Rockville, MD 20852-3804 (US); GENVEC, INC., Gaithersburg, MD 20878 (US)
(72) Inventor: Nabel, Gary J, Washington, DC District of Columbia 20008 (US); Cheng, Cheng, Bethesda, MD Maryland 20892 (US); Kong, Wing-Pui, Gaithersburg, MD Maryland 20878 (US); Gall, Jason G D, Germantown, MD Maryland 20874 (US); King, C. Richter, Washington, DC District of Columbia 20010 (US)
(74) Representative: Grünecker, Kinkeldey, Stockmair & Schwanhäusser

(57) **Abstract**

The invention provides a method of inducing an immune response against a human immunodeficiency virus (HIV) in a mammal. The method comprises administering to the mammal an adenoviral vector composition comprising one or more adenoviral vectors encoding two or more different HIV antigens, the production of which induces an immune response against HIV in the mammal. The invention also provides an adenoviral vector composition comprising four adenoviral vectors encoding an HIV clade A Env protein, an HIV clade B Env protein, an HIV clade C Env protein, and a fusion protein comprising an HIV clade B Gag protein and Pol protein, respectively.

SEQUENCE LISTING

<110> The Government of the United States of America, as
      Represented by the Secretary, Department of Health and Human
      Services

<120> METHOD OF USING ADENOVIRAL VECTORS TO INDUCE AN IMMUNE RESPONSE

<130> EP45466IFZSEpau

<140> not yet assigned
<141> 2012-08-13

<150> EP05778761.6
<151> 2005-04-12

<150> PCT/US2005/012291
<151> 2005-04-12

<150> 60/561,341
<151> 2004-04-12

<160> 7

<170> PatentIn version 3.3

<210> 1
<211> 5
<212> PRT
<213> Artificial

<220>
<223> Synthetic

<400> 1

Cys Arg Gly Asp Cys
1               5


<210> 2
<211> 9
<212> PRT
<213> Artificial

<220>
<223> Synthetic


<220>
<221> MISC_FEATURE
<222> (2)..(2)
<223> Xaa may be any amino acid

<220>
<221> MISC_FEATURE
<222> (8)..(8)
<223> Xaa may be any amino acid

<400> 2

Cys Xaa Cys Arg Gly Asp Cys Xaa Cys
1               5


<210> 3
<211> 9
<212> PRT
<213> Artificial

<220>
<223> Synthetic

<400> 3

Cys Asp Cys Arg Gly Asp Cys Phe Cys

1                    5


<210>  4
<211>  36066
<212>  DNA
<213>  Artificial

<220>
<223>  Synthetic

<400>  4
catcatcaat aatatacctt attttggatt gaagccaata tgataatgag ggggtggagt      60

ttgtgacgtg gcgcggggcg tgggaacggg gcgggtgacg tagtagtgtg gcggaagtgt     120

gatgttgcaa gtgtggcgga acacatgtaa gcgacggatg tggcaaaagt gacgtttttg     180

gtgtgcgccg gtgtacacag gaagtgacaa ttttcgcgcg gttttaggcg gatgttgtag     240

taaatttggg cgtaaccgag taagatttgg ccattttcgc gggaaaactg aataagagga     300

agtgaaatct gaataatttt gtgttactca tagcgcgtaa tatttgtcta gggccgggga     360

tcggtgatca ccgatccaga catgataaga tacattgatg agtttggaca aaccacaact     420

agaatgcagt gaaaaaaatg ctttatttgt gaaatttgtg atgctattgc tttatttgta     480

accattataa gctgcaataa acaagttccc ggatctttct agctagtcta gaattcctag     540

tcctcgtcct ggcggctggc cacgcagtcg tcgccggcca tctgcttgcc gtagtcgcgg     600

atgatcttgg ccttgcggcg gggcaccacc ttgatgtcgc tgttgtcctg gatcaccacg     660

gcgccctcgc ccttccacag cagcttggcg gggcccttcc acacggggtc gcggctgtcg     720

cggtagtaca cgcggaagtt ctggatcttg gtgatctgct tctgcagctc cttggtctgg     780

atgtcggtgg cgatgatgtc cacgatgcgc tcgccggcgc tgtagccgcc gatgccgccc     840

ttgcgcttga agttgtggat gaacacggcc atctgcacgg cggtcttcag gtgctcggcc     900

tggtcgcgca cctggccgat gatcttcttc agctccttgt tcatgctctc gatcacgccc     960

tggctctggg ggttgtaggg gatgccgaac tcctgcttga tgccggccca ccagcaggcg    1020

gccttcacgg tggtgctggt gaagttgctg ccgttgtcgg tgtgcacggt cttcacgggc    1080

cagcggccgg ccagcttcag caggaagtag gcggtctcct ggccggtctc ggcggggatc    1140

acctcggcct cgatgtagcc gctggccacg tgcacggcca ccaggatcac cttgccctcc    1200

aggtgggtgc agtccagctg ccagatgccg gggctgcagt ccacctggcc gtgcatggcc    1260

tcgcccttca gctggcactt gtcgcagctg gccacgatct ccttggccac cacggggggc     1320

aggttgaagt cgctggccat ggcgcgccag ttgctgtggt acttctcgtg ctcctcctgg    1380

gccttgtcga tgccgtccag gaacagcacc ttgcggatgc cggcgctcac caggccgtcc    1440

acctgctcgt tgccgccgat gcccttgtgg gcgggcaccc aggccaggta caccttctcc    1500

ttcttgatca gctgctcgat gatctggctc accagctcgc tctcgctctt gtcgggctgg    1560

gcctggatga tgcccagggc gtactggctg tcggtcacga tgttcacctc caggccgctg    1620

tcctgcaggg ccaggtggat ggcctgcagc tcggtcttct ggttggtggt gtcggtcagg    1680

ggcaccacct tctggcggcc gcggtcggtc acgtagccgg ccttgcccag cttggtctcg    1740

cggttggcgg cgccgtccac gtagaaggtc tcggcgccga tgatgggctc cttctccagc    1800

tggtaccaca gcttcaccag ggggggggtg ttcacgaact cccactcggg gatccaggtg    1860

gcctgccagt actcggtcca ccaggcctcc caggtctcct tctggatggg cagcttgaac    1920

ttgggggtct tgccccagat cacgatgctc tcggtggcga tcttctgcac ggcctcggtc 1980
agctgcttca cgtcgttggt gtgggcgccc ttcatgcggg cgtacttgcc ggtcttcagg 2040
ttcttgaagg gctcctggta gatctggtag gtccactggc cctggccctg cttctggatc 2100
tcggcgatca ggtccttgct ggggtcgtag tacacgccgt gcacgggctc cttcaggatc 2160
tcgcggttct cggccagctc cagctcggcc tcctcggtca ggggcaccac ctcggtcagg 2220
gccttggtgc cgcgcagcag cttgcacagc tggcgcacct tgatgccggc gtagatctgg 2280
ctggcccagt tcagcttgcc caccagcttc tggatgtcgt tcacggtcca gctgtccttc 2340
tcgggcagca cgatgggctg cacggtccac ttgtcggggt gcagctcgta gcccatccac 2400
aggaaggggg gctccttctg gtgcttcttg tcgggggtgg tgaagcccca gcgcagcagg 2460
tgctggcgca gctcctcgat cttggtgcgg tgctggccga tctccaggtc gctgcccacg 2520
tacaggtggt ccatgtactg gtagatcacg atgtcggggt tctgcttgcg gaagggctcc 2580
aggatcttgg tcatgctgca ctggaagatg gcggggctgc ccttccagcc ctggggcagc 2640
acgttgtact ggtagcggat gccgggggtc tcgttgttga tgctggggat ggtgaaggcg 2700
gtgtacttgc ggaagtcctt gtccaggggc acgctgaagt aggcgtcgcc cacgtccagc 2760
acggtcacgc tcttcttctg cttcaggccg gcggggtggg ggatgcccag ctgcacctcc 2820
cagaagtcct gggtgcgctt gttcagctcg cggaagtcca ccagcttgcg ccacttggtg 2880
ctgtccttct tcttgatggc gaacacgggg gtgttgtagg ggttctcggg gccgatcttg 2940
ctgatcttgc cctccttctc catctcggtg cagatctcca ccagggcctt gatcttctcc 3000
tcggtcaggg gccactgctt caccttgggg ccgtccatgc cgggcttcag cttcacgggc 3060
acggtctcga tggggctgat ggggaagttc agggtgcagc cgatctgggt cagcaggttg 3120
cggccgatga tgttcacggg ggtggggccc accagcacgg tgccgatggc cttgtggccg 3180
cagatctcga tcaggatctg gtcgtactgg cccaccttga tgaagccgcc gatgccgccg 3240
atcatcttgg gcttccagcg gccgggcagg ttcatctcct ccagcacggt gtcgtcggcg 3300
ccggtgtcta gaagggcctc cttcagctgg ccccctatct ttattgtgac gaggggtcgc 3360
tgccaaagag tgatctgagg gaagctaaag gatacagttc cttgtctatc ggctcctgct 3420
tctgagaggg agttgttgtc tcttccccaa acctgaagct ctcttctggt ggggctgttg 3480
gctctggtct gctctgaaga aaattccctg gccttccctt gtgggaaggc cagatcttcc 3540
ctattagcct gtcgctcggt gcagtccttc atctggtggc cctccttgcc gcacttccag 3600
cagcccttct tgcggggggc gcggcagttg cgggcggtgt ggccctcctt gccgcagttg 3660
aagcacttca cgatcttgcg ctggttgcgg aagttgccgc gctgcatcat gatggtggcg 3720
ctgttggtca cctggctcat ggcctcggcc agcacgcggg ccttgtggcc ggggccgccc 3780
acgccctggc aggcggtcat catctcctcc agggtggcgg cggggcccag ggccttcagg 3840
atggtcttgc agtcggggtt ggcgttctgc accagcaggg tctcggtcat ccagttcttc 3900
acctcctggc tggcctgctc ggcgcgcagg gtcttgtaga agcggtccac gtagtcgcgg 3960
aagggctcct tggggccctg gcggatgtcc aggatgctgg tggggctgta catgcgcacg 4020
atcttgttca ggcccaggat gatccagcgc ttgtagatct cgcccacggg gatggggggg 4080
ttgttggtca tccagccgat ctgctcctgc agggtgctgg tggtgccggc gatgtcgctg 4140
ccgcggggct cgcgcatctg gccgggggcg atggggccgg cgtgcacggg gtgcacgcgg 4200

tcccactcgg cggcctcctc gttgatggtc tccttcagca tctgcatggc ggcctggtgg 4260
ccgcccacgg tgttcagcat ggtgttcagg tcctgggggg tggcgccctc gctcagggcg 4320
ctgaacatgg ggatcacctc ggggctgaag gccttctcct ccaccacctt cacccaggcg 4380
ttcagggtgc gggggctgat ggcctggtgc accatctggc cctggatgtt ctgcacgatg 4440
gggtagttct ggctcacctg gttgctgtgg ccggtgtcgg cggcggcctg ctgggccttc 4500
ttcttgctct tgttctgctc ctcctcgatc ttgtccaggg cctccttggt gtccttgatc 4560
tcgatgcgct ggtgcacgca gtacagggtg gccacggtgt tgtacaggct gcgcagctcc 4620
tcgctgccgg tctgcaggct gggctgcagc tggcccagga tctggcggca gccctcgctg 4680
gtctccagca ggccggggtt cacggcgaag cgctccagct cgcggctggc ccacacgatg 4740
tgcttcagct tgtacttctt cttgccgccg gggcgcaggc ggatcttctc ccagcggtcc 4800
agctcgccgc cgctcagcac gctggcgcgg gcgcccatgt cgaatcgaat tctgcagtga 4860
tcagggatcc gtatagtgag tcgtattagg taccggctgc agttggacct gggagtggac 4920
acctgtggag agaaaggcaa agtggatgtc attgtcactc aagtgtatgg ccagatctca 4980
agcctgccac acctcaagtg aagccaaggg ggtgggccta tagactctat aggcggtact 5040
tacgtcactc ttggcacggg gaatccgcgt tccaatgcac cgttcccggc cgcggaggct 5100
ggatcggtcc cggtgtcttc tatggaggtc aaaacagcgt ggatggcgtc tccaggcgat 5160
ctgacggttc actaaacgag ctcgtcgacg atctctatca ctgataggga gatctctatc 5220
actgataggg agagctctgc ttatatagac ctcccaccgt acacgcctac cgcccatttg 5280
cgtcaatggg gcggagttgt tacgacattt tggaaagtcc cgttgatttt ggtgccaaaa 5340
caaactccca ttgacgtcaa tggggtggag acttggaaat ccccgtgagt caaaccgcta 5400
tccacgccca ttgatgtact gccaaaaccg catcaccatg gtaatagcga tgactaatac 5460
gtagatgtac tgccaagtag gaaagtccca taaggtcatg tactgggcat aatgccaggc 5520
gggccattta ccgtcattga cgtcaatagg gggcgtactt ggcatatgat acacttgatg 5580
tactgccaag tgggcagttt accgtaaata ctccacccat tgacgtcaat ggaaagtccc 5640
tattggcgtt actatgggaa catacgtcat tattgacgtc aatgggcggg ggtcgttggg 5700
cggtcagcca ggcgggccat ttaccgtaag ttatgtaacg cggaactcca tatatgggct 5760
atgaactaat gaccccgtaa ttgattacta ttaataacta gtactgaaat gtgtgggcgt 5820
ggcttaaggg tgggaaagaa tatataaggt gggggtctta tgtagttttg tatctgtttt 5880
gcagcagccg ccgccgccat gagcaccaac tcgtttgatg gaagcattgt gagctcatat 5940
ttgacaacgc gcatgccccc atgggccggg gtgcgtcaga atgtgatggg ctccagcatt 6000
gatggtcgcc ccgtcctgcc cgcaaactct actaccttga cctacgagac cgtgtctgga 6060
acgccgttgg agactgcagc ctccgccgcc gcttcagccg ctgcagccac cgcccgcggg 6120
attgtgactg actttgcttt cctgagcccg cttgcaagca gtgcagcttc ccgttcatcc 6180
gcccgcgatg acaagttgac ggctcttttg gcacaattgg attctttgac ccgggaactt 6240
aatgtcgttt ctcagcagct gttggatctg cgccagcagg tttctgccct gaaggcttcc 6300
tcccctccca atgcggttta aaacataaat aaaaaaccag actctgtttg gatttggatc 6360
aagcaagtgt cttgctgtct ttatttaggg gttttgcgcg cgcggtaggc ccgggaccag 6420
cggtctcggt cgttgagggt cctgtgtatt ttttccagga cgtggtaaag gtgactctgg 6480

atgttcagat acatgggcat aagcccgtct ctggggtgga ggtagcacca ctgcagagct 6540
tcatgctgcg gggtggtgtt gtagatgatc cagtcgtagc aggagcgctg ggcgtggtgc 6600
ctaaaaatgt ctttcagtag caagctgatt gccaggggca ggcccttggt gtaagtgttt 6660
acaaagcggt taagctggga tgggtgcata cgtggggata tgagatgcat cttggactgt 6720
atttttaggt tggctatgtt cccagccata tccctccggg gattcatgtt gtgcagaacc 6780
accagcacag tgtatccggt gcacttggga aatttgtcat gtagcttaga aggaaatgcg 6840
tggaagaact tggagacgcc cttgtgacct ccaagatttt ccatgcattc gtccataatg 6900
atggcaatgg gcccacgggc ggcggcctgg gcgaagatat ttctgggatc actaacgtca 6960
tagttgtgtt ccaggatgag atcgtcatag gccattttta caaagcgcgg gcggagggtg 7020
ccagactgcg gtataatggt tccatccggc ccaggggcgt agttaccctc acagatttgc 7080
atttcccacg ctttgagttc agatgggggg atcatgtcta cctgcggggc gatgaagaaa 7140
acggtttccg gggtagggga gatcagctgg gaagaaagca ggttcctgag cagctgcgac 7200
ttaccgcagc cggtgggccc gtaaatcaca cctattaccg ggtgcaactg gtagttaaga 7260
gagctgcagc tgccgtcatc cctgagcagg ggggccactt cgttaagcat gtccctgact 7320
cgcatgtttt ccctgaccaa atccgccaga aggcgctcgc cgcccagcga tagcagttct 7380
tgcaaggaag caaagttttt caacggtttg agaccgtccg ccgtaggcat gcttttgagc 7440
gtttgaccaa gcagttccag gcggtcccac agctcggtca cctgctctac ggcatctcga 7500
tccagcatat ctcctcgttt cgcgggttgg ggcggctttc gctgtacggc agtagtcggt 7560
gctcgtccag acgggccagg gtcatgtctt tccacgggcg cagggtcctc gtcagcgtag 7620
tctgggtcac ggtgaagggg tgcgctccgg gctgcgcgct ggccagggtg cgcttgaggc 7680
tggtcctgct ggtgctgaag cgctgccggt cttcgccctg cgcgtcggcc aggtagcatt 7740
tgaccatggt gtcatagtcc agcccctccg cggcgtggcc cttggcgcgc agcttgccct 7800
tggaggaggc gccgcacgag gggcagtgca gacttttgag ggcgtagagc ttgggcgcga 7860
gaaataccga ttccggggag taggcatccg cgccgcaggc cccgcagacg gtctcgcatt 7920
ccacgagcca ggtgagctct ggccgttcgg ggtcaaaaac caggtttccc ccatgctttt 7980
tgatgcgttt cttacctctg gtttccatga gccggtgtcc acgctcggtg acgaaaaggc 8040
tgtccgtgtc cccgtataca gacttgagag gcctgtcctc gagcggtgtt ccgcggtcct 8100
cctcgtatag aaactcggac cactctgaga caaaggctcg cgtccaggcc agcacgaagg 8160
aggctaagtg ggaggggtag cggtcgttgt ccactagggg gtccactcgc tccagggtgt 8220
gaagacacat gtcgccctct tcggcatcaa ggaaggtgat tggtttgtag gtgtaggcca 8280
cgtgaccggg tgttcctgaa ggggggctat aaaagggggt gggggcgcgt tcgtcctcac 8340
tctcttccgc atcgctgtct gcgagggcca gctgttgggg tgagtactcc ctctgaaaag 8400
cgggcatgac ttctgcgcta agattgtcag tttccaaaaa cgaggaggat ttgatattca 8460
cctggcccgc ggtgatgcct ttgagggtgg ccgcatccat ctggtcagaa aagacaatct 8520
ttttgttgtc aagcttggtg gcaaacgacc cgtagagggc gttggacagc aacttggcga 8580
tggagcgcag ggtttggttt ttgtcgcgat cggcgcgctc cttggccgcg atgtttagct 8640
gcacgtattc gcgcgcaacg caccgccatt cgggaaagac ggtggtgcgc tcgtcgggca 8700
ccaggtgcac gcgccaaccg cggttgtgca gggtgacaag gtcaacgctg gtggctacct 8760

ctccgcgtag gcgctcgttg gtccagcaga ggcggccgcc cttgcgcgag cagaatggcg 8820
gtagggggtc tagctgcgtc tcgtccgggg ggtctgcgtc cacggtaaag accccgggca 8880
gcaggcgcgc gtcgaagtag tctatcttgc atccttgcaa gtctagcgcc tgctgccatg 8940
cgcgggcggc aagcgcgcgc tcgtatgggt tgagtggggg accccatggc atggggtggg 9000
tgagcgcgga ggcgtacatg ccgcaaatgt cgtaaacgta gaggggctct ctgagtattc 9060
caagatatgt agggtagcat cttccaccgc ggatgctggc gcgcacgtaa tcgtatagtt 9120
cgtgcgaggg agcgaggagg tcgggaccga ggttgctacg ggcgggctgc tctgctcgga 9180
agactatctg cctgaagatg gcatgtgagt tggatgatat ggttggacgc tggaagacgt 9240
tgaagctggc gtctgtgaga cctaccgcgt cacgcacgaa ggaggcgtag gagtcgcgca 9300
gcttgttgac cagctcggcg gtgacctgca cgtctagggc gcagtagtcc agggtttcct 9360
tgatgatgtc atacttatcc tgtccctttt ttttccacag ctcgcggttg aggacaaact 9420
cttcgcggtc tttccagtac tcttggatcg gaaacccgtc ggcctccgaa cggtaagagc 9480
ctagcatgta gaactggttg acggcctggt aggcgcagca tcccttttct acgggtagcg 9540
cgtatgcctg cgcggccttc cggagcgagg tgtgggtgag cgcaaaggtg tccctgacca 9600
tgactttgag gtactggtat ttgaagtcag tgtcgtcgca tccgccctgc tcccagagca 9660
aaaagtccgt gcgctttttg gaacgcggat ttggcagggc gaaggtgaca tcgttgaaga 9720
gtatctttcc cgcgcgaggc ataaagttgc gtgtgatgcg gaagggtccc ggcacctcgg 9780
aacggttgtt aattacctgg gcggcgagca cgatctcgtc aaagccgttg atgttgtggc 9840
ccacaatgta aagttccaag aagcgcggga tgcccttgat ggaaggcaat tttttaagtt 9900
cctcgtaggt gagctcttca ggggagctga gcccgtgctc tgaaagggcc cagtctgcaa 9960
gatgagggtt ggaagcgacg aatgagctcc acaggtcacg ggccattagc atttgcaggt 10020
ggtcgcgaaa ggtcctaaac tggcgaccta tggccatttt ttctggggtg atgcagtaga 10080
aggtaagcgg gtcttgttcc cagcggtccc atccaaggtt cgcggctagg tctcgcgcgg 10140
cagtcactag aggctcatct ccgccgaact tcatgaccag catgaagggc acgagctgct 10200
tcccaaaggc ccccatccaa gtataggtct ctacatcgta ggtgacaaag agacgctcgg 10260
tgcgaggatg cgagccgatc gggaagaact ggatctcccg ccaccaattg gaggagtggc 10320
tattgatgtg gtgaaagtag aagtccctgc gacgggccga acactcgtgc tggcttttgt 10380
aaaaacgtgc gcagtactgg cagcggtgca cgggctgtac atcctgcacg aggttgacct 10440
gacgaccgcg cacaaggaag cagagtggga atttgagccc ctcgcctggc gggtttggct 10500
ggtggtcttc tacttcggct gcttgtcctt gaccgtctgg ctgctcgagg ggagttacgg 10560
tggatcggac caccacgccg cgcgagccca aagtccagat gtccgcgcgc ggcggtcgga 10620
gcttgatgac aacatcgcgc agatgggagc tgtccatggt ctggagctcc cgcggcgtca 10680
ggtcaggcgg gagctcctgc aggtttacct cgcatagacg ggtcagggcg cgggctagat 10740
ccaggtgata cctaatttcc aggggctggt tggtggcggc gtcgatggct tgcaagaggc 10800
cgcatccccg cggcgcgact acggtaccgc gcggcgggcg gtgggccgcg ggggtgtcct 10860
tggatgatgc atctaaaagc ggtgacgcgg gcgagccccc ggaggtaggg ggggctccgg 10920
acccgccggg agagggggca ggggcacgtc ggcgccgcgc gcgggcagga gctggtgctg 10980
cgcgcgtagg ttgctggcga acgcgacgac gcggcggttg atctcctgaa tctggcgcct 11040

ctgcgtgaag acgacgggcc cggtgagctt gaacctgaaa gagagttcga cagaatcaat 11100
ttcggtgtcg ttgacggcgg cctggcgcaa aatctcctgc acgtctcctg agttgtcttg 11160
ataggcgatc tcggccatga actgctcgat ctcttcctcc tggagatctc cgcgtccggc 11220
tcgctccacg gtggcggcga ggtcgttgga aatgcgggcc atgagctgcg agaaggcgtt 11280
gaggcctccc tcgttccaga cgcggctgta gaccacgccc ccttcggcat cgcgggcgcg 11340
catgaccacc tgcgcgagat tgagctccac gtgccgggcg aagacggcgt agtttcgcag 11400
gcgctgaaag aggtagttga gggtggtggc ggtgtgttct gccacgaaga agtacataac 11460
ccagcgtcgc aacgtggatt cgttgatatc ccccaaggcc tcaaggcgct ccatggcctc 11520
gtagaagtcc acggcgaagt tgaaaaactg ggagttgcgc gccgacacgg ttaactcctc 11580
ctccagaaga cggatgagct cggcgacagt gtcgcgcacc tcgcgctcaa aggctacagg 11640
ggcctcttct tcttcttcaa tctcctcttc cataagggcc tccccttctt cttcttctgg 11700
cggcggtggg ggagggggga cacggcggcg acgacggcgc accgggaggc ggtcgacaaa 11760
gcgctcgatc atctccccgc ggcgacggcg catggtctcg gtgacggcgc ggccgttctc 11820
gcgggggcgc agttggaaga cgccgcccgt catgtcccgg ttatgggttg gcggggggct 11880
gccatgcggc agggatacgg cgctaacgat gcatctcaac aattgttgtg taggtactcc 11940
gccgccgagg gacctgagcg agtccgcatc gaccggatcg gaaaacctct cgagaaaggc 12000
gtctaaccag tcacagtcgc aaggtaggct gagcaccgtg gcgggcggca gcgggcggcg 12060
gtcggggttg tttctggcgg aggtgctgct gatgatgtaa ttaaagtagg cggtcttgag 12120
acggcggatg gtcgacagaa gcaccatgtc cttgggtccg gcctgctgaa tgcgcaggcg 12180
gtcggccatg ccccaggctt cgttttgaca tcggcgcagg tctttgtagt agtcttgcat 12240
gagcctttct accggcactt cttcttctcc ttcctcttgt cctgcatctc ttgcatctat 12300
cgctgcggcg gcggcggagt ttggccgtag gtggcgccct cttcctccca tgcgtgtgac 12360
cccgaagccc ctcatcggct gaagcagggc taggtcggcg acaacgcgct cggctaatat 12420
ggcctgctgc acctgcgtga gggtagactg gaagtcatcc atgtccacaa agcggtggta 12480
tgcgcccgtg ttgatggtgt aagtgcagtt ggccataacg gaccagttaa cggtctggtg 12540
acccggctgc gagagctcgg tgtacctgag acgcgagtaa gccctcgagt caaatacgta 12600
gtcgttgcaa gtccgcacca ggtactggta tcccaccaaa aagtgcggcg gcggctggcg 12660
gtagaggggc cagcgtaggg tggccggggc tccgggggcg agatcttcca acataaggcg 12720
atgatatccg tagatgtacc tggacatcca ggtgatgccg gcggcggtgg tggaggcgcg 12780
cggaaagtcg cggacgcggt tccagatgtt gcgcagcggc aaaaagtgct ccatggtcgg 12840
gacgctctgg ccggtcaggc gcgcgcaatc gttgacgctc tagcgtgcaa aaggagagcc 12900
tgtaagcggg cactcttccg tggtctggtg gataaattcg caagggtatc atggcggacg 12960
accggggttc gagccccgta tccggccgtc cgccgtgatc catgcggtta ccgcccgcgt 13020
gtcgaaccca ggtgtgcgac gtcagacaac gggggagtgc tccttttggc ttccttccag 13080
gcgcggcggc tgctgcgcta gcttttttgg ccactggccg cgcgcagcgt aagcggttag 13140
gctggaaagc gaaagcatta agtggctcgc tccctgtagc cggagggtta ttttccaagg 13200
gttgagtcgc gggacccccg gttcgagtct cggaccggcc ggactgcggc gaacgggggt 13260
ttgcctcccc gtcatgcaag accccgcttg caaattcctc cggaaacagg gacgagcccc 13320

ttttttgctt ttcccagatg catccggtgc tgcggcagat gcgcccccct cctcagcagc 13380
ggcaagagca agagcagcgg cagacatgca gggcaccctc ccctcctcct accgcgtcag 13440
gaggggcgac atccgcggtt gacgcggcag cagatggtga ttacgaaccc ccgcggcgcc 13500
gggcccggca ctacctggac ttggaggagg gcgagggcct ggcgcggcta ggagcgccct 13560
ctcctgagcg gcacccaagg gtgcagctga agcgtgatac gcgtgaggcg tacgtgccgc 13620
ggcagaacct gtttcgcgac cgcgagggag aggagcccga ggagatgcgg gatcgaaagt 13680
tccacgcagg gcgcgagctg cggcatggcc tgaatcgcga gcggttgctg cgcgaggagg 13740
actttgagcc cgacgcgcga accgggatta gtcccgcgcg cgcacacgtg gcggccgccg 13800
acctggtaac cgcatacgag cagacggtga accaggagat taactttcaa aaaagcttta 13860
acaaccacgt gcgtacgctt gtggcgcgcg aggaggtggc tataggactg atgcatctgt 13920
gggactttgt aagcgcgctg gagcaaaacc caaatagcaa gccgctcatg gcgcagctgt 13980
tccttatagt gcagcacagc agggacaacg aggcattcag ggatgcgctg ctaaacatag 14040
tagagcccga gggccgctgg ctgctcgatt tgataaacat cctgcagagc atagtggtgc 14100
aggagcgcag cttgagcctg gctgacaagg tggccgccat caactattcc atgcttagcc 14160
tgggcaagtt ttacgcccgc aagatatacc atacccctta cgttcccata gacaaggagg 14220
taaagatcga ggggttctac atgcgcatgg cgctgaaggt gcttaccttg agcgacgacc 14280
tgggcgttta tcgcaacgag cgcatccaca aggccgtgag cgtgagccgg cggcgcgagc 14340
tcagcgaccg cgagctgatg cacagcctgc aaagggccct ggctggcacg ggcagcggcg 14400
atagagaggc cgagtcctac tttgacgcgg gcgctgacct gcgctgggcc ccaagccgac 14460
gcgccctgga ggcagctggg gccggacctg ggctggcggt ggcacccgcg cgcgctggca 14520
acgtcggcgg cgtggaggaa tatgacgagg acgatgagta cgagccagag gacggcgagt 14580
actaagcggt gatgtttctg atcagatgat gcaagacgca acggacccgg cggtgcgggc 14640
ggcgctgcag agccagccgt ccggccttaa ctccacggac gactggcgcc aggtcatgga 14700
ccgcatcatg tcgctgactg cgcgcaatcc tgacgcgttc cggcagcagc cgcaggccaa 14760
ccggctctcc gcaattctgg aagcggtggt cccggcgcgc gcaaacccca cgcacgagaa 14820
ggtgctggcg atcgtaaacg cgctggccga aaacagggcc atccggcccg acgaggccgg 14880
cctggtctac gacgcgctgc ttcagcgcgt ggctcgttac aacagcggca acgtgcagac 14940
caacctggac cggctggtgg gggatgtgcg cgaggccgtg gcgcagcgtg agcgcgcgca 15000
gcagcagggc aacctgggct ccatggttgc actaaacgcc ttcctgagta cacagcccgc 15060
caacgtgccg cggggacagg aggactacac caactttgtg agcgcactgc ggctaatggt 15120
gactgagaca ccgcaaagtg aggtgtacca gtctgggcca gactattttt tccagaccag 15180
tagacaaggc ctgcagaccg taaacctgag ccaggctttc aaaaacttgc aggggctgtg 15240
gggggtgcgg gctcccacag gcgaccgcgc gaccgtgtct agcttgctga cgcccaactc 15300
gcgcctgttg ctgctgctaa tagcgccctt cacggacagt ggcagcgtgt cccgggacac 15360
atacctaggt cacttgctga cactgtaccg cgaggccata ggtcaggcgc atgtggacga 15420
gcatactttc caggagatta caagtgtcag ccgcgcgctg gggcaggagg acacgggcag 15480
cctggaggca accctaaact acctgctgac caaccggcgg cagaagatcc cctcgttgca 15540
cagtttaaac agcgaggagg agcgcatttt gcgctacgtg cagcagagcg tgagccttaa 15600

cctgatgcgc gacggggtaa cgcccagcgt ggcgctggac atgaccgcgc gcaacatgga 15660
accgggcatg tatgcctcaa accggccgtt tatcaaccgc ctaatggact acttgcatcg 15720
cgcggccgcc gtgaaccccg agtatttcac caatgccatc ttgaacccgc actggctacc 15780
gccccctggt ttctacaccg ggggattcga ggtgcccgag ggtaacgatg gattcctctg 15840
ggacgacata gacgacagcg tgttttcccc gcaaccgcag accctgctag agttgcaaca 15900
gcgcgagcag gcagaggcgg cgctgcgaaa ggaaagcttc cgcaggccaa gcagcttgtc 15960
cgatctaggc gctgcggccc cgcggtcaga tgctagtagc ccatttccaa gcttgatagg 16020
gtctcttacc agcactcgca ccacccgccc gcgcctgctg ggcgaggagg agtacctaaa 16080
caactcgctg ctgcagccgc agcgcgaaaa aaacctgcct ccggcatttc ccaacaacgg 16140
gatagagagc ctagtggaca agatgagtag atggaagacg tacgcgcagg agcacaggga 16200
cgtgccaggc ccgcgcccgc ccacccgtcg tcaaaggcac gaccgtcagc ggggtctggt 16260
gtgggaggac gatgactcgg cagacgacag cagcgtcctg gatttgggag ggagtggcaa 16320
cccgtttgcg caccttcgcc ccaggctggg gagaatgttt taaaaaaaaa aaaagcatga 16380
tgcaaaataa aaaactcacc aaggccatgg caccgagcgt tggttttctt gtattcccct 16440
tagtatgcgg cgcgcggcga tgtatgagga aggtcctcct ccctcctacg agagtgtggt 16500
gagcgcggcg ccagtggcgg cggcgctggg ttctcccttc gatgctcccc tggacccgcc 16560
gtttgtgcct ccgcggtacc tgcggcctac cggggggaga aacagcatcc gttactctga 16620
gttggcaccc ctattcgaca ccacccgtgt gtacctggtg gacaacaagt caacggatgt 16680
ggcatccctg aactaccaga acgaccacag caactttctg accacggtca ttcaaaacaa 16740
tgactacagc ccgggggagg caagcacaca gaccatcaat cttgacgacc ggtcgcactg 16800
gggcggcgac ctgaaaacca tcctgcatac caacatgcca aatgtgaacg agttcatgtt 16860
taccaataag tttaaggcgc gggtgatggt gtcgcgcttg cctactaagg acaatcaggt 16920
ggagctgaaa tacgagtggg tggagttcac gctgcccgag ggcaactact ccgagaccat 16980
gaccatagac cttatgaaca acgcgatcgt ggagcactac ttgaaagtgg gcagacagaa 17040
cggggttctg gaaagcgaca tcggggtaaa gtttgacacc cgcaacttca gactggggtt 17100
tgaccccgtc actggtcttg tcatgcctgg ggtatataca aacgaagcct tccatccaga 17160
catcattttg ctgccaggat gcggggtgga cttcacccac agccgcctga gcaacttgtt 17220
gggcatccgc aagcggcaac ccttccagga gggctttagg atcacctacg atgatctgga 17280
gggtggtaac attcccgcac tgttggatgt ggacgcctac caggcgagct tgaaagatga 17340
caccgaacag ggcgggggtg gcgcaggcgg cagcaacagc agtggcagcg gcgcggaaga 17400
gaactccaac gcggcagccg cggcaatgca gccggtggag gacatgaacg atcatgccat 17460
tcgcggcgac acctttgcca cacgggctga ggagaagcgc gctgaggccg aagcagcggc 17520
cgaagctgcc gcccccgctg cgcaacccga ggtcgagaag cctcagaaga aaccggtgat 17580
caaacccctg acagaggaca gcaagaaacg cagttacaac ctaataagca atgacagcac 17640
cttcacccag taccgcagct ggtaccttgc atacaactac ggcgaccctc agaccggaat 17700
ccgctcatgg accctgcttt gcactcctga cgtaacctgc ggctcggagc aggtctactg 17760
gtcgttgcca gacatgatgc aagaccccgt gaccttccgc tccacgcgcc agatcagcaa 17820
ctttccggtg gtgggcgccg agctgttgcc cgtgcactcc aagagcttct acaacgacca 17880

ggccgtctac tcccaactca tccgccagtt tacctctctg acccacgtgt tcaatcgctt 17940
tcccgagaac cagattttgg cgcgcccgcc agcccccacc atcaccaccg tcagtgaaaa 18000
cgttcctgct ctcacagatc acgggacgct accgctgcgc aacagcatcg gaggagtcca 18060
gcgagtgacc attactgacg ccagacgccg cacctgcccc tacgtttaca aggccctggg 18120
catagtctcg ccgcgcgtcc tatcgagccg cactttttga gcaagcatgt ccatccttat 18180
atcgcccagc aataacacag gctggggcct gcgcttccca agcaagatgt ttggcggggc 18240
caagaagcgc tccgaccaac acccagtgcg cgtgcgcggg cactaccgcg cgccctgggg 18300
cgcgcacaaa cgcggccgca ctgggcgcac caccgtcgat gacgccatcg acgcggtggt 18360
ggaggaggcg cgcaactaca cgcccacgcc gccaccagtg tccacagtgg acgcggccat 18420
tcagaccgtg gtgcgcggag cccggcgcta tgctaaaatg aagagacggc ggaggcgcgt 18480
agcacgtcgc caccgccgcc gacccggcac tgccgcccaa cgcgcggcgg cggccctgct 18540
taaccgcgca cgtcgcaccg gccgacgggc ggccatgcgg gccgctcgaa ggctggccgc 18600
gggtattgtc actgtgcccc ccaggtccag gcgacgagcg gccgccgcag cagccgcggc 18660
cattagtgct atgactcagg gtcgcagggg caacgtgtat tgggtgcgcg actcggttag 18720
cggcctgcgc gtgcccgtgc gcacccgccc cccgcgcaac tagattgcaa gaaaaaacta 18780
cttagactcg tactgttgta tgtatccagc ggcggcggcg cgcaacgaag ctatgtccaa 18840
gcgcaaaatc aaagaagaga tgctccaggt catcgcgccg gagatctatg gccccccgaa 18900
gaaggaagag caggattaca agccccgaaa gctaaagcgg gtcaaaaaga aaaagaaaga 18960
tgatgatgat gaacttgacg acgaggtgga actgctgcac gctaccgcgc ccaggcgacg 19020
ggtacagtgg aaaggtcgac gcgtaaaacg tgttttgcga cccggcacca ccgtagtctt 19080
tacgcccggt gagcgctcca cccgcaccta caagcgcgtg tatgatgagg tgtacggcga 19140
cgaggacctg cttgagcagg ccaacgagcg cctcggggag tttgcctacg gaaagcggca 19200
taaggacatg ctggcgttgc cgctggacga gggcaaccca acacctagcc taaagcccgt 19260
aacactgcag caggtgctgc ccgcgcttgc accgtccgaa gaaaagcgcg gcctaaagcg 19320
cgagtctggt gacttggcac ccaccgtgca gctgatggta cccaagcgcc agcgactgga 19380
agatgtcttg gaaaaaatga ccgtggaacc tgggctggag cccgaggtcc gcgtgcggcc 19440
aatcaagcag gtggcgccgg gactgggcgt gcagaccgtg gacgttcaga tacccactac 19500
cagtagcacc agtattgcca ccgccacaga gggcatggag acacaaacgt ccccggttgc 19560
ctcagcggtg gcggatgccg cggtgcaggc ggtcgctgcg gccgcgtcca agacctctac 19620
ggaggtgcaa acggacccgt ggatgtttcg cgtttcagcc ccccggcgcc cgcgccgttc 19680
gaggaagtac ggcgccgcca gcgcgctact gcccgaatat gccctacatc cttccattgc 19740
gcctaccccc ggctatcgtg gctacaccta ccgccccaga agacgagcaa ctacccgacg 19800
ccgaaccacc actggaaccc gccgccgccg tcgccgtcgc cagcccgtgc tggccccgat 19860
ttccgtgcgc agggtggctc gcgaaggagg caggaccctg gtgctgccaa cagcgcgcta 19920
ccaccccagc atcgtttaaa agccggtctt tgtggttctt gcagatatgg ccctcacctg 19980
ccgcctccgt ttcccggtgc cgggattccg aggaagaatg caccgtagga ggggcatggc 20040
cggccacggc ctgacgggcg gcatgcgtcg tgcgcaccac cggcggcggc gcgcgtcgca 20100
ccgtcgcatg cgcggcggta tcctgcccct ccttattcca ctgatcgccg cggcgattgg 20160

cgccgtgccc ggaattgcat ccgtggcctt gcaggcgcag agacactgat taaaaacaag 20220
ttgcatgtgg aaaaatcaaa ataaaaagtc tggactctca cgctcgcttg gtcctgtaac 20280
tattttgtag aatggaagac atcaactttg cgtctctggc cccgcgacac ggctcgcgcc 20340
cgttcatggg aaactggcaa gatatcggca ccagcaatat gagcggtggc gccttcagct 20400
ggggctcgct gtggagcggc attaaaaatt tcggttccac cgttaagaac tatggcagca 20460
aggcctggaa cagcagcaca ggccagatgc tgagggataa gttgaaagag caaaatttcc 20520
aacaaaaggt ggtagatggc ctggcctctg gcattagcgg ggtggtggac ctggccaacc 20580
aggcagtgca aaataagatt aacagtaagc ttgatccccg ccctcccgta gaggagcctc 20640
caccggccgt ggagacagtg tctccagagg ggcgtggcga aaagcgtccg cgccccgaca 20700
gggaagaaac tctggtgacg caaatagacg agcctccctc gtacgaggag gcactaaagc 20760
aaggcctgcc caccacccgt cccatcgcgc ccatggctac cggagtgctg ggccagcaca 20820
cacccgtaac gctggacctg cctccccccg ccgacaccca gcagaaacct gtgctgccag 20880
gcccgaccgc cgttgttgta acccgtccta gccgcgcgtc cctgcgccgc gccgccagcg 20940
gtccgcgatc gttgcggccc gtagccagtg gcaactggca aagcacactg aacagcatcg 21000
tgggtctggg ggtgcaatcc ctgaagcgcc gacgatgctt ctgatagcta acgtgtcgta 21060
tgtgtgtcat gtatgcgtcc atgtcgccgc cagaggagct gctgagccgc cgcgcgcccg 21120
ctttccaaga tggctacccc ttcgatgatg ccgcagtggt cttacatgca catctcgggc 21180
caggacgcct cggagtacct gagccccggg ctggtgcagt ttgcccgcgc caccgagacg 21240
tacttcagcc tgaataacaa gtttagaaac cccacggtgg cgcctacgca cgacgtgacc 21300
acagaccggt cccagcgttt gacgctgcgg ttcatccctg tggaccgtga ggatactgcg 21360
tactcgtaca aggcgcggtt caccctagct gtgggtgata accgtgtgct ggacatggct 21420
tccacgtact ttgacatccg cggcgtgctg gacaggggcc ctacttttaa gccctactct 21480
ggcactgcct acaacgccct ggctcccaag ggtgccccaa atccttgcga atgggatgaa 21540
gctgctactg ctcttgaaat aaacctagaa gaagaggacg atgacaacga agacgaagta 21600
gacgagcaag ctgagcagca aaaaactcac gtatttgggc aggcgcctta ttctggtata 21660
aatattacaa aggagggtat tcaaataggt gtcgaaggtc aaacacctaa atatgccgat 21720
aaaacatttc aacctgaacc tcaaatagga gaatctcagt ggtacgaaac agaaattaat 21780
catgcagctg ggagagtcct aaaaaagact accccaatga aaccatgtta cggttcatat 21840
gcaaaaccca caaatgaaaa tggagggcaa ggcattcttg taaagcaaca aaatggaaag 21900
ctagaaagtc aagtggaaat gcaatttttc tcaactactg aggcagccgc aggcaatggt 21960
gataacttga ctcctaaagt ggtattgtac agtgaagatg tagatataga aaccccagac 22020
actcatattt cttacatgcc cactattaag gaaggtaact cacgagaact aatgggccaa 22080
caatctatgc ccaacaggcc taattacatt gcttttaggg acaattttat tggtctaatg 22140
tattacaaca gcacgggtaa tatgggtgtt ctggcgggcc aagcatcgca gttgaatgct 22200
gttgtagatt tgcaagacag aaacacagag ctttcatacc agcttttgct tgattccatt 22260
ggtgatagaa ccaggtactt ttctatgtgg aatcaggctg ttgacagcta tgatccagat 22320
gttagaatta ttgaaaatca tggaactgaa gatgaacttc caaattactg ctttccactg 22380
ggaggtgtga ttaatacaga gactcttacc aaggtaaaac ctaaaacagg tcaggaaaat 22440

ggatgggaaa aagatgctac agaattttca gataaaaatg aaataagagt tggaaataat 22500
tttgccatgg aaatcaatct aaatgccaac ctgtggagaa atttcctgta ctccaacata 22560
gcgctgtatt tgcccgacaa gctaaagtac agtccttcca acgtaaaaat ttctgataac 22620
ccaaacacct acgactacat gaacaagcga gtggtggctc ccgggctagt ggactgctac 22680
attaaccttg gagcacgctg gtcccttgac tatatggaca acgtcaaccc atttaaccac 22740
caccgcaatg ctggcctgcg ctaccgctca atgttgctgg gcaatggtcg ctatgtgccc 22800
ttccacatcc aggtgcctca gaagttcttt gccattaaaa acctccttct cctgccgggc 22860
tcatacacct acgagtggaa cttcaggaag gatgttaaca tggttctgca gagctcccta 22920
ggaaatgacc taagggttga cggagccagc attaagtttg atagcatttg cctttacgcc 22980
accttcttcc ccatggccca caacaccgcc tccacgcttg aggccatgct tagaaacgac 23040
accaacgacc agtcctttaa cgactatctc tccgccgcca acatgctcta ccctataccc 23100
gccaacgcta ccaacgtgcc catatccatc ccctcccgca actgggcggc tttccgcggc 23160
tgggccttca cgcgccttaa gactaaggaa accccatcac tgggctcggg ctacgaccct 23220
tattacacct actctggctc tataccctac ctagatggaa ccttttacct caaccacacc 23280
tttaagaagg tggccattac ctttgactct tctgtcagct ggcctggcaa tgaccgcctg 23340
cttaccccca acgagtttga aattaagcgc tcagttgacg gggagggtta caacgttgcc 23400
cagtgtaaca tgaccaaaga ctggttcctg gtacaaatgc tagctaacta taacattggc 23460
taccagggct tctatatccc agagagctac aaggaccgca tgtactcctt ctttagaaac 23520
ttccagccca tgagccgtca ggtggtggat gatactaaat acaaggacta ccaacaggtg 23580
ggcatcctac accaacacaa caactctgga tttgttggct accttgcccc caccatgcgc 23640
gaaggacagg cctaccctgc taacttcccc tatccgctta taggcaagac cgcagttgac 23700
agcattaccc agaaaaagtt tctttgcgat cgcacccttt ggcgcatccc attctccagt 23760
aactttatgt ccatgggcgc actcacagac ctgggccaaa accttctcta cgccaactcc 23820
gcccacgcgc tagacatgac ttttgaggtg gatcccatgg acgagcccac ccttctttat 23880
gttttgtttg aagtctttga cgtggtccgt gtgcaccagc cgcaccgcgg cgtcatcgaa 23940
accgtgtacc tgcgcacgcc cttctcggcc ggcaacgcca caacataaag aagcaagcaa 24000
catcaacaac agctgccgcc atgggctcca gtgagcagga actgaaagcc attgtcaaag 24060
atcttggttg tgggccatat tttttgggca cctatgacaa gcgctttcca ggctttgttt 24120
ctccacacaa gctcgcctgc gccatagtca atacggccgg tcgcgagact gggggcgtac 24180
actggatggc ctttgcctgg aacccgcact caaaaacatg ctacctcttt gagccctttg 24240
gcttttctga ccagcgactc aagcaggttt accagtttga gtacgagtca ctcctgcgcc 24300
gtagcgccat tgcttcttcc cccgaccgct gtataacgct ggaaaagtcc acccaaagcg 24360
tacaggggcc caactcggcc gcctgtggac tattctgctg catgtttctc cacgcctttg 24420
ccaactggcc ccaaactccc atggatcaca accccaccat gaaccttatt accggggtac 24480
ccaactccat gctcaacagt ccccaggtac agcccaccct gcgtcgcaac caggaacagc 24540
tctacagctt cctggagcgc cactcgccct acttccgcag ccacagtgcg cagattagga 24600
gcgccacttc tttttgtcac ttgaaaaaca tgtaaaaata atgtactaga gacactttca 24660
ataaaggcaa atgcttttat ttgtacactc tcgggtgatt atttaccccc acccttgccg 24720

tctgcgccgt ttaaaaatca aaggggttct gccgcgcatc gctatgcgcc actggcaggg 24780
acacgttgcg atactggtgt ttagtgctcc acttaaactc aggcacaacc atccgcggca 24840
gctcggtgaa gttttcactc cacaggctgc gcaccatcac caacgcgttt agcaggtcgg 24900
gcgccgatat cttgaagtcg cagttggggc ctccgccctg cgcgcgcgag ttgcgataca 24960
cagggttgca gcactggaac actatcagcg ccgggtggtg cacgctggcc agcacgctct 25020
tgtcggagat cagatccgcg tccaggtcct ccgcgttgct cagggcgaac ggagtcaact 25080
ttggtagctg ccttcccaaa aagggcgcgt gcccaggctt tgagttgcac tcgcaccgta 25140
gtggcatcaa aaggtgaccg tgcccggtct gggcgttagg atacagcgcc tgcataaaag 25200
ccttgatctg cttaaaagcc acctgagcct ttgcgccttc agagaagaac atgccgcaag 25260
acttgccgga aaactgattg gccggacagg ccgcgtcgtg cacgcagcac cttgcgtcgg 25320
tgttggagat ctgcaccaca tttcggcccc accggttctt cacgatcttg gccttgctag 25380
actgctcctt cagcgcgcgc tgcccgtttt cgctcgtcac atccatttca atcacgtgct 25440
ccttatttat cataatgctt ccgtgtagac acttaagctc gccttcgatc tcagcgcagc 25500
ggtgcagcca caacgcgcag cccgtgggct cgtgatgctt gtaggtcacc tctgcaaacg 25560
actgcaggta cgcctgcagg aatcgcccca tcatcgtcac aaaggtcttg ttgctggtga 25620
aggtcagctg caacccgcgg tgctcctcgt tcagccaggt cttgcatacg gccgccagag 25680
cttccacttg gtcaggcagt agtttgaagt tcgcctttag atcgttatcc acgtggtact 25740
tgtccatcag cgcgcgcgca gcctccatgc ccttctccca cgcagacacg atcggcacac 25800
tcagcgggtt catcaccgta atttcacttt ccgcttcgct gggctcttcc tcttcctctt 25860
gcgtccgcat accacgcgcc actgggtcgt cttcattcag ccgccgcact gtgcgcttac 25920
ctcctttgcc atgcttgatt agcaccggtg ggttgctgaa acccaccatt tgtagcgcca 25980
catcttctct ttcttcctcg ctgtccacga ttacctctgg tgatggcggg cgctcgggct 26040
tgggagaagg gcgcttcttt ttcttcttgg gcgcaatggc caaatccgcc gccgaggtcg 26100
atggccgcgg gctgggtgtg cgcggcacca gcgcgtcttg tgatgagtct tcctcgtcct 26160
cggactcgat acgccgcctc atccgctttt ttgggggcgc ccggggaggc ggcggcgacg 26220
gggacgggga cgacacgtcc tccatggttg ggggacgtcg cgccgcaccg cgtccgcgct 26280
cgggggtggt ttcgcgctgc tcctcttccc gactggccat ttccttctcc tataggcaga 26340
aaaagatcat ggagtcagtc gagaagaagg acagcctaac cgccccctct gagttcgcca 26400
ccaccgcctc caccgatgcc gccaacgcgc ctaccacctt ccccgtcgag gcacccccgc 26460
ttgaggagga ggaagtgatt atcgagcagg acccaggttt tgtaagcgaa gacgacgagg 26520
accgctcagt accaacagag gataaaaagc aagaccagga caacgcagag gcaaacgagg 26580
aacaagtcgg gcggggggac gaaaggcatg gcgactacct agatgtggga gacgacgtgc 26640
tgttgaagca tctgcagcgc cagtgcgcca ttatctgcga cgcgttgcaa gagcgcagcg 26700
atgtgcccct cgccatagcg gatgtcagcc ttgcctacga acgccaccta ttctcaccgc 26760
gcgtaccccc caaacgccaa gaaaacggca catgcgagcc caacccgcgc ctcaacttct 26820
accccgtatt tgccgtgcca gaggtgcttg ccacctatca catctttttc caaaactgca 26880
agatacccct atcctgccgt gccaaccgca gccgagcgga caagcagctg gccttgcggc 26940
agggcgctgt catacctgat atcgcctcgc tcaacgaagt gccaaaaatc tttgagggtc 27000

ttggacgcga cgagaagcgc gcggcaaacg ctctgcaaca ggaaaacagc gaaaatgaaa 27060
gtcactctgg agtgttggtg gaactcgagg gtgacaacgc gcgcctagcc gtactaaaac 27120
gcagcatcga ggtcacccac tttgcctacc cggcacttaa cctacccccc aaggtcatga 27180
gcacagtcat gagtgagctg atcgtgcgcc gtgcgcagcc cctggagagg gatgcaaatt 27240
tgcaagaaca aacagaggag ggcctacccg cagttggcga cgagcagcta gcgcgctggc 27300
ttcaaacgcg cgagcctgcc gacttggagg agcgacgcaa actaatgatg gccgcagtgc 27360
tcgttaccgt ggagcttgag tgcatgcagc ggttctttgc tgacccggag atgcagcgca 27420
agctagagga aacattgcac tacacctttc gacagggcta cgtacgccag gcctgcaaga 27480
tctccaacgt ggagctctgc aacctggtct cctaccttgg aattttgcac gaaaaccgcc 27540
ttgggcaaaa cgtgcttcat tccacgctca agggcgaggc gcgccgcgac tacgtccgcg 27600
actgcgttta cttatttcta tgctacacct ggcagacggc catgggcgtt tggcagcagt 27660
gcttggagga gtgcaacctc aaggagctgc agaaactgct aaagcaaaac ttgaaggacc 27720
tatggacggc cttcaacgag cgctccgtgg ccgcgcacct ggcggacatc attttccccg 27780
aacgcctgct taaaaccctg caacagggtc tgccagactt caccagtcaa agcatgttgc 27840
agaactttag gaactttatc ctagagcgct caggaatctt gcccgccacc tgctgtgcac 27900
ttcctagcga ctttgtgccc attaagtacc gcgaatgccc tccgccgctt tggggccact 27960
gctaccttct gcagctagcc aactaccttg cctaccactc tgacataatg gaagacgtga 28020
gcggtgacgg tctactggag tgtcactgtc gctgcaacct atgcaccccg caccgctccc 28080
tggtttgcaa ttcgcagctg cttaacgaaa gtcaaattat cggtaccttt gagctgcagg 28140
gtccctcgcc tgacgaaaag tccgcggctc cggggttgaa actcactccg gggctgtgga 28200
cgtcggctta ccttcgcaaa tttgtacctg aggactacca cgcccacgag attaggttct 28260
acgaagacca atcccgcccg cctaatgcgg agcttaccgc ctgcgtcatt acccagggcc 28320
acattcttgg ccaattgcaa gccatcaaca aagcccgcca agagtttctg ctacgaaagg 28380
gacggggggt ttacttggac ccccagtccg gcgaggagct caacccaatc cccccgccgc 28440
cgcagcccta tcagcagcag ccgcgggccc ttgcttccca ggatggcacc caaaaagaag 28500
ctgcagctgc cgccgccacc cacggacgag gaggaatact gggacagtca ggcagaggag 28560
gttttggacg aggaggagga ggacatgatg gaagactggg agagcctaga cgaggaagct 28620
tccgaggtcg aagaggtgtc agacgaaaca ccgtcaccct cggtcgcatt cccctcgccg 28680
gcgccccaga aatcggcaac cggttccagc atggctacaa cctccgctcc tcaggcgccg 28740
ccggcactgc ccgttcgccg acccaaccgt agatgggaca ccactggaac cagggccggt 28800
aagtccaagc agccgccgcc gttagcccaa gagcaacaac agcgccaagg ctaccgctca 28860
tggcgcgggc acaagaacgc catagttgct tgcttgcaag actgtggggg caacatctcc 28920
ttcgcccgcc gctttcttct ctaccatcac ggcgtggcct tcccccgtaa catcctgcat 28980
tactaccgtc atctctacag cccatactgc accggcggca gcggcagcaa cagcagcggc 29040
cacacagaag caaaggcgac cggatagcaa gactctgaca aagcccaaga aatccacagc 29100
ggcggcagca gcaggaggag gagcgctgcg tctggcgccc aacgaacccg tatcgacccg 29160
cgagcttaga aacaggattt ttcccactct gtatgctata tttcaacaga gcaggggcca 29220
agaacaagag ctgaaaataa aaaacaggtc tctgcgatcc ctcacccgca gctgcctgta 29280

tcacaaaagc gaagatcagc ttcggcgcac gctggaagac gcggaggctc tcttcagtaa 29340
atactgcgcg ctgactctta aggactagtt tcgcgccctt tctcaaattt aagcgcgaaa 29400
actacgtcat ctccagcggc cacacccggc gccagcacct gttgtcagcg ccattatgag 29460
caaggaaatt cccacgccct acatgtggag ttaccagcca caaatgggac ttgcggctgg 29520
agctgcccaa gactactcaa cccgaataaa ctacatgagc gcgggacccc acatgatatc 29580
ccgggtcaac ggaatacgcg cccaccgaaa ccgaattctc ctggaacagg cggctattac 29640
caccacacct cgtaataacc ttaatccccg tagttggccc gctgccctgg tgtaccagga 29700
aagtcccgct cccaccactg tggtacttcc cagagacgcc caggccgaag ttcagatgac 29760
taactcaggg gcgcagcttg cgggcggctt tcgtcacagg gtgcggtcgc ccgggcaggg 29820
tataactcac ctgacaatca gagggcgagg tattcagctc aacgacgagt cggtgagctc 29880
ctcgcttggt ctccgtccgg acgggacatt tcagatcggc ggcgccggcc gctcttcatt 29940
cacgcctcgt caggcaatcc taactctgca gacctcgtcc tctgagccgc gctctggagg 30000
cattggaact ctgcaattta ttgaggagtt tgtgccatcg gtctacttta accccttctc 30060
gggacctccc ggccactatc cggatcaatt tattcctaac tttgacgcgg taaaggactc 30120
ggcggacggc tacgactgaa tgttaagtgg agaggcagag caactgcgcc tgaaacacct 30180
ggtccactgt cgccgccaca agtgctttgc ccgcgactcc ggtgagtttt gctactttga 30240
attgcccgag gatcatatcg agggcccggc gcacggcgtc cggcttaccg cccagggaga 30300
gcttgcccgt agcctgattc gggagtttac ccagcgcccc ctgctagttg agcgggacag 30360
gggaccctgt gttctcactg tgatttgcaa ctgtcctaac cctggattac atcaagatct 30420
ttgttgccat ctctgtgctg agtataataa atacagaaat taaaatatac tggggctcct 30480
atcgccatcc tgtaaacgcc accgtcttca cccgcccaag caaaccaagg cgaaccttac 30540
ctggtacttt taacatctct ccctctgtga tttacaacag tttcaaccca gacggagtga 30600
gtctacgaga gaacctctcc gagctcagct actccatcag aaaaaacacc accctcctta 30660
cctgccggga acgtacgagt gcgtcaccgg ccgctgcacc acacctaccg cctgaccgta 30720
aaccagactt tttccggaca gacctcaata actctgttta ccagaacagg aggtgagctt 30780
agaaaaccct tagggtatta ggccaaaggc gcagctactg tggggtttat gaacaattca 30840
agcaactcta cgggctattc taattcaggt ttctctagaa atggacggaa ttattacaga 30900
gcagcgcctg ctagaaagac gcagggcagc ggccgagcaa cagcgcatga atcaagagct 30960
ccaagacatg gttaacttgc accagtgcaa aaggggtatc ttttgtctgg taaagcaggc 31020
caaagtcacc tacgacagta ataccaccgg acaccgcctt agctacaagt tgccaaccaa 31080
gcgtcagaaa ttggtggtca tggtgggaga aaagcccatt accataactc agcactcggt 31140
agaaaccgaa ggctgcattc actcaccttg tcaaggacct gaggatctct gcacccttat 31200
taagaccctg tgcggtctca aagatcttat tccctttaac taataaaaaa aaataataaa 31260
gcatcactta cttaaaatca gttagcaaat ttctgtccag tttattcagc agcacctcct 31320
tgccctcctc ccagctctgg tattgcagct tcctcctggc tgcaaacttt ctccacaatc 31380
taaatggaat gtcagtttcc tcctgttcct gtccatccgc acccactatc ttcatgttgt 31440
tgcagatgaa gcgcgcaaga ccgtctgaag ataccttcaa ccccgtgtat ccatatgaca 31500
cggaaaccgg tcctccaact gtgccttttc ttactcctcc ctttgtatcc cccaatgggt 31560

ttcaagagag tccccctggg gtactctctt tgcgcctatc cgaacctcta gttacctcca 31620
atggcatgct tgcgctcaaa atgggcaacg gcctctctct ggacgaggcc ggcaacctta 31680
cctcccaaaa tgtaaccact gtgagcccac ctctcaaaaa aaccaagtca aacataaacc 31740
tggaaatatc tgcacccctc acagttacct cagaagccct aactgtggct gccgccgcac 31800
ctctaatggt cgcgggcaac acactcacca tgcaatcaca ggccccgcta accgtgcacg 31860
actccaaact tagcattgcc acccaaggac ccctcacagt gtcagaagga aagctagccc 31920
tgcaaacatc aggccccctc accaccaccg atagcagtac ccttactatc actgcctcac 31980
cccctctaac tactgccact ggtagcttgg gcattgactt gaaagagccc atttatacac 32040
aaaatggaaa actaggacta aagtacgggg ctcctttgca tgtaacagac gacctaaaca 32100
ctttgaccgt agcaactggt ccaggtgtga ctattaataa tacttccttg caaactaaag 32160
ttactggagc cttgggtttt gattcacaag gcaatatgca acttaatgta gcaggaggac 32220
taaggattga ttctcaaaac agacgcctta tacttgatgt tagttatccg tttgatgctc 32280
aaaaccaact aaatctaaga ctaggacagg gccctctttt tataaactca gcccacaact 32340
tggatattaa ctacaacaaa ggcctttact tgtttacagc ttcaaacaat tccaaaaagc 32400
ttgaggttaa cctaagcact gccaaggggt tgatgtttga cgctacagcc atagccatta 32460
atgcaggaga tgggcttgaa tttggttcac ctaatgcacc aaacacaaat cccctcaaaa 32520
caaaaattgg ccatggccta gaatttgatt caaacaaggc tatggttcct aaactaggaa 32580
ctggccttag ttttgacagc acaggtgcca ttacagtagg aaacaaaaat aatgataagc 32640
taactttgtg gaccacacca gctccatctc ctaactgtag actaaatgca gagaaagatg 32700
ctaaactcac tttggtctta acaaaatgtg gcagtcaaat acttgctaca gtttcagttt 32760
tggctgttaa aggcagtttg gctccaatat ctggaacagt tcaaagtgct catcttatta 32820
taagatttga cgaaaatgga gtgctactaa acaattcctt cctggaccca gaatattgga 32880
actttagaaa tggagatctt actgaaggca cagcctatac aaacgctgtt ggatttatgc 32940
ctaacctatc agcttatcca aaatctcacg gtaaaactgc caaaagtaac attgtcagtc 33000
aagtttactt aaacggagac aaaactaaac ctgtaacact aaccattaca ctaaacggta 33060
cacaggaaac aggagacaca actccaagtg catactctat gtcattttca tgggactggt 33120
ctggccacaa ctacattaat gaaatatttg ccacatcctc ttacactttt tcatacattg 33180
cccaagaata aagaatcgtt tgtgttatgt ttcaacgtgt ttatttttca attgcccggg 33240
atcggtgatc accgatccag acatgataag atacattgat gagtttggac aaaccacaac 33300
tagaatgcag tgaaaaaaat gctttatttg tgaaatttgt gatgctattg ctttatttgt 33360
aaccattata agctgcaata aacaagttcc cggatcgcga tccggcccga ggctgtagcc 33420
gacgatggtg cgccaggaga gttgttgatt cattgtttgc ctccctgctg cggtttttca 33480
ccgaagttca tgccagtcca gcgtttttgc agcagaaaag ccgccgactt cggtttgcgg 33540
tcgcgagtga agatcccttt cttgttaccg ccaacgcgca atatgccttg cgaggtcgca 33600
aaatcggcga aattccatac ctgttcaccg acgacggcgc tgacgcgatc aaagacgcgg 33660
tgatacatat ccagccatgc acactgatac tcttcactcc acatgtcggt gtacattgag 33720
tgcagcccgg ctaacgtatc cacgccgtat tcggtgatga taatcggctg atgcagtttc 33780
tcctgccagg ccagaagttc tttttccagt accttctctg ccgtttccaa atcgccgctt 33840

tggacatacc atccgtaata acggttcagg cacagcacat caaagagatc gctgatggta 33900
tcggtgtgag cgtcgcagaa cattacattg acgcaggtga tcggacgcgt cgggtcgagt 33960
ttacgcgttg cttccgccag tggcgcgaaa tattcccgtg caccttgcgg acgggtatcc 34020
ggttcgttgg caatactcca catcaccacg cttgggtggt ttttgtcacg cgctatcagc 34080
tctttaatcg cctgtaagtg cgcttgctga gtttccccgt tgactgcctc ttcgctgtac 34140
agttctttcg gcttgttgcc cgcttcgaaa ccaatgccta aagagaggtt aaagccgaca 34200
gcagcagttt catcaatcac cacgatgcca tgttcatctg cccagtcgag catctcttca 34260
gcgtaagggt aatgcgaggt acggtaggag ttggccccaa tccagtccat taatgcgtgg 34320
tcgtgcacca tcagcacgtt atcgaatcct ttgccacgca agtccgcatc ttcatgacga 34380
ccaaagccag taaagtagaa cggtttgtgg ttaatcagga actgttcgcc cttcactgcc 34440
actgaccgga tgccgacgcg aagcgggtag atatcacact ctgtctggct tttggctgtg 34500
acgcacagtt catagagata accttcaccc ggttgccaga ggtgcggatt caccacttgc 34560
aaagtcccgc tagtgccttg tccagttgca accacctgtt gatccgcatc acgcagttca 34620
acgctgacat caccattggc caccacctgc cagtcaacag acgcgtggtt acagtcttgc 34680
gcgacatgcg tcaccacggt gatatcgtcc acccaggtgt tcggcgtggt gtagagcatt 34740
acgctgcgat ggattccggc atagttaaag aaatcatgga agtaagactg ctttttcttg 34800
ccgttttcgt cggtaatcac cattcccggc gggatagtct gccagttcag ttcgttgttc 34860
acacaaacgg tgatacgtac acttttcccg gcaataacat acggcgtgac atcggcttca 34920
aatggcgtat agccgccctg atgctccatc acttcctgat tattgaccca cactttgccg 34980
taatgagtga ccgcatcgaa acgcagcacg atacgctggc ctgcccaacc tttcggtata 35040
aagacttcgc gctgatacca gacgttgccc gcataattac gaatatctgc atcggcgaac 35100
tgatcgttaa aactgcctgg cacagcaatt gcccggcttt cttgtaacgc gctttcccac 35160
caacgctgat caattccaca gttttcgcga tccagactga atgcccacag gccgtcgagt 35220
tttttgattt cacgggttgg ggtttctaca ggacggacca tgcgttcgac ctttctcttc 35280
ttttttgggc ccatgatggc agatccgtat agtgagtcgt attagctggt tctttccgcc 35340
tcagaagcca tagagcccac cgcatcccca gcatgcctgc tattgtcttc ccaatcctcc 35400
cccttgctgt cctgccccac cccacccccc agaatagaat gacacctact cagacaatgc 35460
gatgcaattt cctcatttta ttaggaaagg acagtgggag tggcaccttc cagggtcaag 35520
gaaggcacgg gggaggggca aacaacagat ggctggcaac tagaaggcac agtcgaggct 35580
gatcagcgag ctctagatgc atgctcgagc ggccgccagt gtgatggata tctgcagaat 35640
tccagcacac tggcggccgt tactagtgga tccgagctcg gtacccggcc gttataacac 35700
cactcgacac ggcaccagct caatcagtca cagtgtaaaa aagggccaag tgcagagcga 35760
gtatatatag gactaaaaaa tgacgtaacg gttaaagtcc acaaaaaaca cccagaaaac 35820
cgcacgcgaa cctacgccca gaaacgaaag ccaaaaaacc cacaacttcc tcaaatcgtc 35880
acttccgttt tcccacgtta cgtcacttcc cattttaaga aaactacaat tcccaacaca 35940
tacaagttac tccgccctaa aacctacgtc acccgccccg ttcccacgcc ccgcgccacg 36000
tcacaaactc caccccctca ttatcatatt ggcttcaatc caaaataagg tatattattg 36060
atgatg 36066

<210> 5
<211> 33583
<212> DNA
<213> Artificial

<220>
<223> Synthetic

<400> 5
catcatcaat aatatacctt attttggatt gaagccaata tgataatgag ggggtggagt 60

ttgtgacgtg gcgcggggcg tgggaacggg gcgggtgacg tagtagtgtg gcggaagtgt 120

gatgttgcaa gtgtggcgga acacatgtaa gcgacggatg tggcaaaagt gacgtttttg 180

gtgtgcgccg gtgtacacag gaagtgacaa ttttcgcgcg gttttaggcg gatgttgtag 240

taaatttggg cgtaaccgag taagatttgg ccattttcgc gggaaaactg aataagagga 300

agtgaaatct gaataatttt gtgttactca tagcgcgtaa tatttgtcta gggccgggga 360

tcggtgatca ccgatccaga catgataaga tacattgatg agtttggaca aaccacaact 420

agaatgcagt gaaaaaaatg ctttatttgt gaaatttgtg atgctattgc tttatttgta 480

accattataa gctgcaataa acaagttccc ggatctttct agctagtcta gactagctag 540

actcgagagc ggccgcaatc gataagcttg atgatcccac gtgttcacca cagccagcgg 600

ctgatgtcga accagttcca caggctggcc cacttgtcca gggccagcag gtcctgctcg 660

ttcttctcct gctggttctg gctctcctcg atcaggttgt agatgatctg ggtgtagttg 720

ctgatctcct tgtcccactg cagccaggtc atgttgtccc agatctcgag ctgctggtcc 780

ttcaggtagc gctccacggc cagcacgcgg gcctgcagct gcttgatgcc ccacacggtc 840

agcttcagca tgtgctgctg ggcctcgatg gcgcgcagca ggttgctctg ctgctgcacg 900

atgccgctca gcagctggcg ggcctgggcg gtaagcttgg cgcggctggg ggccacgccc 960

aggggctcga tcttcaccac cttgtacttg tacagctcgc tgcgccagtt gtcgcgcatg 1020

ttgccgccgc cggggcggaa gatctcgttg gtgctgttgt tgccgccgtc gcgggtcagc 1080

agcaggccgg tgatgttgct ctcgcagcgg atcacgccct ggatgggggg ggggtacatg 1140

gcctggccca ccttctgcca catgttgatg atctgcttga tgcggcaggt cagggtgatg 1200

gtgtcgttgc tggtggtgtt gttgctctcg gtgctgttgc tctcccaggt gctgttgaac 1260

aggccgctgg tgttgcagta gaagaactcg ccgccgcaga tgaagctgtg ggtggtgatc 1320

tcgatgtcgc cgccgctgct cttctcgaag atgatggtct tgttcttgaa gtgctcgcgc 1380

agcttcttgg ccacgccgcg cagggtgtcg ttccacttgg cgcggctcac gtggcagtgg 1440

gcctggcgga tgtcgccgat gatgccgccg gtggcgtaga aggcctggcc ggggccgatg 1500

cgcacgccct tgcgggtgtt gttgttgggg cgggtgcagt tgatcttcac ggccttatcc 1560

agctgcacga tgatggtctt ggcgttgttg gtgatgttct cgctgcggat ctggatgccc 1620

tcctcggcca ggctgccgtt cagcagcagc tgggtgctga tcaccggtcg gatgccgtgg 1680

gtgcactgca cggtgctcac gttcttgcag gggccggtac cgttgaactc ggtgtccttg 1740

cacttcagga tggcgaagcc ggcgggggcg cagtagtgga tggggatggg ctcgaagctc 1800

accttggggc aggcctgggt gatggcgctg gtgttgcagt tgatcaggcg gtacttgtcg 1860

gtctcgttct tctcgttgat ctgcaccacg tccagcttgt agaacaggct gtacacctgc 1920

tgcttcttgt ccttcagctc ggtggtgatg ttgaagctac agttgcgcat ctcgttggtc 1980

acgttgctgg cggtggcgtt gcagtccagg gtcacgcaca ggggggtcag cttcacgcag 2040
ggcttcaggc tctggtccca caggctgatg atgtcggtgt gcatctgctc caccatgttg 2100
ttgcgccaca tgttgaagtc ctcggtcacg ttctccaggt ggatctcctg ggggttgggg 2160
tcggtgggca cgcaggcgtg ggtctcccac acgttgtgca cctcggtgtc gtaggccttg 2220
gcgtcgctgg cgcagaacag ggtggtctcg gcgtccttcc acacgggcac gccgtagtac 2280
acggccaccc acaggttctc ggcggcgctg tagatcacca gcatgcccag gatcatggtg 2340
ccccagcgcc acaggttctg ccagctggtc tggatgccgc gcacgcgcat ggtggcgata 2400
tctctagatc gaattctgca gtgatcaggg atcccagatc cgtatagtga gtcgtattag 2460
gtaccggctg cagttggacc tgggagtgga cacctgtgga gagaaaggca aagtggatgt 2520
cattgtcact caagtgtatg gccagatctc aagcctgcca cacctcaagt gaagccaagg 2580
gggtgggcct atagactcta taggcggtac ttacgtcact cttggcacgg ggaatccgcg 2640
ttccaatgca ccgttcccgg ccgcggaggc tggatcggtc ccggtgtctt ctatggaggt 2700
caaaacagcg tggatggcgt ctccaggcga tctgacggtt cactaaacga gctctgctta 2760
tatagacctc ccaccgtaca cgcctaccgc ccatttgcgt caatggggcg gagttgttac 2820
gacattttgg aaagtcccgt tgattttggt gccaaaacaa actcccattg acgtcaatgg 2880
ggtggagact tggaaatccc cgtgagtcaa accgctatcc acgcccattg atgtactgcc 2940
aaaaccgcat caccatggta atagcgatga ctaatacgta gatgtactgc caagtaggaa 3000
agtcccataa ggtcatgtac tgggcataat gccaggcggg ccatttaccg tcattgacgt 3060
caataggggg cgtacttggc atatgataca cttgatgtac tgccaagtgg gcagtttacc 3120
gtaaatactc cacccattga cgtcaatgga aagtccctat tggcgttact atgggaacat 3180
acgtcattat tgacgtcaat gggcgggggt cgttgggcgg tcagccaggc gggccattta 3240
ccgtaagtta tgtaacgcgg aactccatat atgggctatg aactaatgac cccgtaattg 3300
attactatta ataactagta ctgaaatgtg tgggcgtggc ttaagggtgg gaaagaatat 3360
ataaggtggg ggtcttatgt agttttgtat ctgttttgca gcagccgccg ccgccatgag 3420
caccaactcg tttgatggaa gcattgtgag ctcatatttg acaacgcgca tgcccccatg 3480
ggccggggtg cgtcagaatg tgatgggctc cagcattgat ggtcgccccg tcctgcccgc 3540
aaactctact accttgacct acgagaccgt gtctggaacg ccgttggaga ctgcagcctc 3600
cgccgccgct tcagccgctg cagccaccgc ccgcgggatt gtgactgact ttgctttcct 3660
gagcccgctt gcaagcagtg cagcttcccg ttcatccgcc cgcgatgaca agttgacggc 3720
tcttttggca caattggatt ctttgacccg ggaacttaat gtcgtttctc agcagctgtt 3780
ggatctgcgc cagcaggttt ctgccctgaa ggcttcctcc cctcccaatg cggtttaaaa 3840
cataaataaa aaaccagact ctgtttggat ttggatcaag caagtgtctt gctgtcttta 3900
tttaggggtt ttgcgcgcgc ggtaggcccg ggaccagcgg tctcggtcgt tgagggtcct 3960
gtgtattttt tccaggacgt ggtaaaggtg actctggatg ttcagataca tgggcataag 4020
cccgtctctg gggtggaggt agcaccactg cagagcttca tgctgcgggg tggtgttgta 4080
gatgatccag tcgtagcagg agcgctgggc gtggtgccta aaaatgtctt tcagtagcaa 4140
gctgattgcc aggggcaggc ccttggtgta agtgtttaca aagcggttaa gctgggatgg 4200
gtgcatacgt ggggatatga gatgcatctt ggactgtatt tttaggttgg ctatgttccc 4260

agccatatcc ctccggggat tcatgttgtg cagaaccacc agcacagtgt atccggtgca 4320
cttgggaaat ttgtcatgta gcttagaagg aaatgcgtgg aagaacttgg agacgccctt 4380
gtgacctcca agattttcca tgcattcgtc cataatgatg gcaatgggcc cacgggcggc 4440
ggcctgggcg aagatatttc tgggatcact aacgtcatag ttgtgttcca ggatgagatc 4500
gtcataggcc atttttacaa agcgcgggcg gagggtgcca gactgcggta taatggttcc 4560
atccggccca ggggcgtagt taccctcaca gatttgcatt tcccacgctt tgagttcaga 4620
tggggggatc atgtctacct gcggggcgat gaagaaaacg gtttccgggg taggggagat 4680
cagctgggaa gaaagcaggt tcctgagcag ctgcgactta ccgcagccgg tgggcccgta 4740
aatcacacct attaccggct gcaactggta gttaagagag ctgcagctgc cgtcatccct 4800
gagcaggggg gccacttcgt taagcatgtc cctgactcgc atgttttccc tgaccaaatc 4860
cgccagaagg cgctcgccgc ccagcgatag cagttcttgc aaggaagcaa agtttttcaa 4920
cggtttgaga ccgtccgccg taggcatgct tttgagcgtt tgaccaagca gttccaggcg 4980
gtcccacagc tcggtcacct gctctacggc atctcgatcc agcatatctc ctcgtttcgc 5040
gggttggggc ggctttcgct gtacggcagt agtcggtgct cgtccagacg ggccagggtc 5100
atgtctttcc acgggcgcag ggtcctcgtc agcgtagtct gggtcacggt gaaggggtgc 5160
gctccgggct gcgcgctggc cagggtgcgc ttgaggctgg tcctgctggt gctgaagcgc 5220
tgccggtctt cgccctgcgc gtcggccagg tagcatttga ccatggtgtc atagtccagc 5280
ccctccgcgg cgtggccctt ggcgcgcagc ttgcccttgg aggaggcgcc gcacgagggg 5340
cagtgcagac ttttgagggc gtagagcttg ggcgcgagaa ataccgattc cggggagtag 5400
gcatccgcgc cgcaggcccc gcagacggtc tcgcattcca cgagccaggt gagctctggc 5460
cgttcggggt caaaaaccag gtttccccca tgctttttga tgcgtttctt acctctggtt 5520
tccatgagcc ggtgtccacg ctcggtgacg aaaaggctgt ccgtgtcccc gtatacagac 5580
ttgagaggcc tgtcctcgag cggtgttccg cggtcctcct cgtatagaaa ctcggaccac 5640
tctgagacaa aggctcgcgt ccaggccagc acgaaggagg ctaagtggga ggggtagcgg 5700
tcgttgtcca ctagggggtc cactcgctcc agggtgtgaa gacacatgtc gccctcttcg 5760
gcatcaagga aggtgattgg tttgtaggtg taggccacgt gaccgggtgt tcctgaaggg 5820
gggctataaa agggggtggg ggcgcgttcg tcctcactct cttccgcatc gctgtctgcg 5880
agggccagct gttggggtga gtactccctc tgaaaagcgg gcatgacttc tgcgctaaga 5940
ttgtcagttt ccaaaaacga ggaggatttg atattcacct ggcccgcggt gatgcctttg 6000
agggtggccg catccatctg gtcagaaaag acaatctttt tgttgtcaag cttggtggca 6060
aacgacccgt agagggcgtt ggacagcaac ttggcgatgg agcgcagggt ttggtttttg 6120
tcgcgatcgg cgcgctcctt ggccgcgatg tttagctgca cgtattcgcg cgcaacgcac 6180
cgccattcgg gaaagacggt ggtgcgctcg tcgggcacca ggtgcacgcg ccaaccgcgg 6240
ttgtgcaggg tgacaaggtc aacgctggtg gctacctctc cgcgtaggcg ctcgttggtc 6300
cagcagaggc ggccgccctt gcgcgagcag aatggcggta gggggtctag ctgcgtctcg 6360
tccggggggt ctgcgtccac ggtaaagacc ccgggcagca ggcgcgcgtc gaagtagtct 6420
atcttgcatc cttgcaagtc tagcgcctgc tgccatgcgc gggcggcaag cgcgcgctcg 6480
tatgggttga gtgggggacc ccatggcatg gggtgggtga gcgcggaggc gtacatgccg 6540

caaatgtcgt aaacgtagag gggctctctg agtattccaa gatatgtagg gtagcatctt 6600
ccaccgcgga tgctggcgcg cacgtaatcg tatagttcgt gcgagggagc gaggaggtcg 6660
ggaccgaggt tgctacgggc gggctgctct gctcggaaga ctatctgcct gaagatggca 6720
tgtgagttgg atgatatggt tggacgctgg aagacgttga agctggcgtc tgtgagacct 6780
accgcgtcac gcacgaagga ggcgtaggag tcgcgcagct tgttgaccag ctcggcggtg 6840
acctgcacgt ctagggcgca gtagtccagg gtttccttga tgatgtcata cttatcctgt 6900
cccttttttt tccacagctc gcggttgagg acaaactctt cgcggtcttt ccagtactct 6960
tggatcggaa acccgtcggc ctccgaacgg taagagccta gcatgtagaa ctggttgacg 7020
gcctggtagg cgcagcatcc cttttctacg ggtagcgcgt atgcctgcgc ggccttccgg 7080
agcgaggtgt gggtgagcgc aaaggtgtcc ctgaccatga ctttgaggta ctggtatttg 7140
aagtcagtgt cgtcgcatcc gccctgctcc cagagcaaaa agtccgtgcg ctttttggaa 7200
cgcggatttg gcagggcgaa ggtgacatcg ttgaagagta tctttcccgc gcgaggcata 7260
aagttgcgtg tgatgcggaa gggtcccggc acctcggaac ggttgttaat tacctgggcg 7320
gcgagcacga tctcgtcaaa gccgttgatg ttgtggccca caatgtaaag ttccaagaag 7380
cgcgggatgc ccttgatgga aggcaatttt ttaagttcct cgtaggtgag ctcttcaggg 7440
gagctgagcc cgtgctctga aagggcccag tctgcaagat gagggttgga agcgacgaat 7500
gagctccaca ggtcacgggc cattagcatt tgcaggtggt cgcgaaaggt cctaaactgg 7560
cgacctatgg ccattttttc tggggtgatg cagtagaagg taagcgggtc ttgttcccag 7620
cggtcccatc caaggttcgc ggctaggtct cgcgcggcag tcactagagg ctcatctccg 7680
ccgaacttca tgaccagcat gaagggcacg agctgcttcc caaaggcccc catccaagta 7740
taggtctcta catcgtaggt gacaaagaga cgctcggtgc gaggatgcga gccgatcggg 7800
aagaactgga tctcccgcca ccaattggag gagtggctat tgatgtggtg aaagtagaag 7860
tccctgcgac gggccgaaca ctcgtgctgg cttttgtaaa aacgtgcgca gtactggcag 7920
cggtgcacgg gctgtacatc ctgcacgagg ttgacctgac gaccgcgcac aaggaagcag 7980
agtgggaatt tgagcccctc gcctggcggg tttggctggt ggtcttctac ttcggctgct 8040
tgtccttgac cgtctggctg ctcgagggga gttacggtgg atcggaccac cacgccgcgc 8100
gagcccaaag tccagatgtc cgcgcgcggc ggtcggagct tgatgacaac atcgcgcaga 8160
tgggagctgt ccatggtctg gagctcccgc ggcgtcaggt caggcgggag ctcctgcagg 8220
tttacctcgc atagacgggt cagggcgcgg gctagatcca ggtgatacct aatttccagg 8280
ggctggttgg tggcggcgtc gatggcttgc aagaggccgc atccccgcgg cgcgactacg 8340
gtaccgcgcg gcgggcggtg ggccgcgggg gtgtccttgg atgatgcatc taaaagcggt 8400
gacgcgggcg agcccccgga ggtagggggg gctccggacc cgccgggaga gggggcaggg 8460
gcacgtcggc gccgcgcgcg ggcaggagct ggtgctgcgc gcgtaggttg ctggcgaacg 8520
cgacgacgcg gcggttgatc tcctgaatct ggcgcctctg cgtgaagacg acgggcccgg 8580
tgagcttgaa cctgaaagag agttcgacag aatcaatttc ggtgtcgttg acggcggcct 8640
ggcgcaaaat ctcctgcacg tctcctgagt tgtcttgata ggcgatctcg gccatgaact 8700
gctcgatctc ttcctcctgg agatctccgc gtccggctcg ctccacggtg gcggcgaggt 8760
cgttggaaat gcgggccatg agctgcgaga aggcgttgag gcctccctcg ttccagacgc 8820

ggctgtagac cacgccccct tcggcatcgc gggcgcgcat gaccacctgc gcgagattga 8880
gctccacgtg ccgggcgaag acggcgtagt ttcgcaggcg ctgaaagagg tagttgaggg 8940
tggtggcggt gtgttctgcc acgaagaagt acataaccca gcgtcgcaac gtggattcgt 9000
tgatatcccc caaggcctca aggcgctcca tggcctcgta gaagtccacg gcgaagttga 9060
aaaactggga gttgcgcgcc gacacggtta actcctcctc cagaagacgg atgagctcgg 9120
cgacagtgtc gcgcacctcg cgctcaaagg ctacaggggc ctcttcttct tcttcaatct 9180
cctcttccat aagggcctcc ccttcttctt cttctggcgg cggtggggga gggggacac 9240
ggcggcgacg acggcgcacc gggaggcggt cgacaaagcg ctcgatcatc tccccgcggc 9300
gacggcgcat ggtctcggtg acggcgcggc cgttctcgcg ggggcgcagt tggaagacgc 9360
cgcccgtcat gtcccggtta tgggttggcg gggggctgcc atgcggcagg gatacggcgc 9420
taacgatgca tctcaacaat tgttgtgtag gtactccgcc gccgagggac ctgagcgagt 9480
ccgcatcgac cggatcggaa aacctctcga gaaaggcgtc taaccagtca cagtcgcaag 9540
gtaggctgag caccgtggcg ggcggcagcg ggcggcggtc ggggttgttt ctggcggagg 9600
tgctgctgat gatgtaatta aagtaggcgg tcttgagacg gcggatggtc gacagaagca 9660
ccatgtcctt gggtccggcc tgctgaatgc gcaggcggtc ggccatgccc caggcttcgt 9720
tttgacatcg gcgcaggtct ttgtagtagt cttgcatgag cctttctacc ggcacttctt 9780
cttctccttc ctcttgtcct gcatctcttg catctatcgc tgcggcggcg gcggagtttg 9840
gccgtaggtg gcgccctctt cctcccatgc gtgtgacccc gaagcccctc atcggctgaa 9900
gcagggctag gtcggcgaca acgcgctcgg ctaatatggc ctgctgcacc tgcgtgaggg 9960
tagactggaa gtcatccatg tccacaaagc ggtggtatgc gcccgtgttg atggtgtaag 10020
tgcagttggc cataacggac cagttaacgg tctggtgacc cggctgcgag agctcggtgt 10080
acctgagacg cgagtaagcc ctcgagtcaa atacgtagtc gttgcaagtc cgcaccaggt 10140
actggtatcc caccaaaaag tgcggcggcg gctggcggta gaggggccag cgtagggtgg 10200
ccggggctcc gggggcgaga tcttccaaca taaggcgatg atatccgtag atgtacctgg 10260
acatccaggt gatgccggcg gcggtggtgg aggcgcgcgg aaagtcgcgg acgcggttcc 10320
agatgttgcg cagcggcaaa aagtgctcca tggtcgggac gctctggccg gtcaggcgcg 10380
cgcaatcgtt gacgctctag cgtgcaaaag gagagcctgt aagcgggcac tcttccgtgg 10440
tctggtggat aaattcgcaa gggtatcatg gcggacgacc ggggttcgag ccccgtatcc 10500
ggccgtccgc cgtgatccat gcggttaccg cccgcgtgtc gaacccaggt gtgcgacgtc 10560
agacaacggg ggagtgctcc ttttggcttc cttccaggcg cggcggctgc tgcgctagct 10620
tttttggcca ctggccgcgc gcagcgtaag cggttaggct ggaaagcgaa agcattaagt 10680
ggctcgctcc ctgtagccgg agggttattt tccaagggtt gagtcgcggg acccccggtt 10740
cgagtctcgg accggccgga ctgcggcgaa cgggggtttg cctccccgtc atgcaagacc 10800
ccgcttgcaa attcctccgg aaacagggac gagccccttt tttgcttttc ccagatgcat 10860
ccggtgctgc ggcagatgcg cccccctcct cagcagcggc aagagcaaga gcagcggcag 10920
acatgcaggg caccctcccc tcctcctacc gcgtcaggag gggcgacatc cgcggttgac 10980
gcggcagcag atggtgatta cgaacccccg cggcgccggg cccggcacta cctggacttg 11040
gaggagggcg agggcctggc gcggctagga gcgccctctc ctgagcggca cccaagggtg 11100

cagctgaagc gtgatacgcg tgaggcgtac gtgccgcggc agaacctgtt tcgcgaccgc 11160
gagggagagg agcccgagga gatgcgggat cgaaagttcc acgcagggcg cgagctgcgg 11220
catggcctga atcgcgagcg gttgctgcgc gaggaggact ttgagcccga cgcgcgaacc 11280
gggattagtc ccgcgcgcgc acacgtggcg gccgccgacc tggtaaccgc atacgagcag 11340
acggtgaacc aggagattaa ctttcaaaaa agctttaaca accacgtgcg tacgcttgtg 11400
gcgcgcgagg aggtggctat aggactgatg catctgtggg actttgtaag cgcgctggag 11460
caaaacccaa atagcaagcc gctcatggcg cagctgttcc ttatagtgca gcacagcagg 11520
gacaacgagg cattcaggga tgcgctgcta aacatagtag agcccgaggg ccgctggctg 11580
ctcgatttga taaacatcct gcagagcata gtggtgcagg agcgcagctt gagcctggct 11640
gacaaggtgg ccgccatcaa ctattccatg cttagcctgg gcaagtttta cgcccgcaag 11700
atataccata ccccttacgt tcccatagac aaggaggtaa agatcgaggg gttctacatg 11760
cgcatggcgc tgaaggtgct taccttgagc gacgacctgg gcgtttatcg caacgagcgc 11820
atccacaagg ccgtgagcgt gagccggcgg cgcgagctca gcgaccgcga gctgatgcac 11880
agcctgcaaa gggccctggc tggcacgggc agcggcgata gagaggccga gtcctacttt 11940
gacgcgggcg ctgacctgcg ctgggcccca agccgacgcg ccctggaggc agctggggcc 12000
ggacctgggc tggcggtggc acccgcgcgc gctggcaacg tcggcggcgt ggaggaatat 12060
gacgaggacg atgagtacga gccagaggac ggcgagtact aagcggtgat gtttctgatc 12120
agatgatgca agacgcaacg gacccggcgg tgcgggcggc gctgcagagc cagccgtccg 12180
gccttaactc cacggacgac tggcgccagg tcatggaccg catcatgtcg ctgactgcgc 12240
gcaatcctga cgcgttccgg cagcagccgc aggccaaccg gctctccgca attctggaag 12300
cggtggtccc ggcgcgcgca aaccccacgc acgagaaggt gctggcgatc gtaaacgcgc 12360
tggccgaaaa cagggccatc cggcccgacg aggccggcct ggtctacgac gcgctgcttc 12420
agcgcgtggc tcgttacaac agcggcaacg tgcagaccaa cctggaccgg ctggtggggg 12480
atgtgcgcga ggccgtggcg cagcgtgagc gcgcgcagca gcagggcaac ctgggctcca 12540
tggttgcact aaacgccttc ctgagtacac agcccgccaa cgtgccgcgg ggacaggagg 12600
actacaccaa ctttgtgagc gcactgcggc taatggtgac tgagacaccg caaagtgagg 12660
tgtaccagtc tgggccagac tattttttcc agaccagtag acaaggcctg cagaccgtaa 12720
acctgagcca ggctttcaaa aacttgcagg ggctgtgggg ggtgcgggct cccacaggcg 12780
accgcgcgac cgtgtctagc ttgctgacgc ccaactcgcg cctgttgctg ctgctaatag 12840
cgcccttcac ggacagtggc agcgtgtccc gggacacata cctaggtcac ttgctgacac 12900
tgtaccgcga ggccataggt caggcgcatg tggacgagca tactttccag gagattacaa 12960
gtgtcagccg cgcgctgggg caggaggaca cgggcagcct ggaggcaacc ctaaactacc 13020
tgctgaccaa ccggcggcag aagatcccct cgttgcacag tttaaacagc gaggaggagc 13080
gcattttgcg ctacgtgcag cagagcgtga gccttaacct gatgcgcgac ggggtaacgc 13140
ccagcgtggc gctggacatg accgcgcgca acatggaacc gggcatgtat gcctcaaacc 13200
ggccgtttat caaccgccta atggactact tgcatcgcgc ggccgccgtg aaccccgagt 13260
atttcaccaa tgccatcttg aacccgcact ggctaccgcc ccctggtttc tacaccgggg 13320
gattcgaggt gcccgagggt aacgatggat tcctctggga cgacatagac gacagcgtgt 13380

tttccccgca accgcagacc ctgctagagt tgcaacagcg cgagcaggca gaggcggcgc 13440
tgcgaaagga aagcttccgc aggccaagca gcttgtccga tctaggcgct gcggccccgc 13500
ggtcagatgc tagtagccca tttccaagct tgatagggtc tcttaccagc actcgcacca 13560
cccgcccgcg cctgctgggc gaggaggagt acctaaacaa ctcgctgctg cagccgcagc 13620
gcgaaaaaaa cctgcctccg gcatttccca acaacgggat agagagccta gtggacaaga 13680
tgagtagatg gaagacgtac gcgcaggagc acagggacgt gccaggcccg cgcccgccca 13740
cccgtcgtca aaggcacgac cgtcagcggg gtctggtgtg ggaggacgat gactcggcag 13800
acgacagcag cgtcctggat ttgggaggga gtggcaaccc gtttgcgcac cttcgcccca 13860
ggctggggag aatgttttaa aaaaaaaaaa agcatgatgc aaaataaaaa actcaccaag 13920
gccatggcac cgagcgttgg ttttcttgta ttccccttag tatgcggcgc gcggcgatgt 13980
atgaggaagg tcctcctccc tcctacgaga gtgtggtgag cgcggcgcca gtggcggcgg 14040
cgctgggttc tcccttcgat gctcccctgg acccgccgtt tgtgcctccg cggtacctgc 14100
ggcctaccgg ggggagaaac agcatccgtt actctgagtt ggcaccccta ttcgacacca 14160
cccgtgtgta cctggtggac aacaagtcaa cggatgtggc atccctgaac taccagaacg 14220
accacagcaa ctttctgacc acggtcattc aaaacaatga ctacagcccg ggggaggcaa 14280
gcacacagac catcaatctt gacgaccggt cgcactgggg cggcgacctg aaaaccatcc 14340
tgcataccaa catgccaaat gtgaacgagt tcatgtttac caataagttt aaggcgcggg 14400
tgatggtgtc gcgcttgcct actaaggaca atcaggtgga gctgaaatac gagtgggtgg 14460
agttcacgct gcccgagggc aactactccg agaccatgac catagacctt atgaacaacg 14520
cgatcgtgga gcactacttg aaagtgggca gacagaacgg ggttctggaa agcgacatcg 14580
gggtaaagtt tgacacccgc aacttcagac tggggtttga ccccgtcact ggtcttgtca 14640
tgcctggggt atatacaaac gaagccttcc atccagacat cattttgctg ccaggatgcg 14700
gggtggactt cacccacagc cgcctgagca acttgttggg catccgcaag cggcaaccct 14760
tccaggaggg ctttaggatc acctacgatg atctggaggg tggtaacatt cccgcactgt 14820
tggatgtgga cgcctaccag gcgagcttga aagatgacac cgaacagggc gggggtggcg 14880
caggcggcag caacagcagt ggcagcggcg cggaagagaa ctccaacgcg gcagccgcgg 14940
caatgcagcc ggtggaggac atgaacgatc atgccattcg cggcgacacc tttgccacac 15000
gggctgagga gaagcgcgct gaggccgaag cagcggccga agctgccgcc cccgctgcgc 15060
aacccgaggt cgagaagcct cagaagaaac cggtgatcaa acccctgaca gaggacagca 15120
agaaacgcag ttacaaccta ataagcaatg acagcacctt cacccagtac cgcagctggt 15180
accttgcata caactacggc gaccctcaga ccggaatccg ctcatggacc ctgctttgca 15240
ctcctgacgt aacctgcggc tcggagcagg tctactggtc gttgccagac atgatgcaag 15300
accccgtgac cttccgctcc acgcgccaga tcagcaactt tccggtggtg ggcgccgagc 15360
tgttgcccgt gcactccaag agcttctaca acgaccaggc cgtctactcc caactcatcc 15420
gccagtttac ctctctgacc cacgtgttca atcgctttcc cgagaaccag attttggcgc 15480
gcccgccagc ccccaccatc accaccgtca gtgaaaacgt tcctgctctc acagatcacg 15540
ggacgctacc gctgcgcaac agcatcggag gagtccagcg agtgaccatt actgacgcca 15600
gacgccgcac ctgcccctac gtttacaagg ccctgggcat agtctcgccg cgcgtcctat 15660

cgagccgcac tttttgagca agcatgtcca tccttatatc gcccagcaat aacacaggct 15720
ggggcctgcg cttcccaagc aagatgtttg gcggggccaa gaagcgctcc gaccaacacc 15780
cagtgcgcgt gcgcgggcac taccgcgcgc cctggggcgc gcacaaacgc ggccgcactg 15840
ggcgcaccac cgtcgatgac gccatcgacg cggtggtgga ggaggcgcgc aactacacgc 15900
ccacgccgcc accagtgtcc acagtggacg cggccattca gaccgtggtg cgcggagccc 15960
ggcgctatgc taaaatgaag agacggcgga ggcgcgtagc acgtcgccac cgccgccgac 16020
ccggcactgc cgcccaacgc gcggcggcgg ccctgcttaa ccgcgcacgt cgcaccggcc 16080
gacgggcggc catgcgggcc gctcgaaggc tggccgcggg tattgtcact gtgcccccca 16140
ggtccaggcg acgagcggcc gccgcagcag ccgcggccat tagtgctatg actcagggtc 16200
gcaggggcaa cgtgtattgg gtgcgcgact cggttagcgg cctgcgcgtg cccgtgcgca 16260
cccgcccccc gcgcaactag attgcaagaa aaaactactt agactcgtac tgttgtatgt 16320
atccagcggc ggcggcgcgc aacgaagcta tgtccaagcg caaaatcaaa gaagagatgc 16380
tccaggtcat cgcgccggag atctatggcc ccccgaagaa ggaagagcag gattacaagc 16440
cccgaaagct aaagcgggtc aaaaagaaaa agaaagatga tgatgatgaa cttgacgacg 16500
aggtggaact gctgcacgct accgcgccca ggcgacgggt acagtggaaa ggtcgacgcg 16560
taaaacgtgt tttgcgaccc ggcaccaccg tagtctttac gcccggtgag cgctccaccc 16620
gcacctacaa gcgcgtgtat gatgaggtgt acggcgacga ggacctgctt gagcaggcca 16680
acgagcgcct cggggagttt gcctacggaa agcggcataa ggacatgctg gcgttgccgc 16740
tggacgaggg caacccaaca cctagcctaa agcccgtaac actgcagcag gtgctgcccg 16800
cgcttgcacc gtccgaagaa aagcgcggcc taaagcgcga gtctggtgac ttggcaccca 16860
ccgtgcagct gatggtaccc aagcgccagc gactggaaga tgtcttggaa aaaatgaccg 16920
tggaacctgg gctggagccc gaggtccgcg tgcggccaat caagcaggtg gcgccgggac 16980
tgggcgtgca gaccgtggac gttcagatac ccactaccag tagcaccagt attgccaccg 17040
ccacagaggg catggagaca caaacgtccc cggttgcctc agcggtggcg gatgccgcgg 17100
tgcaggcggt cgctgcggcc gcgtccaaga cctctacgga ggtgcaaacg gacccgtgga 17160
tgtttcgcgt ttcagccccc cggcgcccgc gccgttcgag gaagtacggc gccgccagcg 17220
cgctactgcc cgaatatgcc ctacatcctt ccattgcgcc tacccccggc tatcgtggct 17280
acacctaccg ccccagaaga cgagcaacta cccgacgccg aaccaccact ggaacccgcc 17340
gccgccgtcg ccgtcgccag cccgtgctgg ccccgatttc cgtgcgcagg gtggctcgcg 17400
aaggaggcag gaccctggtg ctgccaacag cgcgctacca ccccagcatc gtttaaaagc 17460
cggtctttgt ggttcttgca gatatggccc tcacctgccg cctccgtttc ccggtgccgg 17520
gattccgagg aagaatgcac cgtaggaggg gcatggccgg ccacggcctg acgggcggca 17580
tgcgtcgtgc gcaccaccgg cggcggcgcg cgtcgcaccg tcgcatgcgc ggcggtatcc 17640
tgcccctcct tattccactg atcgccgcgg cgattggcgc cgtgcccgga attgcatccg 17700
tggccttgca ggcgcagaga cactgattaa aaacaagttg catgtggaaa aatcaaaata 17760
aaaagtctgg actctcacgc tcgcttggtc ctgtaactat tttgtagaat ggaagacatc 17820
aactttgcgt ctctggcccc gcgacacggc tcgcgcccgt tcatgggaaa ctggcaagat 17880
atcggcacca gcaatatgag cggtggcgcc ttcagctggg gctcgctgtg gagcggcatt 17940

aaaaatttcg gttccaccgt taagaactat ggcagcaagg cctggaacag cagcacaggc 18000
cagatgctga gggataagtt gaaagagcaa aatttccaac aaaaggtggt agatggcctg 18060
gcctctggca ttagcggggt ggtggacctg gccaaccagg cagtgcaaaa taagattaac 18120
agtaagcttg atccccgccc tcccgtagag gagcctccac cggccgtgga gacagtgtct 18180
ccagaggggc gtggcgaaaa gcgtccgcgc cccgacaggg aagaaactct ggtgacgcaa 18240
atagacgagc ctccctcgta cgaggaggca ctaaagcaag gcctgcccac cacccgtccc 18300
atcgcgccca tggctaccgg agtgctgggc cagcacacac ccgtaacgct ggacctgcct 18360
ccccccgccg acacccagca gaaacctgtg ctgccaggcc cgaccgccgt tgttgtaacc 18420
cgtcctagcc gcgcgtccct gcgccgcgcc gccagcggtc cgcgatcgtt gcggcccgta 18480
gccagtggca actggcaaag cacactgaac agcatcgtgg gtctgggggt gcaatccctg 18540
aagcgccgac gatgcttctg atagctaacg tgtcgtatgt gtgtcatgta tgcgtccatg 18600
tcgccgccag aggagctgct gagccgccgc gcgcccgctt tccaagatgg ctaccccttc 18660
gatgatgccg cagtggtctt acatgcacat ctcgggccag gacgcctcgg agtacctgag 18720
ccccgggctg gtgcagtttg cccgcgccac cgagacgtac ttcagcctga ataacaagtt 18780
tagaaacccc acggtggcgc ctacgcacga cgtgaccaca gaccggtccc agcgtttgac 18840
gctgcggttc atccctgtgg accgtgagga tactgcgtac tcgtacaagg cgcggttcac 18900
cctagctgtg ggtgataacc gtgtgctgga catggcttcc acgtactttg acatccgcgg 18960
cgtgctggac aggggcccta cttttaagcc ctactctggc actgcctaca acgccctggc 19020
tcccaagggt gccccaaatc cttgcgaatg ggatgaagct gctactgctc ttgaaataaa 19080
cctagaagaa gaggacgatg acaacgaaga cgaagtagac gagcaagctg agcagcaaaa 19140
aactcacgta tttgggcagg cgccttattc tggtataaat attacaaagg agggtattca 19200
aataggtgtc gaaggtcaaa cacctaaata tgccgataaa acatttcaac ctgaacctca 19260
aataggagaa tctcagtggt acgaaacaga aattaatcat gcagctggga gagtcctaaa 19320
aaagactacc ccaatgaaac catgttacgg ttcatatgca aaacccacaa atgaaaatgg 19380
agggcaaggc attcttgtaa agcaacaaaa tggaaagcta gaaagtcaag tggaaatgca 19440
atttttctca actactgagg cagccgcagg caatggtgat aacttgactc ctaaagtggt 19500
attgtacagt gaagatgtag atatagaaac cccagacact catatttctt acatgcccac 19560
tattaaggaa ggtaactcac gagaactaat gggccaacaa tctatgccca acaggcctaa 19620
ttacattgct tttagggaca attttattgg tctaatgtat tacaacagca cgggtaatat 19680
gggtgttctg gcgggccaag catcgcagtt gaatgctgtt gtagatttgc aagacagaaa 19740
cacagagctt tcataccagc ttttgcttga ttccattggt gatagaacca ggtacttttc 19800
tatgtggaat caggctgttg acagctatga tccagatgtt agaattattg aaaatcatgg 19860
aactgaagat gaacttccaa attactgctt tccactggga ggtgtgatta atacagagac 19920
tcttaccaag gtaaaaccta aaacaggtca ggaaaatgga tgggaaaaag atgctacaga 19980
attttcagat aaaaatgaaa taagagttgg aaataatttt gccatggaaa tcaatctaaa 20040
tgccaacctg tggagaaatt tcctgtactc caacatagcg ctgtatttgc ccgacaagct 20100
aaagtacagt ccttccaacg taaaaatttc tgataaccca aacacctacg actacatgaa 20160
caagcgagtg gtggctcccg ggctagtgga ctgctacatt aaccttggag cacgctggtc 20220

ccttgactat atggacaacg tcaacccatt taaccaccac cgcaatgctg gcctgcgcta 20280
ccgctcaatg ttgctgggca atggtcgcta tgtgcccttc cacatccagg tgcctcagaa 20340
gttctttgcc attaaaaacc tccttctcct gccgggctca tacacctacg agtggaactt 20400
caggaaggat gttaacatgg ttctgcagag ctccctagga aatgacctaa gggttgacgg 20460
agccagcatt aagtttgata gcatttgcct ttacgccacc ttcttcccca tggcccacaa 20520
caccgcctcc acgcttgagg ccatgcttag aaacgacacc aacgaccagt cctttaacga 20580
ctatctctcc gccgccaaca tgctctaccc tatacccgcc aacgctacca acgtgcccat 20640
atccatcccc tcccgcaact gggcggcttt ccgcggctgg gccttcacgc gccttaagac 20700
taaggaaacc ccatcactgg gctcgggcta cgacccttat tacacctact ctggctctat 20760
accctaccta gatggaacct tttacctcaa ccacaccttt aagaaggtgg ccattacctt 20820
tgactcttct gtcagctggc ctggcaatga ccgcctgctt acccccaacg agtttgaaat 20880
taagcgctca gttgacgggg agggttacaa cgttgcccag tgtaacatga ccaaagactg 20940
gttcctggta caaatgctag ctaactataa cattggctac cagggcttct atatcccaga 21000
gagctacaag gaccgcatgt actccttctt tagaaacttc cagcccatga gccgtcaggt 21060
ggtggatgat actaaataca aggactacca acaggtgggc atcctacacc aacacaacaa 21120
ctctggattt gttggctacc ttgcccccac catgcgcgaa ggacaggcct accctgctaa 21180
cttcccctat ccgcttatag gcaagaccgc agttgacagc attacccaga aaaagtttct 21240
ttgcgatcgc accctttggc gcatcccatt ctccagtaac tttatgtcca tgggcgcact 21300
cacagacctg ggccaaaacc ttctctacgc caactccgcc cacgcgctag acatgacttt 21360
tgaggtggat cccatggacg agcccaccct tctttatgtt ttgtttgaag tctttgacgt 21420
ggtccgtgtg caccagccgc accgcggcgt catcgaaacc gtgtacctgc gcacgccctt 21480
ctcggccggc aacgccacaa cataaagaag caagcaacat caacaacagc tgccgccatg 21540
ggctccagtg agcaggaact gaaagccatt gtcaaagatc ttggttgtgg gccatatttt 21600
ttgggcacct atgacaagcg ctttccaggc tttgtttctc cacacaagct cgcctgcgcc 21660
atagtcaata cggccggtcg cgagactggg ggcgtacact ggatggcctt tgcctggaac 21720
ccgcactcaa aaacatgcta cctctttgag ccctttggct tttctgacca gcgactcaag 21780
caggtttacc agtttgagta cgagtcactc ctgcgccgta gcgccattgc ttcttccccc 21840
gaccgctgta taacgctgga aaagtccacc caaagcgtac aggggcccaa ctcggccgcc 21900
tgtggactat tctgctgcat gtttctccac gcctttgcca actggcccca aactcccatg 21960
gatcacaacc ccaccatgaa ccttattacc ggggtaccca actccatgct caacagtccc 22020
caggtacagc ccaccctgcg tcgcaaccag gaacagctct acagcttcct ggagcgccac 22080
tcgccctact tccgcagcca cagtgcgcag attaggagcg ccacttcttt ttgtcacttg 22140
aaaaacatgt aaaaataatg tactagagac actttcaata aaggcaaatg cttttatttg 22200
tacactctcg ggtgattatt tacccccacc cttgccgtct gcgccgttta aaaatcaaag 22260
gggttctgcc gcgcatcgct atgcgccact ggcagggaca cgttgcgata ctggtgttta 22320
gtgctccact taaactcagg cacaaccatc cgcggcagct cggtgaagtt ttcactccac 22380
aggctgcgca ccatcaccaa cgcgtttagc aggtcgggcg ccgatatctt gaagtcgcag 22440
ttggggcctc cgccctgcgc gcgcgagttg cgatacacag ggttgcagca ctggaacact 22500

atcagcgccg ggtggtgcac gctggccagc acgctcttgt cggagatcag atccgcgtcc 22560
aggtcctccg cgttgctcag ggcgaacgga gtcaactttg gtagctgcct tcccaaaaag 22620
ggcgcgtgcc caggctttga gttgcactcg caccgtagtg gcatcaaaag gtgaccgtgc 22680
ccggtctggg cgttaggata cagcgcctgc ataaaagcct tgatctgctt aaaagccacc 22740
tgagcctttg cgccttcaga gaagaacatg ccgcaagact tgccggaaaa ctgattggcc 22800
ggacaggccg cgtcgtgcac gcagcacctt gcgtcggtgt tggagatctg caccacattt 22860
cggccccacc ggttcttcac gatcttggcc ttgctagact gctccttcag cgcgcgctgc 22920
ccgttttcgc tcgtcacatc catttcaatc acgtgctcct tatttatcat aatgcttccg 22980
tgtagacact taagctcgcc ttcgatctca gcgcagcggt gcagccacaa cgcgcagccc 23040
gtgggctcgt gatgcttgta ggtcacctct gcaaacgact gcaggtacgc ctgcaggaat 23100
cgccccatca tcgtcacaaa ggtcttgttg ctggtgaagg tcagctgcaa cccgcggtgc 23160
tcctcgttca gccaggtctt gcatacggcc gccagagctt ccacttggtc aggcagtagt 23220
ttgaagttcg cctttagatc gttatccacg tggtacttgt ccatcagcgc gcgcgcagcc 23280
tccatgccct tctcccacgc agacacgatc ggcacactca gcgggttcat caccgtaatt 23340
tcactttccg cttcgctggg ctcttcctct tcctcttgcg tccgcatacc acgcgccact 23400
gggtcgtctt cattcagccg ccgcactgtg cgcttacctc ctttgccatg cttgattagc 23460
accggtgggt tgctgaaacc caccatttgt agcgccacat cttctctttc ttcctcgctg 23520
tccacgatta cctctggtga tggcgggcgc tcgggcttgg gagaagggcg cttctttttc 23580
ttcttgggcg caatggccaa atccgccgcc gaggtcgatg gccgcgggct gggtgtgcgc 23640
ggcaccagcg cgtcttgtga tgagtcttcc tcgtcctcgg actcgatacg ccgcctcatc 23700
cgcttttttg ggggcgcccg gggaggcggc ggcgacgggg acggggacga cacgtcctcc 23760
atggttgggg gacgtcgcgc cgcaccgcgt ccgcgctcgg gggtggtttc gcgctgctcc 23820
tcttcccgac tggccatttc cttctcctat aggcagaaaa agatcatgga gtcagtcgag 23880
aagaaggaca gcctaaccgc cccctctgag ttcgccacca ccgcctccac cgatgccgcc 23940
aacgcgccta ccaccttccc cgtcgaggca cccccgcttg aggaggagga agtgattatc 24000
gagcaggacc caggttttgt aagcgaagac gacgaggacc gctcagtacc aacagaggat 24060
aaaaagcaag accaggacaa cgcagaggca aacgaggaac aagtcgggcg gggggacgaa 24120
aggcatggcg actacctaga tgtgggagac gacgtgctgt tgaagcatct gcagcgccag 24180
tgcgccatta tctgcgacgc gttgcaagag cgcagcgatg tgcccctcgc catagcggat 24240
gtcagccttg cctacgaacg ccacctattc tcaccgcgcg taccccccaa acgccaagaa 24300
aacggcacat gcgagcccaa cccgcgcctc aacttctacc ccgtatttgc cgtgccagag 24360
gtgcttgcca cctatcacat ctttttccaa aactgcaaga tacccctatc ctgccgtgcc 24420
aaccgcagcc gagcggacaa gcagctggcc ttgcggcagg gcgctgtcat acctgatatc 24480
gcctcgctca acgaagtgcc aaaaatcttt gagggtcttg gacgcgacga gaagcgcgcg 24540
gcaaacgctc tgcaacagga aaacagcgaa aatgaaagtc actctggagt gttggtggaa 24600
ctcgagggtg acaacgcgcg cctagccgta ctaaaacgca gcatcgaggt cacccacttt 24660
gcctacccgg cacttaacct accccccaag gtcatgagca cagtcatgag tgagctgatc 24720
gtgcgccgtg cgcagcccct ggagagggat gcaaatttgc aagaacaaac agaggagggc 24780

ctacccgcag ttggcgacga gcagctagcg cgctggcttc aaacgcgcga gcctgccgac 24840
ttggaggagc gacgcaaact aatgatggcc gcagtgctcg ttaccgtgga gcttgagtgc 24900
atgcagcggt tctttgctga cccggagatg cagcgcaagc tagaggaaac attgcactac 24960
acctttcgac agggctacgt acgccaggcc tgcaagatct ccaacgtgga gctctgcaac 25020
ctggtctcct accttggaat tttgcacgaa aaccgccttg ggcaaaacgt gcttcattcc 25080
acgctcaagg gcgaggcgcg ccgcgactac gtccgcgact gcgtttactt atttctatgc 25140
tacacctggc agacggccat gggcgtttgg cagcagtgct tggaggagtg caacctcaag 25200
gagctgcaga aactgctaaa gcaaaacttg aaggacctat ggacggcctt caacgagcgc 25260
tccgtggccg cgcacctggc ggacatcatt ttccccgaac gcctgcttaa aaccctgcaa 25320
cagggtctgc cagacttcac cagtcaaagc atgttgcaga actttaggaa ctttatccta 25380
gagcgctcag gaatcttgcc cgccacctgc tgtgcacttc ctagcgactt tgtgcccatt 25440
aagtaccgcg aatgccctcc gccgctttgg ggccactgct accttctgca gctagccaac 25500
taccttgcct accactctga cataatggaa gacgtgagcg gtgacggtct actggagtgt 25560
cactgtcgct gcaacctatg caccccgcac cgctccctgg tttgcaattc gcagctgctt 25620
aacgaaagtc aaattatcgg tacctttgag ctgcagggtc cctcgcctga cgaaaagtcc 25680
gcggctccgg ggttgaaact cactccgggg ctgtggacgt cggcttacct tcgcaaattt 25740
gtacctgagg actaccacgc ccacgagatt aggttctacg aagaccaatc ccgcccgcct 25800
aatgcggagc ttaccgcctg cgtcattacc cagggccaca ttcttggcca attgcaagcc 25860
atcaacaaag cccgccaaga gtttctgcta cgaaagggac gggggtttta cttggacccc 25920
cagtccggcg aggagctcaa cccaatcccc ccgccgccgc agccctatca gcagcagccg 25980
cgggcccttg cttcccagga tggcacccaa aaagaagctg cagctgccgc cgccacccac 26040
ggacgaggag gaatactggg acagtcaggc agaggaggtt ttggacgagg aggaggagga 26100
catgatggaa gactgggaga gcctagacga ggaagcttcc gaggtcgaag aggtgtcaga 26160
cgaaacaccg tcaccctcgg tcgcattccc ctcgccggcg ccccagaaat cggcaaccgg 26220
ttccagcatg gctacaacct ccgctcctca ggcgccgccg gcactgcccg ttcgccgacc 26280
caaccgtaga tgggacacca ctggaaccag ggccggtaag tccaagcagc cgccgccgtt 26340
agcccaagag caacaacagc gccaaggcta ccgctcatgg cgcgggcaca agaacgccat 26400
agttgcttgc ttgcaagact gtgggggcaa catctccttc gcccgccgct ttcttctcta 26460
ccatcacggc gtggccttcc cccgtaacat cctgcattac taccgtcatc tctacagccc 26520
atactgcacc ggcggcagcg gcagcaacag cagcggccac acagaagcaa aggcgaccgg 26580
atagcaagac tctgacaaag cccaagaaat ccacagcggc ggcagcagca ggaggaggag 26640
cgctgcgtct ggcgcccaac gaacccgtat cgacccgcga gcttagaaac aggatttttc 26700
ccactctgta tgctatattt caacagagca ggggccaaga acaagagctg aaaataaaaa 26760
acaggtctct gcgatccctc acccgcagct gcctgtatca caaaagcgaa gatcagcttc 26820
ggcgcacgct ggaagacgcg gaggctctct tcagtaaata ctgcgcgctg actcttaagg 26880
actagtttcg cgccctttct caaatttaag cgcgaaaact acgtcatctc cagcggccac 26940
acccggcgcc agcacctgtt gtcagcgcca ttatgagcaa ggaaattccc acgccctaca 27000
tgtggagtta ccagccacaa atgggacttg cggctggagc tgcccaagac tactcaaccc 27060

gaataaacta catgagcgcg ggaccccaca tgatatcccg ggtcaacgga atacgcgccc 27120
accgaaaccg aattctcctg gaacaggcgg ctattaccac cacacctcgt aataacctta 27180
atccccgtag ttggcccgct gccctggtgt accaggaaag tcccgctccc accactgtgg 27240
tacttcccag agacgcccag gccgaagttc agatgactaa ctcaggggcg cagcttgcgg 27300
gcggctttcg tcacagggtg cggtcgcccg ggcagggtat aactcacctg acaatcagag 27360
ggcgaggtat tcagctcaac gacgagtcgg tgagctcctc gcttggtctc cgtccggacg 27420
ggacatttca gatcggcggc gccggccgct cttcattcac gcctcgtcag gcaatcctaa 27480
ctctgcagac ctcgtcctct gagccgcgct ctggaggcat tggaactctg caatttattg 27540
aggagtttgt gccatcggtc tactttaacc ccttctcggg acctcccggc cactatccgg 27600
atcaatttat tcctaacttt gacgcggtaa aggactcggc ggacggctac gactgaatgt 27660
taagtggaga ggcagagcaa ctgcgcctga aacacctggt ccactgtcgc cgccacaagt 27720
gctttgcccg cgactccggt gagttttgct actttgaatt gcccgaggat catatcgagg 27780
gcccggcgca cggcgtccgg cttaccgccc agggagagct tgcccgtagc ctgattcggg 27840
agtttaccca gcgccccctg ctagttgagc gggacagggg accctgtgtt ctcactgtga 27900
tttgcaactg tcctaaccct ggattacatc aagatctttg ttgccatctc tgtgctgagt 27960
ataataaata cagaaattaa aatatactgg ggctcctatc gccatcctgt aaacgccacc 28020
gtcttcaccc gcccaagcaa accaaggcga accttacctg gtacttttaa catctctccc 28080
tctgtgattt acaacagttt caacccagac ggagtgagtc tacgagagaa cctctccgag 28140
ctcagctact ccatcagaaa aaacaccacc ctccttacct gccgggaacg tacgagtgcg 28200
tcaccggccg ctgcaccaca cctaccgcct gaccgtaaac cagacttttt ccggacagac 28260
ctcaataact ctgtttacca gaacaggagg tgagcttaga aaacccttag ggtattaggc 28320
caaaggcgca gctactgtgg ggtttatgaa caattcaagc aactctacgg gctattctaa 28380
ttcaggtttc tctagaaatg gacggaatta ttacagagca gcgcctgcta gaaagacgca 28440
gggcagcggc cgagcaacag cgcatgaatc aagagctcca agacatggtt aacttgcacc 28500
agtgcaaaag gggtatcttt tgtctggtaa agcaggccaa agtcacctac gacagtaata 28560
ccaccggaca ccgccttagc tacaagttgc caaccaagcg tcagaaattg gtggtcatgg 28620
tgggagaaaa gcccattacc ataactcagc actcggtaga aaccgaaggc tgcattcact 28680
caccttgtca aggacctgag gatctctgca cccttattaa gaccctgtgc ggtctcaaag 28740
atcttattcc ctttaactaa taaaaaaaaa taataaagca tcacttactt aaaatcagtt 28800
agcaaatttc tgtccagttt attcagcagc acctccttgc cctcctccca gctctggtat 28860
tgcagcttcc tcctggctgc aaactttctc cacaatctaa atggaatgtc agtttcctcc 28920
tgttcctgtc catccgcacc cactatcttc atgttgttgc agatgaagcg cgcaagaccg 28980
tctgaagata ccttcaaccc cgtgtatcca tatgacacgg aaaccggtcc tccaactgtg 29040
ccttttctta ctcctccctt tgtatccccc aatgggtttc aagagagtcc ccctggggta 29100
ctctctttgc gcctatccga acctctagtt acctccaatg gcatgcttgc gctcaaaatg 29160
ggcaacggcc tctctctgga cgaggccggc aaccttacct cccaaaatgt aaccactgtg 29220
agcccacctc tcaaaaaaac caagtcaaac ataaacctgg aaatatctgc acccctcaca 29280
gttacctcag aagccctaac tgtggctgcc gccgcacctc taatggtcgc gggcaacaca 29340

ctcaccatgc aatcacaggc cccgctaacc gtgcacgact ccaaacttag cattgccacc 29400
caaggacccc tcacagtgtc agaaggaaag ctagccctgc aaacatcagg ccccctcacc 29460
accaccgata gcagtaccct tactatcact gcctcacccc ctctaactac tgccactggt 29520
agcttgggca ttgacttgaa agagcccatt tatacacaaa atggaaaact aggactaaag 29580
tacggggctc ctttgcatgt aacagacgac ctaaacactt tgaccgtagc aactggtcca 29640
ggtgtgacta ttaataatac ttccttgcaa actaaagtta ctggagcctt gggttttgat 29700
tcacaaggca atatgcaact taatgtagca ggaggactaa ggattgattc tcaaaacaga 29760
cgccttatac ttgatgttag ttatccgttt gatgctcaaa accaactaaa tctaagacta 29820
ggacagggcc ctctttttat aaactcagcc cacaacttgg atattaacta caacaaaggc 29880
ctttacttgt ttacagcttc aaacaattcc aaaaagcttg aggttaacct aagcactgcc 29940
aaggggttga tgtttgacgc tacagccata gccattaatg caggagatgg gcttgaattt 30000
ggttcaccta atgcaccaaa cacaaatccc ctcaaaacaa aaattggcca tggcctagaa 30060
tttgattcaa acaaggctat ggttcctaaa ctaggaactg gccttagttt tgacagcaca 30120
ggtgccatta cagtaggaaa caaaaataat gataagctaa ctttgtggac cacaccagct 30180
ccatctccta actgtagact aaatgcagag aaagatgcta aactcacttt ggtcttaaca 30240
aaatgtggca gtcaaatact tgctacagtt tcagttttgg ctgttaaagg cagtttggct 30300
ccaatatctg gaacagttca aagtgctcat cttattataa gatttgacga aaatggagtg 30360
ctactaaaca attccttcct ggacccagaa tattggaact ttagaaatgg agatcttact 30420
gaaggcacag cctatacaaa cgctgttgga tttatgccta acctatcagc ttatccaaaa 30480
tctcacggta aaactgccaa aagtaacatt gtcagtcaag tttacttaaa cggagacaaa 30540
actaaacctg taacactaac cattacacta aacggtacac aggaaacagg agacacaact 30600
ccaagtgcat actctatgtc attttcatgg gactggtctg gccacaacta cattaatgaa 30660
atatttgcca catcctctta cactttttca tacattgccc aagaataaag aatcgtttgt 30720
gttatgtttc aacgtgttta tttttcaatt gcccgggatc ggtgatcacc gatccagaca 30780
tgataagata cattgatgag tttggacaaa ccacaactag aatgcagtga aaaaaatgct 30840
ttatttgtga aatttgtgat gctattgctt tatttgtaac cattataagc tgcaataaac 30900
aagttcccgg atcgcgatcc ggcccgaggc tgtagccgac gatggtgcgc caggagagtt 30960
gttgattcat tgtttgcctc cctgctgcgg tttttcaccg aagttcatgc cagtccagcg 31020
tttttgcagc agaaaagccg ccgacttcgg tttgcggtcg cgagtgaaga tccctttctt 31080
gttaccgcca acgcgcaata tgccttgcga ggtcgcaaaa tcggcgaaat tccatacctg 31140
ttcaccgacg acggcgctga cgcgatcaaa gacgcggtga tacatatcca gccatgcaca 31200
ctgatactct tcactccaca tgtcggtgta cattgagtgc agcccggcta acgtatccac 31260
gccgtattcg gtgatgataa tcggctgatg cagtttctcc tgccaggcca gaagttcttt 31320
ttccagtacc ttctctgccg tttccaaatc gccgctttgg acataccatc cgtaataacg 31380
gttcaggcac agcacatcaa agagatcgct gatggtatcg gtgtgagcgt cgcagaacat 31440
tacattgacg caggtgatcg gacgcgtcgg gtcgagttta cgcgttgctt ccgccagtgg 31500
cgcgaaatat tcccgtgcac cttgcggacg ggtatccggt tcgttggcaa tactccacat 31560
caccacgctt gggtggtttt tgtcacgcgc tatcagctct ttaatcgcct gtaagtgcgc 31620

ttgctgagtt tccccgttga ctgcctcttc gctgtacagt tctttcggct tgttgcccgc 31680

ttcgaaacca atgcctaaag agaggttaaa gccgacagca gcagtttcat caatcaccac 31740

gatgccatgt tcatctgccc agtcgagcat ctcttcagcg taagggtaat gcgaggtacg 31800

gtaggagttg gccccaatcc agtccattaa tgcgtggtcg tgcaccatca gcacgttatc 31860

gaatcctttg ccacgcaagt ccgcatcttc atgacgacca aagccagtaa agtagaacgg 31920

tttgtggtta atcaggaact gttcgccctt cactgccact gaccggatgc cgacgcgaag 31980

cgggtagata tcacactctg tctggctttt ggctgtgacg cacagttcat agagataacc 32040

ttcacccggt tgccagaggt gcggattcac cacttgcaaa gtcccgctag tgccttgtcc 32100

agttgcaacc acctgttgat ccgcatcacg cagttcaacg ctgacatcac cattggccac 32160

cacctgccag tcaacagacg cgtggttaca gtcttgcgcg acatgcgtca ccacggtgat 32220

atcgtccacc caggtgttcg gcgtggtgta gagcattacg ctgcgatgga ttccggcata 32280

gttaaagaaa tcatggaagt aagactgctt tttcttgccg ttttcgtcgg taatcaccat 32340

tcccggcggg atagtctgcc agttcagttc gttgttcaca caaacggtga tacgtacact 32400

tttcccggca ataacatacg gcgtgacatc ggcttcaaat ggcgtatagc cgccctgatg 32460

ctccatcact tcctgattat tgacccacac tttgccgtaa tgagtgaccg catcgaaacg 32520

cagcacgata cgctggcctg cccaaccttt cggtataaag acttcgcgct gataccagac 32580

gttgcccgca taattacgaa tatctgcatc ggcgaactga tcgttaaaac tgcctggcac 32640

agcaattgcc cggctttctt gtaacgcgct ttcccaccaa cgctgatcaa ttccacagtt 32700

ttcgcgatcc agactgaatg cccacaggcc gtcgagtttt ttgatttcac gggttggggt 32760

ttctacagga cggaccatgc gttcgacctt tctcttcttt tttgggccca tgatggcaga 32820

tccgtatagt gagtcgtatt agctggttct ttccgcctca gaagccatag agcccaccgc 32880

atccccagca tgcctgctat tgtcttccca atcctccccc ttgctgtcct gccccacccc 32940

accccccaga atagaatgac acctactcag acaatgcgat gcaatttcct cattttatta 33000

ggaaaggaca gtgggagtgg caccttccag ggtcaaggaa ggcacggggg aggggcaaac 33060

aacagatggc tggcaactag aaggcacagt cgaggctgat cagcgagctc tagatgcatg 33120

ctcgagcggc cgccagtgtg atggatatct gcagaattcc agcacactgg cggccgttac 33180

tagtggatcc gagctcggta cccggccgtt ataacaccac tcgacacggc accagctcaa 33240

tcagtcacag tgtaaaaaag ggccaagtgc agagcgagta tatataggac taaaaaatga 33300

cgtaacggtt aaagtccaca aaaaacaccc agaaaaccgc acgcgaacct acgcccagaa 33360

acgaaagcca aaaaacccac aacttcctca aatcgtcact tccgttttcc cacgttacgt 33420

cacttcccat tttaagaaaa ctacaattcc caacacatac aagttactcc gccctaaaac 33480

ctacgtcacc cgccccgttc ccacgccccg cgccacgtca caaactccac cccctcatta 33540

tcatattggc ttcaatccaa aataaggtat attattgatg atg 33583

<210> 6
<211> 33476
<212> DNA
<213> Artificial

<220>
<223> Synthetic

<400> 6

catcatcaat aatatacctt attttggatt gaagccaata tgataatgag ggggtggagt 60
ttgtgacgtg gcgcggggcg tgggaacggg gcgggtgacg tagtagtgtg gcggaagtgt 120
gatgttgcaa gtgtggcgga acacatgtaa gcgacggatg tggcaaaagt gacgtttttg 180
gtgtgcgccg gtgtacacag gaagtgacaa ttttcgcgcg gttttaggcg gatgttgtag 240
taaatttggg cgtaaccgag taagatttgg ccattttcgc gggaaaactg aataagagga 300
agtgaaatct gaataatttt gtgttactca tagcgcgtaa tatttgtcta gggccgggga 360
tcggtgatca ccgatccaga catgataaga tacattgatg agtttggaca aaccacaact 420
agaatgcagt gaaaaaaatg ctttatttgt gaaatttgtg atgctattgc tttatttgta 480
accattataa gctgcaataa acaagttccc ggatctttct agctagtcta gactagctag 540
actcgagagc ggccgcaatc gataagcttg atatcgaatt ctgcagtgat cagggatcct 600
caccacagcc agttggtgat gttgaaccag ttccacaggc tggcccactt gtccagctcc 660
agcagctcct gctcgttctt ctcgtgctgg ttctggctct cctcgatcag gctgtggatc 720
aggctggtgt agttgttgat ctcgcggtcc cactccatcc aggtggtgtg gttccagatc 780
tgctcgagca gctgctggtc cttcaggtag cgctccacgg ccagggtgcg ggcctgcagc 840
tgcttgatgc cccacacggt cagctgcagc aggtgctgct gggcctcgat ggcgcgcagc 900
aggttgttct gctgctgcac gatgccgctc agcagctggc gggcctggac ggtaagcttg 960
gccttggtgg gggccacgcc caggggctcg atcttcacca ccttgtactt gtacagctcg 1020
ctgcgccagt tgtcgcgcat gtcgccgccg cccaggcgga agatctcgct ctcgttgttg 1080
ctgttgccgc cgtcgcgggt cagcagcagg ccggtgatgt tgctgctgca gcggatctgg 1140
ccgctgatgg ggggggcgta catggccttg cccaccttct gccacatgtt gatgatctgc 1200
ttgatgcggc agggcagggt gatggtgtcg ctgccctcgg tgttgttgct gccctcggtg 1260
ctccaggtgc tgttgaacca ggtgctgttg aacagctggg tgctgttgca gtagaagaac 1320
tcgccgccgc agttgaagct gtgggtcacg atctcggggt cgccgccgct gctgtgcttg 1380
aacacgatgg tcttgttgcc gaactgctcg cgcagcttga tcacgatctt gttcagggtg 1440
tcgttccact tggcgcggct caggttgcag tgggcctggc ggatgtcgcc gatgatctcg 1500
ccggtggtgt agaaggcgcg gccggggccg atgtggatgc tcttgcgggt gttgttgttg 1560
gggcgggtgc agttgatctc cacgctctcg ttcagctgca cgatgatcac cttggcgttg 1620
tcggcgaagt tagcgctgcg gatcaccacc tcctcctcgg ccaggttacc cgtaaccagc 1680
agctgggtgc tcaccacggg gcggatgccg tgggtgcact gcacggtgct cacgttggtg 1740
caggggccct tgccgttgaa cttcttgtcc ttgcacttca ggatggcgaa gccggcgggg 1800
gcgcagtagt ggttggggat gggctcgaag ctcaccttgg ggcaggcctg ggtgatcacg 1860
ctggtgttgc agctggtgct agcgtcggtg cacttcaggc tcacgcacag gggggtcagc 1920
ttcacgcagg gcttcaggct ctggtcccac aggctgatga tgtcctcgtg catctgctcc 1980
accatgtcgt tcttccacat gtcgaagttc tcggtcacgt tcaccagcac cacctcctgg 2040
gggttggggt cggtgggcac gcaggcgtgg gtggcccaca cgttgtgcac ctcggtgtcg 2100
taggccttgg cgtcgctggc gcagagcagg gtggtggtgg cctccttcca cacgggcacg 2160
ccgtagtaca cggtcaccca cagcttctcg gtggcgctgc agatcatcag catgcccagc 2220
agcatggtgc cccagcgcca gccccagcgc cacaggtgct ggtacttctc cttcacgcgc 2280

atggtgtcta gagcggccgc gatcggctgc agttggacct gggagtggac acctgtggag 2340
agaaaggcaa agtggatgtc attgtcactc aagtgtatgg ccagatctca agcctgccac 2400
acctcaagtg aagccaaggg ggtgggccta tagactctat aggcggtact tacgtcactc 2460
ttggcacggg gaatccgcgt tccaatgcac cgttcccggc cgcggaggct ggatcggtcc 2520
cggtgtcttc tatggaggtc aaaacagcgt ggatggcgtc tccaggcgat ctgacggttc 2580
actaaacgag ctcgtcgacg atctctatca ctgataggga gatctctatc actgataggg 2640
agagctctgc ttatatagac ctcccaccgt acacgcctac cgcccatttg cgtcaatggg 2700
gcggagttgt tacgacattt tggaaagtcc cgttgatttt ggtgccaaaa caaactccca 2760
ttgacgtcaa tggggtggag acttggaaat ccccgtgagt caaaccgcta tccacgccca 2820
ttgatgtact gccaaaaccg catcaccatg gtaatagcga tgactaatac gtagatgtac 2880
tgccaagtag gaaagtccca taaggtcatg tactgggcat aatgccaggc gggccattta 2940
ccgtcattga cgtcaatagg gggcgtactt ggcatatgat acacttgatg tactgccaag 3000
tgggcagttt accgtaaata ctccacccat tgacgtcaat ggaaagtccc tattggcgtt 3060
actatgggaa catacgtcat tattgacgtc aatgggcggg ggtcgttggg cggtcagcca 3120
ggcgggccat ttaccgtaag ttatgtaacg cggaactcca tatatgggct atgaactaat 3180
gaccccgtaa ttgattacta ttaataacta gtactgaaat gtgtgggcgt ggcttaaggg 3240
tgggaaagaa tatataaggt gggggtctta tgtagttttg tatctgtttt gcagcagccg 3300
ccgccgccat gagcaccaac tcgtttgatg gaagcattgt gagctcatat ttgacaacgc 3360
gcatgccccc atgggccggg gtgcgtcaga atgtgatggg ctccagcatt gatggtcgcc 3420
ccgtcctgcc cgcaaactct actaccttga cctacgagac cgtgtctgga acgccgttgg 3480
agactgcagc ctccgccgcc gcttcagccg ctgcagccac cgcccgcggg attgtgactg 3540
actttgcttt cctgagcccg cttgcaagca gtgcagcttc ccgttcatcc gcccgcgatg 3600
acaagttgac ggctcttttg gcacaattgg attctttgac ccgggaactt aatgtcgttt 3660
ctcagcagct gttggatctg cgccagcagg tttctgccct gaaggcttcc tcccctccca 3720
atgcggttta aaacataaat aaaaaaccag actctgtttg gatttggatc aagcaagtgt 3780
cttgctgtct ttatttaggg gttttgcgcg cgcggtaggc ccgggaccag cggtctcggt 3840
cgttgagggt cctgtgtatt ttttccagga cgtggtaaag gtgactctgg atgttcagat 3900
acatgggcat aagcccgtct ctggggtgga ggtagcacca ctgcagagct tcatgctgcg 3960
gggtggtgtt gtagatgatc cagtcgtagc aggagcgctg ggcgtggtgc ctaaaaatgt 4020
ctttcagtag caagctgatt gccaggggca ggcccttggt gtaagtgttt acaaagcggt 4080
taagctggga tgggtgcata cgtggggata tgagatgcat cttggactgt atttttaggt 4140
tggctatgtt cccagccata tccctccggg gattcatgtt gtgcagaacc accagcacag 4200
tgtatccggt gcacttggga aatttgtcat gtagcttaga aggaaatgcg tggaagaact 4260
tggagacgcc cttgtgacct ccaagatttt ccatgcattc gtccataatg atggcaatgg 4320
gcccacgggc ggcggcctgg gcgaagatat ttctgggatc actaacgtca tagttgtgtt 4380
ccaggatgag atcgtcatag gccattttta caaagcgcgg gcggagggtg ccagactgcg 4440
gtataatggt tccatccggc ccaggggcgt agttaccctc acagatttgc atttcccacg 4500
ctttgagttc agatgggggg atcatgtcta cctgcggggc gatgaagaaa acggtttccg 4560

gggtagggga gatcagctgg gaagaaagca ggttcctgag cagctgcgac ttaccgcagc 4620
cggtgggccc gtaaatcaca cctattaccg gctgcaactg gtagttaaga gagctgcagc 4680
tgccgtcatc cctgagcagg ggggccactt cgttaagcat gtccctgact cgcatgtttt 4740
ccctgaccaa atccgccaga aggcgctcgc cgcccagcga tagcagttct tgcaaggaag 4800
caaagttttt caacggtttg agaccgtccg ccgtaggcat gcttttgagc gtttgaccaa 4860
gcagttccag gcggtcccac agctcggtca cctgctctac ggcatctcga tccagcatat 4920
ctcctcgttt cgcgggttgg ggcggctttc gctgtacggc agtagtcggt gctcgtccag 4980
acgggccagg gtcatgtctt tccacgggcg cagggtcctc gtcagcgtag tctgggtcac 5040
ggtgaagggg tgcgctccgg gctgcgcgct ggccagggtg cgcttgaggc tggtcctgct 5100
ggtgctgaag cgctgccggt cttcgccctg cgcgtcggcc aggtagcatt tgaccatggt 5160
gtcatagtcc agcccctccg cggcgtggcc cttggcgcgc agcttgccct tggaggaggc 5220
gccgcacgag gggcagtgca gacttttgag ggcgtagagc ttgggcgcga gaaataccga 5280
ttccggggag taggcatccg cgccgcaggc cccgcagacg gtctcgcatt ccacgagcca 5340
ggtgagctct ggccgttcgg ggtcaaaaac caggtttccc ccatgctttt tgatgcgttt 5400
cttacctctg gtttccatga gccggtgtcc acgctcggtg acgaaaaggc tgtccgtgtc 5460
cccgtataca gacttgagag gcctgtcctc gagcggtgtt ccgcggtcct cctcgtatag 5520
aaactcggac cactctgaga caaaggctcg cgtccaggcc agcacgaagg aggctaagtg 5580
ggaggggtag cggtcgttgt ccactagggg gtccactcgc tccagggtgt gaagacacat 5640
gtcgccctct tcggcatcaa ggaaggtgat tggtttgtag gtgtaggcca cgtgaccggg 5700
tgttcctgaa ggggggctat aaaagggggt gggggcgcgt tcgtcctcac tctcttccgc 5760
atcgctgtct gcgagggcca gctgttgggg tgagtactcc ctctgaaaag cgggcatgac 5820
ttctgcgcta agattgtcag tttccaaaaa cgaggaggat ttgatattca cctggcccgc 5880
ggtgatgcct ttgagggtgg ccgcatccat ctggtcagaa aagacaatct ttttgttgtc 5940
aagcttggtg gcaaacgacc cgtagagggc gttggacagc aacttggcga tggagcgcag 6000
ggtttggttt ttgtcgcgat cggcgcgctc cttggccgcg atgtttagct gcacgtattc 6060
gcgcgcaacg caccgccatt cgggaaagac ggtggtgcgc tcgtcgggca ccaggtgcac 6120
gcgccaaccg cggttgtgca gggtgacaag gtcaacgctg gtggctacct ctccgcgtag 6180
gcgctcgttg gtccagcaga ggcggccgcc cttgcgcgag cagaatggcg gtagggggtc 6240
tagctgcgtc tcgtccgggg ggtctgcgtc cacggtaaag accccgggca gcaggcgcgc 6300
gtcgaagtag tctatcttgc atccttgcaa gtctagcgcc tgctgccatg cgcgggcggc 6360
aagcgcgcgc tcgtatgggt tgagtggggg accccatggc atggggtggg tgagcgcgga 6420
ggcgtacatg ccgcaaatgt cgtaaacgta gaggggctct ctgagtattc caagatatgt 6480
agggtagcat cttccaccgc ggatgctggc gcgcacgtaa tcgtatagtt cgtgcgaggg 6540
agcgaggagg tcgggaccga ggttgctacg ggcgggctgc tctgctcgga agactatctg 6600
cctgaagatg gcatgtgagt tggatgatat ggttggacgc tggaagacgt tgaagctggc 6660
gtctgtgaga cctaccgcgt cacgcacgaa ggaggcgtag gagtcgcgca gcttgttgac 6720
cagctcggcg gtgacctgca cgtctagggc gcagtagtcc agggtttcct tgatgatgtc 6780
atacttatcc tgtccctttt ttttccacag ctcgcggttg aggacaaact cttcgcggtc 6840

tttccagtac tcttggatcg gaaacccgtc ggcctccgaa cggtaagagc ctagcatgta 6900
gaactggttg acggcctggt aggcgcagca tcccttttct acgggtagcg cgtatgcctg 6960
cgcggccttc cggagcgagg tgtgggtgag cgcaaaggtg tccctgacca tgactttgag 7020
gtactggtat ttgaagtcag tgtcgtcgca tccgccctgc tcccagagca aaaagtccgt 7080
gcgctttttg gaacgcggat ttggcagggc gaaggtgaca tcgttgaaga gtatctttcc 7140
cgcgcgaggc ataaagttgc gtgtgatgcg gaagggtccc ggcacctcgg aacggttgtt 7200
aattacctgg gcggcgagca cgatctcgtc aaagccgttg atgttgtggc ccacaatgta 7260
aagttccaag aagcgcggga tgcccttgat ggaaggcaat tttttaagtt cctcgtaggt 7320
gagctcttca ggggagctga gcccgtgctc tgaaagggcc cagtctgcaa gatgagggtt 7380
ggaagcgacg aatgagctcc acaggtcacg ggccattagc atttgcaggt ggtcgcgaaa 7440
ggtcctaaac tggcgaccta tggccatttt ttctggggtg atgcagtaga aggtaagcgg 7500
gtcttgttcc cagcggtccc atccaaggtt cgcggctagg tctcgcgcgg cagtcactag 7560
aggctcatct ccgccgaact tcatgaccag catgaagggc acgagctgct tcccaaaggc 7620
ccccatccaa gtataggtct ctacatcgta ggtgacaaag agacgctcgg tgcgaggatg 7680
cgagccgatc gggaagaact ggatctcccg ccaccaattg gaggagtggc tattgatgtg 7740
gtgaaagtag aagtccctgc gacgggccga acactcgtgc tggcttttgt aaaaacgtgc 7800
gcagtactgg cagcggtgca cgggctgtac atcctgcacg aggttgacct gacgaccgcg 7860
cacaaggaag cagagtggga atttgagccc ctcgcctggc gggtttggct ggtggtcttc 7920
tacttcggct gcttgtcctt gaccgtctgg ctgctcgagg ggagttacgg tggatcggac 7980
caccacgccg cgcgagccca aagtccagat gtccgcgcgc ggcggtcgga gcttgatgac 8040
aacatcgcgc agatgggagc tgtccatggt ctggagctcc cgcggcgtca ggtcaggcgg 8100
gagctcctgc aggtttacct cgcatagacg ggtcagggcg cgggctagat ccaggtgata 8160
cctaatttcc aggggctggt tggtggcggc gtcgatggct tgcaagaggc cgcatccccg 8220
cggcgcgact acggtaccgc gcggcgggcg gtgggccgcg ggggtgtcct tggatgatgc 8280
atctaaaagc ggtgacgcgg gcgagccccc ggaggtaggg ggggctccgg acccgccggg 8340
agagggggca ggggcacgtc ggcgccgcgc gcgggcagga gctggtgctg cgcgcgtagg 8400
ttgctggcga acgcgacgac gcggcggttg atctcctgaa tctggcgcct ctgcgtgaag 8460
acgacgggcc cggtgagctt gaacctgaaa gagagttcga cagaatcaat ttcggtgtcg 8520
ttgacggcgg cctggcgcaa aatctcctgc acgtctcctg agttgtcttg ataggcgatc 8580
tcggccatga actgctcgat ctcttcctcc tggagatctc cgcgtccggc tcgctccacg 8640
gtggcggcga ggtcgttgga aatgcgggcc atgagctgcg agaaggcgtt gaggcctccc 8700
tcgttccaga cgcggctgta gaccacgccc ccttcggcat cgcgggcgcg catgaccacc 8760
tgcgcgagat tgagctccac gtgccgggcg aagacggcgt agtttcgcag gcgctgaaag 8820
aggtagttga gggtggtggc ggtgtgttct gccacgaaga agtacataac ccagcgtcgc 8880
aacgtggatt cgttgatatc ccccaaggcc tcaaggcgct ccatggcctc gtagaagtcc 8940
acggcgaagt tgaaaaactg ggagttgcgc gccgacacgg ttaactcctc ctccagaaga 9000
cggatgagct cggcgacagt gtcgcgcacc tcgcgctcaa aggctacagg ggcctcttct 9060
tcttcttcaa tctcctcttc cataagggcc tccccttctt cttcttctgg cggcggtggg 9120

ggagggggga cacggcggcg acgacggcgc accgggaggc ggtcgacaaa gcgctcgatc 9180
atctccccgc ggcgacggcg catggtctcg gtgacggcgc ggccgttctc gcgggggcgc 9240
agttggaaga cgccgcccgt catgtcccgg ttatgggttg gcggggggct gccatgcggc 9300
agggatacgg cgctaacgat gcatctcaac aattgttgtg taggtactcc gccgccgagg 9360
gacctgagcg agtccgcatc gaccggatcg gaaaacctct cgagaaaggc gtctaaccag 9420
tcacagtcgc aaggtaggct gagcaccgtg gcgggcggca gcgggcggcg gtcggggttg 9480
tttctggcgg aggtgctgct gatgatgtaa ttaaagtagg cggtcttgag acggcggatg 9540
gtcgacagaa gcaccatgtc cttgggtccg gcctgctgaa tgcgcaggcg gtcggccatg 9600
ccccaggctt cgttttgaca tcggcgcagg tctttgtagt agtcttgcat gagcctttct 9660
accggcactt cttcttctcc ttcctcttgt cctgcatctc ttgcatctat cgctgcggcg 9720
gcggcggagt ttggccgtag gtggcgccct cttcctccca tgcgtgtgac cccgaagccc 9780
ctcatcggct gaagcagggc taggtcggcg acaacgcgct cggctaatat ggcctgctgc 9840
acctgcgtga gggtagactg gaagtcatcc atgtccacaa agcggtggta tgcgcccgtg 9900
ttgatggtgt aagtgcagtt ggccataacg gaccagttaa cggtctggtg acccggctgc 9960
gagagctcgg tgtacctgag acgcgagtaa gccctcgagt caaatacgta gtcgttgcaa 10020
gtccgcacca ggtactggta tcccaccaaa aagtgcggcg gcggctggcg gtagaggggc 10080
cagcgtaggg tggccggggc tccgggggcg agatcttcca acataaggcg atgatatccg 10140
tagatgtacc tggacatcca ggtgatgccg gcggcggtgg tggaggcgcg cggaaagtcg 10200
cggacgcggt tccagatgtt gcgcagcggc aaaaagtgct ccatggtcgg gacgctctgg 10260
ccggtcaggc gcgcgcaatc gttgacgctc tagcgtgcaa aaggagagcc tgtaagcggg 10320
cactcttccg tggtctggtg gataaattcg caagggtatc atggcggacg accggggttc 10380
gagccccgta tccggccgtc cgccgtgatc catgcggtta ccgcccgcgt gtcgaaccca 10440
ggtgtgcgac gtcagacaac gggggagtgc tccttttggc ttccttccag gcgcggcggc 10500
tgctgcgcta gcttttttgg ccactggccg cgcgcagcgt aagcggttag gctggaaagc 10560
gaaagcatta agtggctcgc tccctgtagc cggagggtta ttttccaagg gttgagtcgc 10620
gggacccccg gttcgagtct cggaccggcc ggactgcggc gaacgggggt ttgcctcccc 10680
gtcatgcaag accccgcttg caaattcctc cggaaacagg gacgagcccc ttttttgctt 10740
ttcccagatg catccggtgc tgcggcagat gcgcccccct cctcagcagc ggcaagagca 10800
agagcagcgg cagacatgca gggcaccctc ccctcctcct accgcgtcag gaggggcgac 10860
atccgcggtt gacgcggcag cagatggtga ttacgaaccc ccgcggcgcc gggcccggca 10920
ctacctggac ttggaggagg gcgagggcct ggcgcggcta ggagcgccct ctcctgagcg 10980
gcacccaagg gtgcagctga agcgtgatac gcgtgaggcg tacgtgccgc ggcagaacct 11040
gtttcgcgac cgcgagggag aggagcccga ggagatgcgg gatcgaaagt tccacgcagg 11100
gcgcgagctg cggcatggcc tgaatcgcga gcggttgctg cgcgaggagg actttgagcc 11160
cgacgcgcga accgggatta gtcccgcgcg cgcacacgtg gcggccgccg acctggtaac 11220
cgcatacgag cagacggtga accaggagat taactttcaa aaaagcttta acaaccacgt 11280
gcgtacgctt gtggcgcgcg aggaggtggc tataggactg atgcatctgt gggactttgt 11340
aagcgcgctg gagcaaaacc caaatagcaa gccgctcatg gcgcagctgt tccttatagt 11400

gcagcacagc agggacaacg aggcattcag ggatgcgctg ctaaacatag tagagcccga 11460
gggccgctgg ctgctcgatt tgataaacat cctgcagagc atagtggtgc aggagcgcag 11520
cttgagcctg gctgacaagg tggccgccat caactattcc atgcttagcc tgggcaagtt 11580
ttacgcccgc aagatatacc atacccctta cgttcccata gacaaggagg taaagatcga 11640
ggggttctac atgcgcatgg cgctgaaggt gcttaccttg agcgacgacc tgggcgttta 11700
tcgcaacgag cgcatccaca aggccgtgag cgtgagccgg cggcgcgagc tcagcgaccg 11760
cgagctgatg cacagcctgc aaagggccct ggctggcacg ggcagcggcg atagagaggc 11820
cgagtcctac tttgacgcgg gcgctgacct gcgctgggcc ccaagccgac gcgccctgga 11880
ggcagctggg gccggacctg ggctggcggt ggcacccgcg cgcgctggca acgtcggcgg 11940
cgtggaggaa tatgacgagg acgatgagta cgagccagag gacggcgagt actaagcggt 12000
gatgtttctg atcagatgat gcaagacgca acggacccgg cggtgcgggc ggcgctgcag 12060
agccagccgt ccggccttaa ctccacggac gactggcgcc aggtcatgga ccgcatcatg 12120
tcgctgactg cgcgcaatcc tgacgcgttc cggcagcagc cgcaggccaa ccggctctcc 12180
gcaattctgg aagcggtggt cccggcgcgc gcaaacccca cgcacgagaa ggtgctggcg 12240
atcgtaaacg cgctggccga aaacagggcc atccggcccg acgaggccgg cctggtctac 12300
gacgcgctgc ttcagcgcgt ggctcgttac aacagcggca acgtgcagac caacctggac 12360
cggctggtgg gggatgtgcg cgaggccgtg gcgcagcgtg agcgcgcgca gcagcagggc 12420
aacctgggct ccatggttgc actaaacgcc ttcctgagta cacagcccgc caacgtgccg 12480
cggggacagg aggactacac caactttgtg agcgcactgc ggctaatggt gactgagaca 12540
ccgcaaagtg aggtgtacca gtctgggcca gactattttt tccagaccag tagacaaggc 12600
ctgcagaccg taaacctgag ccaggctttc aaaaacttgc aggggctgtg gggggtgcgg 12660
gctcccacag gcgaccgcgc gaccgtgtct agcttgctga cgcccaactc gcgcctgttg 12720
ctgctgctaa tagcgccctt cacggacagt ggcagcgtgt cccgggacac atacctaggt 12780
cacttgctga cactgtaccg cgaggccata ggtcaggcgc atgtggacga gcatactttc 12840
caggagatta caagtgtcag ccgcgcgctg gggcaggagg acacgggcag cctggaggca 12900
accctaaact acctgctgac caaccggcgg cagaagatcc cctcgttgca cagtttaaac 12960
agcgaggagg agcgcatttt gcgctacgtg cagcagagcg tgagccttaa cctgatgcgc 13020
gacggggtaa cgcccagcgt ggcgctggac atgaccgcgc gcaacatgga accgggcatg 13080
tatgcctcaa accggccgtt tatcaaccgc ctaatggact acttgcatcg cgcggccgcc 13140
gtgaaccccg agtatttcac caatgccatc ttgaacccgc actggctacc gccccctggt 13200
ttctacaccg ggggattcga ggtgcccgag ggtaacgatg gattcctctg ggacgacata 13260
gacgacagcg tgttttcccc gcaaccgcag accctgctag agttgcaaca gcgcgagcag 13320
gcagaggcgg cgctgcgaaa ggaaagcttc cgcaggccaa gcagcttgtc cgatctaggc 13380
gctgcggccc cgcggtcaga tgctagtagc ccatttccaa gcttgatagg gtctcttacc 13440
agcactcgca ccacccgccc gcgcctgctg ggcgaggagg agtacctaaa caactcgctg 13500
ctgcagccgc agcgcgaaaa aaacctgcct ccggcatttc ccaacaacgg gatagagagc 13560
ctagtggaca agatgagtag atggaagacg tacgcgcagg agcacaggga cgtgccaggc 13620
ccgcgcccgc ccacccgtcg tcaaaggcac gaccgtcagc ggggtctggt gtgggaggac 13680

gatgactcgg cagacgacag cagcgtcctg gatttgggag ggagtggcaa cccgtttgcg 13740
caccttcgcc ccaggctggg gagaatgttt taaaaaaaaa aaaagcatga tgcaaaataa 13800
aaaactcacc aaggccatgg caccgagcgt tggttttctt gtattcccct tagtatgcgg 13860
cgcgcggcga tgtatgagga aggtcctcct ccctcctacg agagtgtggt gagcgcggcg 13920
ccagtggcgg cggcgctggg ttctcccttc gatgctcccc tggacccgcc gtttgtgcct 13980
ccgcggtacc tgcggcctac cggggggaga aacagcatcc gttactctga gttggcaccc 14040
ctattcgaca ccacccgtgt gtacctggtg gacaacaagt caacggatgt ggcatccctg 14100
aactaccaga acgaccacag caactttctg accacggtca ttcaaaacaa tgactacagc 14160
ccgggggagg caagcacaca gaccatcaat cttgacgacc ggtcgcactg gggcggcgac 14220
ctgaaaacca tcctgcatac caacatgcca aatgtgaacg agttcatgtt taccaataag 14280
tttaaggcgc gggtgatggt gtcgcgcttg cctactaagg acaatcaggt ggagctgaaa 14340
tacgagtggg tggagttcac gctgcccgag ggcaactact ccgagaccat gaccatagac 14400
cttatgaaca acgcgatcgt ggagcactac ttgaaagtgg gcagacagaa cggggttctg 14460
gaaagcgaca tcggggtaaa gtttgacacc cgcaacttca gactggggtt tgaccccgtc 14520
actggtcttg tcatgcctgg ggtatataca aacgaagcct tccatccaga catcattttg 14580
ctgccaggat gcggggtgga cttcacccac agccgcctga gcaacttgtt gggcatccgc 14640
aagcggcaac ccttccagga gggctttagg atcacctacg atgatctgga gggtggtaac 14700
attcccgcac tgttggatgt ggacgcctac caggcgagct tgaaagatga caccgaacag 14760
ggcgggggtg gcgcaggcgg cagcaacagc agtggcagcg gcgcggaaga gaactccaac 14820
gcggcagccg cggcaatgca gccggtggag gacatgaacg atcatgccat tcgcggcgac 14880
acctttgcca cacgggctga ggagaagcgc gctgaggccg aagcagcggc cgaagctgcc 14940
gcccccgctg cgcaacccga ggtcgagaag cctcagaaga aaccggtgat caaacccctg 15000
acagaggaca gcaagaaacg cagttacaac ctaataagca atgacagcac cttcacccag 15060
taccgcagct ggtaccttgc atacaactac ggcgaccctc agaccggaat ccgctcatgg 15120
accctgcttt gcactcctga cgtaacctgc ggctcggagc aggtctactg gtcgttgcca 15180
gacatgatgc aagaccccgt gaccttccgc tccacgcgcc agatcagcaa ctttccggtg 15240
gtgggcgccg agctgttgcc cgtgcactcc aagagcttct acaacgacca ggccgtctac 15300
tcccaactca tccgccagtt tacctctctg acccacgtgt tcaatcgctt tcccgagaac 15360
cagattttgg cgcgcccgcc agcccccacc atcaccaccg tcagtgaaaa cgttcctgct 15420
ctcacagatc acgggacgct accgctgcgc aacagcatcg gaggagtcca gcgagtgacc 15480
attactgacg ccagacgccg cacctgcccc tacgtttaca aggccctggg catagtctcg 15540
ccgcgcgtcc tatcgagccg cactttttga gcaagcatgt ccatccttat atcgcccagc 15600
aataacacag gctggggcct gcgcttccca agcaagatgt ttggcggggc caagaagcgc 15660
tccgaccaac acccagtgcg cgtgcgcggg cactaccgcg cgccctgggg cgcgcacaaa 15720
cgcggccgca ctgggcgcac caccgtcgat gacgccatcg acgcggtggt ggaggaggcg 15780
cgcaactaca cgcccacgcc gccaccagtg tccacagtgg acgcggccat tcagaccgtg 15840
gtgcgcggag cccggcgcta tgctaaaatg aagagacggc ggaggcgcgt agcacgtcgc 15900
caccgccgcc gacccggcac tgccgcccaa cgcgcggcgg cggccctgct taaccgcgca 15960

cgtcgcaccg gccgacgggc ggccatgcgg gccgctcgaa ggctggccgc gggtattgtc 16020
actgtgcccc ccaggtccag gcgacgagcg gccgccgcag cagccgcggc cattagtgct 16080
atgactcagg gtcgcagggg caacgtgtat tgggtgcgcg actcggttag cggcctgcgc 16140
gtgcccgtgc gcacccgccc cccgcgcaac tagattgcaa gaaaaaacta cttagactcg 16200
tactgttgta tgtatccagc ggcggcggcg cgcaacgaag ctatgtccaa gcgcaaaatc 16260
aaagaagaga tgctccaggt catcgcgccg gagatctatg gccccccgaa gaaggaagag 16320
caggattaca agccccgaaa gctaaagcgg gtcaaaaaga aaaagaaaga tgatgatgat 16380
gaacttgacg acgaggtgga actgctgcac gctaccgcgc ccaggcgacg ggtacagtgg 16440
aaaggtcgac gcgtaaaacg tgttttgcga cccggcacca ccgtagtctt tacgcccggt 16500
gagcgctcca cccgcaccta caagcgcgtg tatgatgagg tgtacggcga cgaggacctg 16560
cttgagcagg ccaacgagcg cctcggggag tttgcctacg gaaagcggca taaggacatg 16620
ctggcgttgc cgctggacga gggcaaccca acacctagcc taaagcccgt aacactgcag 16680
caggtgctgc ccgcgcttgc accgtccgaa gaaaagcgcg gcctaaagcg cgagtctggt 16740
gacttggcac ccaccgtgca gctgatggta cccaagcgcc agcgactgga agatgtcttg 16800
gaaaaaatga ccgtggaacc tgggctggag cccgaggtcc gcgtgcggcc aatcaagcag 16860
gtggcgccgg gactgggcgt gcagaccgtg gacgttcaga tacccactac cagtagcacc 16920
agtattgcca ccgccacaga gggcatggag acacaaacgt ccccggttgc ctcagcggtg 16980
gcggatgccg cggtgcaggc ggtcgctgcg gccgcgtcca agacctctac ggaggtgcaa 17040
acggacccgt ggatgtttcg cgtttcagcc ccccggcgcc cgcgccgttc gaggaagtac 17100
ggcgccgcca gcgcgctact gcccgaatat gccctacatc cttccattgc gcctaccccc 17160
ggctatcgtg gctacaccta ccgccccaga agacgagcaa ctacccgacg ccgaaccacc 17220
actggaaccc gccgccgccg tcgccgtcgc cagcccgtgc tggccccgat ttccgtgcgc 17280
agggtggctc gcgaaggagg caggaccctg gtgctgccaa cagcgcgcta ccaccccagc 17340
atcgtttaaa agccggtctt tgtggttctt gcagatatgg ccctcacctg ccgcctccgt 17400
ttcccggtgc cgggattccg aggaagaatg caccgtagga ggggcatggc cggccacggc 17460
ctgacgggcg gcatgcgtcg tgcgcaccac cggcggcggc gcgcgtcgca ccgtcgcatg 17520
cgcggcggta tcctgcccct ccttattcca ctgatcgccg cggcgattgg cgccgtgccc 17580
ggaattgcat ccgtggcctt gcaggcgcag agacactgat taaaaacaag ttgcatgtgg 17640
aaaaatcaaa ataaaaagtc tggactctca cgctcgcttg gtcctgtaac tattttgtag 17700
aatggaagac atcaactttg cgtctctggc cccgcgacac ggctcgcgcc cgttcatggg 17760
aaactggcaa gatatcggca ccagcaatat gagcggtggc gccttcagct ggggctcgct 17820
gtggagcggc attaaaaatt tcggttccac cgttaagaac tatggcagca aggcctggaa 17880
cagcagcaca ggccagatgc tgagggataa gttgaaagag caaaatttcc aacaaaaggt 17940
ggtagatggc ctggcctctg gcattagcgg ggtggtggac ctggccaacc aggcagtgca 18000
aaataagatt aacagtaagc ttgatccccg ccctcccgta gaggagcctc caccggccgt 18060
ggagacagtg tctccagagg ggcgtggcga aaagcgtccg cgccccgaca gggaagaaac 18120
tctggtgacg caaatagacg agcctccctc gtacgaggag gcactaaagc aaggcctgcc 18180
caccacccgt cccatcgcgc ccatggctac cggagtgctg ggccagcaca cacccgtaac 18240

gctggacctg cctccccccg ccgacaccca gcagaaacct gtgctgccag gcccgaccgc 18300
cgttgttgta acccgtccta gccgcgcgtc cctgcgccgc gccgccagcg gtccgcgatc 18360
gttgcggccc gtagccagtg gcaactggca aagcacactg aacagcatcg tgggtctggg 18420
ggtgcaatcc ctgaagcgcc gacgatgctt ctgatagcta acgtgtcgta tgtgtgtcat 18480
gtatgcgtcc atgtcgccgc cagaggagct gctgagccgc cgcgcgcccg ctttccaaga 18540
tggctacccc ttcgatgatg ccgcagtggt cttacatgca catctcgggc caggacgcct 18600
cggagtacct gagccccggg ctggtgcagt ttgcccgcgc caccgagacg tacttcagcc 18660
tgaataacaa gtttagaaac cccacggtgg cgcctacgca cgacgtgacc acagaccggt 18720
cccagcgttt gacgctgcgg ttcatccctg tggaccgtga ggatactgcg tactcgtaca 18780
aggcgcggtt caccctagct gtgggtgata accgtgtgct ggacatggct tccacgtact 18840
ttgacatccg cggcgtgctg gacaggggcc ctacttttaa gccctactct ggcactgcct 18900
acaacgccct ggctcccaag ggtgccccaa atccttgcga atgggatgaa gctgctactg 18960
ctcttgaaat aaacctagaa gaagaggacg atgacaacga agacgaagta gacgagcaag 19020
ctgagcagca aaaaactcac gtatttgggc aggcgcctta ttctggtata aatattacaa 19080
aggagggtat tcaaataggt gtcgaaggtc aaacacctaa atatgccgat aaaacatttc 19140
aacctgaacc tcaaatagga gaatctcagt ggtacgaaac agaaattaat catgcagctg 19200
ggagagtcct aaaaaagact accccaatga aaccatgtta cggttcatat gcaaaaccca 19260
caaatgaaaa tggagggcaa ggcattcttg taaagcaaca aaatggaaag ctagaaagtc 19320
aagtggaaat gcaatttttc tcaactactg aggcagccgc aggcaatggt gataacttga 19380
ctcctaaagt ggtattgtac agtgaagatg tagatataga aaccccagac actcatattt 19440
cttacatgcc cactattaag gaaggtaact cacgagaact aatgggccaa caatctatgc 19500
ccaacaggcc taattacatt gcttttaggg acaattttat tggtctaatg tattacaaca 19560
gcacgggtaa tatgggtgtt ctggcgggcc aagcatcgca gttgaatgct gttgtagatt 19620
tgcaagacag aaacacagag ctttcatacc agcttttgct tgattccatt ggtgatagaa 19680
ccaggtactt ttctatgtgg aatcaggctg ttgacagcta tgatccagat gttagaatta 19740
ttgaaaatca tggaactgaa gatgaacttc caaattactg ctttccactg ggaggtgtga 19800
ttaatacaga gactcttacc aaggtaaaac ctaaaacagg tcaggaaaat ggatgggaaa 19860
aagatgctac agaattttca gataaaaatg aaataagagt tggaaataat tttgccatgg 19920
aaatcaatct aaatgccaac ctgtggagaa atttcctgta ctccaacata gcgctgtatt 19980
tgcccgacaa gctaaagtac agtccttcca acgtaaaaat ttctgataac ccaaacacct 20040
acgactacat gaacaagcga gtggtggctc ccgggctagt ggactgctac attaaccttg 20100
gagcacgctg gtcccttgac tatatggaca acgtcaaccc atttaaccac caccgcaatg 20160
ctggcctgcg ctaccgctca atgttgctgg gcaatggtcg ctatgtgccc ttccacatcc 20220
aggtgcctca gaagttcttt gccattaaaa acctccttct cctgccgggc tcatacacct 20280
acgagtggaa cttcaggaag gatgttaaca tggttctgca gagctcccta ggaaatgacc 20340
taagggttga cggagccagc attaagtttg atagcatttg cctttacgcc accttcttcc 20400
ccatggccca caacaccgcc tccacgcttg aggccatgct tagaaacgac accaacgacc 20460
agtcctttaa cgactatctc tccgccgcca acatgctcta ccctataccc gccaacgcta 20520

ccaacgtgcc catatccatc ccctcccgca actgggcggc tttccgcggc tgggccttca 20580
cgcgccttaa gactaaggaa accccatcac tgggctcggg ctacgaccct tattacacct 20640
actctggctc tataccctac ctagatggaa ccttttacct caaccacacc tttaagaagg 20700
tggccattac ctttgactct tctgtcagct ggcctggcaa tgaccgcctg cttaccccca 20760
acgagtttga aattaagcgc tcagttgacg gggagggtta caacgttgcc cagtgtaaca 20820
tgaccaaaga ctggttcctg gtacaaatgc tagctaacta taacattggc taccagggct 20880
tctatatccc agagagctac aaggaccgca tgtactcctt ctttagaaac ttccagccca 20940
tgagccgtca ggtggtggat gatactaaat acaaggacta ccaacaggtg ggcatcctac 21000
accaacacaa caactctgga tttgttggct accttgcccc caccatgcgc gaaggacagg 21060
cctaccctgc taacttcccc tatccgctta taggcaagac cgcagttgac agcattaccc 21120
agaaaaagtt tctttgcgat cgcacccttt ggcgcatccc attctccagt aactttatgt 21180
ccatgggcgc actcacagac ctgggccaaa accttctcta cgccaactcc gcccacgcgc 21240
tagacatgac ttttgaggtg gatcccatgg acgagcccac ccttctttat gttttgtttg 21300
aagtctttga cgtggtccgt gtgcaccagc cgcaccgcgg cgtcatcgaa accgtgtacc 21360
tgcgcacgcc cttctcggcc ggcaacgcca caacataaag aagcaagcaa catcaacaac 21420
agctgccgcc atgggctcca gtgagcagga actgaaagcc attgtcaaag atcttggttg 21480
tgggccatat tttttgggca cctatgacaa gcgctttcca ggctttgttt ctccacacaa 21540
gctcgcctgc gccatagtca atacggccgg tcgcgagact gggggcgtac actggatggc 21600
ctttgcctgg aacccgcact caaaaacatg ctacctcttt gagccctttg gcttttctga 21660
ccagcgactc aagcaggttt accagtttga gtacgagtca ctcctgcgcc gtagcgccat 21720
tgcttcttcc cccgaccgct gtataacgct ggaaaagtcc acccaaagcg tacaggggcc 21780
caactcggcc gcctgtggac tattctgctg catgtttctc cacgcctttg ccaactggcc 21840
ccaaactccc atggatcaca accccaccat gaaccttatt accggggtac ccaactccat 21900
gctcaacagt ccccaggtac agcccaccct gcgtcgcaac caggaacagc tctacagctt 21960
cctggagcgc cactcgccct acttccgcag ccacagtgcg cagattagga gcgccacttc 22020
tttttgtcac ttgaaaaaca tgtaaaaata atgtactaga gacactttca ataaaggcaa 22080
atgcttttat ttgtacactc tcgggtgatt atttaccccc acccttgccg tctgcgccgt 22140
ttaaaaatca aaggggttct gccgcgcatc gctatgcgcc actggcaggg acacgttgcg 22200
atactggtgt ttagtgctcc acttaaactc aggcacaacc atccgcggca gctcggtgaa 22260
gttttcactc cacaggctgc gcaccatcac caacgcgttt agcaggtcgg gcgccgatat 22320
cttgaagtcg cagttggggc ctccgccctg cgcgcgcgag ttgcgataca cagggttgca 22380
gcactggaac actatcagcg ccgggtggtg cacgctggcc agcacgctct tgtcggagat 22440
cagatccgcg tccaggtcct ccgcgttgct cagggcgaac ggagtcaact ttggtagctg 22500
ccttcccaaa aagggcgcgt gcccaggctt tgagttgcac tcgcaccgta gtggcatcaa 22560
aaggtgaccg tgcccggtct gggcgttagg atacagcgcc tgcataaaag ccttgatctg 22620
cttaaaagcc acctgagcct ttgcgccttc agagaagaac atgccgcaag acttgccgga 22680
aaactgattg gccggacagg ccgcgtcgtg cacgcagcac cttgcgtcgg tgttggagat 22740
ctgcaccaca tttcggcccc accggttctt cacgatcttg gccttgctag actgctcctt 22800

cagcgcgcgc tgcccgtttt cgctcgtcac atccatttca atcacgtgct ccttatttat 22860
cataatgctt ccgtgtagac acttaagctc gccttcgatc tcagcgcagc ggtgcagcca 22920
caacgcgcag cccgtgggct cgtgatgctt gtaggtcacc tctgcaaacg actgcaggta 22980
cgcctgcagg aatcgcccca tcatcgtcac aaaggtcttg ttgctggtga aggtcagctg 23040
caacccgcgg tgctcctcgt tcagccaggt cttgcatacg gccgccagag cttccacttg 23100
gtcaggcagt agtttgaagt tcgcctttag atcgttatcc acgtggtact tgtccatcag 23160
cgcgcgcgca gcctccatgc ccttctccca cgcagacacg atcggcacac tcagcgggtt 23220
catcaccgta atttcacttt ccgcttcgct gggctcttcc tcttcctctt gcgtccgcat 23280
accacgcgcc actgggtcgt cttcattcag ccgccgcact gtgcgcttac ctcctttgcc 23340
atgcttgatt agcaccggtg ggttgctgaa acccaccatt tgtagcgcca catcttctct 23400
ttcttcctcg ctgtccacga ttacctctgg tgatggcggg cgctcgggct tgggagaagg 23460
gcgcttcttt ttcttcttgg gcgcaatggc caaatccgcc gccgaggtcg atggccgcgg 23520
gctgggtgtg cgcggcacca gcgcgtcttg tgatgagtct tcctcgtcct cggactcgat 23580
acgccgcctc atccgctttt ttgggggcgc ccggggaggc ggcggcgacg gggacgggga 23640
cgacacgtcc tccatggttg ggggacgtcg cgccgcaccg cgtccgcgct cgggggtggt 23700
ttcgcgctgc tcctcttccc gactggccat ttccttctcc tataggcaga aaaagatcat 23760
ggagtcagtc gagaagaagg acagcctaac cgccccctct gagttcgcca ccaccgcctc 23820
caccgatgcc gccaacgcgc ctaccacctt ccccgtcgag gcacccccgc ttgaggagga 23880
ggaagtgatt atcgagcagg acccaggttt tgtaagcgaa gacgacgagg accgctcagt 23940
accaacagag gataaaaagc aagaccagga caacgcagag gcaaacgagg aacaagtcgg 24000
gcggggggac gaaaggcatg gcgactacct agatgtggga gacgacgtgc tgttgaagca 24060
tctgcagcgc cagtgcgcca ttatctgcga cgcgttgcaa gagcgcagcg atgtgcccct 24120
cgccatagcg gatgtcagcc ttgcctacga acgccaccta ttctcaccgc gcgtaccccc 24180
caaacgccaa gaaaacggca catgcgagcc caacccgcgc ctcaacttct accccgtatt 24240
tgccgtgcca gaggtgcttg ccacctatca catctttttc caaaactgca agatacccct 24300
atcctgccgt gccaaccgca gccgagcgga caagcagctg gccttgcggc agggcgctgt 24360
catacctgat atcgcctcgc tcaacgaagt gccaaaaatc tttgagggtc ttggacgcga 24420
cgagaagcgc gcggcaaacg ctctgcaaca ggaaaacagc gaaaatgaaa gtcactctgg 24480
agtgttggtg gaactcgagg gtgacaacgc gcgcctagcc gtactaaaac gcagcatcga 24540
ggtcacccac tttgcctacc cggcacttaa cctacccccc aaggtcatga gcacagtcat 24600
gagtgagctg atcgtgcgcc gtgcgcagcc cctggagagg gatgcaaatt tgcaagaaca 24660
aacagaggag ggcctacccg cagttggcga cgagcagcta gcgcgctggc ttcaaacgcg 24720
cgagcctgcc gacttggagg agcgacgcaa actaatgatg gccgcagtgc tcgttaccgt 24780
ggagcttgag tgcatgcagc ggttctttgc tgacccggag atgcagcgca agctagagga 24840
aacattgcac tacacctttc gacagggcta cgtacgccag gcctgcaaga tctccaacgt 24900
ggagctctgc aacctggtct cctaccttgg aattttgcac gaaaaccgcc ttgggcaaaa 24960
cgtgcttcat tccacgctca agggcgaggc gcgccgcgac tacgtccgcg actgcgttta 25020
cttatttcta tgctacacct ggcagacggc catgggcgtt tggcagcagt gcttggagga 25080

gtgcaacctc aaggagctgc agaaactgct aaagcaaaac ttgaaggacc tatggacggc 25140
cttcaacgag cgctccgtgg ccgcgcacct ggcggacatc attttccccg aacgcctgct 25200
taaaaccctg caacagggtc tgccagactt caccagtcaa agcatgttgc agaactttag 25260
gaactttatc ctagagcgct caggaatctt gcccgccacc tgctgtgcac ttcctagcga 25320
ctttgtgccc attaagtacc gcgaatgccc tccgccgctt tggggccact gctaccttct 25380
gcagctagcc aactaccttg cctaccactc tgacataatg gaagacgtga gcggtgacgg 25440
tctactggag tgtcactgtc gctgcaacct atgcaccccg caccgctccc tggtttgcaa 25500
ttcgcagctg cttaacgaaa gtcaaattat cggtaccttt gagctgcagg gtccctcgcc 25560
tgacgaaaag tccgcggctc cggggttgaa actcactccg gggctgtgga cgtcggctta 25620
ccttcgcaaa tttgtacctg aggactacca cgcccacgag attaggttct acgaagacca 25680
atcccgcccg cctaatgcgg agcttaccgc ctgcgtcatt acccagggcc acattcttgg 25740
ccaattgcaa gccatcaaca aagcccgcca agagtttctg ctacgaaagg gacggggggt 25800
ttacttggac ccccagtccg gcgaggagct caacccaatc cccccgccgc cgcagcccta 25860
tcagcagcag ccgcgggccc ttgcttccca ggatggcacc caaaaagaag ctgcagctgc 25920
cgccgccacc cacggacgag gaggaatact gggacagtca ggcagaggag gttttggacg 25980
aggaggagga ggacatgatg gaagactggg agagcctaga cgaggaagct tccgaggtcg 26040
aagaggtgtc agacgaaaca ccgtcaccct cggtcgcatt cccctcgccg gcgccccaga 26100
aatcggcaac cggttccagc atggctacaa cctccgctcc tcaggcgccg ccggcactgc 26160
ccgttcgccg acccaaccgt agatgggaca ccactggaac cagggccggt aagtccaagc 26220
agccgccgcc gttagcccaa gagcaacaac agcgccaagg ctaccgctca tggcgcgggc 26280
acaagaacgc catagttgct tgcttgcaag actgtggggg caacatctcc ttcgcccgcc 26340
gctttcttct ctaccatcac ggcgtggcct tcccccgtaa catcctgcat tactaccgtc 26400
atctctacag cccatactgc accggcggca gcggcagcaa cagcagcggc cacacagaag 26460
caaaggcgac cggatagcaa gactctgaca aagcccaaga aatccacagc ggcggcagca 26520
gcaggaggag gagcgctgcg tctggcgccc aacgaacccg tatcgacccg cgagcttaga 26580
aacaggattt ttcccactct gtatgctata tttcaacaga gcaggggcca agaacaagag 26640
ctgaaaataa aaaacaggtc tctgcgatcc ctcacccgca gctgcctgta tcacaaaagc 26700
gaagatcagc ttcggcgcac gctggaagac gcggaggctc tcttcagtaa atactgcgcg 26760
ctgactctta aggactagtt tcgcgccctt tctcaaattt aagcgcgaaa actacgtcat 26820
ctccagcggc cacacccggc gccagcacct gttgtcagcg ccattatgag caaggaaatt 26880
cccacgccct acatgtggag ttaccagcca caaatgggac ttgcggctgg agctgcccaa 26940
gactactcaa cccgaataaa ctacatgagc gcgggacccc acatgatatc ccgggtcaac 27000
ggaatacgcg cccaccgaaa ccgaattctc ctggaacagg cggctattac caccacacct 27060
cgtaataacc ttaatccccg tagttggccc gctgccctgg tgtaccagga aagtcccgct 27120
cccaccactg tggtacttcc cagagacgcc caggccgaag ttcagatgac taactcaggg 27180
gcgcagcttg cgggcggctt tcgtcacagg gtgcggtcgc ccgggcaggg tataactcac 27240
ctgacaatca gagggcgagg tattcagctc aacgacgagt cggtgagctc ctcgcttggt 27300
ctccgtccgg acgggacatt tcagatcggc ggcgccggcc gctcttcatt cacgcctcgt 27360

caggcaatcc taactctgca gacctcgtcc tctgagccgc gctctggagg cattggaact 27420
ctgcaattta ttgaggagtt tgtgccatcg gtctacttta accccttctc gggacctccc 27480
ggccactatc cggatcaatt tattcctaac tttgacgcgg taaaggactc ggcggacggc 27540
tacgactgaa tgttaagtgg agaggcagag caactgcgcc tgaaacacct ggtccactgt 27600
cgccgccaca agtgctttgc ccgcgactcc ggtgagtttt gctactttga attgcccgag 27660
gatcatatcg agggcccggc gcacggcgtc cggcttaccg cccagggaga gcttgcccgt 27720
agcctgattc gggagtttac ccagcgcccc ctgctagttg agcgggacag gggaccctgt 27780
gttctcactg tgatttgcaa ctgtcctaac cctggattac atcaagatct ttgttgccat 27840
ctctgtgctg agtataataa atacagaaat taaaatatac tggggctcct atcgccatcc 27900
tgtaaacgcc accgtcttca cccgcccaag caaaccaagg cgaaccttac ctggtacttt 27960
taacatctct ccctctgtga tttacaacag tttcaaccca gacggagtga gtctacgaga 28020
gaacctctcc gagctcagct actccatcag aaaaaacacc accctcctta cctgccggga 28080
acgtacgagt gcgtcaccgg ccgctgcacc acacctaccg cctgaccgta aaccagactt 28140
tttccggaca gacctcaata actctgttta ccagaacagg aggtgagctt agaaaaccct 28200
tagggtatta ggccaaaggc gcagctactg tggggtttat gaacaattca agcaactcta 28260
cgggctattc taattcaggt ttctctagaa atggacggaa ttattacaga gcagcgcctg 28320
ctagaaagac gcagggcagc ggccgagcaa cagcgcatga atcaagagct ccaagacatg 28380
gttaacttgc accagtgcaa aaggggtatc ttttgtctgg taaagcaggc caaagtcacc 28440
tacgacagta ataccaccgg acaccgcctt agctacaagt tgccaaccaa gcgtcagaaa 28500
ttggtggtca tggtgggaga aaagcccatt accataactc agcactcggt agaaaccgaa 28560
ggctgcattc actcaccttg tcaaggacct gaggatctct gcacccttat taagaccctg 28620
tgcggtctca aagatcttat tccctttaac taataaaaaa aaataataaa gcatcactta 28680
cttaaaatca gttagcaaat ttctgtccag tttattcagc agcacctcct tgccctcctc 28740
ccagctctgg tattgcagct tcctcctggc tgcaaacttt ctccacaatc taaatggaat 28800
gtcagtttcc tcctgttcct gtccatccgc acccactatc ttcatgttgt tgcagatgaa 28860
gcgcgcaaga ccgtctgaag ataccttcaa ccccgtgtat ccatatgaca cggaaaccgg 28920
tcctccaact gtgccttttc ttactcctcc ctttgtatcc cccaatgggt ttcaagagag 28980
tccccctggg gtactctctt tgcgcctatc cgaacctcta gttacctcca atggcatgct 29040
tgcgctcaaa atgggcaacg gcctctctct ggacgaggcc ggcaacctta cctcccaaaa 29100
tgtaaccact gtgagcccac ctctcaaaaa aaccaagtca aacataaacc tggaaatatc 29160
tgcacccctc acagttacct cagaagccct aactgtggct gccgccgcac ctctaatggt 29220
cgcgggcaac acactcacca tgcaatcaca ggccccgcta accgtgcacg actccaaact 29280
tagcattgcc acccaaggac ccctcacagt gtcagaagga aagctagccc tgcaaacatc 29340
aggccccctc accaccaccg atagcagtac ccttactatc actgcctcac cccctctaac 29400
tactgccact ggtagcttgg gcattgactt gaaagagccc atttatacac aaaatggaaa 29460
actaggacta aagtacgggg ctcctttgca tgtaacagac gacctaaaca ctttgaccgt 29520
agcaactggt ccaggtgtga ctattaataa tacttccttg caaactaaag ttactggagc 29580
cttgggtttt gattcacaag gcaatatgca acttaatgta gcaggaggac taaggattga 29640

ttctcaaaac agacgcctta tacttgatgt tagttatccg tttgatgctc aaaaccaact 29700
aaatctaaga ctaggacagg gccctctttt tataaactca gcccacaact tggatattaa 29760
ctacaacaaa ggcctttact tgtttacagc ttcaaacaat tccaaaaagc ttgaggttaa 29820
cctaagcact gccaaggggt tgatgtttga cgctacagcc atagccatta atgcaggaga 29880
tgggcttgaa tttggttcac ctaatgcacc aaacacaaat cccctcaaaa caaaaattgg 29940
ccatggccta gaatttgatt caaacaaggc tatggttcct aaactaggaa ctggccttag 30000
ttttgacagc acaggtgcca ttacagtagg aaacaaaaat aatgataagc taactttgtg 30060
gaccacacca gctccatctc ctaactgtag actaaatgca gagaaagatg ctaaactcac 30120
tttggtctta acaaaatgtg gcagtcaaat acttgctaca gtttcagttt tggctgttaa 30180
aggcagtttg gctccaatat ctggaacagt tcaaagtgct catcttatta taagatttga 30240
cgaaaatgga gtgctactaa acaattcctt cctggaccca gaatattgga actttagaaa 30300
tggagatctt actgaaggca cagcctatac aaacgctgtt ggatttatgc ctaacctatc 30360
agcttatcca aaatctcacg gtaaaactgc caaaagtaac attgtcagtc aagtttactt 30420
aaacggagac aaaactaaac ctgtaacact aaccattaca ctaaacggta cacaggaaac 30480
aggagacaca actccaagtg catactctat gtcattttca tgggactggt ctggccacaa 30540
ctacattaat gaaatatttg ccacatcctc ttacactttt tcatacattg cccaagaata 30600
aagaatcgtt tgtgttatgt ttcaacgtgt ttatttttca attgcccggg atcggtgatc 30660
accgatccag acatgataag atacattgat gagtttggac aaaccacaac tagaatgcag 30720
tgaaaaaaat gctttatttg tgaaatttgt gatgctattg ctttatttgt aaccattata 30780
agctgcaata aacaagttcc cggatcgcga tccggcccga ggctgtagcc gacgatggtg 30840
cgccaggaga gttgttgatt cattgtttgc ctccctgctg cggtttttca ccgaagttca 30900
tgccagtcca gcgtttttgc agcagaaaag ccgccgactt cggtttgcgg tcgcgagtga 30960
agatcccttt cttgttaccg ccaacgcgca atatgccttg cgaggtcgca aaatcggcga 31020
aattccatac ctgttcaccg acgacggcgc tgacgcgatc aaagacgcgg tgatacatat 31080
ccagccatgc acactgatac tcttcactcc acatgtcggt gtacattgag tgcagcccgg 31140
ctaacgtatc cacgccgtat tcggtgatga taatcggctg atgcagtttc tcctgccagg 31200
ccagaagttc tttttccagt accttctctg ccgtttccaa atcgccgctt tggacatacc 31260
atccgtaata acggttcagg cacagcacat caaagagatc gctgatggta tcggtgtgag 31320
cgtcgcagaa cattacattg acgcaggtga tcggacgcgt cgggtcgagt ttacgcgttg 31380
cttccgccag tggcgcgaaa tattcccgtg caccttgcgg acgggtatcc ggttcgttgg 31440
caatactcca catcaccacg cttgggtggt ttttgtcacg cgctatcagc tctttaatcg 31500
cctgtaagtg cgcttgctga gtttccccgt tgactgcctc ttcgctgtac agttctttcg 31560
gcttgttgcc cgcttcgaaa ccaatgccta aagagaggtt aaagccgaca gcagcagttt 31620
catcaatcac cacgatgcca tgttcatctg cccagtcgag catctcttca gcgtaagggt 31680
aatgcgaggt acggtaggag ttggccccaa tccagtccat taatgcgtgg tcgtgcacca 31740
tcagcacgtt atcgaatcct ttgccacgca agtccgcatc ttcatgacga ccaaagccag 31800
taaagtagaa cggtttgtgg ttaatcagga actgttcgcc cttcactgcc actgaccgga 31860
tgccgacgcg aagcgggtag atatcacact ctgtctggct tttggctgtg acgcacagtt 31920

catagagata accttcaccc ggttgccaga ggtgcggatt caccacttgc aaagtcccgc 31980

tagtgccttg tccagttgca accacctgtt gatccgcatc acgcagttca acgctgacat 32040

caccattggc caccacctgc cagtcaacag acgcgtggtt acagtcttgc gcgacatgcg 32100

tcaccacggt gatatcgtcc acccaggtgt tcggcgtggt gtagagcatt acgctgcgat 32160

ggattccggc atagttaaag aaatcatgga agtaagactg ctttttcttg ccgttttcgt 32220

cggtaatcac cattcccggc gggatagtct gccagttcag ttcgttgttc acacaaacgg 32280

tgatacgtac acttttcccg gcaataacat acggcgtgac atcggcttca aatggcgtat 32340

agccgccctg atgctccatc acttcctgat tattgaccca cactttgccg taatgagtga 32400

ccgcatcgaa acgcagcacg atacgctggc ctgcccaacc tttcggtata aagacttcgc 32460

gctgatacca gacgttgccc gcataattac gaatatctgc atcggcgaac tgatcgttaa 32520

aactgcctgg cacagcaatt gcccggcttt cttgtaacgc gctttcccac caacgctgat 32580

caattccaca gttttcgcga tccagactga atgcccacag gccgtcgagt tttttgattt 32640

cacgggttgg ggtttctaca ggacggacca tgcgttcgac ctttctcttc ttttttgggc 32700

ccatgatggc agatccgtat agtgagtcgt attagctggt tctttccgcc tcagaagcca 32760

tagagcccac cgcatcccca gcatgcctgc tattgtcttc ccaatcctcc cccttgctgt 32820

cctgccccac cccacccccc agaatagaat gacacctact cagacaatgc gatgcaattt 32880

cctcatttta ttaggaaagg acagtgggag tggcaccttc cagggtcaag gaaggcacgg 32940

gggaggggca aacaacagat ggctggcaac tagaaggcac agtcgaggct gatcagcgag 33000

ctctagatgc atgctcgagc ggccgccagt gtgatggata tctgcagaat tccagcacac 33060

tggcggccgt tactagtgga tccgagctcg gtacccggcc gttataacac cactcgacac 33120

ggcaccagct caatcagtca cagtgtaaaa aagggccaag tgcagagcga gtatatatag 33180

gactaaaaaa tgacgtaacg gttaaagtcc acaaaaaaca cccagaaaac cgcacgcgaa 33240

cctacgccca gaaacgaaag ccaaaaaacc cacaacttcc tcaaatcgtc acttccgttt 33300

tcccacgtta cgtcacttcc cattttaaga aaactacaat tcccaacaca tacaagttac 33360

tccgccctaa aacctacgtc acccgccccg ttcccacgcc ccgcgccacg tcacaaactc 33420

caccccctca ttatcatatt ggcttcaatc caaaataagg tatattattg atgatg 33476

<210> 7
<211> 33589
<212> DNA
<213> Artificial

<220>
<223> Synthetic

<400> 7
catcatcaat aatatacctt attttggatt gaagccaata tgataatgag ggggtggagt 60

ttgtgacgtg gcgcggggcg tgggaacggg gcgggtgacg tagtagtgtg gcggaagtgt 120

gatgttgcaa gtgtggcgga acacatgtaa gcgacggatg tggcaaaagt gacgtttttg 180

gtgtgcgccg gtgtacacag gaagtgacaa ttttcgcgcg gttttaggcg gatgttgtag 240

taaatttggg cgtaaccgag taagatttgg ccattttcgc gggaaaactg aataagagga 300

agtgaaatct gaataatttt gtgttactca tagcgcgtaa tatttgtcta gggccgggga 360

tcggtgatca ccgatccaga catgataaga tacattgatg agtttggaca aaccacaact 420

agaatgcagt gaaaaaaatg ctttatttgt gaaatttgtg atgctattgc tttatttgta 480
accattataa gctgcaataa acaagttccc ggatctttct agctagtcta gactagctag 540
actcgagagc ggccgcaatc gataagcttg atctagagat gatcctcacc acagccagtt 600
ggagatgtcg aaccaggacc acaggttttt ccaggagtcc agagccagca ggtctttttc 660
gtttttttcc tgctgggtct gggagtcttc cagcagacgg tagatggtgt cggtgtagtt 720
ggagatttca cggtcccatt ccatccaggt catgttgttc cagatctcga gctgctggtc 780
tttcaggtaa cgttcgatag ccagaacacg ggtctgcagc tgtttgatac cccaaacggt 840
cagctgcagc atgtgctgct gagcttcgat agcacgcagc aggttggact gctgctgaac 900
gatggaggac agcagctgac gagcctgaac ggtaagctta gcaccggtcg gagcgatacc 960
cagcggtttc agttcgataa ctttgtattt gtacagttcg gaacgccagt tgtctttcat 1020
gttaccacca cccggacgga agatttcttc ggttttgttg tcttcaccac cgtcacgaac 1080
cagcagcaga ccggtgatgt tggatttgca ggtgatgtta ccagcgatcg gcggagcgta 1140
catagcacga ccaacaccct gccacatgtt gatgatctgt ttgatacggc acggcagggt 1200
gatggtttcg tcttcggtag cgttgttgtt gaacagacgg gtggtgttgc agtagaagaa 1260
ttcaccacgg cagttgaagg agtgggtggt gatttccagg tcaccaccgg aggacggagc 1320
gaatttgatg gttttgttgt tgttgtagtt ttcctgcagt ttttctttaa cacgtttcag 1380
ggtttcgttc catttggaac cggagatgtt gcagtaagcc tgacggatgt caccgatgat 1440
gtcaccggta gcgtagaagg tctgacccgg accgatacgc atggatttac gggtgttgtt 1500
gttcggacgg gtgcaaacga tttcaacgga tttgttcagg tgaacgatga tggttttaac 1560
gttgtcggtc aggttttcgg aacggatgat gatttctttt tcagccaggg aaccgttcag 1620
cagcagctgg gtggaaacaa ccggtttgat accatgggtg cactgaacgg tggaaacgtt 1680
gttgcacgga cctttaccgg agaaggtttt gttgttgcat ttcaggatag cgtaaccagc 1740
cggagcgcag tagtggatcg ggatcgggtc gaagttaact ttcgggcaag cctgggtgat 1800
ggtggaagcg ttgcagttga tcaggatgta ttcggagttg ttggagttgt tacggttttc 1860
tttcagcaga acgatgtccg gacggtagaa cagagcgtaa ccctgctgtt ttttgtcacg 1920
gatttcggtg gtggtgttga aggagcagtt acggatttct ttgttcatgt cgttggtaac 1980
gttgtttttg aaggtagcgt tggtgcagtg cagggtaacg cacagcgggg tcagtttaac 2040
gcacggtttc agggactggt cccacaggga gatgatgtct tcgtgcatct ggtcaaccat 2100
gtcgtttttc cacatgttga agttttcggt aacgttttcc agaacgattt cctgcgggtt 2160
cgggtcggtc ggaacgcaag cgtgggtagc ccaaacgttg tgaacttcac ggtcgtaggc 2220
tttggtgtcg gaagcgcaga acagggtggt tttagcgtcg gtccaaaccg gaacaccgta 2280
gtaaacggta acccacatgt taccaacaac acggcagatg atgatcatcc agaaacccag 2340
gataccccac atccaccact gcggccagtt acgcgggata ccacgaacac gcatggtggc 2400
gatatctcta gtcatcgaat tctgcagtga tcagggatcc cagatccgta tagtgagtcg 2460
tattaggtac cggctgcagt tggacctggg agtggacacc tgtggagaga aaggcaaagt 2520
ggatgtcatt gtcactcaag tgtatggcca gatctcaagc ctgccacacc tcaagtgaag 2580
ccaagggggt gggcctatag actctatagg cggtacttac gtcactcttg gcacggggaa 2640
tccgcgttcc aatgcaccgt tcccggccgc ggaggctgga tcggtcccgg tgtcttctat 2700

ggaggtcaaa acagcgtgga tggcgtctcc aggcgatctg acggttcact aaacgagctc 2760
tgcttatata gacctcccac cgtacacgcc taccgcccat ttgcgtcaat ggggcggagt 2820
tgttacgaca ttttggaaag tcccgttgat tttggtgcca aaacaaactc ccattgacgt 2880
caatggggtg gagacttgga aatccccgtg agtcaaaccg ctatccacgc ccattgatgt 2940
actgccaaaa ccgcatcacc atggtaatag cgatgactaa tacgtagatg tactgccaag 3000
taggaaagtc ccataaggtc atgtactggg cataatgcca ggcgggccat ttaccgtcat 3060
tgacgtcaat agggggcgta cttggcatat gatacacttg atgtactgcc aagtgggcag 3120
tttaccgtaa atactccacc cattgacgtc aatggaaagt ccctattggc gttactatgg 3180
gaacatacgt cattattgac gtcaatgggc gggggtcgtt gggcggtcag ccaggcgggc 3240
catttaccgt aagttatgta acgcggaact ccatatatgg gctatgaact aatgaccccg 3300
taattgatta ctattaataa ctagtactga aatgtgtggg cgtggcttaa gggtgggaaa 3360
gaatatataa ggtgggggtc ttatgtagtt ttgtatctgt tttgcagcag ccgccgccgc 3420
catgagcacc aactcgtttg atggaagcat tgtgagctca tatttgacaa cgcgcatgcc 3480
cccatgggcc ggggtgcgtc agaatgtgat gggctccagc attgatggtc gccccgtcct 3540
gcccgcaaac tctactacct tgacctacga gaccgtgtct ggaacgccgt tggagactgc 3600
agcctccgcc gccgcttcag ccgctgcagc caccgcccgc gggattgtga ctgactttgc 3660
tttcctgagc ccgcttgcaa gcagtgcagc ttcccgttca tccgcccgcg atgacaagtt 3720
gacggctctt ttggcacaat tggattcttt gacccgggaa cttaatgtcg tttctcagca 3780
gctgttggat ctgcgccagc aggtttctgc cctgaaggct tcctcccctc ccaatgcggt 3840
ttaaaacata aataaaaaac cagactctgt ttggatttgg atcaagcaag tgtcttgctg 3900
tctttattta ggggttttgc gcgcgcggta ggcccgggac cagcggtctc ggtcgttgag 3960
ggtcctgtgt attttttcca ggacgtggta aaggtgactc tggatgttca gatacatggg 4020
cataagcccg tctctggggt ggaggtagca ccactgcaga gcttcatgct gcggggtggt 4080
gttgtagatg atccagtcgt agcaggagcg ctgggcgtgg tgcctaaaaa tgtctttcag 4140
tagcaagctg attgccaggg gcaggccctt ggtgtaagtg tttacaaagc ggttaagctg 4200
ggatgggtgc atacgtgggg atatgagatg catcttggac tgtattttta ggttggctat 4260
gttcccagcc atatccctcc ggggattcat gttgtgcaga accaccagca cagtgtatcc 4320
ggtgcacttg ggaaatttgt catgtagctt agaaggaaat gcgtggaaga acttggagac 4380
gcccttgtga cctccaagat tttccatgca ttcgtccata atgatggcaa tgggcccacg 4440
ggcggcggcc tgggcgaaga tatttctggg atcactaacg tcatagttgt gttccaggat 4500
gagatcgtca taggccattt ttacaaagcg cgggcggagg gtgccagact gcggtataat 4560
ggttccatcc ggcccagggg cgtagttacc ctcacagatt tgcatttccc acgctttgag 4620
ttcagatggg gggatcatgt ctacctgcgg ggcgatgaag aaaacggttt ccggggtagg 4680
ggagatcagc tgggaagaaa gcaggttcct gagcagctgc gacttaccgc agccggtggg 4740
cccgtaaatc acacctatta ccggctgcaa ctggtagtta agagagctgc agctgccgtc 4800
atccctgagc aggggggcca cttcgttaag catgtccctg actcgcatgt tttccctgac 4860
caaatccgcc agaaggcgct cgccgcccag cgatagcagt tcttgcaagg aagcaaagtt 4920
tttcaacggt ttgagaccgt ccgccgtagg catgcttttg agcgtttgac caagcagttc 4980

caggcggtcc cacagctcgg tcacctgctc tacggcatct cgatccagca tatctcctcg 5040
tttcgcgggt tggggcggct ttcgctgtac ggcagtagtc ggtgctcgtc cagacgggcc 5100
agggtcatgt ctttccacgg gcgcagggtc ctcgtcagcg tagtctgggt cacggtgaag 5160
gggtgcgctc cgggctgcgc gctggccagg gtgcgcttga ggctggtcct gctggtgctg 5220
aagcgctgcc ggtcttcgcc ctgcgcgtcg gccaggtagc atttgaccat ggtgtcatag 5280
tccagcccct ccgcggcgtg gcccttggcg cgcagcttgc ccttggagga ggcgccgcac 5340
gaggggcagt gcagactttt gagggcgtag agcttgggcg cgagaaatac cgattccggg 5400
gagtaggcat ccgcgccgca ggccccgcag acggtctcgc attccacgag ccaggtgagc 5460
tctggccgtt cggggtcaaa aaccaggttt cccccatgct ttttgatgcg tttcttacct 5520
ctggtttcca tgagccggtg tccacgctcg gtgacgaaaa ggctgtccgt gtccccgtat 5580
acagacttga gaggcctgtc ctcgagcggt gttccgcggt cctcctcgta tagaaactcg 5640
gaccactctg agacaaaggc tcgcgtccag gccagcacga aggaggctaa gtgggagggg 5700
tagcggtcgt tgtccactag ggggtccact cgctccaggg tgtgaagaca catgtcgccc 5760
tcttcggcat caaggaaggt gattggtttg taggtgtagg ccacgtgacc gggtgttcct 5820
gaaggggggc tataaaaggg ggtgggggcg cgttcgtcct cactctcttc cgcatcgctg 5880
tctgcgaggg ccagctgttg gggtgagtac tccctctgaa aagcgggcat gacttctgcg 5940
ctaagattgt cagtttccaa aaacgaggag gatttgatat tcacctggcc cgcggtgatg 6000
cctttgaggg tggccgcatc catctggtca gaaaagacaa tctttttgtt gtcaagcttg 6060
gtggcaaacg acccgtagag ggcgttggac agcaacttgg cgatggagcg cagggtttgg 6120
tttttgtcgc gatcggcgcg ctccttggcc gcgatgttta gctgcacgta ttcgcgcgca 6180
acgcaccgcc attcgggaaa gacggtggtg cgctcgtcgg gcaccaggtg cacgcgccaa 6240
ccgcggttgt gcagggtgac aaggtcaacg ctggtggcta cctctccgcg taggcgctcg 6300
ttggtccagc agaggcggcc gcccttgcgc gagcagaatg gcggtagggg gtctagctgc 6360
gtctcgtccg gggggtctgc gtccacggta aagaccccgg gcagcaggcg cgcgtcgaag 6420
tagtctatct tgcatccttg caagtctagc gcctgctgcc atgcgcgggc ggcaagcgcg 6480
cgctcgtatg ggttgagtgg gggaccccat ggcatggggt gggtgagcgc ggaggcgtac 6540
atgccgcaaa tgtcgtaaac gtagaggggc tctctgagta ttccaagata tgtagggtag 6600
catcttccac cgcggatgct ggcgcgcacg taatcgtata gttcgtgcga gggagcgagg 6660
aggtcgggac cgaggttgct acgggcgggc tgctctgctc ggaagactat ctgcctgaag 6720
atggcatgtg agttggatga tatggttgga cgctggaaga cgttgaagct ggcgtctgtg 6780
agacctaccg cgtcacgcac gaaggaggcg taggagtcgc gcagcttgtt gaccagctcg 6840
gcggtgacct gcacgtctag ggcgcagtag tccagggttt ccttgatgat gtcatactta 6900
tcctgtccct tttttttcca cagctcgcgg ttgaggacaa actcttcgcg gtctttccag 6960
tactcttgga tcggaaaccc gtcggcctcc gaacggtaag agcctagcat gtagaactgg 7020
ttgacggcct ggtaggcgca gcatcccttt tctacgggta gcgcgtatgc ctgcgcggcc 7080
ttccggagcg aggtgtgggt gagcgcaaag gtgtccctga ccatgacttt gaggtactgg 7140
tatttgaagt cagtgtcgtc gcatccgccc tgctcccaga gcaaaaagtc cgtgcgcttt 7200
ttggaacgcg gatttggcag ggcgaaggtg acatcgttga agagtatctt tcccgcgcga 7260

ggcataaagt tgcgtgtgat gcggaagggt cccggcacct cggaacggtt gttaattacc 7320
tgggcggcga gcacgatctc gtcaaagccg ttgatgttgt ggcccacaat gtaaagttcc 7380
aagaagcgcg ggatgccctt gatggaaggc aattttttaa gttcctcgta ggtgagctct 7440
tcaggggagc tgagcccgtg ctctgaaagg gcccagtctg caagatgagg gttggaagcg 7500
acgaatgagc tccacaggtc acgggccatt agcatttgca ggtggtcgcg aaaggtccta 7560
aactggcgac ctatggccat tttttctggg gtgatgcagt agaaggtaag cgggtcttgt 7620
tcccagcggt cccatccaag gttcgcggct aggtctcgcg cggcagtcac tagaggctca 7680
tctccgccga acttcatgac cagcatgaag ggcacgagct gcttcccaaa ggcccccatc 7740
caagtatagg tctctacatc gtaggtgaca aagagacgct cggtgcgagg atgcgagccg 7800
atcgggaaga actggatctc ccgccaccaa ttggaggagt ggctattgat gtggtgaaag 7860
tagaagtccc tgcgacgggc cgaacactcg tgctggcttt tgtaaaaacg tgcgcagtac 7920
tggcagcggt gcacgggctg tacatcctgc acgaggttga cctgacgacc gcgcacaagg 7980
aagcagagtg ggaatttgag cccctcgcct ggcgggtttg gctggtggtc ttctacttcg 8040
gctgcttgtc cttgaccgtc tggctgctcg aggggagtta cggtggatcg gaccaccacg 8100
ccgcgcgagc ccaaagtcca gatgtccgcg cgcggcggtc ggagcttgat gacaacatcg 8160
cgcagatggg agctgtccat ggtctggagc tcccgcggcg tcaggtcagg cgggagctcc 8220
tgcaggttta cctcgcatag acgggtcagg gcgcgggcta gatccaggtg atacctaatt 8280
tccaggggct ggttggtggc ggcgtcgatg gcttgcaaga ggccgcatcc ccgcggcgcg 8340
actacggtac cgcgcggcgg gcggtgggcc gcgggggtgt ccttggatga tgcatctaaa 8400
agcggtgacg cgggcgagcc cccggaggta gggggggctc cggacccgcc gggagagggg 8460
gcaggggcac gtcggcgccg cgcgcgggca ggagctggtg ctgcgcgcgt aggttgctgg 8520
cgaacgcgac gacgcggcgg ttgatctcct gaatctggcg cctctgcgtg aagacgacgg 8580
gcccggtgag cttgaacctg aaagagagtt cgacagaatc aatttcggtg tcgttgacgg 8640
cggcctggcg caaaatctcc tgcacgtctc ctgagttgtc ttgataggcg atctcggcca 8700
tgaactgctc gatctcttcc tcctggagat ctccgcgtcc ggctcgctcc acggtggcgg 8760
cgaggtcgtt ggaaatgcgg gccatgagct gcgagaaggc gttgaggcct ccctcgttcc 8820
agacgcggct gtagaccacg cccccttcgg catcgcgggc gcgcatgacc acctgcgcga 8880
gattgagctc cacgtgccgg gcgaagacgg cgtagtttcg caggcgctga aagaggtagt 8940
tgagggtggt ggcggtgtgt tctgccacga agaagtacat aacccagcgt cgcaacgtgg 9000
attcgttgat atcccccaag gcctcaaggc gctccatggc ctcgtagaag tccacggcga 9060
agttgaaaaa ctgggagttg cgcgccgaca cggttaactc ctcctccaga agacggatga 9120
gctcggcgac agtgtcgcgc acctcgcgct caaaggctac aggggcctct tcttcttctt 9180
caatctcctc ttccataagg gcctcccctt cttcttcttc tggcggcggt gggggagggg 9240
ggacacggcg gcgacgacgg cgcaccggga ggcggtcgac aaagcgctcg atcatctccc 9300
cgcggcgacg gcgcatggtc tcggtgacgg cgcggccgtt ctcgcggggg cgcagttgga 9360
agacgccgcc cgtcatgtcc cggttatggg ttggcggggg gctgccatgc ggcagggata 9420
cggcgctaac gatgcatctc aacaattgtt gtgtaggtac tccgccgccg agggacctga 9480
gcgagtccgc atcgaccgga tcggaaaacc tctcgagaaa ggcgtctaac cagtcacagt 9540

cgcaaggtag gctgagcacc gtggcgggcg gcagcgggcg gcggtcgggg ttgtttctgg 9600
cggaggtgct gctgatgatg taattaaagt aggcggtctt gagacggcgg atggtcgaca 9660
gaagcaccat gtccttgggt ccggcctgct gaatgcgcag gcggtcggcc atgccccagg 9720
cttcgttttg acatcggcgc aggtctttgt agtagtcttg catgagcctt tctaccggca 9780
cttcttcttc tccttcctct tgtcctgcat ctcttgcatc tatcgctgcg gcggcggcgg 9840
agtttggccg taggtggcgc cctcttcctc ccatgcgtgt gaccccgaag cccctcatcg 9900
gctgaagcag ggctaggtcg gcgacaacgc gctcggctaa tatggcctgc tgcacctgcg 9960
tgagggtaga ctggaagtca tccatgtcca caaagcggtg gtatgcgccc gtgttgatgg 10020
tgtaagtgca gttggccata acggaccagt taacggtctg gtgacccggc tgcgagagct 10080
cggtgtacct gagacgcgag taagccctcg agtcaaatac gtagtcgttg caagtccgca 10140
ccaggtactg gtatcccacc aaaaagtgcg gcggcggctg gcggtagagg ggccagcgta 10200
gggtggccgg ggctccgggg gcgagatctt ccaacataag gcgatgatat ccgtagatgt 10260
acctggacat ccaggtgatg ccggcggcgg tggtggaggc gcgcggaaag tcgcggacgc 10320
ggttccagat gttgcgcagc ggcaaaaagt gctccatggt cgggacgctc tggccggtca 10380
ggcgcgcgca atcgttgacg ctctagcgtg caaaaggaga gcctgtaagc gggcactctt 10440
ccgtggtctg gtggataaat tcgcaagggt atcatggcgg acgaccgggg ttcgagcccc 10500
gtatccggcc gtccgccgtg atccatgcgg ttaccgcccg cgtgtcgaac ccaggtgtgc 10560
gacgtcagac aacgggggag tgctcctttt ggcttccttc caggcgcggc ggctgctgcg 10620
ctagcttttt tggccactgg ccgcgcgcag cgtaagcggt taggctggaa agcgaaagca 10680
ttaagtggct cgctccctgt agccggaggg ttattttcca agggttgagt cgcgggaccc 10740
ccggttcgag tctcggaccg gccggactgc ggcgaacggg ggtttgcctc cccgtcatgc 10800
aagaccccgc ttgcaaattc ctccggaaac agggacgagc cccttttttg cttttcccag 10860
atgcatccgg tgctgcggca gatgcgcccc cctcctcagc agcggcaaga gcaagagcag 10920
cggcagacat gcagggcacc ctcccctcct cctaccgcgt caggaggggc gacatccgcg 10980
gttgacgcgg cagcagatgg tgattacgaa cccccgcggc gccgggcccg gcactacctg 11040
gacttggagg agggcgaggg cctggcgcgg ctaggagcgc cctctcctga gcggcaccca 11100
agggtgcagc tgaagcgtga tacgcgtgag gcgtacgtgc cgcggcagaa cctgtttcgc 11160
gaccgcgagg gagaggagcc cgaggagatg cgggatcgaa agttccacgc agggcgcgag 11220
ctgcggcatg gcctgaatcg cgagcggttg ctgcgcgagg aggactttga gcccgacgcg 11280
cgaaccggga ttagtcccgc gcgcgcacac gtggcggccg ccgacctggt aaccgcatac 11340
gagcagacgg tgaaccagga gattaacttt caaaaaagct ttaacaacca cgtgcgtacg 11400
cttgtggcgc gcgaggaggt ggctatagga ctgatgcatc tgtgggactt tgtaagcgcg 11460
ctggagcaaa acccaaatag caagccgctc atggcgcagc tgttccttat agtgcagcac 11520
agcagggaca acgaggcatt cagggatgcg ctgctaaaca tagtagagcc cgagggccgc 11580
tggctgctcg atttgataaa catcctgcag agcatagtgg tgcaggagcg cagcttgagc 11640
ctggctgaca aggtggccgc catcaactat tccatgctta gcctgggcaa gttttacgcc 11700
cgcaagatat accatacccc ttacgttccc atagacaagg aggtaaagat cgaggggttc 11760
tacatgcgca tggcgctgaa ggtgcttacc ttgagcgacg acctgggcgt ttatcgcaac 11820

gagcgcatcc acaaggccgt gagcgtgagc cggcggcgcg agctcagcga ccgcgagctg 11880
atgcacagcc tgcaaagggc cctggctggc acgggcagcg gcgatagaga ggccgagtcc 11940
tactttgacg cgggcgctga cctgcgctgg gccccaagcc gacgcgccct ggaggcagct 12000
ggggccggac ctgggctggc ggtggcaccc gcgcgcgctg gcaacgtcgg cggcgtggag 12060
gaatatgacg aggacgatga gtacgagcca gaggacggcg agtactaagc ggtgatgttt 12120
ctgatcagat gatgcaagac gcaacggacc cggcggtgcg ggcggcgctg cagagccagc 12180
cgtccggcct taactccacg gacgactggc gccaggtcat ggaccgcatc atgtcgctga 12240
ctgcgcgcaa tcctgacgcg ttccggcagc agccgcaggc caaccggctc tccgcaattc 12300
tggaagcggt ggtcccggcg cgcgcaaacc ccacgcacga gaaggtgctg gcgatcgtaa 12360
acgcgctggc cgaaaacagg gccatccggc ccgacgaggc cggcctggtc tacgacgcgc 12420
tgcttcagcg cgtggctcgt tacaacagcg gcaacgtgca gaccaacctg gaccggctgg 12480
tgggggatgt gcgcgaggcc gtggcgcagc gtgagcgcgc gcagcagcag ggcaacctgg 12540
gctccatggt tgcactaaac gccttcctga gtacacagcc cgccaacgtg ccgcggggac 12600
aggaggacta caccaacttt gtgagcgcac tgcggctaat ggtgactgag acaccgcaaa 12660
gtgaggtgta ccagtctggg ccagactatt ttttccagac cagtagacaa ggcctgcaga 12720
ccgtaaacct gagccaggct ttcaaaaact tgcaggggct gtgggggtg cgggctccca 12780
caggcgaccg cgcgaccgtg tctagcttgc tgacgcccaa ctcgcgcctg ttgctgctgc 12840
taatagcgcc cttcacggac agtggcagcg tgtcccggga cacataccta ggtcacttgc 12900
tgacactgta ccgcgaggcc ataggtcagg cgcatgtgga cgagcatact ttccaggaga 12960
ttacaagtgt cagccgcgcg ctggggcagg aggacacggg cagcctggag gcaaccctaa 13020
actacctgct gaccaaccgg cggcagaaga tcccctcgtt gcacagttta aacagcgagg 13080
aggagcgcat tttgcgctac gtgcagcaga gcgtgagcct taacctgatg cgcgacgggg 13140
taacgcccag cgtggcgctg gacatgaccg cgcgcaacat ggaaccgggc atgtatgcct 13200
caaaccggcc gtttatcaac cgcctaatgg actacttgca tcgcgcggcc gccgtgaacc 13260
ccgagtattt caccaatgcc atcttgaacc cgcactggct accgccccct ggtttctaca 13320
ccgggggatt cgaggtgccc gagggtaacg atggattcct ctgggacgac atagacgaca 13380
gcgtgttttc cccgcaaccg cagaccctgc tagagttgca acagcgcgag caggcagagg 13440
cggcgctgcg aaaggaaagc ttccgcaggc caagcagctt gtccgatcta ggcgctgcgg 13500
ccccgcggtc agatgctagt agcccatttc caagcttgat agggtctctt accagcactc 13560
gcaccacccg cccgcgcctg ctgggcgagg aggagtacct aaacaactcg ctgctgcagc 13620
cgcagcgcga aaaaaacctg cctccggcat ttcccaacaa cgggatagag agcctagtgg 13680
acaagatgag tagatggaag acgtacgcgc aggagcacag ggacgtgcca ggcccgcgcc 13740
cgcccacccg tcgtcaaagg cacgaccgtc agcggggtct ggtgtgggag gacgatgact 13800
cggcagacga cagcagcgtc ctggatttgg gagggagtgg caacccgttt gcgcaccttc 13860
gccccaggct ggggagaatg ttttaaaaaa aaaaaaagca tgatgcaaaa taaaaaactc 13920
accaaggcca tggcaccgag cgttggtttt cttgtattcc ccttagtatg cggcgcgcgg 13980
cgatgtatga ggaaggtcct cctccctcct acgagagtgt ggtgagcgcg gcgccagtgg 14040
cggcggcgct gggttctccc ttcgatgctc ccctggaccc gccgtttgtg cctccgcggt 14100

acctgcggcc taccgggggg agaaacagca tccgttactc tgagttggca cccctattcg 14160
acaccacccg tgtgtacctg gtggacaaca agtcaacgga tgtggcatcc ctgaactacc 14220
agaacgacca cagcaacttt ctgaccacgg tcattcaaaa caatgactac agcccggggg 14280
aggcaagcac acagaccatc aatcttgacg accggtcgca ctggggcggc gacctgaaaa 14340
ccatcctgca taccaacatg ccaaatgtga acgagttcat gtttaccaat aagtttaagg 14400
cgcgggtgat ggtgtcgcgc ttgcctacta aggacaatca ggtggagctg aaatacgagt 14460
gggtggagtt cacgctgccc gagggcaact actccgagac catgaccata gaccttatga 14520
acaacgcgat cgtggagcac tacttgaaag tgggcagaca gaacggggtt ctggaaagcg 14580
acatcggggt aaagtttgac acccgcaact tcagactggg gtttgacccc gtcactggtc 14640
ttgtcatgcc tggggtatat acaaacgaag ccttccatcc agacatcatt ttgctgccag 14700
gatgcggggt ggacttcacc cacagccgcc tgagcaactt gttgggcatc cgcaagcggc 14760
aacccttcca ggagggcttt aggatcacct acgatgatct ggagggtggt aacattcccg 14820
cactgttgga tgtggacgcc taccaggcga gcttgaaaga tgacaccgaa cagggcgggg 14880
gtggcgcagg cggcagcaac agcagtggca gcggcgcgga agagaactcc aacgcggcag 14940
ccgcggcaat gcagccggtg gaggacatga acgatcatgc cattcgcggc gacacctttg 15000
ccacacgggc tgaggagaag cgcgctgagg ccgaagcagc ggccgaagct gccgcccccg 15060
ctgcgcaacc cgaggtcgag aagcctcaga agaaaccggt gatcaaaccc ctgacagagg 15120
acagcaagaa acgcagttac aacctaataa gcaatgacag caccttcacc cagtaccgca 15180
gctggtacct tgcatacaac tacggcgacc ctcagaccgg aatccgctca tggaccctgc 15240
tttgcactcc tgacgtaacc tgcggctcgg agcaggtcta ctggtcgttg ccagacatga 15300
tgcaagaccc cgtgaccttc cgctccacgc gccagatcag caactttccg gtggtgggcg 15360
ccgagctgtt gcccgtgcac tccaagagct tctacaacga ccaggccgtc tactcccaac 15420
tcatccgcca gtttacctct ctgacccacg tgttcaatcg ctttcccgag aaccagattt 15480
tggcgcgccc gccagccccc accatcacca ccgtcagtga aaacgttcct gctctcacag 15540
atcacgggac gctaccgctg cgcaacagca tcggaggagt ccagcgagtg accattactg 15600
acgccagacg ccgcacctgc ccctacgttt acaaggccct gggcatagtc tcgccgcgcg 15660
tcctatcgag ccgcactttt tgagcaagca tgtccatcct tatatcgccc agcaataaca 15720
caggctgggg cctgcgcttc ccaagcaaga tgtttggcgg ggccaagaag cgctccgacc 15780
aacacccagt gcgcgtgcgc gggcactacc gcgcgccctg gggcgcgcac aaacgcggcc 15840
gcactgggcg caccaccgtc gatgacgcca tcgacgcggt ggtggaggag gcgcgcaact 15900
acacgcccac gccgccacca gtgtccacag tggacgcggc cattcagacc gtggtgcgcg 15960
gagcccggcg ctatgctaaa atgaagagac ggcggaggcg cgtagcacgt cgccaccgcc 16020
gccgacccgg cactgccgcc caacgcgcgg cggcggccct gcttaaccgc gcacgtcgca 16080
ccggccgacg ggcggccatg cgggccgctc gaaggctggc cgcgggtatt gtcactgtgc 16140
cccccaggtc caggcgacga gcggccgccg cagcagccgc ggccattagt gctatgactc 16200
agggtcgcag gggcaacgtg tattgggtgc gcgactcggt tagcggcctg cgcgtgcccg 16260
tgcgcacccg ccccccgcgc aactagattg caagaaaaaa ctacttagac tcgtactgtt 16320
gtatgtatcc agcggcggcg gcgcgcaacg aagctatgtc caagcgcaaa atcaaagaag 16380

agatgctcca ggtcatcgcg ccggagatct atggcccccc gaagaaggaa gagcaggatt 16440
acaagccccg aaagctaaag cgggtcaaaa agaaaaagaa agatgatgat gatgaacttg 16500
acgacgaggt ggaactgctg cacgctaccg cgcccaggcg acgggtacag tggaaaggtc 16560
gacgcgtaaa acgtgttttg cgacccggca ccaccgtagt ctttacgccc ggtgagcgct 16620
ccacccgcac ctacaagcgc gtgtatgatg aggtgtacgg cgacgaggac ctgcttgagc 16680
aggccaacga gcgcctcggg gagtttgcct acggaaagcg gcataaggac atgctggcgt 16740
tgccgctgga cgagggcaac ccaacaccta gcctaaagcc cgtaacactg cagcaggtgc 16800
tgcccgcgct tgcaccgtcc gaagaaaagc gcggcctaaa gcgcgagtct ggtgacttgg 16860
cacccaccgt gcagctgatg gtacccaagc gccagcgact ggaagatgtc ttggaaaaaa 16920
tgaccgtgga acctgggctg gagcccgagg tccgcgtgcg gccaatcaag caggtggcgc 16980
cgggactggg cgtgcagacc gtggacgttc agatacccac taccagtagc accagtattg 17040
ccaccgccac agagggcatg gagacacaaa cgtccccggt tgcctcagcg gtggcggatg 17100
ccgcggtgca ggcggtcgct gcggccgcgt ccaagacctc tacggaggtg caaacggacc 17160
cgtggatgtt tcgcgtttca gccccccggc gcccgcgccg ttcgaggaag tacggcgccg 17220
ccagcgcgct actgcccgaa tatgccctac atccttccat tgcgcctacc cccggctatc 17280
gtggctacac ctaccgcccc agaagacgag caactacccg acgccgaacc accactggaa 17340
cccgccgccg ccgtcgccgt cgccagcccg tgctggcccc gatttccgtg cgcagggtgg 17400
ctcgcgaagg aggcaggacc ctggtgctgc caacagcgcg ctaccacccc agcatcgttt 17460
aaaagccggt ctttgtggtt cttgcagata tggccctcac ctgccgcctc cgtttcccgg 17520
tgccgggatt ccgaggaaga atgcaccgta ggaggggcat ggccggccac ggcctgacgg 17580
gcggcatgcg tcgtgcgcac caccggcggc ggcgcgcgtc gcaccgtcgc atgcgcggcg 17640
gtatcctgcc cctccttatt ccactgatcg ccgcggcgat tggcgccgtg cccggaattg 17700
catccgtggc cttgcaggcg cagagacact gattaaaaac aagttgcatg tggaaaaatc 17760
aaaataaaaa gtctggactc tcacgctcgc ttggtcctgt aactattttg tagaatggaa 17820
gacatcaact ttgcgtctct ggccccgcga cacggctcgc gcccgttcat gggaaactgg 17880
caagatatcg gcaccagcaa tatgagcggt ggcgccttca gctggggctc gctgtggagc 17940
ggcattaaaa atttcggttc caccgttaag aactatggca gcaaggcctg gaacagcagc 18000
acaggccaga tgctgaggga taagttgaaa gagcaaaatt tccaacaaaa ggtggtagat 18060
ggcctggcct ctggcattag cggggtggtg gacctggcca accaggcagt gcaaaataag 18120
attaacagta agcttgatcc ccgccctccc gtagaggagc ctccaccggc cgtggagaca 18180
gtgtctccag aggggcgtgg cgaaaagcgt ccgcgccccg acagggaaga aactctggtg 18240
acgcaaatag acgagcctcc ctcgtacgag gaggcactaa agcaaggcct gcccaccacc 18300
cgtcccatcg cgcccatggc taccggagtg ctgggccagc acacacccgt aacgctggac 18360
ctgcctcccc ccgccgacac ccagcagaaa cctgtgctgc caggcccgac cgccgttgtt 18420
gtaacccgtc ctagccgcgc gtccctgcgc cgcgccgcca gcggtccgcg atcgttgcgg 18480
cccgtagcca gtggcaactg gcaaagcaca ctgaacagca tcgtgggtct gggggtgcaa 18540
tccctgaagc gccgacgatg cttctgatag ctaacgtgtc gtatgtgtgt catgtatgcg 18600
tccatgtcgc cgccagagga gctgctgagc cgccgcgcgc ccgctttcca agatggctac 18660

cccttcgatg atgccgcagt ggtcttacat gcacatctcg ggccaggacg cctcggagta 18720
cctgagcccc gggctggtgc agtttgcccg cgccaccgag acgtacttca gcctgaataa 18780
caagtttaga aaccccacgg tggcgcctac gcacgacgtg accacagacc ggtcccagcg 18840
tttgacgctg cggttcatcc ctgtggaccg tgaggatact gcgtactcgt acaaggcgcg 18900
gttcacccta gctgtgggtg ataaccgtgt gctggacatg gcttccacgt actttgacat 18960
ccgcggcgtg ctggacaggg gccctacttt taagccctac tctggcactg cctacaacgc 19020
cctggctccc aagggtgccc caaatccttg cgaatgggat gaagctgcta ctgctcttga 19080
aataaaccta gaagaagagg acgatgacaa cgaagacgaa gtagacgagc aagctgagca 19140
gcaaaaaact cacgtatttg ggcaggcgcc ttattctggt ataaatatta caaaggaggg 19200
tattcaaata ggtgtcgaag gtcaaacacc taaatatgcc gataaaacat ttcaacctga 19260
acctcaaata ggagaatctc agtggtacga aacagaaatt aatcatgcag ctgggagagt 19320
cctaaaaaag actaccccaa tgaaaccatg ttacggttca tatgcaaaac ccacaaatga 19380
aaatggaggg caaggcattc ttgtaaagca acaaaatgga aagctagaaa gtcaagtgga 19440
aatgcaattt ttctcaacta ctgaggcagc cgcaggcaat ggtgataact tgactcctaa 19500
agtggtattg tacagtgaag atgtagatat agaaacccca gacactcata tttcttacat 19560
gcccactatt aaggaaggta actcacgaga actaatgggc caacaatcta tgcccaacag 19620
gcctaattac attgctttta gggacaattt tattggtcta atgtattaca acagcacggg 19680
taatatgggt gttctggcgg gccaagcatc gcagttgaat gctgttgtag atttgcaaga 19740
cagaaacaca gagctttcat accagctttt gcttgattcc attggtgata gaaccaggta 19800
cttttctatg tggaatcagg ctgttgacag ctatgatcca gatgttagaa ttattgaaaa 19860
tcatggaact gaagatgaac ttccaaatta ctgctttcca ctgggaggtg tgattaatac 19920
agagactctt accaaggtaa aacctaaaac aggtcaggaa aatggatggg aaaaagatgc 19980
tacagaattt tcagataaaa atgaaataag agttggaaat aattttgcca tggaaatcaa 20040
tctaaatgcc aacctgtgga gaaatttcct gtactccaac atagcgctgt atttgcccga 20100
caagctaaag tacagtcctt ccaacgtaaa aatttctgat aacccaaaca cctacgacta 20160
catgaacaag cgagtggtgg ctcccgggct agtggactgc tacattaacc ttggagcacg 20220
ctggtccctt gactatatgg acaacgtcaa cccatttaac caccaccgca atgctggcct 20280
gcgctaccgc tcaatgttgc tgggcaatgg tcgctatgtg cccttccaca tccaggtgcc 20340
tcagaagttc tttgccatta aaaacctcct tctcctgccg ggctcataca cctacgagtg 20400
gaacttcagg aaggatgtta acatggttct gcagagctcc ctaggaaatg acctaagggt 20460
tgacggagcc agcattaagt ttgatagcat ttgcctttac gccaccttct tccccatggc 20520
ccacaacacc gcctccacgc ttgaggccat gcttagaaac gacaccaacg accagtcctt 20580
taacgactat ctctccgccg ccaacatgct ctaccctata cccgccaacg ctaccaacgt 20640
gcccatatcc atcccctccc gcaactgggc ggctttccgc ggctgggcct tcacgcgcct 20700
taagactaag gaaaccccat cactgggctc gggctacgac ccttattaca cctactctgg 20760
ctctataccc tacctagatg gaacctttta cctcaaccac acctttaaga aggtggccat 20820
tacctttgac tcttctgtca gctggcctgg caatgaccgc ctgcttaccc ccaacgagtt 20880
tgaaattaag cgctcagttg acggggaggg ttacaacgtt gcccagtgta acatgaccaa 20940

agactggttc ctggtacaaa tgctagctaa ctataacatt ggctaccagg gcttctatat 21000
cccagagagc tacaaggacc gcatgtactc cttctttaga aacttccagc ccatgagccg 21060
tcaggtggtg gatgatacta aatacaagga ctaccaacag gtgggcatcc tacaccaaca 21120
caacaactct ggatttgttg gctaccttgc ccccaccatg cgcgaaggac aggcctaccc 21180
tgctaacttc ccctatccgc ttataggcaa gaccgcagtt gacagcatta cccagaaaaa 21240
gtttctttgc gatcgcaccc tttggcgcat cccattctcc agtaacttta tgtccatggg 21300
cgcactcaca gacctgggcc aaaaccttct ctacgccaac tccgcccacg cgctagacat 21360
gacttttgag gtggatccca tggacgagcc cacccttctt tatgttttgt ttgaagtctt 21420
tgacgtggtc cgtgtgcacc agccgcaccg cggcgtcatc gaaaccgtgt acctgcgcac 21480
gcccttctcg gccggcaacg ccacaacata aagaagcaag caacatcaac aacagctgcc 21540
gccatgggct ccagtgagca ggaactgaaa gccattgtca aagatcttgg ttgtgggcca 21600
tatttttggg gcacctatga caagcgcttt ccaggctttg tttctccaca caagctcgcc 21660
tgcgccatag tcaatacggc cggtcgcgag actgggggcg tacactggat ggcctttgcc 21720
tggaacccgc actcaaaaac atgctacctc tttgagccct ttggcttttc tgaccagcga 21780
ctcaagcagg tttaccagtt tgagtacgag tcactcctgc gccgtagcgc cattgcttct 21840
tcccccgacc gctgtataac gctggaaaag tccacccaaa gcgtacaggg gcccaactcg 21900
gccgcctgtg gactattctg ctgcatgttt ctccacgcct ttgccaactg gccccaaact 21960
cccatggatc acaaccccac catgaacctt attaccgggg tacccaactc catgctcaac 22020
agtccccagg tacagcccac cctgcgtcgc aaccaggaac agctctacag cttcctggag 22080
cgccactcgc cctacttccg cagccacagt gcgcagatta ggagcgccac ttctttttgt 22140
cacttgaaaa acatgtaaaa ataatgtact agagacactt tcaataaagg caaatgcttt 22200
tatttgtaca ctctcgggtg attatttacc cccacccttg ccgtctgcgc cgtttaaaaa 22260
tcaaaggggt tctgccgcgc atcgctatgc gccactggca gggacacgtt gcgatactgg 22320
tgtttagtgc tccacttaaa ctcaggcaca accatccgcg gcagctcggt gaagttttca 22380
ctccacaggc tgcgcaccat caccaacgcg tttagcaggt cgggcgccga tatcttgaag 22440
tcgcagttgg ggcctccgcc ctgcgcgcgc gagttgcgat acacagggtt gcagcactgg 22500
aacactatca gcgccgggtg gtgcacgctg gccagcacgc tcttgtcgga gatcagatcc 22560
gcgtccaggt cctccgcgtt gctcagggcg aacggagtca actttggtag ctgccttccc 22620
aaaaagggcg cgtgcccagg ctttgagttg cactcgcacc gtagtggcat caaaaggtga 22680
ccgtgcccgg tctgggcgtt aggatacagc gcctgcataa aagccttgat ctgcttaaaa 22740
gccacctgag cctttgcgcc ttcagagaag aacatgccgc aagacttgcc ggaaaactga 22800
ttggccggac aggccgcgtc gtgcacgcag caccttgcgt cggtgttgga gatctgcacc 22860
acatttcggc cccaccggtt cttcacgatc ttggccttgc tagactgctc cttcagcgcg 22920
cgctgcccgt tttcgctcgt cacatccatt tcaatcacgt gctccttatt tatcataatg 22980
cttccgtgta gacacttaag ctcgccttcg atctcagcgc agcggtgcag ccacaacgcg 23040
cagcccgtgg gctcgtgatg cttgtaggtc acctctgcaa acgactgcag gtacgcctgc 23100
aggaatcgcc ccatcatcgt cacaaaggtc ttgttgctgg tgaaggtcag ctgcaacccg 23160
cggtgctcct cgttcagcca ggtcttgcat acggccgcca gagcttccac ttggtcaggc 23220

agtagtttga agttcgcctt tagatcgtta tccacgtggt acttgtccat cagcgcgcgc 23280
gcagcctcca tgcccttctc ccacgcagac acgatcggca cactcagcgg gttcatcacc 23340
gtaatttcac tttccgcttc gctgggctct tcctcttcct cttgcgtccg cataccacgc 23400
gccactgggt cgtcttcatt cagccgccgc actgtgcgct tacctccttt gccatgcttg 23460
attagcaccg gtgggttgct gaaacccacc atttgtagcg ccacatcttc tctttcttcc 23520
tcgctgtcca cgattacctc tggtgatggc gggcgctcgg gcttgggaga agggcgcttc 23580
tttttcttct tgggcgcaat ggccaaatcc gccgccgagg tcgatggccg cgggctgggt 23640
gtgcgcggca ccagcgcgtc ttgtgatgag tcttcctcgt cctcggactc gatacgccgc 23700
ctcatccgct tttttggggg cgcccgggga ggcggcggcg acggggacgg ggacgacacg 23760
tcctccatgg ttgggggacg tcgcgccgca ccgcgtccgc gctcgggggt ggtttcgcgc 23820
tgctcctctt cccgactggc catttccttc tcctataggc agaaaaagat catggagtca 23880
gtcgagaaga aggacagcct aaccgccccc tctgagttcg ccaccaccgc ctccaccgat 23940
gccgccaacg cgcctaccac cttccccgtc gaggcacccc cgcttgagga ggaggaagtg 24000
attatcgagc aggacccagg ttttgtaagc gaagacgacg aggaccgctc agtaccaaca 24060
gaggataaaa agcaagacca ggacaacgca gaggcaaacg aggaacaagt cgggcggggg 24120
gacgaaaggc atggcgacta cctagatgtg ggagacgacg tgctgttgaa gcatctgcag 24180
cgccagtgcg ccattatctg cgacgcgttg caagagcgca gcgatgtgcc cctcgccata 24240
gcggatgtca gccttgccta cgaacgccac ctattctcac cgcgcgtacc ccccaaacgc 24300
caagaaaacg gcacatgcga gcccaacccg cgcctcaact tctaccccgt atttgccgtg 24360
ccagaggtgc ttgccaccta tcacatcttt ttccaaaact gcaagatacc cctatcctgc 24420
cgtgccaacc gcagccgagc ggacaagcag ctggccttgc ggcagggcgc tgtcatacct 24480
gatatcgcct cgctcaacga agtgccaaaa atctttgagg gtcttggacg cgacgagaag 24540
cgcgcggcaa acgctctgca acaggaaaac agcgaaaatg aaagtcactc tggagtgttg 24600
gtggaactcg agggtgacaa cgcgcgccta gccgtactaa aacgcagcat cgaggtcacc 24660
cactttgcct acccggcact taacctaccc cccaaggtca tgagcacagt catgagtgag 24720
ctgatcgtgc gccgtgcgca gcccctggag agggatgcaa atttgcaaga acaaacagag 24780
gagggcctac ccgcagttgg cgacgagcag ctagcgcgct ggcttcaaac gcgcgagcct 24840
gccgacttgg aggagcgacg caaactaatg atggccgcag tgctcgttac cgtggagctt 24900
gagtgcatgc agcggttctt tgctgacccg gagatgcagc gcaagctaga ggaaacattg 24960
cactacacct ttcgacaggg ctacgtacgc caggcctgca agatctccaa cgtggagctc 25020
tgcaacctgg tctcctacct tggaattttg cacgaaaacc gccttgggca aaacgtgctt 25080
cattccacgc tcaagggcga ggcgcgccgc gactacgtcc gcgactgcgt ttacttattt 25140
ctatgctaca cctggcagac ggccatgggc gtttggcagc agtgcttgga ggagtgcaac 25200
ctcaaggagc tgcagaaact gctaaagcaa aacttgaagg acctatggac ggccttcaac 25260
gagcgctccg tggccgcgca cctggcggac atcattttcc ccgaacgcct gcttaaaacc 25320
ctgcaacagg gtctgccaga cttcaccagt caaagcatgt tgcagaactt taggaacttt 25380
atcctagagc gctcaggaat cttgcccgcc acctgctgtg cacttcctag cgactttgtg 25440
cccattaagt accgcgaatg ccctccgccg ctttggggcc actgctacct tctgcagcta 25500

gccaactacc ttgcctacca ctctgacata atggaagacg tgagcggtga cggtctactg 25560
gagtgtcact gtcgctgcaa cctatgcacc ccgcaccgct ccctggtttg caattcgcag 25620
ctgcttaacg aaagtcaaat tatcggtacc tttgagctgc agggtccctc gcctgacgaa 25680
aagtccgcgg ctccggggtt gaaactcact ccggggctgt ggacgtcggc ttaccttcgc 25740
aaatttgtac ctgaggacta ccacgcccac gagattaggt tctacgaaga ccaatcccgc 25800
ccgcctaatg cggagcttac cgcctgcgtc attacccagg gccacattct tggccaattg 25860
caagccatca acaaagcccg ccaagagttt ctgctacgaa agggacgggg ggtttacttg 25920
gacccccagt ccggcgagga gctcaaccca atccccccgc cgccgcagcc ctatcagcag 25980
cagccgcggg cccttgcttc ccaggatggc acccaaaaag aagctgcagc tgccgccgcc 26040
acccacggac gaggaggaat actgggacag tcaggcagag gaggttttgg acgaggagga 26100
ggaggacatg atggaagact gggagagcct agacgaggaa gcttccgagg tcgaagaggt 26160
gtcagacgaa acaccgtcac cctcggtcgc attcccctcg ccggcgcccc agaaatcggc 26220
aaccggttcc agcatggcta caacctccgc tcctcaggcg ccgccggcac tgcccgttcg 26280
ccgacccaac cgtagatggg acaccactgg aaccagggcc ggtaagtcca agcagccgcc 26340
gccgttagcc caagagcaac aacagcgcca aggctaccgc tcatggcgcg ggcacaagaa 26400
cgccatagtt gcttgcttgc aagactgtgg gggcaacatc tccttcgccc gccgctttct 26460
tctctaccat cacggcgtgg ccttcccccg taacatcctg cattactacc gtcatctcta 26520
cagcccatac tgcaccggcg gcagcggcag caacagcagc ggccacacag aagcaaaggc 26580
gaccggatag caagactctg acaaagccca agaaatccac agcggcggca gcagcaggag 26640
gaggagcgct gcgtctggcg cccaacgaac ccgtatcgac ccgcgagctt agaaacagga 26700
tttttcccac tctgtatgct atatttcaac agagcagggg ccaagaacaa gagctgaaaa 26760
taaaaaacag gtctctgcga tccctcaccc gcagctgcct gtatcacaaa agcgaagatc 26820
agcttcggcg cacgctggaa gacgcggagg ctctcttcag taaatactgc gcgctgactc 26880
ttaaggacta gtttcgcgcc ctttctcaaa tttaagcgcg aaaactacgt catctccagc 26940
ggccacaccc ggcgccagca cctgttgtca gcgccattat gagcaaggaa attcccacgc 27000
cctacatgtg gagttaccag ccacaaatgg gacttgcggc tggagctgcc caagactact 27060
caacccgaat aaactacatg agcgcgggac cccacatgat atcccgggtc aacggaatac 27120
gcgcccaccg aaaccgaatt ctcctggaac aggcggctat taccaccaca cctcgtaata 27180
accttaatcc ccgtagttgg cccgctgccc tggtgtacca ggaaagtccc gctcccacca 27240
ctgtggtact tcccagagac gcccaggccg aagttcagat gactaactca ggggcgcagc 27300
ttgcgggcgg ctttcgtcac agggtgcggt cgcccgggca gggtataact cacctgacaa 27360
tcagagggcg aggtattcag ctcaacgacg agtcggtgag ctcctcgctt ggtctccgtc 27420
cggacgggac atttcagatc ggcggcgccg gccgctcttc attcacgcct cgtcaggcaa 27480
tcctaactct gcagacctcg tcctctgagc cgcgctctgg aggcattgga actctgcaat 27540
ttattgagga gtttgtgcca tcggtctact ttaacccctt ctcgggacct cccggccact 27600
atccggatca atttattcct aactttgacg cggtaaagga ctcggcggac ggctacgact 27660
gaatgttaag tggagaggca gagcaactgc gcctgaaaca cctggtccac tgtcgccgcc 27720
acaagtgctt tgcccgcgac tccggtgagt tttgctactt tgaattgccc gaggatcata 27780

tcgagggccc ggcgcacggc gtccggctta ccgcccaggg agagcttgcc cgtagcctga 27840
ttcgggagtt tacccagcgc cccctgctag ttgagcggga caggggaccc tgtgttctca 27900
ctgtgatttg caactgtcct aaccctggat tacatcaaga tctttgttgc catctctgtg 27960
ctgagtataa taaatacaga aattaaaata tactggggct cctatcgcca tcctgtaaac 28020
gccaccgtct tcacccgccc aagcaaacca aggcgaacct tacctggtac ttttaacatc 28080
tctccctctg tgatttacaa cagtttcaac ccagacggag tgagtctacg agagaacctc 28140
tccgagctca gctactccat cagaaaaaac accaccctcc ttacctgccg ggaacgtacg 28200
agtgcgtcac cggccgctgc accacaccta ccgcctgacc gtaaaccaga ctttttccgg 28260
acagacctca ataactctgt ttaccagaac aggaggtgag cttagaaaac ccttagggta 28320
ttaggccaaa ggcgcagcta ctgtggggtt tatgaacaat tcaagcaact ctacgggcta 28380
ttctaattca ggtttctcta gaaatggacg gaattattac agagcagcgc ctgctagaaa 28440
gacgcagggc agcggccgag caacagcgca tgaatcaaga gctccaagac atggttaact 28500
tgcaccagtg caaaaggggt atcttttgtc tggtaaagca ggccaaagtc acctacgaca 28560
gtaataccac cggacaccgc cttagctaca agttgccaac caagcgtcag aaattggtgg 28620
tcatggtggg agaaaagccc attaccataa ctcagcactc ggtagaaacc gaaggctgca 28680
ttcactcacc ttgtcaagga cctgaggatc tctgcaccct tattaagacc ctgtgcggtc 28740
tcaaagatct tattcccttt aactaataaa aaaaaataat aaagcatcac ttacttaaaa 28800
tcagttagca aatttctgtc cagtttattc agcagcacct ccttgccctc ctcccagctc 28860
tggtattgca gcttcctcct ggctgcaaac tttctccaca atctaaatgg aatgtcagtt 28920
tcctcctgtt cctgtccatc cgcacccact atcttcatgt tgttgcagat gaagcgcgca 28980
agaccgtctg aagatacctt caaccccgtg tatccatatg acacggaaac cggtcctcca 29040
actgtgcctt ttcttactcc tccctttgta tcccccaatg ggtttcaaga gagtccccct 29100
ggggtactct ctttgcgcct atccgaacct ctagttacct ccaatggcat gcttgcgctc 29160
aaaatgggca acggcctctc tctggacgag gccggcaacc ttacctccca aaatgtaacc 29220
actgtgagcc cacctctcaa aaaaaccaag tcaaacataa acctggaaat atctgcaccc 29280
ctcacagtta cctcagaagc cctaactgtg gctgccgccg cacctctaat ggtcgcgggc 29340
aacacactca ccatgcaatc acaggccccg ctaaccgtgc acgactccaa acttagcatt 29400
gccacccaag gacccctcac agtgtcagaa ggaaagctag ccctgcaaac atcaggcccc 29460
ctcaccacca ccgatagcag tacccttact atcactgcct caccccctct aactactgcc 29520
actggtagct tgggcattga cttgaaagag cccatttata cacaaaatgg aaaactagga 29580
ctaaagtacg gggctccttt gcatgtaaca gacgacctaa acactttgac cgtagcaact 29640
ggtccaggtg tgactattaa taatacttcc ttgcaaacta aagttactgg agccttgggt 29700
tttgattcac aaggcaatat gcaacttaat gtagcaggag gactaaggat tgattctcaa 29760
aacagacgcc ttatacttga tgttagttat ccgtttgatg ctcaaaacca actaaatcta 29820
agactaggac agggccctct ttttataaac tcagcccaca acttggatat taactacaac 29880
aaaggccttt acttgtttac agcttcaaac aattccaaaa agcttgaggt taacctaagc 29940
actgccaagg ggttgatgtt tgacgctaca gccatagcca ttaatgcagg agatgggctt 30000
gaatttggtt cacctaatgc accaaacaca aatcccctca aaacaaaaat tggccatggc 30060

ctagaatttg attcaaacaa ggctatggtt cctaaactag gaactggcct tagttttgac 30120
agcacaggtg ccattacagt aggaaacaaa aataatgata agctaacttt gtggaccaca 30180
ccagctccat ctcctaactg tagactaaat gcagagaaag atgctaaact cactttggtc 30240
ttaacaaaat gtggcagtca aatacttgct acagtttcag ttttggctgt taaaggcagt 30300
ttggctccaa tatctggaac agttcaaagt gctcatctta ttataagatt tgacgaaaat 30360
ggagtgctac taaacaattc cttcctggac ccagaatatt ggaactttag aaatggagat 30420
cttactgaag gcacagccta tacaaacgct gttggattta tgcctaacct atcagcttat 30480
ccaaaatctc acggtaaaac tgccaaaagt aacattgtca gtcaagttta cttaaacgga 30540
gacaaaacta aacctgtaac actaaccatt acactaaacg gtacacagga aacaggagac 30600
acaactccaa gtgcatactc tatgtcattt tcatgggact ggtctggcca caactacatt 30660
aatgaaatat ttgccacatc ctcttacact ttttcataca ttgcccaaga ataaagaatc 30720
gtttgtgtta tgtttcaacg tgtttatttt tcaattgccc gggatcggtg atcaccgatc 30780
cagacatgat aagatacatt gatgagtttg gacaaaccac aactagaatg cagtgaaaaa 30840
aatgctttat ttgtgaaatt tgtgatgcta ttgctttatt tgtaaccatt ataagctgca 30900
ataaacaagt tcccggatcg cgatccggcc cgaggctgta gccgacgatg gtgcgccagg 30960
agagttgttg attcattgtt tgcctccctg ctgcggtttt tcaccgaagt tcatgccagt 31020
ccagcgtttt tgcagcagaa aagccgccga cttcggtttg cggtcgcgag tgaagatccc 31080
tttcttgtta ccgccaacgc gcaatatgcc ttgcgaggtc gcaaaatcgg cgaaattcca 31140
tacctgttca ccgacgacgg cgctgacgcg atcaaagacg cggtgataca tatccagcca 31200
tgcacactga tactcttcac tccacatgtc ggtgtacatt gagtgcagcc cggctaacgt 31260
atccacgccg tattcggtga tgataatcgg ctgatgcagt ttctcctgcc aggccagaag 31320
ttctttttcc agtaccttct ctgccgtttc caaatcgccg ctttggacat accatccgta 31380
ataacggttc aggcacagca catcaaagag atcgctgatg gtatcggtgt gagcgtcgca 31440
gaacattaca ttgacgcagg tgatcggacg cgtcgggtcg agtttacgcg ttgcttccgc 31500
cagtggcgcg aaatattccc gtgcaccttg cggacgggta tccggttcgt tggcaatact 31560
ccacatcacc acgcttgggt ggtttttgtc acgcgctatc agctctttaa tcgcctgtaa 31620
gtgcgcttgc tgagtttccc cgttgactgc ctcttcgctg tacagttctt tcggcttgtt 31680
gcccgcttcg aaaccaatgc ctaaagagag gttaaagccg acagcagcag tttcatcaat 31740
caccacgatg ccatgttcat ctgcccagtc gagcatctct tcagcgtaag ggtaatgcga 31800
ggtacggtag gagttggccc caatccagtc cattaatgcg tggtcgtgca ccatcagcac 31860
gttatcgaat cctttgccac gcaagtccgc atcttcatga cgaccaaagc cagtaaagta 31920
gaacggtttg tggttaatca ggaactgttc gcccttcact gccactgacc ggatgccgac 31980
gcgaagcggg tagatatcac actctgtctg gcttttggct gtgacgcaca gttcatagag 32040
ataaccttca cccggttgcc agaggtgcgg attcaccact tgcaaagtcc cgctagtgcc 32100
ttgtccagtt gcaaccacct gttgatccgc atcacgcagt tcaacgctga catcaccatt 32160
ggccaccacc tgccagtcaa cagacgcgtg gttacagtct tgcgcgacat gcgtcaccac 32220
ggtgatatcg tccacccagg tgttcggcgt ggtgtagagc attacgctgc gatggattcc 32280
ggcatagtta aagaaatcat ggaagtaaga ctgctttttc ttgccgtttt cgtcggtaat 32340

caccattccc ggcgggatag tctgccagtt cagttcgttg ttcacacaaa cggtgatacg 32400
tacacttttc ccggcaataa catacggcgt gacatcggct tcaaatggcg tatagccgcc 32460
ctgatgctcc atcacttcct gattattgac ccacactttg ccgtaatgag tgaccgcatc 32520
gaaacgcagc acgatacgct ggcctgccca acctttcggt ataaagactt cgcgctgata 32580
ccagacgttg cccgcataat tacgaatatc tgcatcggcg aactgatcgt taaaactgcc 32640
tggcacagca attgcccggc tttcttgtaa cgcgctttcc caccaacgct gatcaattcc 32700
acagttttcg cgatccagac tgaatgccca caggccgtcg agttttttga tttcacgggt 32760
tggggtttct acaggacgga ccatgcgttc gacctttctc ttcttttttg ggcccatgat 32820
ggcagatccg tatagtgagt cgtattagct ggttctttcc gcctcagaag ccatagagcc 32880
caccgcatcc ccagcatgcc tgctattgtc ttcccaatcc tcccccttgc tgtcctgccc 32940
caccccaccc cccagaatag aatgacacct actcagacaa tgcgatgcaa tttcctcatt 33000
ttattaggaa aggacagtgg gagtggcacc ttccagggtc aaggaaggca cgggggaggg 33060
gcaaacaaca gatggctggc aactagaagg cacagtcgag gctgatcagc gagctctaga 33120
tgcatgctcg agcggccgcc agtgtgatgg atatctgcag aattccagca cactggcggc 33180
cgttactagt ggatccgagc tcggtacccg gccgttataa caccactcga cacggcacca 33240
gctcaatcag tcacagtgta aaaaagggcc aagtgcagag cgagtatata taggactaaa 33300
aaatgacgta acggttaaag tccacaaaaa acacccagaa aaccgcacgc gaacctacgc 33360
ccagaaacga aagccaaaaa acccacaact tcctcaaatc gtcacttccg ttttcccacg 33420
ttacgtcact tcccatttta agaaaactac aattcccaac acatacaagt tactccgccc 33480
taaaacctac gtcacccgcc ccgttcccac gccccgcgcc acgtcacaaa ctccaccccc 33540
tcattatcat attggcttca atccaaaata aggtatatta ttgatgatg 33589

## Description

### CROSS-REFERENCE TO RELATED APPLICATIONS

This patent application claims the benefit of U.S. Provisional Patent Application No. 60/561,341, filed April 12, 2004.

### STATEMENT REGARDING

### FEDERALLY SPONSORED RESEARCH AND DEVELOPMENT

This invention was made in part with Government support under Cooperative Research and Development Agreement (CRADA) Number AI-1034, and amendments thereto, executed between GenVec, Inc. and the U.S. Public Health Service representing the National Institute of Allergy and Infectious Diseases. The Government may have certain rights in this invention.

### BACKGROUND OF THE INVENTION

The Centers for Disease Control and Prevention (CDC) estimate that in the United States, 850,000 to 950,000 people are living with HIV infection and approximately 25% are unaware of their infection (CDC, Morb. Mortal. Wkly. Rep., 52(47), 1145-8 (2003)). Worldwide, the rate of new HIV infections continues to increase at an unacceptably high level. Although new AIDS diagnoses and deaths have fallen significantly in developed countries since the advent of highly active antiretroviral therapy (HAART), in the developing world the HIV/AIDS epidemic continues to accelerate. The global impact of the epidemic is considerable. According to the Joint United Nations Programme on HIV/AIDS and the World Health Organization, as of the end of 2002, 40-42 million people were estimated to be living with HIV/AIDS, with 95% of the global total residing in the developing world (WHO, Treating 3 Million by 2005: The WHO Strategy, Geneva, Switzerland. p. 1-53 (2003), and UNAIDS, AIDS Epidemic Update December 2003). Worldwide there were an estimated 2.5-3.5 million deaths due to HIV/AIDS in 2003 (UNAIDS, AIDS Epidemic Update December 2003) and there have been as many as 30 million deaths as a result of HIV infection since the beginning of the epidemic (WHO, Treating 3 Million by 2005: The WHO Strategy, Geneva, Switzerland. p. 1-53 (2003)). Beyond the human tragedy of HIV/AIDS, the costs of the epidemic pose a significant impediment to the economic growth and Political stability of many countries. In developing countries and in segments of the U.S. population, anti-HIV therapies are frequently beyond financial reach. Accordingly, effective, low-cost tools for HIV prevention, such as a vaccine, are urgently needed to bring the HIV epidemic under control.

Delivery of proteins as therapeutics or for inducing an immune response in biologically relevant amounts has been an obstacle to drug and vaccine development for decades. One solution that has proven to be a successful alternative to traditional antigen delivery approaches is delivery of exogenous nucleic acid sequences for production of antigenic molecules *in vivo.* Gene transfer vectors ideally enter a wide variety of cell types, have the capacity to accept large nucleic acid sequences, are safe, and can be produced in quantities required for treating patients. Viral vectors have these advantageous properties and are used in a variety of protocols to treat or prevent biological disorders.

Despite their advantageous properties, widespread use of viral gene transfer vectors is hindered by several factors. In this regard, certain cells are not readily amenable to gene delivery by currently available viral vectors. For example, lymphocytes are impaired in the uptake of adenoviruses (Silver et al., Virology 165, 377-387 (1988); Horvath et al., J. Virology, 62(1), 341-345 (1988)).

The use of viral gene transfer vectors also is impeded by the immunogenicity of viral vectors. A majority of the U.S. population has been exposed to wild-type forms of many of the viruses currently under development as gene transfer vectors (e.g., adenovirus). As a result, much of the U.S. population has developed pre-existing immunity to certain virus-based gene transfer vectors. Such vectors are quickly cleared from the bloodstream, thereby reducing the effectiveness of the vector in delivering biologically relevant amounts of a gene product. Moreover, the immunogenicity of certain viral vectors prevents efficient repeat dosing, which can be advantageous for "boosting" the immune system against pathogens, and results in only a small fraction of a dose of the viral vector delivering its payload to host cells.

In addition, a major challenge in the design of viral vectors as HIV vaccines is to identify and target viral structures that are the critical determinants for protective humoral and cellular immune responses across the widest possible range of diversity. The use of multivalent vaccines, containing a defined mixture of immunogens from a number of prevalent HIV subtypes, might be a feasible approach to achieve broadly protective HIV vaccines.

Thus, there remains a need for improved methods and compositions for inducing immune responses against HIV. The invention provides such a method and composition. These and other advantages of the invention, as well as additional inventive features, will be apparent from the description of the invention provided herein.

### BRIEF SUMMARY OF THE INVENTION

The invention provides a method of inducing an immune response against a human immunodeficiency virus (HIV) in a mammal. The method comprises administering to the mammal an adenoviral vector composition, wherein the adenoviral vector composition comprises one or more adenoviral vectors encoding two or more different HIV antigens, whereupon the HIV antigens are produced in the mammal and an immune response against HIV is induced.

The invention also provides an adenoviral vector composition comprising (a) an adenoviral vector comprising a nucleic acid encoding an HIV clade A Env protein, (b) an adenoviral vector comprising a nucleic acid encoding an HIV clade B Env protein, (c) an adenoviral vector comprising a nucleic acid encoding an HIV clade C Env protein, and (d) an adenoviral vector comprising a nucleic acid encoding a fusion protein comprising an HIV clade B Gag protein and Pol protein.

### DETAILED DESCRIPTION OF THE INVENTION

The invention provides a method of inducing an immune response against a human immunodeficiency virus (HIV) in a mammal. The method comprises administering to the mammal an adenoviral vector composition, wherein the adenoviral vector composition comprises one or more adenoviral vectors encoding two or more different HIV antigens.

The invention also provides an adenoviral vector composition. The adenoviral vector composition comprises (a) an adenoviral vector comprising a nucleic acid encoding an HIV clade A Env protein, (b) an adenoviral vector comprising a nucleic acid encoding an HIV clade B Env protein, (c) an adenoviral vector comprising a nucleic acid encoding an HIV clade C Env protein, and (d) an adenoviral vector comprising a nucleic acid encoding a fusion protein comprising an HIV clade B Gag protein and Pol protein

An "antigen" is a molecule that triggers an immune response in a mammal. An "immune response" can entail, for example, antibody production and/or the activation of immune effector cells. An HIV antigen in the context of the invention can comprise any proteinaceous HIV molecule or portion thereof that provokes an immune response in mammal. An "HIV molecule" is a molecule that is a part of a human immunodeficiency virus, is encoded by a nucleic acid sequence of a human immunodeficiency virus, or is derived from or synthetically based upon any such molecule. Administration of an HIV antigen that provokes an immune response in accordance with the invention preferably leads to protective immunity against HIV. In this regard, an "immune response" to HIV is an immune response to any one or more HIV antigens.

Examples of suitable HIV antigens include all or part of an HIV Gag, Env, Pol, Tat, Reverse Transcriptase (RT), Vif, Vpr, Vpu, Vpo, Integrase, or Nef proteins. Preferably, each of the two or more HIV antigens comprises all or part of an HIV Gag, Env, and/or Pol protein. Suitable Env proteins are known in the art and include, for example, gp160, gp120, gp41, gp145, and gp140. In addition, an HIV antigen can be a modified Env protein that exhibits enhanced immunogenicity *in vivo.* For example, the antigen can be an Env protein comprising mutations in the cleavage site, fusion peptide, or interhelical coiled-coil domains of the Env protein (ΔCFI Env proteins) (see, e.g., Cao et al., J. Virol., 71, 9808-9812 (1997), and Yang et al., J. Virol., 78, 4029-4036 (2004)).

Any clade of HIV is appropriate for antigen selection, including HIV clades A, B, C, D, E, MN, and the like. Thus, it will be appreciated that the following HIV antigens can be used in the inventive method: HIV clade A gp140, Gag, Env, and/or Pol; HIV clade B gp140, Gag, Env, and/or Pol proteins; HIV clade C gp140, Gag, Env, and/or Pol proteins; and HIV clade MN gp140, Gag, Env, and/or Pol proteins. While it is preferred that the antigen is a Gag, Env, and/or Pol protein, any HIV protein or portion thereof capable of inducing an immune response in a mammal can be used in connection with the inventive method. HIV Gag, Env, and Pol proteins from the different HIV clades (e.g., HIV clades A, B, C, MN, etc.), as well as nucleic acid sequences encoding such proteins and methods for the manipulation and insertion of such nucleic acid sequences into vectors, are known (see, e.g., HIV Sequence Compendium, Division of AIDS, National Institute of Allergy and Infectious Diseases (2003), HIV Sequence Database (http://hiv-web.lanl.gov/content/hiv-db/mainpage.html), Sambrook et al., Molecular Cloning, a Laboratory Manual, 2d edition, Cold Spring Harbor Press, Cold Spring Harbor, N.Y. (1989), and Ausubel et al., Current Protocols in Molecular Biology, Greene Publishing Associates and John Wiley & Sons, New York, N.Y. (1994)).

It will be appreciated that an entire, intact HIV protein is not required to produce an immune response. Indeed, most antigenic epitopes of HIV proteins are relatively small in size. Thus, fragments (e.g., epitopes or other antigenic fragments) of an HIV protein, such as any of the HIV proteins described herein, can be used as an HIV antigen. Antigenic fragments and epitopes of the HIV Gag, Env, and Pol proteins, as well as nucleic acid sequences encoding such antigenic fragments and epitopes, are known (see, e.g., HIV Immunology and HIV/SIV Vaccine Databases, Vol. 1, Division of AIDS, National Institute of Allergy and Infectious Diseases (2003)).

HIV antigens also include fusion proteins and polyproteins. A fusion protein can comprise one or more antigenic HIV protein fragments (e.g., epitopes) fused to one another, or fused to all or part of a different HIV protein or other polypeptide. The fusion protein can comprise all or part of any of the HIV antigens described herein. For example, all or part of an HIV Env protein (e.g., gp120 or gp 160), can be fused to all or part of the HIV Pol protein, or all or part of HIV Gag protein can be fused to all or part of the HIV Pol protein. Such fusion proteins effectively provide multiple HIV antigens in the context of the invention, and can be used to generate a more complete immune response against a given HIV pathogen as compared to that generated by a single HIV antigen. Similarly, polyproteins also can provide multiple HIV antigens. Polyproteins useful in conjunction with the invention include those that provide two or more HIV antigens, such as two or more of any of the HIV antigens described herein. Delivery of fusion proteins or polyproteins via adenoviral vector to a mammal allows exposure of an immune system to multiple antigens using a single nucleic acid sequence and, thus, conveniently allows a single composition to provide immunity against multiple HIV antigens or multiple epitopes of a single antigen. Nucleic acid sequences encoding fusion proteins and polyproteins of HIV antigens can be prepared and inserted into vectors by known methods (see, e.g., U.S. Patents 5,130,247 and 5,130,248, Sambrook et al., *supra,* and Ausubel et al., *supra*).

The adenoviral vector composition comprises one or more adenoviral vectors encoding two or more different HIV antigens. It is understood that adenoviral vectors "encode" an antigen by way of a nucleic acid sequence that has been inserted into the adenoviral vector. HIV antigens are "different" if they comprise a different antigenic amino acid sequence. The two or more different HIV antigens can be any HIV antigens, such as two or more of the HIV antigens described herein. Preferably, the adenoviral vector composition comprises one or more adenoviral vectors encoding three or more, or even four or more different HIV antigens. It will be appreciated that exposing the immune system of a mammal to a "cocktail" of different HIV antigens can elicit a broader and more effective immune response than exposing the immune system to only a single HIV antigen.

The two or more different HIV antigens can be provided by two or more antigens from different HIV proteins (e.g., HIV Gag, Env, Pol, etc.) or different HIV clades (e.g., HIV clades A, B, C, D, E, MN, etc.). For example, an HIV Gag protein and Pol protein are different antigens. Similarly, HIV clade A Env protein and an HIV clade B Env protein are different HIV antigens. Preferably, the two or more different HIV antigens comprise HIV antigens from two or more different HIV clades. More preferably the adenoviral vector composition comprises adenoviral vectors encoding three or more different HIV antigens from three or more different HIV clades, or even four or more different HIV antigens from four or more different HIV clades. Alternatively, at least one of the two or more HIV antigens can be a chimeric antigen, which comprises amino acid sequences derived from the same antigen obtained from two or more different HIV clades. For example, a chimeric Env protein can comprise a portion of an Env amino acid sequence obtained from a clade A HIV and a portion of an Env amino acid sequence obtained from a clade B HIV.

The adenoviral vector composition can be provided, for example, by a composition comprising one or more adenoviral vectors (e.g., a single adenoviral vector) that each encode two or more different HIV antigens, or by a composition that comprises two or more adenoviral vectors (e.g., multiple adenoviral vectors) that each encode one or more different HIV antigens and, thereby, collectively encode two or more different HIV antigens. When the adenoviral vector composition comprises one or more adenoviral vectors (e.g., a single adenoviral vector) that each encode two or more HIV antigens, each adenoviral vector can comprise (i) a nucleic acid sequence that encodes two or more different HIV antigens (e.g., a Polyprotein or fusion protein), or (ii) two or more nucleic acid sequences that each encode a different HIV antigen. Consistent with configuration (i), it is within the scope of the invention two use an adenoviral vector comprising a nucleic acid sequence that encodes more than two different HIV antigens (e.g., three or more, four or more, or even five or more different HIV antigens) or encodes multiple copies of the same antigen, provided that it encodes at least two or more different HIV antigens. Likewise, consistent with configuration (ii), it is within the scope of the invention to use an adenoviral vector comprising several nucleic acid sequences (e.g., three or more, four or more, or even five or more different nucleic acid sequences) each encoding different HIV antigens or multiple copies of the same antigen, provided that the adenoviral vector encodes at least two different HIV antigens. Whether by configuration (i) or (ii), the adenoviral vector composition preferably comprises one or more adenoviral vectors encoding three or more, or even four or more, different HIV antigens (e.g., wherein each vector comprises a nucleic acid sequence that encodes three or more, or four or more different HIV antigens, or wherein each vector comprises three or more, or four or more nucleic acid sequences, and each nucleic acid sequence encodes a different HIV antigen). Desirably, the two or more, three or more, or four or more different HIV antigens are from two or more, three or more, or four or more different HIV clades.

Preferably, the adenoviral vector composition comprises two or more adenoviral vectors encoding the two or more different HIV antigens, and each adenoviral vector comprises a nucleic acid sequence that encodes at least one of the two or more different HIV antigens. Although the adenoviral vector composition comprises two or more adenoviral vectors encoding two or more different HIV antigens, there is no upper limit to the number of adenoviral vectors use or the number of different HIV antigens encoded thereby. Preferably, the adenoviral vector composition comprises three or more adenoviral vectors encoding three or more different HIV antigens, and each adenoviral vector comprises a nucleic acid sequence that encodes at least one of the three or more different HIV antigens. Most preferably, the adenoviral vector composition comprises four or more adenoviral vectors encoding four or more different HIV antigens, and each adenoviral vector comprises a nucleic acid sequence that encodes at least one of the four or more different HIV antigens. Desirably, the two or more, three or more, or four or more different HIV antigens are from two or more, three or more, or four or more different HIV clades.

Of course, a combination of the above configurations of adenoviral vectors can be used without departing from the spirit and scope of the invention. For example, the adenoviral vector composition used in accordance with the invention can comprise a first adenoviral vector encoding a single HIV antigen and a second adenoviral vector encoding two or more HIV antigens that are different from the HIV antigen encoded by the first adenoviral vector. Other similar combinations and permutations of the adenoviral vector configurations disclosed herein are apparent and can be used in accordance with the invention.

When the adenoviral vector composition comprises two or more adenoviral vectors, the relative amount of each of the two or more adenoviral vectors included in the composition will depend upon a number of factors, including the immunogenicity of a particular HIV antigen compared to the other HIV antigens. The adenoviral vector composition can comprise equal amounts of each of the two or more adenoviral vectors. Alternatively, the adenoviral vector composition can comprise different amounts of each of the two or more adenoviral vectors.

In a particularly preferred embodiment of the invention, the adenoviral vector composition comprises four adenoviral vectors each comprising a nucleic acid sequence encoding a clade B Gag-Pol fusion protein, clade A gp140, clade B gp140, and clade C gp140, respectively. Most preferably, the adenoviral vector composition comprises four adenoviral vectors having the nucleic acid sequence of SEQ ID NO: 4, SEQ ID NO: 5, SEQ ID NO: 6, and SEQ ID NO: 7. SEQ ID NO: 4 is the nucleic acid sequence of an E1/E4-deficient adenoviral vector encoding a clade B Gag-Pol fusion protein. SEQ ID NO: 5 is the nucleic acid sequence of an E1/E4-deficient adenoviral vector encoding a clade A gp140 protein. SEQ ID NO: 6 is the nucleic acid sequence of an E1/E4-deficient adenoviral vector encoding a clade B gp140 protein. SEQ ID NO: 7 is the nucleic acid sequence of a clade C gp140 protein. Desirably, the adenoviral vector composition comprises the following adenoviral vectors in a 3:1:1:1 ratio by weight, respectively: an adenoviral vector comprising a nucleic acid sequence encoding clade B Gag-Pol fusion protein, an adenoviral vector comprising a nucleic acid sequence encoding clade A gp140, an adenoviral vector comprising a nucleic acid sequence encoding clade B gp140, and an adenoviral vector comprising a nucleic acid sequence encoding clade C gp140.

Typically, the adenoviral vector comprises a nucleic acid encoding one or more HIV antigens as part of an expression cassette, i.e., a particular nucleotide sequence that possesses functions which facilitate subcloning and recovery of a nucleic acid sequence (e.g., one or more restriction sites) or expression of a nucleic acid sequence (e.g., Polyadenylation or splice sites). The nucleic acid preferably is located in the E1 region (e.g., replaces the E1 region in whole or in part) or the E4 region of the adenoviral genome. For example, the E1 region can be replaced by a promoter-variable expression cassette comprising a nucleic acid encoding an antigen. The expression cassette optionally can be inserted in a 3'-5' orientation, e.g., oriented such that the direction of transcription of the expression cassette is opposite that of the surrounding adjacent adenoviral genome. However, it is also appropriate for the expression cassette to be inserted in a 5'-3' orientation with respect to the direction of transcription of the surrounding genome. In addition to the expression cassette comprising the nucleic acid encoding an antigen, the adenoviral vector can comprise other expression cassettes containing other exogenous nucleic acids, which cassettes can replace any of the deleted regions of the adenoviral genome. The insertion of an expression cassette into the adenoviral genome (e.g., into the E1 region of the genome) can be facilitated by known methods, for example, by the introduction of a unique restriction site at a given position of the adenoviral genome. As set forth above, preferably all or part of the E3 region of the adenoviral vector also is deleted.

Preferably, the antigen-encoding nucleic acid is operably linked to (i.e., under the transcriptional control of) one or more promoter and/or enhancer elements, for example, as part of a promoter-variable expression cassette. Techniques for operably linking sequences together are well known in the art. A "promoter" is a DNA sequence that directs the binding of RNA Polymerase and thereby promotes RNA synthesis. A nucleic acid sequence is "operably linked" to a promoter when the promoter is capable of directing transcription of that nucleic acid sequence. A promoter can be native or non-native to the nucleic acid sequence to which it is operably linked.

Any promoter (i.e., whether isolated from nature or produced by recombinant DNA or synthetic techniques) can be used in connection with the invention to provide for transcription of the nucleic acid sequence. The promoter preferably is capable of directing transcription in a eukaryotic (desirably mammalian) cell. The functioning of the promoter can be altered by the presence of one or more enhancers and/or silencers present on the vector. "Enhancers" are cis-acting elements of DNA that stimulate or inhibit transcription of adjacent genes. An enhancer that inhibits transcription also is termed a "silencer." Enhancers differ from DNA-binding sites for sequence-specific DNA binding proteins found only in the promoter (which also are termed "promoter elements") in that enhancers can function in either orientation, and over distances of up to several kilobase pairs (kb), even from a position downstream of a transcribed region.

Promoter regions can vary in length and sequence and can further encompass one or more DNA binding sites for sequence-specific DNA binding proteins and/or an enhancer or silencer. Enhancers and/or silencers can similarly be present on a nucleic acid sequence outside of the promoter per se. Desirably, a cellular or viral enhancer, such as the cytomegalovirus (CMV) immediate-early enhancer, is positioned in the proximity of the promoter to enhance promoter activity. In addition, splice acceptor and donor sites can be present on a nucleic acid sequence to enhance transcription.

Any suitable promoter or enhancer sequence can be used in the context of the invention. In this respect, the antigen-encoding nucleic acid sequence can be operably linked to a viral promoter. Suitable viral promoters include, for instance, cytomegalovirus (CMV) promoters, such as the CMV immediate-early promoter (described in, for example, U.S. Patents 5,168,062 and 5,385,839), promoters derived from human immunodeficiency virus (HIV), such as the HIV long terminal repeat promoter, Rous sarcoma virus (RSV) promoters, such as the RSV long terminal repeat, mouse mammary tumor virus (MMTV) promoters, HSV promoters, such as the Lap2 promoter or the herpes thymidine kinase promoter (Wagner et al., Proc. Natl. Acad. Sci., 78, 144-145 (1981)), promoters derived from SV40 or Epstein Barr virus, an adeno-associated viral promoter, such as the p5 promoter, and the like.

Alternatively, the invention employs a cellular promoter, i.e., a promoter that drives expression of a cellular protein. Preferred cellular promoters for use in the invention will depend on the desired expression profile to produce the antigen(s). In one aspect, the cellular promoter is preferably a constitutive promoter that works in a variety of cell types, such as immune cells described herein. Suitable constitutive promoters can drive expression of genes encoding transcription factors, housekeeping genes, or structural genes common to eukaryotic cells. For example, the Ying Yang 1 (YY1) transcription factor (also referred to as NMP-1, NF-E1, and UCRBP) is a ubiquitous nuclear transcription factor that is an intrinsic component of the nuclear matrix (Guo et al., PNAS, 92, 10526-10530 (1995)). While the promoters described herein are considered constitutive promoters, it is understood in the art that constitutive promoters can be upregulated. Promoter analysis shows that the elements critical for basal transcription reside from -277 to +475 of the YY1 gene relative to the transcription start site from the promoter, and include a TATA and CCAAT box. JEM-1 (also known as HGMW and BLZF-1) also is a ubiquitous nuclear transcription factor identified in normal and tumorous tissues (Tong et al., Leukemia, 12(11), 1733-1740 (1998), and Tong et al., Genomics, 69(3), 380-390 (2000)). JEM-1 is involved in cellular growth control and maturation, and can be upregulated by retinoic acids. Sequences responsible for maximal activity of the JEM-1 promoter has been located at -432 to +101 of the JEM-1 gene relative the transcription start site of the promoter. Unlike the YY1 promoter, the JEM-1 promoter does not comprise a TATA box. The ubiquitin promoter, specifically UbC, is a strong constitutively active promoter functional in several species. The UbC promoter is further characterized in Marinovic et al., J. Biol. Chem., 277(19), 16673-16681 (2002).

Many of the above-described promoters are constitutive promoters. Instead of being a constitutive promoter, the promoter can be a regulatable promoter, i.e., a promoter that is up- and/or down-regulated in response to appropriate signals. The use of a regulatable promoter or expression control sequence is particularly applicable to DNA vaccine development as antigenic proteins, including viral and parasite antigens, frequently are toxic to complementing cell lines. In one embodiment, the regulatory sequences operably linked to the antigen-encoding nucleic acid sequence include components of the tetracycline expression system, e.g., tet operator sites. For instance, the antigen-encoding nucleic acid sequence is operably linked to a promoter which is operably linked to one or more tet operator sites. An adenoviral vector comprising such an expression cassette can be propagated in a complementing cell line, such as 293-ORF6 described in, for example, U.S. Patent 5,994,106 and International Patent Application Publication WO 95/34671, which comprises a nucleic acid sequence encoding a tet repressor protein. By producing the tet repressor protein in the complementing cell line, antigen production is inhibited and propagation proceeds without any associated antigen-mediated toxicity. Suitable regulatable promoter systems also include, but are not limited to, the IL-8 promoter, the metallothionine inducible promoter system, the bacterial lacZYA expression system, and the T7 Polymerase system. Further, promoters that are selectively activated at different developmental stages (e.g., globin genes are differentially transcribed from globin-associated promoters in embryos and adults) can be employed. The promoter sequence can contain at least one regulatory sequence responsive to regulation by an exogenous agent. The regulatory sequences are preferably responsive to exogenous agents such as, but not limited to, drugs, hormones, radiation, or other gene products.

The promoter can be a tissue-specific promoter, i.e., a promoter that is preferentially activated in a given tissue and results in expression of a gene product in the tissue where activated. A tissue-specific promoter suitable for use in the invention can be chosen by the ordinarily skilled artisan based upon the target tissue or cell-type. Preferred tissue-specific promoters for use in the inventive method are specific to immune cells, such as the dendritic-cell specific Dectin-2 promoter described in Morita et al., Gene Ther., 8, 1729-37 (2001).

In yet another embodiment, the promoter can be a chimeric promoter. A promoter is "chimeric" in that it comprises at least two nucleic acid sequence portions obtained from, derived from, or based upon at least two different sources (e.g., two different regions of an organism's genome, two different organisms, or an organism combined with a synthetic sequence). Preferably, the two different nucleic acid sequence portions exhibit less than about 40%, more preferably less than about 25%, and even more preferably less than about 10% nucleic acid sequence identity to one another (which can be determined by methods described elsewhere herein). Any suitable chimeric promoter can be used in the inventive method.

A promoter can be selected for use in the invention by matching its particular pattern of activity with the desired pattern and level of expression of the antigen(s). For example, in embodiments where the adenoviral vector comprises two or more nucleic acid sequences that encode different antigens, each nucleic acid sequence can be operably linked to different promoters displaying distinct expression profiles. For example, a first promoter is selected to mediate an initial peak of antigen production, thereby priming the immune system against an encoded antigen. A second promoter is selected to drive production of the same or different antigen such that expression peaks several days after that of the first promoter, thereby "boosting" the immune system against the antigen. Alternatively, a chimeric promoter can be constructed which combines the desirable aspects of multiple promoters. For example, a CMV-RSV hybrid promoter combining the CMV promoter's initial rush of activity with the RSV promoter's high maintenance level of activity is especially preferred for use in many embodiments of the inventive method. In that antigens can be toxic to eukaryotic cells, it may be advantageous to modify the promoter to decrease activity in complementing cell lines used to propagate the adenoviral vector.

To optimize protein production, preferably the antigen-encoding nucleic acid sequence further comprises a polyadenylation site following the coding sequence of the antigen-encoding nucleic acid sequence. Any suitable polyadenylation sequence can be used, including a synthetic optimized sequence, as well as the polyadenylation sequence of BGH (Bovine Growth Hormone), polyoma virus, TK (Thymidine Kinase), EBV (Epstein Barr Virus), and the papillomaviruses, including human papillomaviruses and BPV (Bovine Papilloma Virus). A preferred polyadenylation sequence is the SV40 (Human Sarcoma Virus-40) polyadenylation sequence. Also, preferably all the proper transcription signals (and translation signals, where appropriate) are correctly arranged such that the nucleic acid sequence is properly expressed in the cells into which it is introduced. If desired, the nucleic acid sequence also can incorporate splice sites (i.e., splice acceptor and splice donor sites) to facilitate mRNA production.

If the antigen-encoding nucleic acid sequence encodes a processed or secreted protein or peptide, or a protein that acts intracellularly, preferably the antigen-encoding nucleic acid sequence further comprises the appropriate sequences for processing, secretion, intracellular localization, and the like. The antigen-encoding nucleic acid sequence can be operably linked to a signal sequence, which targets a protein to cellular machinery for secretion. Appropriate signal sequences include, but are not limited to, leader sequences for immunoglobulin heavy chains and cytokines, (see, for example, Ladunga, Current Opinions in Biotechnology, 11, 13-18 (2000)). Other protein modifications can be required to secrete a protein from a host cell, which can be determined using routine laboratory techniques. Preparing expression constructs encoding antigens and signal sequences is further described in, for example, U.S. Patent 6,500,641. Methods of secreting non-secretable proteins are further described in, for example, U.S. Patent 6,472,176, and International Patent Application Publication WO 02/48377.

An antigen protein encoded by the nucleic acid sequence of the adenoviral vector also can be modified to attach or incorporate the antigen on the host cell surface. In this respect, the antigen can comprise a membrane anchor, such as a gpi-anchor, for conjugation onto the cell surface. A transmembrane domain can be fused to the antigen to incorporate a terminus of the antigen protein into the cell membrane. Other strategies for displaying peptides on a cell surface are known in the art and are appropriate for use in the context of the invention.

In accordance with the invention, the adenoviral vector composition is administered to an animal, preferably a mammal (e.g., a human), wherein each antigen-encoding nucleic acid sequence is expressed to induce an immune response against the antigen. The immune response can be a humoral immune response, a cell-mediated immune response, or, desirably, a combination of humoral and cell-mediated immunity. Ideally, the immune response provides protection upon subsequent challenge with the infectious agent comprising the antigen. However, protective immunity is not required in the context of the invention. The inventive method further can be used for antibody production and harvesting.

To enhance the immune response generated against an HIV antigen, the adenoviral vector composition can also comprise a nucleic acid sequence that encodes an immune stimulator, such as a cytokine, a chemokine, or a chaperone. Cytokines include, for example, Macrophage Colony Stimulating Factor (e.g., GM-CSF), Interferon Alpha (IFN-α), Interferon Beta (IFN-β), Interferon Gamma (IFN-γ), interleukins (IL-1, IL-2, IL-4, IL-5, IL-6, IL-8, IL-10, IL-12, IL-13, IL-15, IL-16, and IL-18), the TNF family of proteins, Intercellular Adhesion Molecule-1 (ICAM-1), Lymphocyte Function-Associated antigen-3 (LFA-3), B7-1, B7-2, FMS-related tyrosine kinase 3 ligand, (Flt3L), vasoactive intestinal peptide (VIP), and CD40 ligand. Chemokines include, for example, B Cell-Attracting chemokine-1 (BCA-1), Fractalkine, Melanoma Growth Stimulatory Activity protein (MGSA), Hemofiltrate CC chemokine 1 (HCC-1), Interleukin 8 (IL8), Interferon-stimulated T-cell alpha chemoattractant (I-TAC), Lymphotactin, Monocyte Chemotactic Protein 1 (MCP-1), Monocyte Chemotactic Protein 3 (MCP-3), Monocyte Chemotactic Protein 4 (MCP-4), Macrophage-Derived Chemokine (MDC), a macrophage inflammatory protein (MIP), Platelet Factor 4 (PF4), RANTES, BRAK, eotaxin, exodus 1-3, and the like. Chaperones include, for example, the heat shock proteins Hsp170, Hsc70, and Hsp40. Cytokines and chemokines are generally described in the art, including the Invivogen catalog (2002), San Diego, CA.

Administration of the adenoviral vector composition can be one component of a multistep regimen for inducing an immune response against HIV in a mammal. In this respect, the inventive method further comprises administering to the mammal a primer composition comprising one or more nucleic acid sequences that encode at least one HIV antigen that is the same as an HIV antigen encoded by an adenoviral vector of the adenoviral vector composition, wherein the administration of the primer composition is performed at least one week before the administration of the adenoviral vector composition. Thus, this embodiment of the invention represents one arm of a prime and boost immunization regimen, wherein an immune response is "primed" by administration of the primer composition, and is "boosted" by administration of the adenoviral vector composition. The one or more nucleic acid sequences of the primer composition can be administered as part of a gene transfer vector or as naked DNA. Any gene transfer vector can be employed in the primer composition, including viral and non-viral gene transfer vectors. Examples of suitable viral gene transfer vectors include, but are not limited to, retroviral vectors, adeno-associated virus vectors, vaccinia virus vectors, herpesvirus vectors, or adenoviral vectors. Examples of suitable non-viral vectors include, but are not limited to, plasmids, liposomes, and molecular conjugates (e.g., transferrin). Ideally, the gene transfer vector is a plasmid or an adenoviral vector. Alternatively, an immune response can be primed or boosted by administration of the antigen itself, e.g., an antigenic protein, inactivated pathogen, and the like.

While the antigen encoded by the one or more nucleic acid sequences of the primer composition preferably is the same as an HIV antigen encoded by an adenoviral vector of the adenoviral vector composition, in some embodiments it may be appropriate to use a primer composition comprising one or more nucleic acid sequences encoding an HIV antigen that is different from the antigen(s) encoded by the adenoviral vector composition. Preferably, the primer composition comprises one or more nucleic acid sequences that encode two or more HIV antigens that are the same as the HIV antigens encoded by the one or more adenoviral vectors of the adenoviral vector composition. More preferably, the primer composition comprises one or more nucleic acid sequences that encode all of the HIV antigens encoded by the one or more adenoviral vectors of the adenoviral vector composition.

The primer composition is administered to the mammal to prime the immune response to HIV. More than one dose of primer composition can be provided in any suitable timeframe (e.g., at least about 1 week, 2 weeks, 4 weeks, 8 weeks, 12 weeks, 16 weeks, or more prior to boosting). Preferably, the primer composition is administered to the mammal at least three months (e.g., three, six, nine, twelve, or more months) before administration of the adenoviral vector composition. Most preferably, the primer composition is administered to the mammal at least about six months to about nine months before administration of the adenoviral vector composition. The adenoviral vector composition is administered to the mammal to boost the immune response to HIV. More than one dose of adenoviral vector composition can be provided in any suitable timeframe to maintain immunity.

The adenoviral vector composition and/or the primer composition desirably is administered in a pharmaceutically acceptable (e.g., physiologically acceptable) composition, which comprises a carrier, preferably a physiologically (e.g., pharmaceutically) acceptable carrier and the adenoviral vector composition. Any suitable carrier can be used within the context of the invention, and such carriers are well known in the art. The choice of carrier will be determined, in part, by the particular site to which the composition is to be administered and the particular method used to administer the composition. Ideally, in the context of adenoviral vectors, the pharmaceutical composition preferably is free of replication-competent adenovirus. The pharmaceutical composition can optionally be sterile or sterile with the exception of the one or more adenoviral vectors.

Suitable formulations for the pharmaceutical composition include aqueous and non-aqueous solutions, isotonic sterile solutions, which can contain anti-oxidants, buffers, and bacteriostats, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets. Preferably, the carrier is a buffered saline solution. More preferably, the pharmaceutical composition for use in the inventive method is formulated to protect the adenoviral vectors from damage prior to administration. For example, the pharmaceutical composition can be formulated to reduce loss of the adenoviral vectors on devices used to prepare, store, or administer the expression vector, such as glassware, syringes, or needles. The pharmaceutical composition can be formulated to decrease the light sensitivity and/or temperature sensitivity of the adenoviral vectors. To this end, the pharmaceutical composition preferably comprises a pharmaceutically acceptable liquid carrier, such as, for example, those described above, and a stabilizing agent selected from the group consisting of Polysorbate 80, L-arginine, polyvinylpyrrolidone, trehalose, and combinations thereof. Use of such a composition will extend the shelf life of the vector, facilitate administration, and increase the efficiency of the inventive method. Formulations for adenoviral vector-containing compositions are further described in, for example, U.S. Patent 6,225,289, 6,514,943, U.S. Patent Application Publication No. 2003/0153065 A1, and International Patent Application Publication WO 00/34444. A pharmaceutical composition also can be formulated to enhance transduction efficiency of the adenoviral vector. In addition, one of ordinary skill in the art will appreciate that the pharmaceutical composition can comprise other therapeutic or biologically-active agents. For example, factors that control inflammation, such as ibuprofen or steroids, can be part of the pharmaceutical composition to reduce swelling and inflammation associated with *in vivo* administration of the adenoviral vectors. As discussed herein, immune system stimulators can be administered to enhance any immune response to the antigens. Antibiotics, i.e., microbicides and fungicides, can be present to treat existing infection and/or reduce the risk of future infection, such as infection associated with gene transfer procedures.

Any route of administration can be used to deliver the pharmaceutical composition to the mammal. Indeed, although more than one route can be used to administer the pharmaceutical composition, a particular route can provide a more immediate and more effective reaction than another route. Preferably, the pharmaceutical composition is administered via intramuscular injection. The pharmaceutical composition also can be applied or instilled into body cavities, absorbed through the skin (e.g., via a transdermal patch), inhaled, ingested, topically applied to tissue, or administered parenterally via, for instance, intravenous, peritoneal, or intraarterial administration.

The pharmaceutical composition can be administered in or on a device that allows controlled or sustained release, such as a sponge, biocompatible meshwork, mechanical reservoir, or mechanical implant. Implants (see, e.g., U.S. Patent 5,443,505), devices (see, e.g., U.S. Patent 4,863,457), such as an implantable device, e.g., a mechanical reservoir or an implant or a device comprised of a polymeric composition, are particularly useful for administration of the pharmaceutical composition. The pharmaceutical composition also can be administered in the form of sustained-release formulations (see, e.g., U.S. Patent 5,378,475) comprising, for example, gel foam, hyaluronic acid, gelatin, chondroitin sulfate, a polyphosphoester, such as bis-2-hydroxyethyl-terephthalate (BHET), and/or a polylactic-glycolic acid.

The dose of the pharmaceutical composition administered to the mammal will depend on a number of factors, including the size of a target tissue, the extent of any side-effects, the particular route of administration, and the like. The dose ideally comprises an "effective amount" of adenoviral vector composition and/or the primer composition, i.e., a dose of adenoviral vector composition and/or the primer composition which provokes a desired immune response in the mammal. The desired immune response can entail production of antibodies, protection upon subsequent challenge, immune tolerance, immune cell activation, and the like. In embodiments where the adenoviral vector composition comprises two or more adenoviral vectors, it will be appreciated that the pharmaceutical composition of the inventive method comprises a dose of adenoviral vector that is the combined dose of each of the two or more adenoviral vectors contained therein.

Desirably, the adenoviral vector composition comprises a single dose of adenoviral vector comprising at least about 1x10⁵ particles (which also is referred to as particle units) of adenoviral vector. The dose preferably is at least about 1x10⁶ particles (e.g., about 1x10⁶-1x10¹² particles), more preferably at least about 1x10⁷ particles, more preferably at least about 1x10⁸ particles (e.g., about 1x10⁸-1x10¹¹ particles or about 1x10⁸-1x10¹² particles), and most preferably at least about 1x10⁹ particles (e.g., about 1x10⁹-1x10¹⁰ particles or about 1x10⁹-1x10¹² particles), or even at least about 1x10¹⁰ particles (e.g., about 1x10¹⁰-1x10¹² particles) of the adenoviral vector. Alternatively, the dose comprises no more than about 1x10¹⁴ particles, preferably no more than about 1x10¹³ particles, even more preferably no more than about 1x10¹² particles, even more preferably no more than about 1x10¹¹ particles, and most preferably no more than about 1x10¹⁰ particles (e.g., no more than about 1x10⁹ particles). In other words, the adenoviral vector composition can comprise a single dose of adenoviral vector comprising, for example, about 1x10⁶ particle units (pu), 2x10⁶ pu, 4x10⁶ pu, 1x10⁷ pu, 2x10⁷ pu, 4x10⁷ pu, 1x10⁸ pu, 2x10⁸ pu, 4x10⁸ pu, 1x10⁹ pu, 2x10⁹ pu, 4x10⁹ pu, 1x10¹⁰ pu, 2x10¹⁰ pu, 4x10¹⁰ pu, 1x10¹¹ pu, 2x10¹¹ pu, 4x10¹¹ pu, 1x10¹² pu, 2x10¹² pu, or 4x10¹² pu of adenoviral vector.

The primer composition desirably comprises at least about 1 mg of nucleic acid, typically and preferably DNA. The primer composition preferably comprises 1 mg or more of nucleic acid (e.g., about 1 mg, 2 mg, 3 mg, 4 mg, 5 mg, or more). In a preferred embodiment, the primer composition comprises about 2 mg to about 5 mg nucleic acid (e.g., about 3 mg or 4 mg), more preferably about 3 mg to about 5 mg nucleic acid (e.g., about 3.5 mg), and most preferably about 4 mg to about 5 mg nucleic acid (e.g., about 4.5 mg).

Modified viruses have proven convenient vector systems for investigative and therapeutic gene transfer applications, and adenoviral vector systems present several advantages for such uses. Adenoviruses are generally associated with benign pathologies in humans, and the 36 kilobase (kb) adenoviral genome has been extensively studied. Adenoviral vectors can be produced in high titers (e.g., about 10¹³ particle forming units (pfu)), and such vectors can transfer genetic material to nonreplicating, as well as replicating, cells; in contrast with, e.g., retroviral vectors, which only transfer genetic material to replicating cells. The adenoviral genome can be manipulated to carry a large amount of exogenous DNA (up to about 8 kb), and the adenoviral capsid can potentiate the transfer of even longer sequences (Curiel et al., Hum. Gene Ther., 3, 147-154 (1992)). Additionally, adenoviruses generally do not integrate into the host cell chromosome, but rather are maintained as a linear episome, thus minimizing the likelihood that a recombinant adenovirus will interfere with normal cell function. In addition to being a superior vehicle for transferring genetic material to a wide variety of cell types, adenoviral vectors represent a safe choice for gene transfer, a particular concern for therapeutic applications.

Adenovirus from various origins, subtypes, or mixture of subtypes can be used as the source of the viral genome for the adenoviral vector. While non-human adenovirus (e.g., simian, avian, canine, ovine, or bovine adenoviruses) can be used to generate the adenoviral vector, a human adenovirus preferably is used as the source of the viral genome for the adenoviral vector of the inventive method. Adenovirus can be of various subgroups or serotypes. For instance, an adenovirus can be of subgroup A (e.g., serotypes 12, 18, and 31), subgroup B (e.g., serotypes 3, 7, 11, 14, 16, 21, 34, 35, and 50), subgroup C (e.g., serotypes 1, 2, 5, and 6), subgroup D (e.g., serotypes 8, 9, 10, 13, 15, 17, 19, 20, 22-30, 32, 33, 36-39, and 42-48), subgroup E (e.g., serotype 4), subgroup F (e.g., serotypes 40 and 41), an unclassified serogroup (e.g., serotypes 49 and 51), or any other adenoviral serotype. Adenoviral serotypes 1 through 51 are available from the American Type Culture Collection (ATCC, Manassas, VA). Preferably, in the context of the inventive method, the adenoviral vector is of human subgroup C, especially serotype 2 or even more desirably serotype 5. However, non-group C adenoviruses can be used to prepare adenoviral gene transfer vectors for delivery of gene products to host cells. Preferred adenoviruses used in the construction of non-group C adenoviral gene transfer vectors include Ad12 (group A), Ad7 and Ad35 (group B), Ad30 and Ad36 (group D), Ad4 (group E), and Ad41 (group F). Non-group C adenoviral vectors, methods of producing non-group C adenoviral vectors, and methods of using non-group C adenoviral vectors are disclosed in, for example, U.S. Patents 5,801,030, 5,837,511, and 5,849,561 and International Patent Applications WO 97/12986 and WO 98/53087.

The adenoviral vector can comprise a mixture of subtypes and thereby be a "chimeric" adenoviral vector. A chimeric adenoviral vector can comprise an adenoviral genome that is derived from two or more (e.g., 2, 3, 4, etc.) different adenovirus serotypes. In the context of the invention, a chimeric adenoviral vector can comprise approximately equal amounts of the genome of each of the two or more different adenovirus serotypes. When the chimeric adenoviral vector genome is comprised of the genomes of two different adenovirus serotypes, the chimeric adenoviral vector genome preferably comprises no more than about 70% (e.g., no more than about 65%, about 50%, or about 40%) of the genome of one of the adenovirus serotypes, with the remainder of the chimeric adenovirus genome being derived from the genome of the other adenovirus serotype. In one embodiment, the chimeric adenoviral vector can contain an adenoviral genome comprising a portion of a serotype 2 genome and a portion of a serotype 5 genome. For example, the 5' region of an adenoviral serotype 5 genome (i.e., the region of the genome 5' to the adenoviral E1 region) can be replaced with the corresponding region of an adenoviral serotype 2 genome (e.g., the Ad5 genome region 5' to the E1 region of the adenoviral genome is replaced with nucleotides 1-456 of the Ad2 genome).

The adenoviral vector of the invention can be replication-competent. For example, the adenoviral vector can have a mutation (e.g., a deletion, an insertion, or a substitution) in the adenoviral genome that does not inhibit viral replication in host cells. The inventive adenoviral vector also can be conditionally replication-competent. Preferably, however, the adenoviral vector is replication-deficient in host cells.

By "replication-deficient" is meant that the adenoviral vector requires complementation of one or more regions of the adenoviral genome that are required for replication, as a result of, for example a deficiency in at least one replication-essential gene function (i.e., such that the adenoviral vector does not replicate in typical host cells, especially those in a human patient that could be infected by the adenoviral vector in the course of the inventive method). A deficiency in a gene, gene function, or genomic region, as used herein, is defined as a deletion of sufficient genetic material of the viral genome to obliterate or impair the function of the gene (e.g., such that the function of the gene product is reduced by at least about 2-fold, 5-fold, 10-fold, 20-fold, 30-fold, or 50-fold) whose nucleic acid sequence was deleted in whole or in part. Deletion of an entire gene region often is not required for disruption of a replication-essential gene function. However, for the purpose of providing sufficient space in the adenoviral genome for one or more transgenes, removal of a majority of a gene region may be desirable. While deletion of genetic material is preferred, mutation of genetic material by addition or substitution also is appropriate for disrupting gene function. Replication-essential gene functions are those gene functions that are required for replication (e.g., propagation) and are encoded by, for example, the adenoviral early regions (e.g., the E1, E2, and E4 regions), late regions (e.g., the L1-L5 regions), genes involved in viral packaging (e.g., the IVa2 gene), and virus-associated RNAs (e.g., VA-RNA1 and/or VA-RNA-2).

The replication-deficient adenoviral vector desirably requires complementation of at least one replication-essential gene function of one or more regions of the adenoviral genome. Preferably, the adenoviral vector requires complementation of at least one gene function of the E1A region, the E1B region, or the E4 region of the adenoviral genome required for viral replication (denoted an E1-deficient or E4-deficient adenoviral vector). In addition to a deficiency in the E1 region, the recombinant adenovirus also can have a mutation in the major late promoter (MLP), as discussed in International Patent Application Publication WO 00/00628. Most preferably, the adenoviral vector is deficient in at least one replication-essential gene function (desirably all replication-essential gene functions) of the E1 region and at least one gene function of the nonessential E3 region (e.g., an Xba I deletion of the E3 region) (denoted an E1/E3-deficient adenoviral vector). With respect to the E1 region, the adenoviral vector can be deficient in part or all of the E1A region and/or part or all of the E1B region, e.g., in at least one replication-essential gene function of each of the E1A and E1B regions, thus requiring complementation of the E1A region and the E1B region of the adenoviral genome for replication. The adenoviral vector also can require complementation of the E4 region of the adenoviral genome for replication, such as through a deficiency in one or more replication-essential gene functions of the E4 region.

When the adenoviral vector is E1-deficient, the adenoviral vector genome can comprise a deletion beginning at any nucleotide between nucleotides 335 to 375 (e.g., nucleotide 356) and ending at any nucleotide between nucleotides 3,310 to 3,350 (e.g., nucleotide 3,329) or even ending at any nucleotide between 3,490 and 3,530 (e.g., nucleotide 3,510) (based on the adenovirus serotype 5 genome).

When E2A-deficient, the adenoviral vector genome can comprise a deletion beginning at any nucleotide between nucleotides 22,425 to 22,465 (e.g., nucleotide 22,443) and ending at any nucleotide between nucleotides 24,010 to 24,050 (e.g., nucleotide 24,032) (based on the adenovirus serotype 5 genome). When E3-deficient, the adenoviral vector genome can comprise a deletion beginning at any nucleotide between nucleotides 28,575 to 29,615 (e.g., nucleotide 28,593) and ending at any nucleotide between nucleotides 30,450 to 30,490 (e.g., nucleotide 30,470) (based on the adenovirus serotype 5 genome).

When the adenoviral vector is deficient in at least one replication-essential gene function in one region of the adenoviral genome (e.g., an E1- or E1/E3-deficient adenoviral vector), the adenoviral vector is referred to as "singly replication-deficient." A particularly preferred singly replication-deficient adenoviral vector is, for example, a replication-deficient adenoviral vector requiring, at most, complementation of the E1 region of the adenoviral genome, so as to propagate the adenoviral vector (e.g., to form adenoviral vector particles).

The adenoviral vector of the invention can be "multiply replication-deficient," meaning that the adenoviral vector is deficient in one or more replication-essential gene functions in each of two or more regions of the adenoviral genome, and requires complementation of those functions for replication. For example, the aforementioned E1-deficient or E1/E3-deficient adenoviral vector can be further deficient in at least one replication-essential gene function of the E4 region (denoted an E1/E4- or E1/E3/E4-deficient adenoviral vector), and/or the E2 region (denoted an E1/E2- or E1/E2/E3-deficient adenoviral vector), preferably the E2A region (denoted an E1/E2A- or E1/E2A/E3-deficient adenoviral vector). An adenoviral vector deleted of the entire E4 region can elicit a lower host immune response. When E4-deficient, the adenoviral vector genome can comprise a deletion beginning at, for example, any nucleotide between nucleotides 32,805 to 32,845 (e.g., nucleotide 32,826) and ending at, for example, any nucleotide between nucleotides 35,540 to 35,580 (e.g., nucleotide 35,561) (based on the adenovirus serotype 5 genome), optionally in addition to deletions in the E1 region (e.g., nucleotides 356 to 3,329 or nucleotides 356 to 3,510) (based on the adenovirus serotype 5 genome) and/or deletions in the E3 region (e.g., nucleotides 28,594 to 30,469 or nucleotides 28,593 to 30,470) (based on the adenovirus serotype 5 genome). The endpoints defining the deleted nucleotide portions can be difficult to precisely determine and typically will not significantly affect the nature of the adenoviral vector, i.e., each of the aforementioned nucleotide numbers can be +/- 1, 2, 3, 4, 5, or even 10 or 20 nucleotides.

If the adenoviral vector of the invention is deficient in a replication-essential gene function of the E2A region, the vector preferably does not comprise a complete deletion of the E2A region, which deletion preferably is less than about 230 base pairs in length. Generally, the E2A region of the adenovirus codes for a DBP (DNA binding protein), a Polypeptide required for DNA replication. DBP is composed of 473 to 529 amino acids depending on the viral serotype. It is believed that DBP is an asymmetric protein that exists as a prolate ellipsoid consisting of a globular Ct with an extended Nt domain. Studies indicate that the Ct domain is responsible for DBP's ability to bind to nucleic acids, bind to zinc, and function in DNA synthesis at the level of DNA chain elongation. However, the Nt domain is believed to function in late gene expression at both transcriptional and post-transcriptional levels, is responsible for efficient nuclear localization of the protein, and also may be involved in enhancement of its own expression. Deletions in the Nt domain between amino acids 2 to 38 have indicated that this region is important for DBP function (Brough et al., Virology, 196, 269-281 (1993)). While deletions in the E2A region coding for the Ct region of the DBP have no effect on viral replication, deletions in the E2A region which code for amino acids 2 to 38 of the Nt domain of the DBP impair viral replication. It is preferable that any multiply replication-deficient adenoviral vector contains this portion of the E2A region of the adenoviral genome. In particular, for example, the desired portion of the E2A region to be retained is that portion of the E2A region of the adenoviral genome which is defined by the 5' end of the E2A region, specifically positions Ad5(23816) to Ad5(24032) of the E2A region of the adenoviral genome of serotype Ad5. This portion of the adenoviral genome desirably is included in the adenoviral vector because it is not complemented in current E2A cell lines so as to provide the desired level of viral propagation.

While the above-described deletions are described with respect to an adenovirus serotype 5 genome, one of ordinary skill in the art can determine the nucleotide coordinates of the same regions of other adenovirus serotypes, such as an adenovirus serotype 2 genome, without undue experimentation, based on the similarity between the genomes of various adenovirus serotypes, particularly adenovirus serotypes 2 and 5.

In one embodiment of the inventive method, the adenoviral vector can comprise an adenoviral genome deficient in one or more replication-essential gene functions of each of the E1 and E4 regions (i.e., the adenoviral vector is an E1/E4-deficient adenoviral vector), preferably with the entire coding region of the E4 region having been deleted from the adenoviral genome. In other words, all the open reading frames (ORFs) of the E4 region have been removed. Most preferably, the adenoviral vector is rendered replication-deficient by deletion of all of the E1 region and by deletion of a portion of the E4 region. The E4 region of the adenoviral vector can retain the native E4 promoter, Polyadenylation sequence, and/or the right-side inverted terminal repeat (ITR).

It should be appreciated that the deletion of different regions of the adenoviral vector can alter the immune response of the mammal. In particular, deletion of different regions can reduce the inflammatory response generated by the adenoviral vector. Furthermore, the adenoviral vector's coat protein can be modified so as to decrease the adenoviral vector's ability or inability to be recognized by a neutralizing antibody directed against the wild-type coat protein, as described in International Patent Application WO 98/40509. Such modifications are useful for long-term treatment of persistent ocular disorders.

The adenoviral vector, when multiply replication-deficient, especially in replication-essential gene functions of the E1 and E4 regions, can include a spacer sequence to provide viral growth in a complementing cell line similar to that achieved by singly replication-deficient adenoviral vectors, particularly an E1-deficient adenoviral vector. In a preferred E4-deficient adenoviral vector of the invention wherein the L5 fiber region is retained, the spacer is desirably located between the L5 fiber region and the right-side ITR. More preferably in such an adenoviral vector, the E4 Polyadenylation sequence alone or, most preferably, in combination with another sequence exists between the L5 fiber region and the right-side ITR, so as to sufficiently separate the retained L5 fiber region from the right-side ITR, such that viral production of such a vector approaches that of a singly replication-deficient adenoviral vector, particularly a singly replication-deficient E1 deficient adenoviral vector.

The spacer sequence can contain any nucleotide sequence or sequences which are of a desired length, such as sequences at least about 15 base pairs (e.g., between about 15 base pairs and about 12,000 base pairs), preferably about 100 base pairs to about 10,000 base pairs, more preferably about 500 base pairs to about 8,000 base pairs, even more preferably about 1,500 base pairs to about 6,000 base pairs, and most preferably about 2,000 to about 3,000 base pairs in length. The spacer sequence can be coding or non-coding and native or non-native with respect to the adenoviral genome, but does not restore the replication-essential function to the deficient region. The spacer can also contain a promoter-variable expression cassette. More preferably, the spacer comprises an additional Polyadenylation sequence and/or a passenger gene. Preferably, in the case of a spacer inserted into a region deficient for E4, both the E4 Polyadenylation sequence and the E4 promoter of the adenoviral genome or any other (cellular or viral) promoter remain in the vector. The spacer is located between the E4 Polyadenylation site and the E4 promoter, or, if the E4 promoter is not present in the vector, the spacer is proximal to the right-side ITR. The spacer can comprise any suitable Polyadenylation sequence. Examples of suitable Polyadenylation sequences include synthetic optimized sequences, BGH (Bovine Growth Hormone), Polyoma virus, TK (Thymidine Kinase), EBV (Epstein Barr Virus) and the papillomaviruses, including human papillomaviruses and BPV (Bovine Papilloma Virus). Preferably, particularly in the E4 deficient region, the spacer includes an SV40 Polyadenylation sequence. The SV40 Polyadenylation sequence allows for higher virus production levels of multiply replication deficient adenoviral vectors. In the absence of a spacer, production of fiber protein and/or viral growth of the multiply replication-deficient adenoviral vector is reduced by comparison to that of a singly replication-deficient adenoviral vector. However, inclusion of the spacer in at least one of the deficient adenoviral regions, preferably the E4 region, can counteract this decrease in fiber protein production and viral growth. Ideally, the spacer comprises the glucuronidase gene. The use of a spacer in an adenoviral vector is further described in, for example, U.S. Patent 5,851,806 and International Patent Application WO 97/21826.

It has been observed that an at least E4-deficient adenoviral vector expresses a transgene at high levels for a limited amount of time *in vivo* and that persistence of expression of a transgene in an at least E4-deficient adenoviral vector can be modulated through the action of a trans-acting factor, such as HSV ICP0, Ad pTP, CMV-IE2, CMV-IE86, HIV tat, HTLV-tax, HBV-X, AAV Rep 78, the cellular factor from the U205 osteosarcoma cell line that functions like HSV ICP0, or the cellular factor in PC12 cells that is induced by nerve growth factor, among others, as described in for example, U.S. Patents 6,225,113, 6,649,373, and 6,660,521, and International Patent Application Publication WO 00/34496. In view of the above, a multiply deficient adenoviral vector (e.g., the at least E4-deficient adenoviral vector) or a second expression vector can comprise a nucleic acid sequence encoding a trans-acting factor that modulates the persistence of expression of the nucleic acid sequence. Persistent expression of antigenic DNA can be desired when generating immune tolerance.

Desirably, the adenoviral vector requires, at most, complementation of replication-essential gene functions of the E1, E2A, and/or E4 regions of the adenoviral genome for replication (i.e., propagation). However, the adenoviral genome can be modified to disrupt one or more replication-essential gene functions as desired by the practitioner, so long as the adenoviral vector remains deficient and can be propagated using, for example, complementing cells and/or exogenous DNA (e.g., helper adenovirus) encoding the disrupted replication-essential gene functions. In this respect, the adenoviral vector can be deficient in replication-essential gene functions of only the early regions of the adenoviral genome, only the late regions of the adenoviral genome, and both the early and late regions of the adenoviral genome. An adenoviral vector also can have essentially the entire adenoviral genome removed, in which case it is preferred that at least either the viral inverted terminal repeats (ITRs) and one or more promoters or the viral ITRs and a packaging signal are left intact (i.e., an adenoviral amplicon). Suitable replication-deficient adenoviral vectors, including multiply replication-deficient adenoviral vectors, are disclosed in U.S. Patents 5,837,511; 5,851,806; 5,994,106; 6,127,175; and 6,482,616; U.S. Patent Application Publications 2001/0043922 A1, 2002/0004040 A1, 2002/0031831 A1, 2002/0110545 A1, and 2004/0161848 A1, and International Patent Application Publications WO 94/28152, WO 95/02697, WO 95/16772, WO 95/34671, WO 96/22378, WO 97/12986, WO 97/21826, and WO 03/022311.

Ideally, the adenoviral vector administered to a mammal is in the form of an adenoviral vector composition, especially a pharmaceutical composition, which is virtually free of replication-competent adenovirus (RCA) contamination (e.g., the composition comprises less than about 1% of RCA contamination). Most desirably, the composition is RCA-free. Adenoviral vector compositions and stocks that are RCA-free are described in U.S. Patents 5,944,106 and 6,482,616, U.S. Published Patent Application 2002/0110545 A1, and International Patent Application WO 95/34671.

By removing all or part of, for example, the E1, E3, and E4 regions of the adenoviral genome, the resulting adenoviral vector is able to accept inserts of exogenous nucleic acid sequences while retaining the ability to be packaged into adenoviral capsids. The nucleic acid sequence can be positioned in the E1 region, the E3 region, or the E4 region of the adenoviral genome. Indeed, the nucleic acid sequence can be inserted anywhere in the adenoviral genome so long as the position does not prevent expression of the nucleic acid sequence or interfere with packaging of the adenoviral vector.

If the adenoviral vector is not replication-deficient, ideally the adenoviral vector is manipulated to limit replication of the vector to within a target tissue. The adenoviral vector can be a conditionally-replicating adenoviral vector, which is engineered to replicate under conditions pre-determined by the practitioner. For example, replication-essential gene functions, e.g., gene functions encoded by the adenoviral early regions, can be operably linked to an inducible, repressible, or tissue-specific transcription control sequence, e.g., promoter. In this embodiment, replication requires the presence or absence of specific factors that interact with the transcription control sequence. In autoimmune disease treatment, it can be advantageous to control adenoviral vector replication in, for instance, lymph nodes, to obtain continual antigen production and control immune cell production. Conditionally-replicating adenoviral vectors are described further in U.S. Patent 5,998,205.

In addition to modification (e.g., deletion, mutation, or replacement) of adenoviral sequences encoding replication-essential gene functions, the adenoviral genome can contain benign or non-lethal modifications, i.e., modifications which do not render the adenovirus replication-deficient, or, desirably, do not adversely affect viral functioning and/or production of viral proteins, even if such modifications are in regions of the adenoviral genome that otherwise contain replication-essential gene functions. Such modifications commonly result from DNA manipulation or serve to facilitate expression vector construction. For example, it can be advantageous to remove or introduce restriction enzyme sites in the adenoviral genome. Such benign mutations often have no detectable adverse effect on viral functioning. For example, the adenoviral vector can comprise a deletion of nucleotides 10,594 and 10,595 (based on the adenoviral serotype 5 genome), which are associated with VA-RNA-1 transcription, but the deletion of which does not prohibit production of VA-RNA-1.

Similarly, the coat protein of an adenoviral vector can be manipulated to alter the binding specificity or recognition of the adenovirus for a viral receptor on a potential host cell. For adenovirus, such manipulations can include deletion of regions of the fiber, penton, or hexon, insertions of various native or non-native ligands into portions of the coat protein, and the like. Manipulation of the coat protein can broaden the range of cells infected by an adenoviral vector or enable targeting of an adenoviral vector to a specific cell type.

For example, in one embodiment, the adenoviral vector comprises a chimeric coat protein (e.g., a fiber, hexon pIX, pIIIa, or penton protein), which differs from the wild-type (i.e., native) coat protein by the introduction of a nonnative amino acid sequence, preferably at or near the carboxyl terminus. Preferably, the nonnative amino acid sequence is inserted into or in place of an internal coat protein sequence. One of ordinary skill in the art will understand that the nonnative amino acid sequence can be inserted within the internal coat protein sequence or at the end of the internal coat protein sequence. The resultant chimeric viral coat protein is able to direct entry into cells of the adenoviral vector comprising the coat protein that is more efficient than entry into cells of a vector that is identical except for comprising a wild-type adenoviral coat protein rather than the chimeric adenoviral coat protein. Preferably, the chimeric adenovirus coat protein binds a novel endogenous binding site present on the cell surface that is not recognized, or is poorly recognized, by a vector comprising a wild-type coat protein. One direct result of this increased efficiency of entry is that the adenovirus can bind to and enter numerous cell types which an adenovirus comprising wild-type coat protein typically cannot enter or can enter with only a low efficiency.

In another embodiment of the invention, the adenoviral vector comprises a chimeric virus coat protein not selective for a specific type of eukaryotic cell. The chimeric coat protein differs from the wild-type coat protein by an insertion of a nonnative amino acid sequence into or in place of an internal coat protein sequence. In this embodiment, the chimeric adenovirus coat protein efficiently binds to a broader range of eukaryotic cells than a wild-type adenovirus coat, such as described in International Patent Application WO 97/20051.

Specificity of binding of an adenovirus to a given cell can also be adjusted by use of an adenovirus comprising a short-shafted adenoviral fiber gene, as discussed in U.S. Patent 5,962,311. Use of an adenovirus comprising a short-shafted adenoviral fiber gene reduces the level or efficiency of adenoviral fiber binding to its cell-surface receptor and increases adenoviral penton base binding to its cell-surface receptor, thereby increasing the specificity of binding of the adenovirus to a given cell. Alternatively, use of an adenovirus comprising a short-shafted fiber enables targeting of the adenovirus to a desired cell-surface receptor by the introduction of a nonnative amino acid sequence either into the penton base or the fiber knob.

In yet another embodiment, the nucleic acid residues encoding amino acid residues associated with native substrate binding can be changed, supplemented or deleted (see, e.g., International Patent Application Publication WO 00/15823; Einfeld et al., J. Virol., 75(23), 11284-11291 (2001); and van Beusechem et al., J. Virol., 76(6), 2753-2762 (2002)), such that the adenoviral vector incorporating the mutated nucleic acid residues (or having the fiber protein encoded thereby) is less able to bind its native substrate. For example, the native CAR and integrin binding sites of a serotype 5 or serotype 2 adenoviral vector, such as the knob domain of the adenoviral fiber protein and an Arg-Gly-Asp (RGD) sequence located in the adenoviral penton base, respectively, can be removed or disrupted. Any suitable amino acid residue(s) of a fiber protein that mediates or assists in the interaction between the knob and CAR can be mutated or removed, so long as the fiber protein is able to trimerize. Similarly, amino acids can be added to the fiber knob as long as the fiber protein retains the ability to trimerize. Suitable residues include amino acids within the exposed loops of the fiber protein, such as, for example, the AB loop, the DE loop, and the FG loop of the serotype 5 fiber knob domain, which are further described in, for example, Roelvink et al., Science, 286, 1568-1571 (1999), and U.S. Patent 6,455,314. Any suitable amino acid residue(s) of a penton base protein that mediates or assists in the interaction between the penton base and integrins can be mutated or removed. Suitable residues include, for example, one or more of the five RGD amino acid sequence motifs located in the hypervariable region of the Ad5 penton base protein (as described, for example, U.S. Patent 5,731,190). The native integrin binding sites on the penton base protein also can be disrupted by modifying the nucleic acid sequence encoding the native RGD motif such that the native RGD amino acid sequence is conformationally inaccessible for binding to the αv integrin receptor, such as by inserting a DNA sequence into or adjacent to the nucleic acid sequence encoding the adenoviral penton base protein. Preferably, the adenoviral vector comprises a fiber protein and a penton base protein that do not bind to CAR and integrins, respectively. Alternatively, the adenoviral vector comprises fiber protein and a penton base protein that bind to CAR and integrins, respectively, but with less affinity than the corresponding wild type coat proteins. The adenoviral vector exhibits reduced binding to CAR and integrins if a modified adenoviral fiber protein and penton base protein binds CAR and integrins, respectively, with at least about 5-fold, 10-fold, 20-fold, 30-fold, 50-fold, or 100-fold less affinity than a non-modified adenoviral fiber protein and penton base protein of the same serotype.

Although preferred, native binding of an adenovirus to host cells need not be ablated. In some instances, such as use of an adenoviral vector to deliver an antigen coding sequence to host cells, the broad host range of adenovirus can be advantageous.

An adenoviral vector also can comprise a chimeric coat protein comprising a non-native amino acid sequence that binds a substrate (i.e., a ligand). The non-native amino acid sequence of the chimeric adenoviral coat protein allows an adenoviral vector comprising the chimeric coat protein to bind and, desirably, infect host cells not naturally infected by the corresponding adenovirus without the non-native amino acid sequence (i.e., host cells not infected by the corresponding wild-type adenovirus), to bind to host cells naturally infected by the corresponding adenovirus with greater affinity than the corresponding adenovirus without the non-native amino acid sequence, or to bind to particular target cells with greater affinity than non-target cells. A "non-native" amino acid sequence can comprise an amino acid sequence not naturally present in the adenoviral coat protein or an amino acid sequence found in the adenoviral coat but located in a non-native position within the capsid. By "preferentially binds" is meant that the non-native amino acid sequence binds a receptor, such as, for instance, αvβ3 integrin, with at least about 3-fold greater affinity (e.g., at least about 5-fold, 10-fold, 15-fold, 20-fold, 25-fold, 35-fold, 45-fold, or 50-fold greater affinity) than the non-native ligand binds a different receptor, such as, for instance, αvβ1 integrin.

The non-native amino acid sequence can be conjugated to any of the adenoviral coat proteins to form a chimeric coat protein. Therefore, for example, the non-native amino acid sequence can be conjugated to, inserted into, or attached to a fiber protein, a penton base protein, a hexon protein, proteins IX, VI, or IIIa, etc. The sequences of such proteins, and methods for employing them in recombinant proteins, are well known in the art (see, e.g., U.S. Patents 5,543,328; 5,559,099; 5,712,136; 5,731,190; 5,756,086; 5,770,442; 5,846,782; 5,962,311; 5,965,541; 5,846,782; 6,057,155; 6,127,525; 6,153,435; 6,329,190; 6,455,314; 6,465,253; and 6,576,456; U.S. Patent Application Publication 2001/0047081 and 2003/0099619; and International Patent Applications WO 96/07734, WO 96/26281, WO 97/20051, WO 98/07877, WO 98/07865, WO 98/40509, WO 98/54346, WO 00/15823, WO 01/58940, and WO 01/92549). The coat protein portion of the chimeric coat protein can be a full-length adenoviral coat protein to which the ligand domain is appended, or it can be truncated, e.g., internally or at the C- and/or N- terminus. The coat protein portion need not, itself, be native to the adenoviral vector. For example, the coat protein can be an adenoviral serotype 4 (Ad4) fiber protein incorporated into an adenoviral serotype 5 vector, wherein the native CAR binding motif of the Ad4 fiber is preferably ablated. However modified (including the presence of the non-native amino acid), the chimeric coat protein preferably is able to incorporate into an adenoviral capsid as its native counterpart coat protein. Once a given non-native amino acid sequence is identified, it can be incorporated into any location of the virus capable of interacting with a substrate (i.e., the viral surface). For example, the ligand can be incorporated into the fiber, the penton base, the hexon, protein IX, VI, or IIIa, or other suitable location. Where the ligand is attached to the fiber protein, preferably it does not disturb the interaction between viral proteins or fiber monomers. Thus, the non-native amino acid sequence preferably is not itself an oligomerization domain, as such can adversely interact with the trimerization domain of the adenovirus fiber. Preferably the ligand is added to the virion protein, and is incorporated in such a manner as to be readily exposed to the substrate (e.g., at the N- or C- terminus of the protein, attached to a residue facing the substrate, positioned on a peptide spacer to contact the substrate, etc.) to maximally present the non-native amino acid sequence to the substrate. Ideally, the non-native amino acid sequence is incorporated into an adenoviral fiber protein at the C-terminus of the fiber protein (and attached via a spacer) or incorporated into an exposed loop (e.g., the HI loop) of the fiber to create a chimeric coat protein. Where the non-native amino acid sequence is attached to or replaces a portion of the penton base, preferably it is within the hypervariable regions to ensure that it contacts the substrate. Where the non-native amino acid sequence is attached to the hexon, preferably it is within a hypervariable region (Miksza et al., J. Virol., 70(3), 1836-44 (1996)). Use of a spacer sequence to extend the non-native amino acid sequence away from the surface of the adenoviral particle can be advantageous in that the non-native amino acid sequence can be more available for binding to a receptor and any steric interactions between the non-native amino acid sequence and the adenoviral fiber monomers is reduced.

The non-native amino acid sequence can bind a particular cellular receptor present on a narrow class of cell types (e.g., tumor cells, cardiac muscle, skeletal muscle, smooth muscle, etc.) or a broader group encompassing several cell types. In other embodiments (e.g., to facilitate purification or propagation within a specific engineered cell type), a non-native amino acid (e.g., ligand) can bind a compound other than a cell-surface protein. Thus, the ligand can bind blood- and/or lymph-borne proteins (e.g., albumin), synthetic peptide sequences such as Polyamino acids (e.g., Polylysine, Polyhistidine, etc.), artificial peptide sequences (e.g., FLAG), and RGD peptide fragments (Pasqualini et al., J. Cell. Biol., 130, 1189(1995)).

Examples of suitable non-native amino acid sequences and their substrates include, but are not limited to, short (e.g., 6 amino acids or less) linear stretches of amino acids recognized by integrins, as well as Polyamino acid sequences such as Polylysine, Polyarginine, etc. Inserting multiple lysines and/or arginines provides for recognition of heparin and DNA. Suitable non-native amino acid sequences for generating chimeric adenoviral coat proteins are further described in U.S. Patent 6,455,314 and International Patent Application WO 01/92549.

Preferably, the adenoviral coat protein comprises a non-native amino acid sequence that binds αvβ3, αvβ5, or αvβ6 integrins. Adenoviral vectors displaying ligands specific for αvβ3 integrin, such as an RGD motif, infect cells with a greater number of αvβ3 integrin moieties on the cell surface compared to cells that do not express the integrin to such a degree, thereby targeting the vectors to specific cells of interest.

In another embodiment of the invention, the adenoviral vector can comprise a chimeric fiber protein comprising an amino acid sequence (e.g., a non-native amino acid sequence) comprising an RGD motif including, but not limited to, CRGDC (SEQ ID NO: 1), CXCRGDCXC (SEQ ID NO: 2), wherein X represents any amino acid, and CDCRGDCFC (SEQ ID NO: 3). The RGD motif can be inserted into the adenoviral fiber knob region, preferably in an exposed loop of the adenoviral knob, such as the HI loop. The RGD amino acid sequence can replace a region of the HI loop, or can be inserted into the HI loop without removal of native amino acids. The RGD motif also can be appended to the C-terminus of the adenoviral fiber protein, optionally via a spacer sequence. The spacer sequence preferably comprises between one and two-hundred amino acids, and can (but need not) have an intended function. In one embodiment, the chimeric fiber protein recognizes a coxsackievirus and adenovirus receptor (CAR). Ideally, native CAR binding of the fiber protein is not affected by mutation or modification of the fiber protein. In addition, the adenoviral vector can comprise an adenoviral coat wherein penton base proteins retain their ability to bind integrins. However, as discussed herein, native binding by the penton base proteins of the adenoviral coat protein can be ablated if desired. In another embodiment, the RGD motif preferably is flanked by one or two sets of cysteine residues.

An adenoviral vector can comprise a chimeric virus coat protein not selective for a specific type of eukaryotic cell. The chimeric coat protein differs from a wild-type coat protein by an insertion of a nonnative amino acid sequence into or in place of an internal coat protein sequence, or attachment of a non-native amino acid sequence to the N- or C- terminus of the coat protein. For example, a ligand comprising about five to about nine lysine residues (preferably seven lysine residues) is attached to the C-terminus of the adenoviral fiber protein via a non-coding spacer sequence. In this embodiment, the chimeric virus coat protein efficiently binds to a broader range of eukaryotic cells than a wild-type virus coat, such as described in International Patent Application WO 97/20051.

Of course, the ability of an adenoviral vector to recognize a potential host cell can be modulated without genetic manipulation of the coat protein. For instance, complexing an adenovirus with a bispecific molecule comprising a penton base-binding domain and a domain that selectively binds a particular cell surface binding site enables one of ordinary skill in the art to target the vector to a particular cell type.

Replication-deficient adenoviral vectors are typically produced in complementing cell lines that provide gene functions not present in the replication-deficient adenoviral vectors, but required for viral propagation, at appropriate levels in order to generate high titers of viral vector stock. Desirably, the complementing cell line comprises, integrated into the cellular genome, adenoviral nucleic acid sequences which encode gene functions required for adenoviral propagation. A preferred cell line complements for at least one and preferably all replication-essential gene functions not present in a replication-deficient adenovirus. The complementing cell line can complement for a deficiency in at least one replication-essential gene function encoded by the early regions, late regions, viral packaging regions, virus-associated RNA regions, or combinations thereof, including all adenoviral functions (e.g., to enable propagation of adenoviral amplicons). Most preferably, the complementing cell line complements for a deficiency in at least one replication-essential gene function (e.g., two or more replication-essential gene functions) of the E1 region of the adenoviral genome, particularly a deficiency in a replication-essential gene function of each of the E1A and E1B regions. In addition, the complementing cell line can complement for a deficiency in at least one replication-essential gene function of the E2 (particularly as concerns the adenoviral DNA Polymerase and terminal protein) and/or E4 regions of the adenoviral genome. Desirably, a cell that complements for a deficiency in the E4 region comprises the E4-ORF6 gene sequence and produces the E4-ORF6 protein. Such a cell desirably comprises at least ORF6 and no other ORF of the E4 region of the adenoviral genome. The cell line preferably is further characterized in that it contains the complementing genes in a non-overlapping fashion with the adenoviral vector, which minimizes, and practically eliminates, the possibility of the vector genome recombining with the cellular DNA. Accordingly, the presence of replication competent adenoviruses (RCA) is minimized if not avoided in the vector stock, which, therefore, is suitable for certain therapeutic purposes, especially vaccination purposes. The lack of RCA in the vector stock avoids the replication of the adenoviral vector in non-complementing cells. Construction of such a complementing cell lines involve standard molecular biology and cell culture techniques, such as those described by Sambrook et al., *supra,* and Ausubel et al., *supra*).

Complementing cell lines for producing the adenoviral vector include, but are not limited to, 293 cells (described in, e.g., Graham et al., J. Gen. Virol., 36, 59-72 (1977)), PER.C6 cells (described in, e.g., International Patent Application Publication WO 97/00326, and U.S. Patents 5,994,128 and 6,033,908), and 293-ORF6 cells (described in, e.g., International Patent Application Publication WO 95/34671 and Brough et al., J. Virol., 71, 9206-9213 (1997)). Additional complementing cells are described in, for example, U.S. Patents 6,677,156 and 6,682,929, and International Patent Application Publication WO 03/20879. In some instances, the cellular genome need not comprise nucleic acid sequences, the gene products of which complement for all of the deficiencies of a replication-deficient adenoviral vector. One or more replication-essential gene functions lacking in a replication-deficient adenoviral vector can be supplied by a helper virus, e.g., an adenoviral vector that supplies in trans one or more essential gene functions required for replication of the desired adenoviral vector. Helper virus is often engineered to prevent packaging of infectious helper virus. For example, one or more replication-essential gene functions of the E1 region of the adenoviral genome are provided by the complementing cell, while one or more replication-essential gene functions of the E4 region of the adenoviral genome are provided by a helper virus.

Suitable modifications to an adenoviral vector are described in U.S. Patents 5,543,328; 5,559,099; 5,712,136; 5,731,190; 5,756,086; 5,770,442; 5,846,782; 5,871,727; 5,885,808; 5,922,315; 5,962,311; 5,965,541; 6,057,155; 6,127,525; 6,153,435; 6,329,190; 6,455,314; 6,465,253; 6,576,456; 6,649,407; 6,740,525, and International Patent Applications WO 95/02697, WO 95/16772, WO 95/34671, WO 96/07734, WO 96/22378, WO 96/26281, WO 97/20051, WO 98/07865, WO 98/07877, WO 98/40509, WO 98/54346, WO 00/15823, WO 01/58940, and WO 01/92549. Similarly, it will be appreciated that numerous adenoviral vectors are available commercially. Construction of adenoviral vectors is well understood in the art. Adenoviral vectors can be constructed and/or purified using methods known in the art (e.g., using complementing cell lines, such as the 293 cell line, Per.C6 cell line, or 293-ORF6 cell line) and methods set forth, for example, in U.S. Patents 5,965,358; 5,994,128; 6,033,908; 6,168,941; 6,329,200; 6,383,795; 6,440,728; 6,447,995; and 6,475,757; U.S. Patent Application Publication 2002/0034735 A1, and International Patent Applications WO 98/53087, WO 98/56937, WO 99/15686, WO 99/54441, WO 00/12765, WO 01/77304, and WO 02/29388, as well as the other references identified herein.

The following examples further illustrate the invention but, of course, should not be construed as in any way limiting its scope.

### EXAMPLE 1

This example demonstrates the production of a composition comprising four adenoviral vectors each encoding a different HIV antigen.

Adenoviral vectors were constructed using a rapid vector construction system (AdFAST™, GenVec, Inc.). AdFAST™ was used to generate four adenoviral vectors each of which express one of the four HIV antigens: gp140(clade A), gp140(clade B)dv12, gp140(clade C), and GagPol (clade B). Expression of the antigen was driven by the cytomegalovirus (CMV) immediate-early promoter. The GV11 adenoviral backbone was chosen to reduce the risk of replication-competent adenovirus (RCA) generation during clinical production. The GV11 backbone contains deletions of the essential E1 and E4 regions, as well as a partial E3 deletion that render the adenoviral vector replication-deficient.

### AdtGagPol(B).11D Plasmid

A synthetic Polyprotein-encoding version of the Gag/Pol genes using codons optimized for expression in human cells was created using sequences of the Gag and Pol proteins from an HIV-1 clade B were used to create. The synthetic Gag gene was from HIV-1 clade B strain HXB2 (GenBank accession number K03455), and the synthetic Pol gene (Pol/h) was from HIV-1 clade B NL4-3 (GenBank accession number M19921). The Pol gene was non-functional because it was present as a fusion protein comprising reverse transriptase, protease, and integrase proteins. Point mutations were introduced in the nucleic acid sequences encoding the protease and reverse transcriptase genes of the plasmid. The protease modification prevented processing of the Pol gene product, and reduced the potential for functional protease, reverse transcriptase, and integrase enzymatic activity. No modifications were made to the Gag protein. The nucleic acid sequence encoding the Gag/Pol fusion Polyprotein was subcloned using standard recombinant DNA techniques into an expression cassette in an E1-shuttle plasmid for insertion into the adenoviral vector.

### Adgp140(A).11D Plasmid

A synthetic version of the HIV-1 clade A gene gp140delCFI using codons altered for expression in human cells was created using the protein sequence of the Envelope Polyprotein (gp160) from HIV-1 clade A strain 92rw020 (CCR5-tropic, GenBank accession number U08794). In this regard, plasmids expressing the HIV-1 gene were made synthetically with sequences designed to disrupt viral RNA structures that limit protein expression by using codons typically found in human cells. The nucleic acid sequence encoding the clade A gp140delCFI gene was subcloned using standard recombinant DNA techniques into an expression cassette in an E1-shuttle plasmid for insertion into the adenoviral vector.

### Adtgp140dv12(B).11D Plasmid

A synthetic version of the HIV-1 clade B gene X4gp160/h using codons optimized for expression in human cells was generated using the protein sequence of the Envelope Polyprotein (gp160) from HIV-1 clade B strain HXB2 (X4-tropic, GenBank accession number K03455). To produce a CCR5-tropic version of the Envelope protein R5gp160/h, the region encoding HIV-1 Envelope Polyprotein amino acids 275 to 361 from HIV-1 strain X4gp160/h was replaced with the corresponding region from the BaL strain of HIV-1 (GenBank accession number M68893). The full-length CCR5-tropic version of the Envelope protein gene from pR5gp160/h was terminated after the codon for amino acid 680. The truncated Env glycoprotein (gp140) contains the entire surface protein and the ectodomain of gp41 including the fusion domain, and regions important for oligomer formation, specifically two helical coiled coil motifs. The Env V1 and V2 loops were deleted to improve the stability and yield of the vector in the producer cell line. Two additional amino acids were incorporated immediately after the deletion due to creation of a restriction enzyme site. The nucleic acid sequence encoding the gp140dv12 gene was subcloned standard recombinant DNA techniques into an expression cassette in an E1-shuttle plasmid for insertion into the adenoviral vector.

### Adgp140(C).11 D Plasmid

A synthetic version of the HIV-1 clade C gene gp140delCFI using codons optimized for expression in human cells using the protein sequence of the Envelope Polyprotein gpl40delCFI from HIV-1 strain 97ZA012 (CCR5-tropic, GenBank accession number AF286227). The nucleic acid sequence encoding the synthetic gp140delCFI gene was subcloned using standard recombinant DNA techniques into an expression cassette in an E1-shuttle plasmid for insertion into the adenoviral vector.

### Adenoviral Vectors

The four E1-shuttle plasmids, AdtGagPol(B).11D, Adgp140(A).11D, Adtgp140dv12(B).11D, and Adgp140(C).11D were recombined in *E. coli* BjDE3 bacteria with the GV11 adenovector based AdFASTTM plasmid pAdE1(BN)E3(10)E4(TIS1) to generate the adenoviral vector plasmids. The replication-deficient adenoviral vectors AdtGagPol(B).11D, Adgp140(A).11D, Adtgp140dv12(B).11D, and Adgp140(C).11D were then generated by introducing the adenoviral vector plasmids into the packaging cell line, 293-ORF6.

### Adenoviral Vector Composition

The four adenoviral vector constructs were purified and dialyzed against a final formulation buffer (FFB; 10 mM Tris pH 7.8, 75 mM NaCl, 5% Trehalose, 25 ppm Polysorbate 80, 1 mM MgCl₂) custom manufactured at BioWhittaker (Frederick, MD). The adenoviral vector composition, designated VRC-HIVADV014-00-VP, was prepared from a blend of the each of the four adenoviral vectors at a 3:1:1:1 ratio by weight of AdtGagPol(B).11D, Adgp140(A).11D, Adtgp140dv12(B).11D, Adgp140(C).11D, respectively.

### EXAMPLE 2

This example demonstrates the biodistribution of an adenoviral vector composition administered to a mammal.

A single-dose biodistribution study using intramuscular injections delivered by a needle and syringe was conducted in New Zealand White rabbits to evaluate the distribution of the adenoviral vector composition VRC-HIVADV014-00-VP. The vector composition was administered as a single dose to rabbits (0.95 x 10¹¹ pu), and tissues were tested for the presence of adenoviral vectors at 9, 61, and 91 days post vector administration.

Tissues were tested for the presence of the adenoviral vector using a GLP validated Taqman™ Polymerase chain reaction (PCR), developed and qualified to detect a specific target sequence in each of the four different adenoviral vectors of VRC-HIVADV014-00-VP. The assay detects an amplicon from each of the adenoviral vectors. The 5'- PCR primers, 3'-PCR primers and fluorescently labeled probes span regions containing the insert, Polylinker, and promoter. The lower limit of detection for this assay was 10 copies of VRC-HIVADV014-00-VP DNA, and the lower limit of quantification for the assay was 50 copies of VRC-HIVADV014-00-VP DNA.

The PCR data from the three timepoints showed the presence of VRC-HIVADV014-00-VP DNA in the injection site (subcutis and muscle) and liver at 9 and 61 days post administration and spleen at all timepoints. The number of copies and the number of positive tissues decreased considerably between study day (SD) 9 and SD 61 for tissues with positive findings, and between SD 61 and SD 91 for the liver and injection site. No clinical signs of toxicity or gross lesions were observed.

### EXAMPLE 3

This example demonstrates the immunogenicity of an adenoviral vector composition administered to a mammal.

The adenoviral vector composition VRC-HIVADV014-00-VP was administered a single dose (1 x 10¹¹ pu) to mice and twice administered to rabbits. Tissues were analyzed for immunogenicity at 4 weeks post administration for mice, and at 36 days post administration for rabbits.

Cellular immune responses were tested by the interferon gamma (IFN-γ) ELISPOT assay and the flow cytometry-based intracellular cytokine staining (ICS) assay. The IFN-γ ELISPOT quantitatively measures the production of IFN-γ by peripheral blood mononuclear cells (PBMC) from immunized animals. The cells were exposed *in vitro* to HIV-1 antigens (i.e., a series of short, overlapping peptides that span the length of the protein expressed in the adenoviral vector). The IFN-γ molecules produced by antigen-sensitized T-lymphocytes are bound to antibodies coating an assay plate and may be counted colorimetrically as spot forming cells (SFC) by using an alkaline phosphatase conjugated read-out system. Similarly, the ICS assay uses a flow cytometry-based system to measure IFN-γ (and sometimes additional cytokines) produced by antigen-stimulated cells. In this system, the stimulated cells are further characterized by phenotypic lymphocyte markers, allowing for precise quantification of the type of cells (for example CD4+ or CD8+ T-lymphocytes) responding to the vaccine antigens. Humoral immune responses were measured using ELISA assays or a modified assay where the antigens expressed by the adenoviral vectors were bound to the test plate using a lectin capture system.

Immunization with VRC-HIVADV014-00-VP elicited humoral and cellular immune responses in mice, and elicited humoral immune responses in rabbits.

### EXAMPLE 4

This example demonstrates the biodistribution of an adenoviral vector composition administered to a mammal.

Male and female New Zealand White rabbits, approximately 15 weeks old, were divided into two treatment groups. Group 1 consisted of three rabbits of each sex, and group 2 consisted of 15 rabbits of each sex, for a total of 36 rabbits. Group 1 animals received a single intramuscular injection (right thigh muscle) of final formulation buffer (FFB) (0.5 mL/animal) using a needle and syringe on study day ("SD") 1. Group 2 animals received a single intramuscular injection (right thigh muscle) of a 1.0 x 10¹¹ pu dose of VRC-HIVADV014-00-VP.

Animals were observed at least twice daily for moribundity and mortality and clinical signs of toxicity (cageside). A detailed examination was performed at the time animals were weighed (pre-treatment, weekly thereafter, and at necropsy) in lieu of the cageside observations. Clinical signs evaluated included, but were not limited to, skin and fur characteristics, eye and mucus membranes, respiratory, circulatory, autonomic and central nervous systems, and somatomotor and behavior patterns.

Five animals per sex from the test group (Group 2) and one animal per sex from the vehicle control group (Group 1) were sacrificed on study days 9, 61 and 91. Prior to euthanasia, 0.6 mL of blood was collected by puncture of the medial auricular artery into sterile ethylene diamine tetra-acetic acid (EDTA) tubes. Each animal was euthanized by Nembutal sodium injection and exsanguinated. The following organs were collected from each animal using a clean set of instruments for each organ collected: blood, gonads, heart, lung, liver, kidney, lymph nodes, spleen, thymus, subcutis and thigh muscle (at injection site), bone marrow (from femur on side of injection) and brain. The tissues were immediately placed in sterile vials, snap-frozen in liquid nitrogen, and stored at -75° ± 10°C.

An adenoviral vector-specific PCR assay (Taqman™ Polymerase Chain Reaction) was used to detect the presence of the four adenoviral vectors in each tissue sample. The lower limit of detection of the assay was 10 copies of target/µg DNA and the lower limit of quantification was 50 copies of target/µg DNA. Samples that were above the lower limit of detection but below the lower limit of quantification were designated non-quantifiable (NQ). The PCR evaluations were taken from samples harvested on study days 9, 61, and 91. A summary of the tissues exhibiting positive biodistribution results is set forth in Table 1.

No treatment related changes in mortality, clinical signs of toxicity, body weights, or body weight changes were observed. Food consumption in the male group receiving VRC-HIVADV014-00-VP was decreased during the 24-hour period following the injection, but returned to normal after that period.

**Table 1**

| | Marrow | Liver | Spleen | Subcutis | Muscle |
|---|---|---|---|---|---|
| Day 9 | | | | | |
| # positives | 1/10 | 9/10 | 10/10 | 5/10 | 4/10 |
| Avg. copy # | 23 | 945 | 1934 | 8088 | 2751 |
| Day 61 | | | | | |
| # positives | 0/10 | 2/10 | 6/10 | 2/10 | 0/10 |
| Avg. copy # | N/A | 118 | 113 | 232 | N/A |
| Day 91 | | | | | |
| # positives | 0/10 | 0/10 | 5/10 | 0/10 | 0/10 |
| Avg. copy # | N/A | N/A | 124 | N/A | N/A |

The results of this example demonstrate that the composition comprising multiple adenoviral vectors transduces a variety of tissues while exhibiting minimal toxicity.

### EXAMPLE 5

This example demonstrates the immunogenicity of an adenoviral vector composition administered to a mammal.

Two groups of female BALB/c mice were immunized with either an empty adenoviral vector or the VRC-HIVADV014-00-VP adenoviral vector composition diluted in normal saline. Specifically, five mice received an intramuscular injection of 1 x 10¹⁰ pu/animal of empty adenoviral vector, and ten mice received an intramuscular injection of 1 x 10¹⁰ pu/animal of VRC-HIVADV014-00-VP. The total volume injected for each mouse was 200 µL. Ten days after the injection, the mice were bled and sera were collected and stored at 4 °C until tested. Spleens were removed aseptically, gently homogenized to a single-cell suspension, washed, and resuspended to a final concentration of 10⁶ cells/mL.

96-well ELISA plates were coated with 100 µL/well of Lectin-Galanthaus Nivalis (Sigma) and incubated overnight at 4 °C. The lectin was removed and each well was blocked with 200 µL PBS containing 10% fetal bovine serum (FBS) for 2 hours at room temperature. The plates were washed twice with PBS containing 0.2% Tween-20 (PBS-T), and 50 µL of a 1:4 dilution of protein supernatant (~ 1 µg/mL) from 293 cells was added to each well. The supernatant was prepared from 293 cells transfected with DNA plasmids expressing the same HIV-1 clade A, B and C Envelope antigens as the adenoviral vector construct. Total protein from extracts of 293 cells transfected with empty p2000 vector was used as a negative control.

The plates were incubated for one hour at room temperature and washed four times with PBS-T. 50 µL of either control serum (from mice immunized with the control plasmid p2000) or serum from the test plasmid vaccinated mice were added in four-fold serial dilutions to each well, beginning at a dilution of 1:100. The plates were incubated for 1 hour at room temperature, washed, and 50 µL of horseradish peroxidase-conjugated goat antimouse IgG was added to each well. The plates were incubated for 1 hour at room temperature, washed, and 50 µL of substrate (Fast o-Phenylenediamine dihydrochloride, Sigma) were added to each well. The plates were then incubated for 30 minutes at room temperature. The reaction was stopped by the addition of 50 µL of 1(N) H₂SO₄, and optical density was read at 450 nm.

Harvested spleen cells (10⁶ cells/peptide pool) were stimulated for 6 hours. The last five hours of stimulation occurred in the presence of 10 µg/mL brefeldin A (Sigma), with peptide pools having the same amino acid sequences as those expressed by the adenoviral vectors. All peptides used were 15-mers overlapping by 11 amino acids that spanned the complete sequence of the genes tested. Cells were permeabilized, fixed and stained with monoclonal antibodies (rat anti-mouse cell surfaces antigens CD3, CD4 and CD8 (Pharmingen)), followed by multiparametric flow cytometry to detect the IFN-γ and TNF-α positive cells in the CD4+ or CD8+ T-cell population. Statistical analyses in observed CD4+ and CD8+ responses between control plasmid-vaccinated and test article-vaccinated mice were performed by the Mann-Whitney test using Prism 3.0 software (San Diego, CA).

HIV-1-specific cellular immune responses in vaccinated mice were demonstrated by intracellular flow cytometry. Assuming a frequency of greater than 0.1% cytokine producing cells represented a positive result, then CD4+ responses were observed in 3/10 (Gag), 7/10 (Pol), 8/10 (Env-A), 10/10 (Env-B), and 9/10 (Env-C) mice. CD8+ responses were observed in 9/10 (Gag), 10/10 (Pol), 6/10 (Env-A), 6/10 (Env-B), and 7/10 (Env-C) mice. All mice had demonstrable antibody titers (measured by ELISA) to HIV-1 proteins following immunization with VRC-HIVADV014-00-VP.

These results demonstrate that the adenoviral vector composition elicited an immune response in mice.

### EXAMPLE 6

This example demonstrates the immunogenicity of an adenoviral vector composition administered to a mammal.

VRC-HIVADV014-00-VP (1 x 10¹¹ pu) was administered intramuscularly by needle and syringe to a group of 20 rabbits (Group 2), and an equal sized placebo group was used as a control (Group 1). A third group of rabbits (Group 3) was administered a primer composition (VRC-HIVDNA009-00-VP) (4 mg) comprising six plasmids each encoding a clade B Gag, clade B Pol, clade B nef, and Env gp145 from clades A, B and C, respectively. The clade B Pol plasmid also encoded a fusion protein comprising reverse transriptase, protease, and integrase proteins. Point mutations were introduced in the nucleic acid sequences encoding the protease and reverse transcriptase genes of the plasmid, which rendered the reverse transcriptase, protease, and integrase proteins non-functional. Following administration of the primer composition, a dose of VRC-HIVADV014-00-VP (1 x 10¹¹ pu) was administered to the rabbits of Group 3. Group 3 animals were compared to an equal sized placebo group (Group 4).

Following immunization, humoral immune responses were assessed by an ELISA assay. Specifically, plasmids produced at the Vaccine Research Center, National Institutes of Health (Bethesda, MD) (VRC) (i.e., plasmid nos. 5304, 2801, and 5308) which code for HIV-Env A, B, and C, respectively were expressed in 293 cells and purified for the major protein product. Optimized concentrations of the recombinant antigens were coated on microtiter plates and kept at 4°C overnight. The microtiter plates were washed and blocked with 20% FBS/1% BSA buffered solution and incubated. Duplicate wells of serial dilutions of the rabbit sera were incubated followed by Biotin labeled goat and rabbit, Streptavidin-HRPO, and TMB substrate. Color development was stopped and plates were read within 30 minutes at 450 nm, with the reported result based upon the average of duplicate wells.

All serum samples from rabbits in Group 1 and prebleeds for Group 2 exhibited low raw optical densities (OD), with an average OD ± standard deviation of 0.159 ± 0.105 (n= 480) at dilutions of 1:100 and 1:1000. All samples from Group 2 rabbits at day 24 post administration exhibited evidence of seroconversion at serum dilutions of 1:1000. Specifically, raw optical densities for all antigens were greater than 0.21, with the average OD ± standard deviation of 2.71 ± 1.07 (n= 160). All rabbits in Group 2 exhibited detectable antibody concentrations for HIV-ENV-A, ENV-B, ENV-C and GAG.

All samples from rabbits in Group 3 and prebleeds for Group 4 animals exhibited low raw optical densities (OD), with the average prevaccination OD ± standard deviation of 0.099 ± 0.065 (Group 3, n=160 samples) and 0.129 ± 0.138 (Group 4, n=160 samples). In addition, there were very high OD values for all antigens post vaccination for rabbits in Group 4. While some rabbits in Group 4 exhibited higher OD values pre-vaccination, elevated OD values were observed at day 108 (OD = 3.529 ± 0.812), which is indicative of induced immune responses.

This example demonstrates the ability of the inventive method to induce an immune response against HIV in mammals.

### EXAMPLE 7

This example demonstrates the immunogenicity of an adenoviral vector composition administered alone or as part of a DNA prime/adenovirus boost regimen in a mammal.

Outbred adult rhesus monkeys (*Macaca mulatta*) were injected intramuscularly with an adenoviral vector encoding SIVmac239Gag/Pol and HIV-1 Env protein (single or multiclade) (1 x 10¹² pu or 3.3 x 10¹¹ pu) (VRC/NIH, Bethesda, MD) either alone, or in combination with a mixture of research grade SIVmac 239 Gag/Pol-nef plasmid and single or multiclade HIV-1 Env plasmids (VRC/NIH, Bethesda, MD). In each case, vaccine materials were mixed together in sterile saline and delivered as two 0.5 mL injections in the quadriceps muscles using a No. 3 Biojector syringe (Bioject). Animals were immunized at weeks 0, 8, and 26 for the adenoviral vector alone. For the DNA/adenoviral vector prime-boost regimen, monkeys were administered plasmid at weeks 0, 4, 8 and adenoviral vector at week 26. Monkeys were bled every 2-4 weeks through week 90 post-immunization.

ELISPOT assays were utilized to monitor the emergence of vaccine-elicited T cell immune responses to multiple viral antigens. Separate assays were performed for each animal using pools of 15 amino acid peptides overlapping by 11 amino acids spanning the SIV Gag protein, pools of 20 amino acid peptides overlapping by 10 amino acids spanning the HIV-1 Env 89.6P protein (a heterologous clade B Env), and the Mamu-A*01 restricted CTL epitope peptides p11c, p41a, and p68a. 96-well multiscreen plates were coated overnight with 100 µl/well of 5 µg/mL anti-human IFN-γ (B27; BD Pharmingen) in endotoxin-free Dulbecco's PBS (D-PBS). The plates were then washed three times with D-PBS containing 0.25% Tween-20 (D-PBS/Tween), blocked for two h with D-PBS containing 5% FBS at 37°C, washed three times with D-PBS/Tween, rinsed with RPMI 1640 containing 10% FBS to remove the Tween-20, and incubated with peptide pools and 2x10⁵ PBMC in triplicate in 100 µl reaction volumes. Following an 18 h incubation at 37°C, the plates were washed nine times with D-PBS/Tween and once with distilled water. The plates were then incubated with 2 µ/mL biotinylated rabbit anti-human IFN-γ (Biosource) for two hours at room temperature, washed six times with Coulter Wash (Beckman-Coulter), and incubated for 2.5 hours with a 1:500 dilution of streptavidin-AP (Southern Biotechnology). Following five washes with Coulter Wash and one with PBS, the plates were developed with NBT/BCIP chromogen (Pierce), stopped by washing with tap water, air dried, and read using an ELISPOT reader (Hitech Instruments). Spot-forming cells (SFC) per 10⁶ PBMC were calculated. Media backgrounds consistently exhibited less than 15 spot-forming cells per 10⁶ PBMC.

Following a single adenoviral vector immunization, responses to the Gag and Env peptide pools were detected in both monkeys. Four weeks post-immunization, total spot forming cells (SFC) per 10⁶ PBMCs were 2,560 and 2,160 for monkeys Aw13 and AV83, respectively. While monkey AV83 generated enhanced Gag and Env-specific cellular immune responses following the second adenoviral vector immunization on week 8, no change in the responses of monkey Aw13 were observed. Neither monkey demonstrated augmented responses to the third adenoviral vector immunization on week 26. Cellular responses against these vaccine encoded antigens remained durable through week 52 post-immunization in monkeys Aw13 and AV83.

Cellular immune responses directed against the Gag and Env vector-encoded antigens were also analyzed by pooled peptide ELISPOT assays following immunization with a DNA prime/adenoviral vector boost regimen. Following adenoviral vector boost at week 26, cellular immune responses to the Gag and Env peptide pools increased 5-6-fold higher compared to DNA vaccination alone in monkeys Aw2P and Aw28. At Week 30, i.e., four weeks post-immunization, total SFC per 10⁶ PBMCs were 7010 and 7805 for monkeys Aw2P and Aw28, respectively. Cellular responses against these vector-encoded antigens remained durable through week 58 post-immunization, with 4265 and 3000 SFC per 10⁶ PBMC measured in monkeys Aw2P and Aw28.

To assess the contribution of antigen-specific CD4+ and CD8+ T lymphocytes in cellular immunity elicited by the adenoviral vector construct, peptide ELISPOT assays were performed using unfractionated and CD8+ T lymphocyte-depleted PBL on week 28, two weeks following the final adenoviral vector immunization. While potent cellular immune responses were measured against Gag and Env peptide pools using whole PBL, these responses were substantially reduced when CD8+ T lymphocytes were removed from the PBL population, demonstrating that immunizations with adenoviral vectors elicit potent cellular immune responses that are predominantly CD8+ T lymphocyte mediated.

A direct enzyme-linked immunosorbent assay (ELISA) was used to measure plasma titers of anti-gp120 (HIV-MN) and anti-p27 SIVmac239 antibodies (see, e.g., VanCott et al., *J Virol., 73*(6), 4640-50(1999)). Both monkeys had demonstrable antibody titers (measured by ELISA) to gp140 89.6 Envelope proteins following adenoviral vector immunization. Strong homologous neutralizing antibody titers were also measured in all four immunized animals but the magnitude of the responses in the DNA prime/adenovirus boosted animals was several fold higher than those observed after adenoviral vector vaccination alone.

A flow based neutralization assay was used to measure plasma-mediated virus neutralization. Plasma samples were heat-inactivated to deplete complement proteins and tested at a 1:5 dilution. Percent neutralization mediated by week 28 and week 32 were calculated by comparison to the week 0 pre-immune plasma (see, e.g., Mascola et al., J. Virol., 76(10), 4810-21 (2002)). Neutralizing antibodies against HIV-1 89.8 Envelope antigen were also demonstrated. The magnitude of neutralizing antibody responses in the DNA prime/adenoviral vector boosted vaccinated animals was higher than in the adenoviral vector vaccinated animals.

These results show that the adenoviral vector composition can elicit an immune response in a mammal when administered alone, and that the immune response can be enhanced when the adenoviral vector composition is used as part of a DNA prime/adenovirus boost regimen in a mammal.

### EXAMPLE 8

This example demonstrates the use of the inventive method to induce protective immunity against an HIV antigen that is not present in the adenoviral vector composition or the primer composition.

Twenty-four outbred adult Indian-origin rhesus monkeys (*Macaca mulatta*) were injected intramuscularly with DNA constructs expressing SIVmac 239 Gag/Pol DNA, HIV-1 89.6P Env DNA (VRC/NIH, Bethesda, MD), or HXB2/Bal Env DNA, followed by a boost administration of a recombinant adenoviral vector. Because of instability, the research grade adenoviral vector was constructed without Nef (see Letvin et al., Journal of Virology, In press).

In each case, vaccine constructs were mixed together in sterile saline and delivered as two 0.5 mL injections in the quadriceps muscles using a No. 3 Biojector syringe (Bioject). DNA immunization occurred at weeks 0, 4, 8 and adenoviral vector immunization occurred at week 26 (1 x 10¹²pu) for the DNA/adenoviral vector prime-boost regimen. Monkeys were bled every 2-4 weeks through week 90 post-immunization. The following four experimental groups were tested: (1) control, (2) Gag/Pol/Nef DNA and Gag/Pol adenoviral vector with no Env (mock), (3) Gag/Pol/Nef DNA and Gag/Pol adenoviral vector with SHIV-89.6P Env, or 4) Gag/Pol/Nef DNA and Gag/Pol adenoviral vector with HXB2/Bal Env.

All monkeys were challenged intravenously with monkey infectious dose 50 (MID50) SHIV-89.6P on week 38, i.e., 12 weeks following the adenoviral vector boost. Monkeys were bled every 2-4 weeks following both immunization and challenge.

Freshly isolated peripheral blood mononuclear cells (PBMC) were assessed for interferon gamma ELISPOT responses to SIVmac after *in vitro* exposure to peptide pools spanning the SIVmac Gag/Pol/Nef and HIV-1 Env proteins. All Env-specific responses were assessed using peptides that were matched to the Env immunogen. Test systems are described in Letvin et al., *supra.*

ELISPOT responses from the PBMCs of all monkeys receiving experimental immunogens were robust. Cellular immunity to SIV Gag, Pol and Nef was generated in all groups of vaccinated monkeys, and to HIV-1 89.6P and HXB2/Bal Env in monkeys receiving these respective immunogens. Mean total vaccine-elicited ELISPOT responses to all viral proteins two weeks after the final plasmid DNA inoculations were 1,588±554 standard error of the mean (SEM) spot forming cells (SFC) in the mismatched Env group. Two weeks after boosting with recombinant adenoviral vectors, there was a >2.5-fold increase over the cellular immunity elicited by DNA priming alone.

Following challenge with monkey infectious dose 50 (MID50) SHIV-89.6P on week 38, a profound loss of CD4+ T lymphocyte was observed in all controls, while substantial blunting of that CD4+ T lymphocyte depletion was seen in all vaccinated animals. This blunting was most significant in the monkeys that received HIV-1 Env in addition to SIV Gag/Pol-Nef, documenting statistically significant protection against CD4+T lymphocytes loss afforded by inclusion of Env component in the vaccine. Importantly, monkeys that received the mismatched Env immunogens showed comparable protection to those injected with the matched immunogens. The group of monkeys that received the SIV Gag/Pol/Nef + mismatched Env immunogens also demonstrated better containment of virus, indicated by reduced viral loads.

These results show that the adenoviral vector composition can be used to elicit an immune response to HIV in a mammal.

### EXAMPLE 9

This example demonstrates the cellular immune responses elicited by an adenoviral vector composition administered as part of a DNA prime/recombinant adenovirus boost regimen in a mammal.

Outbred adult rhesus monkeys (*Macaca mulatta*) were injected intramuscularly with mixtures of GLP grade plasmid DNA vectors encoding SIV Gag/Pol/Nef proteins and multiclade A, B, and C HIV-1 Env proteins contained in a composition designatedVRC-HIVDNA009-00-VP. An adenoviral vector encoding SIVmac 239 Gag/Pol and an adenoviral vector encoding HIV-1 clade A, B, and C Env were used to boost.

In each case, plasmids or adenoviral vector were mixed together in sterile saline and delivered as two 0.5 mL injections in the quadricep muscles using a No. 3 Biojector syringe (Bioject). Animals were immunized at weeks 0, 4, and 8 with plasmid DNA, and week 26 with adenoviral vector. Animals were bled every 2-4 weeks through week 42. The specific prime and boost immunizations are set forth in Tables 2 and 3, respectively.

**Table 2**

| **Group** | **Number of Animals** | **SIV Gag/Pol/Nef Plasmid** | **HIV-1 Env Plasmid(s)** | **Sham Plasmid** |
|---|---|---|---|---|
| 1 | 6 | 4.5 mg | 4.5 mg (clade B) | - |
| 2 | 6 | 4.5 mg | 4.5 mg (clade C) | - |
| 3 | 6 | 4.5 mg | 1.5 mg (clade A) 1.5 mg (clade B) | - |
| | | | 1.5 mg (clade C) | |
| 4 | 6 | 4.5 mg | 1.5 mg (clade B) | 3.0 mg |
| 5 | 6 | - | - | 9.0 mg |

**Table 3**

| **Group** | **Number of Animals** | **SIV Gag/Pol adenoviral vector (pu)** | **HIV-1 Env adenoviral vector(s) (pu)** | **Sham adenoviral vector** |
|---|---|---|---|---|
| 1 | 6 | 1.0 x 10¹² | 1.0 x 10¹² (clade B) | - |
| 2 | 6 | 1.0 x 10¹² | 1.0 x 10¹² (clade C) | - |
| 3 | 6 | 1.0 x 10¹² | 3.3 x 10¹¹ (clade A) | - |
| | | | 3.3 x 10¹¹ (clade B) | |
| | | | 3.3 x 10¹¹ (clade C) | |
| 4 | 6 | 1.0 x 10¹² | 3.3 x 10¹¹ (clade B) | 6.6 x 10¹¹ |
| 5 | 6 | - | - | 2.0 x 10¹² |

ELISPOT assays were utilized to monitor the emergence of vaccine-elicited T cell immune responses to multiple viral antigens. Separate assays were performed for each animal using pools of 15 amino acid peptides overlapping by 11 amino acids spanning the SIV Gag, SIV Pol, SIV Nef, HIV-1 Env clade A, HIV-1 Env clade B, and HIV-1 Env clade C proteins matching the sequences of the immunogens encoded by the adenoviral vectors. Assays were also performed using pools of 20 amino acid peptides overlapping by 10 amino acids spanning HIV-1 Env 89.6P, which is a clade B Env sequence heterologous to the immunogens encoded by the adenoviral vectors. 96-well multiscreen plates were coated overnight with 100 µl/well of 5 µg/mL anti-human IFN-γ (B27; BD Pharmingen) in endotoxin-free Dulbecco's PBS (D-PBS). The plates were then washed three times with D-PBS containing 0.25% Tween-20 (D-PBS/Tween), blocked for 2 hours with D-PBS containing 5% FBS at 37 °C, washed three times with D-PBS/Tween, rinsed with RPMI 1640 containing 10% FBS to remove the Tween-20, and incubated with peptide pools and 2 x 10⁵ PBMC in triplicate in 100 µl reaction volumes. Following 18 hours incubation at 37 °C, the plates were washed nine times with D-PBS/Tween and once with distilled water.

The plates were then incubated with 2 µg/mL biotinylated rabbit anti-human IFN-γ (Biosource) for 2 hours at room temperature, washed six times with Coulter Wash (Beckman-Coulter), and incubated for 2.5 hours with a 1:500 dilution of streptavidin-AP (Southern Biotechnology). Following five washes with Coulter Wash and one with PBS, the plates were developed with NBT/BCIP chromogen (Pierce), stopped by washing with tap water, air dried, and read using an ELISPOT reader (Hitech Instruments). Spot-forming cells (SFC) per 10⁶ PBMC were calculated. Media backgrounds were consistently less than 15 spot-forming cells per 10⁶ PBMC.

The extent of cross-clade reactivity of cellular immune responses elicited by single clade Env immunization was investigated by assessing responses in Group 1 (high clade B Env) and Group 2 (high clade C Env). For the DNA prime immunizations, monkeys received 4.5 mg Gag/Pol/Nef plasmid with 4.5 mg of Env plasmid from clade B (Group 1) or clade C (Group 2). PBMCs were tested for Env-specific cellular immune responses by pooled peptide ELISPOT assays using peptide pools from Env clade A, Env clade B, Env clade C, and Env 89.6P (a heterologous clade B Env). Monkeys in Group 1 that received the Env clade B plasmid generated responses to all Env peptide pools, demonstrating a degree of cross-clade reactivity. However, clade B peptide responses were higher than clade A or clade C responses. The DNA primed cellular immune responses of monkeys in Group 1 were dramatically augmented following the boost immunization with 1.0 x 10¹² pu Gag/Pol and 1.0 x 10¹² pu clade B Env adenoviral vector. While responses to all Env peptide pools were observed from these monkeys following the adenoviral vector boost immunization, all six animals demonstrated the highest response to clade B Env.

Similarly, monkeys in Group 2 that received the Env plasmid and adenoviral vector from clade C generated responses to all Env peptide pools. Clade C responses were higher than clade A or clade B responses in all six animals following the DNA prime immunizations and following the adenoviral vector boost. These data demonstrate that DNA prime/adenoviral vector boost immunization with single clade Env immunogens elicits Env-specific cellular immune responses with partial cross-clade reactivity, but that the highest responses were generally against the Env clade matching the immunogen.

The Env-specific cellular immune responses of monkeys in Group 4 (low clade B Env) were comparable with responses of monkeys in Group 1 (high clade B Env). Monkeys in Group 4 received 4.5 mg Gag/Pol/Nef plasmid with 1.5 mg Env plasmid from clade B for the DNA prime immunizations, and 1.0 x 10¹² pu Gag/Pol adenoviral vector with 3.3 x 10¹¹ PU clade B Env adenoviral vector for the boost immunization. These observations suggest that lowering the dose of a single Env plasmid or adenoviral vector threefold does not result in major reductions in immunogenicity. Minimal background responses were observed in monkeys in Group 5 that received only sham plasmids and adenoviral vector.

The breadth and magnitude of cellular immune responses elicited by the multiclade Env immunizations were investigated by assessing responses in Group 3 (clade A+B+C Env). For the DNA prime immunizations, these monkeys received 4.5 mg Gag/Pol/Nef plasmid with 1.5 mg of each Env plasmid from Clades A, B, and C (4.5 mg Env plasmids total). Similar magnitude and broad cellular immune responses to Env clade A, B, and C were observed. These data demonstrate that the mixture of the three Env plasmids in Group 3 resulted in increased breadth without loss of magnitude of the responses, despite the fact that each Env plasmid component in Group 3 was given at the 1.5 mg rather than the 4.5 mg dose. Following the boost immunization with 1.0 X 10¹² pu Gag/Pol adenoviral vector and 3.3 x 10¹¹ pu Env adenoviral vector of each clade A, B, and C, all six monkeys demonstrated similar magnitude responses to clade A, B, and C Env peptide pools. These data demonstrate that the magnitude of each individual clade-specific response in Group 3 was comparable with the optimal clade-specific response elicited in Groups 1 and 2.

Cellular immune responses to SIV Gag and Pol were observed in all vaccinated monkeys following DNA immunization and following adenoviral vector boost. Monkeys receiving the four-component multiclade vaccine product (Group 3) elicited similar magnitude cellular immune responses to SIV Gag and SIV Pol as observed in monkeys receiving single clade Env immunogens. The four-component multiclade vaccine (Group 3) thus resulted in broader responses to all vaccine-encoded antigens without loss of immunogenicity as compared with the single clade vaccines (Groups 1, 2, and 4). Furthermore, cellular immune responses to these antigens were found to be durable following both DNA prime and adenoviral vector boost immunization.

The humoral immune responses elicited by single clade and multiclade Env immunizations were investigated by assessing Env-specific antibody titers from monkeys following adenoviral vector boost immunization. Plasma samples were tested for Env clade A, clade B, or clade C specific antibody binding activity as measured by ELISA.

Endpoint titers were determined for week 10 (post DNA) and week 40 (post adenoviral vector) as the last dilution with pre-immunization corrected optical density (OD) greater than 0.2. Wells were coated with 37.5 ng purified Env antigen overnight at 4°C. Plates were washed, and blocked (20%FBS/1%BSA buffered solution) for 1 hour at 37°C. Duplicate wells of serial dilutions of the sera were incubated 2 hours at 37°C followed by Biotin labeled goat anti-monkey (1 hour 37°C), streptavidin-HRPO (30 minutes, room temperature (RT)), and TMB substrate (30 minutes, RT). Color development was stopped by adding sulfuric acid and plates were read within 30 minutes at 450 nm, with reported results based upon the average of duplicate wells.

Monkeys in Group 1 (high clade B Env) generated antibody responses that were capable of binding to all three Env antigens, demonstrating a degree of cross-clade reactivity. While robust responses were measured against the clade B and C Env antigens, the highest antibody titers were detected against the homologous clade B Env. Monkeys in Group 4 (low clade B Env) exhibited Env-specific antibody titers that were similar in breadth and magnitude as to those measured in Group 1 monkeys, demonstrating that lowering the dose of Env immunogen threefold did not result in reduced immunogenicity. Monkeys in Group 2 (high clade C Env) similarly elicited antibody responses capable of recognizing all three Env antigens, but highest titers were detected against the homologous clade C Env. Monkeys immunized with the mixture of clade A, clade B, and clade C Env antigens (Group 3), however, demonstrated high magnitude antibody titers to all three Env antigens.

These data suggest that multiclade Env immunization resulted in increased breadth of the humoral immune response without a loss of immunogenicity when compared to responses elicited by single clade Env immunization.

### EXAMPLE 10

This example demonstrates the cellular and humoral immune responses elicited by an adenoviral vector composition administered as part of a DNA prime/recombinant adenovirus boost regimen in a mammal.

Outbred adult Cynomolgus macaques were injected intramuscularly with mixtures of vaccine plasmids or adenoviral vector constructs. Specifically, GLP plasmid DNA expressing Gag/Pol/Nef proteins and multiclade A, B, and C HIV-1 Env proteins contained in composition VRC-HIVDNA009-00-VP (Example 8) were used for the prime immunization. GMP grade VRC-HIVADV014-00-VP (Example 1) was used as the adenoviral vector boost.

To achieve the required volumes for the three scheduled injections in the animal study, three lots of formulated material were prepared. The three lots were combined in a 50 mL conical tube. Following inversion of the tube several times to mix, 15.6-15.7 mL of the mixture was aliquotted into each of three 50 mL conical tubes. Tubes were labeled and stored at -20 °C until distributed.

8 mg of the DNA composition was delivered intramuscularly (i.m.) at weeks 0, 4, and 8 by Biojector and 10¹¹ pu total adenoviral vector vaccine construct was delivered i.m. by needle and syringe at week 38. Animals were bled every 2-4 weeks through week 42.

ELISPOT assays were utilized to monitor the emergence of vaccine-elicited T cell immune responses to multiple viral antigens as described in Example 8. A direct ELISA was used to measure plasma titers of Env clade A, clade B, and clade C antibodies as described in Example 8.

Monkeys that received the DNA plasmid vaccine prime and adenoviral vector boost generated responses to clade A, B, and C Env peptide pools in all six animals following the DNA prime immunizations and following the adenoviral vector boost. Five of six animals developed antibody responses to all three Envelope antigens (clade A, B, and C). One animal developed a humoral immune responses to clade A and C Envelope only. All six monkeys had strong Env antibody responses after adenovirus boost.

These data demonstrate that the clinical DNA prime/adenoviral vector product is immunogenic and induces cellular immune responses against clade A, B, C Env as well as Gag and Pol, and antibody responses against clade A, B, and C Env as well as Gag. Adenovector boosting increases the immune responses several fold.

### EXAMPLE 11

This example demonstrates cellular immune responses elicited by recombinant adenovirus boost immunizations in Cynomolgus monkeys. Six cynomolgus macaques (*Mauritius* origin) were immunized once intramuscularly with a 1 x 10¹¹ pu dose of the adenoviral vector composition VRC-HIVADV014-00-VP (Example 1). The composition was delivered as two 0.5 mL injections in the quadricep muscles using a needle and syringe. Monkeys were bled every 2-4 weeks through week 4 post-immunization. ELISPOT assays were utilized to monitor the emergence of vaccine-elicited T cell immune responses to multiple viral antigens, as described in Example 8.

Monkeys that received the adenoviral vector generated responses to clade A, B, and C Env peptide pools in all six animals. These data demonstrate that the clinical adenoviral vector product is immunogenic and induces cellular immune responses against clade A, B, C Env, as well as Gag and Pol.

The animals were clinically evaluated by a Laboratory Animal Medicine certified veterinarian after chemical anesthesia by ketamine hydrochloride at pre-immunization (week -1), week 0 (1^{st} immunization), and 1, 2, 3, 4, 5, and 8 week time points. Serum chemistries and complete blood count were determined at weeks -1, 3, and 5. Subject animals were found to be healthy and in excellent condition at all time points evaluated. Physical examination included auscultation, palpation, and determination of body temperature, pulse, and respiration. Body temperatures, pulse, and respiration were within normal limits. A pea-sized inguinal lymph node was detected in two monkeys (CO 7422 and CO 7414) at weeks 1 and 5, respectively, on the ipsilateral side of the inoculation. White blood counts and hematocrit values were generally within normal limits and with minimal variation between pre- and post-immunization time points for all animals. Serum electrolytes, blood urea nitrogen and creatinine were also within normal limits.

All animals had aspartate aminotransferase/glutamic oxaloacetic transaminase (AST/GOT), alkaline phosphatase, and total bilbubin levels with normal limits in pre-immunization serum as well as at immunization. Animal CO 7412 had a pre-immunization alanine aminotransferase/glutamic pyruvic transaminase (ALT/GPT) of 97 U/L (normal range 0-138 U/L). The ALT was slightly elevated after immunization (177 U/L at 3 weeks, 166 U/L at 5 weeks), but within normal limits (136 U/L) at 8 weeks post immunization. Enzymes creatinine kinase and lactate dehydrogenase were minimally increased at week 5 in animals CO 7423 and CO 7420, which most likely represented ketamine induced muscle damage. The values returned to normal at week 8.

These data demonstrate that the inventive method elicited potent and broad cellular immune responses against all viral antigens in cynomolgus macaques.

### EXAMPLE 12

This example demonstrates the safety of an adenoviral vector composition administered to a mammal.

Female and male New Zealand white rabbits were administered via intramuscular injection a DNA priming construct (VRC-HIVDNA009-00-VP) and the adenoviral vector construct VRC-HIVADV014-00-VP as a boost, or VRC-HIVADV014-00-VP alone. VRC-HIVADV014-00-VP was produced as described in Example 1.

For the DNA prime/adenovirus boost method, 4 mg of VRC-HIVDNA009-00-VP or the PBS control (study day 1, 22) were administered via two intramuscular injections (0.5 mL/injection site; dose volume for each injection was not adjusted for body weight) per day of dosing into the thigh muscle (two injections spaced approximately 1 inch apart) using a Biojector 2000® Needle-Free Injection Management System™ (Bioject). Injections were administered on alternate sides for each time point. Each injection was administered at a shaved/marked site. The site was re-shaved and re-marked as needed in order to visualize the injection site.

For both the DNA prime/adenovirus boost study and the study involving only the adenoviral vector, VRC-HIVADV014-00-VP (1 x 10¹¹ pu) or the diluent control (VRC-DILUENT013-DIL-VP) injections were administered as two 0.5 mL injections per day of dosing into the hind thigh muscle with a needle and syringe. Each injection was administered at a shaved/marked site. The site was re-shaved and re-marked as needed in order to visualize the injection site.

Animals were randomly assigned to treatment groups. The treatment period was 22 days, and the study duration was 36 days. Injections were administered on alternate sides for each time point. 1.0 mL was administered regardless of body weight for DNA and adenovector doses and their respective controls.

Blood samples (approximately 2 mL) were isolated from all animals prior to administration of the first dose. The samples were subjected to hematology, chemistry, coagulation, and immunology analyses. Serum was isolated and stored at -75°C ± 10°C for transfer on dry ice. Some or all of these samples were analyzed for seroconversion as an indication of exposure to the test article.

Following terminal blood collection, all animals were euthanized by sodium pentobarbital or equivalent injection and exsanguinated. Animals were necropsied as close as possible to the time of sacrifice. Scheduled necropsies were conducted under the supervision of a veterinary pathologist.

All required animals were subjected to a full gross necropsy, which included examination of the external surface of the body, the injection/treatment site, all orifices, and the cranial, thoracic, and abdominal cavities and their contents. Two bone marrow smears were prepared from the sternum of each animal. Slides were air-dried, fixed in methanol, and archived for possible future evaluation. The following organs (sex appropriate) were weighed as soon as possible from all required animals at scheduled necropsy: adrenal glands, heart, lung, brain, spleen, kidneys, liver (with drained gallbladder), testes/ovaries, pituitary, thymus, uterus, and thyroids/parathyroids. Paired organs were weighed together.

All tissues from each necropsied animal were preserved in 10% neutral buffered formalin (NBF). The tissues were embedded in paraffin, sectioned, stained with hematoxylin and eosin, and examined microscopically by a board certified veterinary pathologist. Tissues from each animal and from gross lesions (from all groups) were analyzed.

Quantitative results were analyzed using the Kolmogorov-Smirnov test for normality, the Levene Median test for equal variance, and by one-way Analysis of Variance (ANOVA). If either the normality or equal variance test failed, then the analysis employed the non-parametric Kruskal-Wallis ANOVA on rank-transformed data. For parametric data, if the ANOVA indicated statistical significance among experimental groups then the Dunnett's t-test was used to delineate which groups (if any) differed from the control. For non-parametric data, if the ANOVA indicated statistical significance among experimental groups then the Dunn's test was used to delineate which groups (if any) differed from the control. The probability value of less than 0.05 (two-tailed) was used as the critical level of significance for all tests. Statistical analysis utilized SigmaStat™ Statistical Software (Jandel Scientific, San Rafael, CA).

For the immunization strategy involving administration of VRC-HIVADV014-00-VP alone, all animals survived to the scheduled termination and no treatment-related effects were noted in the following parameters: mortality, clinical and cageside observations, Draiz observations, body weights, ophthalmology, clinical pathology, and organ weights (with exception of an increased spleen weight, which is likely an expected result of exposure to an immunostimulatory agent) or organ weight ratios. There were increased body temperatures in the treated animals 24 hours after the first injection. There was also decreased food consumption in the treated animals for the 24-48 hour period after each injection. Transient inflammation at the injection site in treated animals was observed, as was recoverable, chronic inflammation in the connective tissue around the sciatic nerve and adjacent lymphatics and blood capillaries. Transient increases in cholesterol and triglyceride levels at SD 3 were not associated with clinical symptoms or pathology, and the transient increase in CPK at SD 24 was possibly related to muscle inflammation.

For the DNA prime/adenoviral vector boost strategy, recoverable inflammation at the injection sites (observed by Draize scoring and histopathologically) and perineural tissue around the sciatic nerves (seen only histopathologically) were observed. In addition, fevers were noted in immunized rabbits in the 24 hours subsequent to the initial and in immunized females in the three hours subsequent to the second adenovector boost. Food consumption was also reduced in the 24-48 hours subsequent to each vaccination, although this resolved and did not impact body weights or weight gain in males. However, treated females did have reduced body weights and weight gains compared to control females, which became statistically significant after SD71 (body weight) and after the initial adenovector boost (weight gains), but which began to be observed as early as SD36 (during the DNA priming series).

This example demonstrates that the inventive method induces minimal toxicity in rabbits.

### EXAMPLE 13

This example demonstrates the administration of an adenoviral vector composition to humans.

A randomized, placebo-controlled, double-blinded, dose escalation study was initiated to examine safety, tolerability and immune response in humans following a single injection of VRC-HIVADVO14-00-VP at a dose of 1 x 10⁹ pu, 1 x 10¹⁰ pu, or 1 x 10¹¹ pu. Each treatment group included 12 subjects (10 vaccines; 2 placebos). The study was initiated on July 19, 2004 and the study completed enrollment of 36 subjects on November 10, 2004. The NIAID Intramural Data and Safety Monitoring Board (DSMB) reviewed the preliminary safety data through 14 days of follow-up prior to each dose escalation. The preliminary data indicated that VRC-HIVADV014-00-VP appears to be safe for healthy subjects at the three dose levels evaluated. The 1 x 10⁹ pu and 1 x 10¹⁰ pu dose levels were associated with less reactogenicity than the 1 x 10¹¹ pu dose level. In both the 1 x 10⁹ pu and 1 x 10¹⁰ pu dose groups, the local and systemic symptoms recorded on the 5-day diary card were none to mild in severity, and none of the subjects experienced fever. In the 1 x 10¹¹ pu dose group, four subjects reported fever on Day 1 (3 mild and 1 moderate in severity). Each of the four subjects with fever also reported moderate headache on Day 1 and three of these subjects also reported at least one other moderate systemic parameter (e.g., malaise, myalgia, and chills). Two subjects without fever reported at least one moderate systemic symptom (e.g., malaise, myalgia, and nausea). One subject in the 1 x 10¹¹ pu dose group reported moderate injection site pain; injection site reactogenicity was otherwise none or mild.

As of January 31, 2005, there was one grade 4 (potentially life-threatening) event. There were three grade 2 (moderate) adverse events that were possibly related to vaccination. The study was blinded to vaccine vs. placebo injection assignments. The grade 4 adverse event was a seizure that occurred 64 days after study injection in a healthy subject in the 1 x 10¹¹ pu dose group who had a history of a single seizure three years prior to study enrollment. Given history of a prior seizure and the timing of the event more than 2 months after study injection, it seemed unlikely that the seizure was related to study agent. The grade 2 adverse events possibly related to study agent included: (1) asymptomatic neutropenia noted 21 days after study injection in a subject known to sometimes have asymptomatic low neutrophil counts prior to enrollment, (2) diarrhea (duration one day) in a different subject on the third day after study injection, and (3) steatohepatitis (fatty liver) diagnosed after extensive evaluation to identify the cause of a persistent grade 1 ALT (alanine aminotransferase) elevation that was noted starting 25 days after the study vaccination in a clinically asymptomatic subject. A hepatology consultant reported an impression that the condition likely existed prior to study enrollment. Contributing factors to the persistent grade 1 ALT may be alcohol consumption and recent weight gain. A diagnosis of steatohepatitis is overall considered to be a grade 2 condition, but as of January 31, 2005, the liver function tests were not more than grade 1 in severity.

Although more reactogenicity was observed with the 1 x 10¹¹ pu dose, it appeared to be a well-tolerated dose and analgesic/antipyretic nonprescription medications can be self administered for relief of the short-term symptoms. A protocol-specified interim immunogenicity analysis is in progress to compare the placebo and three dosage groups. The blinded immunogenicity data suggest a dose effect with increasing immune response at higher doses. The number of subjects with vaccine-induced ELISA at study week 12 by commercial HIV-antibody assay increased from 3 in the 1 x 10⁹ pu group, to 6 in the 1 x 10¹⁰ pu group, and to 9 in the 1 x 10¹¹ pu group among the 12 subjects (two placebos and ten vaccine recipients) per group (study assignments were blinded). The reactogenicity data are summarized in Table 4 below.

**Table 4**

| **Reactogenicity** | **10⁹ pu or placebo (N=12)** | **10¹⁰ pu or placebo (N=12)** | **10¹¹ pu or placebo (N=12)** |
|---|---|---|---|
| *Local Symptoms* | | | |
| None | 9 (75%) | 3 (25%) | 2(16.7%) |
| Mild | 3 (25%) | 9 (75%) | 9 (75.0%) |
| Moderate | 0 | 0 | 1 (8.3%) |
| Severe | 0 | 0 | 0 |
| | | | |

| *Systemic Symptoms* | | | |
|---|---|---|---|
| None | 10 (83.3%) | 4 (33.3%) | 3 (25%) |
| Mild | 2 (16.7%) | 8 (66.7%) | 3 (25%) |
| Moderate | 0 | 0 | 6 (50%) |
| Severe | 0 | 0 | 0 |

These results indicate that the inventive method is well tolerated in humans.

### EXAMPLE 14

This example demonstrates the administration of VRC-HIVADV014-00-VP to humans.

A second Phase I study of the adenoviral vector composition VRC-HIVADV014-00-VP as single agent in uninfected adult subjects is currently in progress. This blinded, dose escalation study is designed to enroll two groups of 24 subjects with low Ad5 antibody titer (<1:12), who will be randomized to VRC-HIVADV014-00-VP or placebo in a 5:1 ratio. The first group of vaccines will receive 1 x 10¹⁰ pu VRC-HIVADV014-00-VP and the second group will receive 1 x 10¹¹ pu VRC-HIVADV014-00-VP.

### EXAMPLE 15

This example demonstrates the administration of VRC-HIVADV014-00-VP to humans as a booster following immunization with a DNA molecule.

A Phase I, blinded, placebo-controlled study has been initiated, which provides a single adenoviral vector boost of VRC-HIVADV014-00-VP at 1 x 10¹⁰ pu (or placebo) to participants who complete a DNA injection regimen with VRC-HIVDNA009-00-VP. The adenoviral vector vaccine boost will be given at an interval of six to nine months after the initial DNA vaccination with VRC-HIVDNA009-00-VP (or placebo injection). The first participant was enrolled on November 22, 2004. As of December 21, 2004, 11 participants have received their boost injection. Of these participants, six have experienced mild pain and/or tenderness at the injection site. There have been no other reports of local reactogenicity events. Five participants reported either mild or moderate systemic symptoms including headache, malaise and nausea. There have been no reports of fever, no grade 3 events, and no serious adverse events.

These results indicate that the inventive method is well tolerated in humans.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.
The present invention relates to the following items:
1. A method of inducing an immune response against a human immunodeficiency virus (HIV) in a mammal comprising administering to the mammal an adenoviral vector composition, wherein the adenoviral vector composition comprises one or more adenoviral vectors encoding two or more different HIV antigens, whereupon the HIV antigens are produced in the mammal and an immune response against HIV is induced.
2. The method of item 1, wherein the adenoviral vector composition comprises two or more adenoviral vectors encoding the two or more different HIV antigens, and each adenoviral vector comprises a nucleic acid sequence that encodes at least one of the two or more different HIV antigens.
3. The method of item 2, wherein the adenoviral vector composition comprises three or more adenoviral vectors encoding three or more different HIV antigens, and each adenoviral vector comprises a nucleic acid sequence that encodes at least one of the three or more different HIV antigens.
4. The method of item 3, wherein the adenoviral vector composition comprises four or more adenoviral vectors encoding four or more different HIV antigens, and each adenoviral vector comprises a nucleic acid sequence that encodes at least one of the four or more different HIV antigens.
5. The method of item 1, wherein the one or more adenoviral vectors each comprise (i) a nucleic acid sequence that encodes two or more different HIV antigens, or (ii) two or more nucleic acid sequences that each encode a different HIV antigen.
6. The method of item 5, wherein the one or more adenoviral vectors each comprise a nucleic acid sequence that encodes two or more different HIV antigens.
7. The method of item 5, wherein the one or more adenoviral vectors each comprise two or more nucleic acid sequences that each encode a different HIV antigen.
8. The method of any of items 1-7, further comprising administering to the mammal a primer composition comprising one or more nucleic acid sequences that encode at least one HIV antigen that is the same as an HIV antigen encoded by an adenoviral vector of the adenoviral vector composition, wherein the administration of the primer composition is performed at least one week before the administration of the adenoviral vector composition.
9. The method of item 8, wherein the primer composition comprises one or more nucleic acid sequences that encode two or more HIV antigens that are the same as the HIV antigens encoded by the one or more adenoviral vectors of the adenoviral vector composition.
10. The method of item 8 or item 9, wherein the administration of the primer composition is performed about six months to about nine months before the administration of the adenoviral vector composition.
11. The method of any of items 8-10, wherein the primer composition comprises one or more plasmids, naked DNA molecules, or viral vectors comprising the one or more nucleic acid sequences.
12. The method of any of items 1-11, wherein the adenoviral vectors are replication-deficient.
13. The method of item 12, wherein the adenoviral vectors are deficient in one or more essential gene functions of the E1 region of the adenoviral genome.
14. The method of item 12 or 13, wherein the adenoviral vectors are deficient in one or more essential gene functions of the E4 region of the adenoviral genome.
15. The method of any of items 1-14, wherein the adenoviral vectors are deficient in one or more gene functions of the E3 region of the adenoviral genome.
16. The method of any of items 1-15, wherein at least one HIV antigen is selected from the group consisting of an HIV Gag protein, HIV Pol protein, HIV Env protein, HIV Tat protein, HIV Reverse Transcriptase (RT) protein, HIV Vif protein, HIV Vpr protein, HIV Vpu protein, HIV Vpo protein, HIV Integrase protein, HIV Nef protein, and a fusion protein comprising all or part of an HIV Gag protein, HIV Pol protein, or HIV Env protein.
17. The method of item 16, wherein at least one HIV antigen is HIV gp140 or gp140dv12.
18. The method of item 16, wherein at least one HIV antigen is a fusion protein that comprises all or part of an HIV Gag protein and all or part of an HIV Pol protein.
19. The method of any of items 1-18, wherein the HIV antigens comprise at least one member selected from the group consisting of an HIV clade A antigen, HIV clade B antigen, HIV clade C antigen, and HIV clade MN antigen.
20. The method of item 19, wherein the HIV antigens comprise at least two members selected from the group consisting of an HIV clade A antigen, HIV clade B antigen, HIV clade C antigen, and HIV clade MN antigen.
21. The method of item 20, wherein the adenoviral vectors encode three or more different HIV antigens, and the HIV antigens comprise at least three members selected from the group consisting of an HIV clade A antigen, HIV clade B antigen, HIV clade C antigen, and HIV clade MN antigen.
22. The method of any of items 1-21, wherein the adenoviral vector composition is administered as part of a pharmaceutical composition comprising a pharmaceutically acceptable carrier.
23. The method of item 22, wherein the pharmaceutical composition is administered in two or more doses.
24. The method of item 22 or 23, wherein the pharmaceutical composition is administered in a dose comprising 1×10⁸ to 1×10¹² particle units (pu) adenoviral vector.
25. The method of item 24, wherein the pharmaceutical composition is administered in a dose comprising 1x10⁸ to 1x10¹⁰ pu adenoviral vector.
26. The method of item 24, wherein the pharmaceutical composition is administered in a dose comprising 1x10⁹ to 1x10¹¹ pu adenoviral vector.
27. The method of item 24, wherein the pharmaceutical composition is administered in a dose comprising 1x10¹⁰ to 1x10¹² pu adenoviral vector.
28. The method of any of items 1-27, wherein the adenoviral vector composition comprises (a) an adenoviral vector comprising a nucleic acid encoding a fusion protein comprising an HIV clade B Gag protein and Pol protein, (b) an adenoviral vector comprising a nucleic acid encoding an HIV clade A Env protein, (c) an adenoviral vector comprising a nucleic acid encoding an HIV clade B Env protein, and (d) an adenoviral vector comprising a nucleic acid encoding an HIV clade C Env protein.
29. The method of item 28, wherein the fusion protein comprising an HIV clade B Gag protein and Pol protein is encoded by a nucleic acid sequence that further encodes HIV Protease, Reverse Transcriptase (RT), and Integrase proteins, and wherein the nucleic acid molecule comprises one or more point mutations, which point mutations render the Protease, RT, and Integrase proteins non-functional.
30. The method of item 28, wherein the Env protein is gp140 or gp140dv12.
31. The method of item 1, wherein the adenoviral vector composition comprises four adenoviral vectors having the nucleic acid sequences of SEQ ID NO: 4, SEQ ID NO: 5, SEQ ID NO: 6, and SEQ ID NO: 7, respectively.
32. An adenoviral vector composition comprising (a) an adenoviral vector comprising a nucleic acid encoding a fusion protein comprising an HIV clade B Gag protein and Pol protein, (b) an adenoviral vector comprising a nucleic acid encoding an HIV clade A Env protein, (c) an adenoviral vector comprising a nucleic acid encoding an HIV clade B Env protein, and (d) an adenoviral vector comprising a nucleic acid encoding an HIV clade C Env protein.
33. The adenoviral vector composition of item 32, wherein the fusion protein comprising an HIV clade B Gag protein and Pol protein is encoded by a nucleic acid sequence that further encodes HIV Protease, Reverse Transcriptase (RT), and Integrase proteins, and wherein the nucleic acid molecule comprises one or more point mutations, which point mutations render the Protease, RT, and Integrase proteins non-functional.
34. The adenoviral vector composition of item 32, wherein the Env protein is gp140 or gp140dv12.
35. The adenoviral vector composition of item 32, wherein (a), (b), (c), and (d) have the nucleic acid sequences of SEQ ID NO: 4, SEQ ID NO: 5, SEQ ID NO: 6, and SEQ ID NO: 7, respectively.
36. The adenoviral vector composition of any of items 32-35, wherein the adenoviral vectors are replication-deficient.
37. The adenoviral vector composition of item 36, wherein the adenoviral vectors are deficient in one or more essential gene functions of the E1 region of the adenoviral genome.
38. The adenoviral vector composition of item 36 or 37, wherein the adenoviral vectors are deficient in one or more essential gene functions of the E4 region of the adenoviral genome.
39. The adenoviral vector composition of any of items 32-38, wherein the adenoviral vectors are deficient in one or more gene functions of the E3 region of the adenoviral genome.
40. The adenoviral vector composition of any of items 32-39, wherein (a), (b), (c), and (d) are present in the composition in a ratio of 3:1:1:1 by weight.
41. A pharmaceutical composition comprising the adenoviral vector composition of any of items 32-40 and a pharmaceutically acceptable carrier.
42. The pharmaceutical composition of item 41, wherein (a), (b), (c), and (d) have the nucleic acid sequences of SEQ NO: 4, SEQ NO: 5, SEQ NO: 6, and SEQ ID NO: 7, respectively.
43. The pharmaceutical composition of item 41 or 42, comprising about 1x10⁸ to 1x10¹² particle units (pu) adenoviral vector.
44. The pharmaceutical composition of item 43, comprising about 1x10⁸ to 1x10¹⁰ pu adenoviral vector.
45. The pharmaceutical composition of item 43, comprising about 1x10⁹ to 1x10¹¹ pu adenoviral vector.
46. The pharmaceutical composition of item 43, comprising about 1x10¹⁰ to 1x10¹² pu adenoviral vector.
Further, the present invention also relates to the following items:
1. Use of an adenoviral vector composition for the manufacture of a medicament for inducing an immune response against a human immunodeficiency virus (HIV) in a mammal, wherein the adenoviral vector composition comprises one or more adenoviral vectors encoding two or more different HIV antigens, wherein said adenoviral vector composition is administered to the mammal, whereupon the HIV antigens are produced in the mammal and an immune response against HIV is induced.
2. The use of item 1, wherein the adenoviral vector composition comprises two or more adenoviral vectors encoding the two or more different HIV antigens, and each adenoviral vector comprises a nucleic acid sequence that encodes at least one of the two or more different HIV antigens.
3. The use of item 2, wherein the adenoviral vector composition comprises three or more adenoviral vectors encoding three or more different HIV antigens, and each adenoviral vector comprises a nucleic acid sequence that encodes at least one of the three or more different HIV antigens.
4. The use of item 3, wherein the adenoviral vector composition comprises four or more adenoviral vectors encoding four or more different HIV antigens, and each adenoviral vector comprises a nucleic acid sequence that encodes at least one of the four or more different HIV antigens.
5. The use of any of items 1 to 4, wherein the one or more adenoviral vectors each comprise (i) a nucleic acid sequence that encodes two or more different HIV antigens, or (ii) two or more nucleic acid sequences that each encode a different HIV antigen.
6. The use of item 5, wherein the one or more adenoviral vectors each comprise a nucleic acid sequence that encodes two or more different HIV antigens.
7. The use of item 5, wherein the one or more adenoviral vectors each comprise two or more nucleic acid sequences that each encode a different HIV antigen.
8. The use of any of items 1-7, further comprising administering to the mammal a primer composition comprising one or more nucleic acid sequences that encode at least one HIV antigen that is the same as an HIV antigen encoded by an adenoviral vector of the adenoviral vector composition, wherein the administration of the primer composition is performed at least one week before the administration of the adenoviral vector composition.
9. The use of item 8, wherein the primer composition comprises one or more nucleic acid sequences that encode two or more HIV antigens that are the same as the HIV antigens encoded by the one or more adenoviral vectors of the adenoviral vector composition.
10. The use of item 8 or item 9, wherein the administration of the primer composition is performed about six months to about nine months before the administration of the adenoviral vector composition.
11. The use of any of items 8-10, wherein the primer composition comprises one or more plasmids, naked DNA molecules, or viral vectors comprising the one or more nucleic acid sequences.
12. The use of any of items 1-11, wherein the adenoviral vectors are replication-deficient.
13. The use of item 12, wherein the adenoviral vectors are deficient in one or more essential gene functions of the E1 region of the adenoviral genome.
14. The use of item 12 or 13, wherein the adenoviral vectors are deficient in one or more essential gene functions of the E4 region of the adenoviral genome.
15. The use of any of items 1-14, wherein the adenoviral vectors are deficient in one or more gene functions of the E3 region of the adenoviral genome.
16. The use of any of items 1-15, wherein at least one HIV antigen is selected from the group consisting of an HIV Gag protein, HIV Pol protein, HIV Env protein, HIV Tat protein, HIV Reverse Transcriptase (RT) protein, HIV Vif protein, HIV Vpr protein, HIV Vpu protein, HIV Vpo protein, HIV Integrase protein, HIV Nef protein, and a fusion protein comprising all or part of an HIV Gag protein, HIV Pol protein, or HIV Env protein.
17. The use of item 16, wherein at least one HIV antigen is HIV gp140 or gp140dv12.
18. The use of item 16, wherein at least one HIV antigen is a fusion protein that comprises all or part of an HIV Gag protein and all or part of an HIV Pol protein.
19. The use of any of items 1-18, wherein the HIV antigens comprise at least one member selected from the group consisting of an HIV clade A antigen, HIV clade B antigen, HIV clade C antigen, and HIV clade MN antigen.
20. The use of item 19, wherein the HIV antigens comprise at least two members selected from the group consisting of an HIV clade A antigen, HIV clade B antigen, HIV clade C antigen, and HIV clade MN antigen.
21. The use of item 20, wherein the adenoviral vectors encode three or more different HIV antigens, and the HIV antigens comprise at least three members selected from the group consisting of an HIV clade A antigen, HIV clade B antigen, HIV clade C antigen, and HIV clade MN antigen.
22. The use of any of items 1-21, wherein the adenoviral vector composition is administered as part of a pharmaceutical composition comprising a pharmaceutically acceptable carrier.
23. The use of item 22, wherein the pharmaceutical composition is administered in two or more doses.
24. The use of item 22 or 23, wherein the pharmaceutical composition is administered in a dose comprising 1x10⁸ to 1x10¹² particle units (pu) adenoviral vector.
25. The use of item 24, wherein the pharmaceutical composition is administered in a dose comprising 1x10⁸ to 1x10¹⁰ pu adenoviral vector.
26. The use of item 24, wherein the pharmaceutical composition is administered in a dose comprising 1x10⁹ to 1x10¹¹ pu adenoviral vector.
27. The use of item 24, wherein the pharmaceutical composition is administered in a dose comprising 1x10¹⁰ to 1x10¹² pu adenoviral vector.
28. The use of any of items 1-27, wherein the adenoviral vector composition comprises (a) an adenoviral vector comprising a nucleic acid encoding a fusion protein comprising an HIV clade B Gag protein and Pol protein, (b) an adenoviral vector comprising a nucleic acid encoding an HIV clade A Env protein, (c) an adenoviral vector comprising a nucleic acid encoding an HIV clade B Env protein, and (d) an adenoviral vector comprising a nucleic acid encoding an HIV clade C Env protein.
29. The use of item 28, wherein the fusion protein comprising an HIV clade B Gag protein and Pol protein is encoded by a nucleic acid sequence that further encodes HIV Protease, Reverse Transcriptase (RT), and Integrase proteins, and wherein the nucleic acid molecule comprises one or more point mutations, which point mutations render the Protease, RT, and Integrase proteins non-functional.
30. The use of item 28, wherein the Env protein is gp140 or gp140dv12.
31. The use of item 1, wherein the adenoviral vector composition comprises four adenoviral vectors having the nucleic acid sequences of SEQ ID NO: 4, SEQ ID NO: 5, SEQ ID NO: 6, and SEQ ID NO: 7, respectively.
32. An adenoviral vector composition comprising (a) an adenoviral vector comprising a nucleic acid encoding a fusion protein comprising an HIV clade B Gag protein and Pol protein, (b) an adenoviral vector comprising a nucleic acid encoding an HIV clade A Env protein, (c) an adenoviral vector comprising a nucleic acid encoding an HIV clade B Env protein, and (d) an adenoviral vector comprising a nucleic acid encoding an HIV clade C Env protein.
33. The adenoviral vector composition of item 32, wherein the fusion protein comprising an HIV clade B Gag protein and Pol protein is encoded by a nucleic acid sequence that further encodes HIV Protease, Reverse Transcriptase (RT), and Integrase proteins, and wherein the nucleic acid molecule comprises one or more point mutations, which point mutations render the Protease, RT, and Integrase proteins non-functional.
34. The adenoviral vector composition of item 32, wherein the Env protein is gp140 or gp140dv12.
35. The adenoviral vector composition of item 32, wherein (a), (b), (c), and (d) have the nucleic acid sequences of SEQ ID NO: 4, SEQ ID NO: 5, SEQ ID NO: 6, and SEQ ID NO: 7, respectively.
36. The adenoviral vector composition of any of items 32-35, wherein the adenoviral vectors are replication-deficient.
37. The adenoviral vector composition of item 36, wherein the adenoviral vectors are deficient in one or more essential gene functions of the E1 region of the adenoviral genome.
38. The adenoviral vector composition of item 36 or 37, wherein the adenoviral vectors are deficient in one or more essential gene functions of the E4 region of the adenoviral genome.
39. The adenoviral vector composition of any of items 32-38, wherein the adenoviral vectors are deficient in one or more gene functions of the E3 region of the adenoviral genome.
40. The adenoviral vector composition of any of items 32-39, wherein (a), (b), (c), and (d) are present in the composition in a ratio of 3:1:1:1 1 by weight.
41. A pharmaceutical composition comprising the adenoviral vector composition of any of items 32-40 and a pharmaceutically acceptable carrier.
42. The pharmaceutical composition of item 41, wherein (a), (b), (c), and (d) have the nucleic acid sequences of SEQ ID NO: 4, SEQ ID NO: 5, SEQ ID NO: 6, and SEQ ID NO: 7, respectively.
43. The pharmaceutical composition of item 41 or 42, comprising about 1x10⁸ to 1x10¹² particle units (pu) adenoviral vector.
44. The pharmaceutical composition of item 43, comprising about 1x10⁸ to 1x10¹⁰ pu adenoviral vector.
45. The pharmaceutical composition of item 43, comprising about 1x10⁹ to 1x10¹¹ pu adenoviral vector.
46. The pharmaceutical composition of item 43, comprising about 1x10¹⁰ to 1x10¹² pu adenoviral vector.

## Claims

1. An adenoviral vector composition comprising (a) an adenoviral vector comprising a nucleic acid encoding a fusion protein comprising an HIV clade B Gag protein and Pol protein, (b) an adenoviral vector comprising a nucleic acid encoding an HIV clade A Env protein, (c) an adenoviral vector comprising a nucleic acid encoding an HIV clade B Env protein, and (d) an adenoviral vector comprising a nucleic acid encoding an HIV clade C Env protein.

2. The adenoviral vector composition of claim 1, wherein the fusion protein comprising an HIV clade B Gag protein and Pol protein is encoded by a nucleic acid sequence that further encodes HIV Protease, Reverse Transcriptase (RT), and Integrase proteins, and wherein the nucleic acid molecule comprises one or more point mutations, which point mutations render the Protease, RT, and Integrase proteins non-functional.

3. The adenoviral vector composition of claim 1, wherein the Env protein is gp140 or gp140dv12.

4. The adenoviral vector composition of claim 1, wherein (a), (b), (c), and (d) have the nucleic acid sequences of SEQ ID NO: 4, SEQ ID NO: 5, SEQ ID NO: 6, and SEQ ID NO: 7, respectively.

5. The adenoviral vector composition of any of claims 1-4, wherein the adenoviral vectors are replication-deficient.

6. The adenoviral vector composition of claim 5, wherein the adenoviral vectors are deficient in one or more essential gene functions of the E1 region of the adenoviral genome.

7. The adenoviral vector composition of claim 5 or claim 6, wherein the adenoviral vectors are deficient in one or more essential gene functions of the E4 region of the adenoviral genome.

8. The adenoviral vector composition of any of claims 1-7, wherein the adenoviral vectors are deficient in one or more gene functions of the E3 region of the adenoviral genome.

9. The adenoviral vector composition of any of claims 1-8, wherein (a), (b), (c), and (d) are present in the composition in a ratio of 3:1:1:1 by weight.

10. A pharmaceutical composition comprising the adenoviral vector composition of any of claims 1-9 and a pharmaceutically acceptable carrier.

11. The pharmaceutical composition of claim 10, wherein (a), (b), (c), and (d) have the nucleic acid sequences of SEQ ID NO: 4, SEQ ID NO: 5, SEQ ID NO: 6, and SEQ ID NO: 7, respectively.

12. The pharmaceutical composition of claim 10 or claim 11, comprising about 1x10⁸ to 1x10¹² particle units (pu) adenoviral vector.

13. The pharmaceutical composition of claim 12, comprising about 1x10⁸ to 1x10¹⁰ pu adenoviral vector.

14. The pharmaceutical composition of claim 12, comprising about 1x10⁹ to 1x10¹¹ pu adenoviral vector.

15. The pharmaceutical composition of claim 12, comprising about 1x10¹⁰ to 1x10¹² pu adenoviral vector.
